# EUROPEAN PATENT APPLICATION

(11) **EP 1 939 199 A1**
(43) Date of publication of application: **02.07.2008**
(21) Application number: 06798200.9
(22) Date of filing: 21.09.2006
(51) Int. Cl.: C07D 473/18, A61K 31/522, A61P 11/02, A61P 11/06, A61P 17/00, A61P 27/02, A61P 31/12, A61P 31/18, A61P 35/00, A61P 37/02, A61P 37/08, A61P 43/00

(54) **NOVEL ADENINE COMPOUND**

(30) Priority: 22.09.2005 JP 2005276172
(71) Applicant: Dainippon Sumitomo Pharma Co., Ltd., Osaka 541-8524 (JP); AstraZeneca AB, 151 85 Södertälje (SE)
(72) Inventor: HASHIMOTO, Kazuki, Osaka-shi, Osaka 554-0022 (JP); NAKAMURA, Tomoaki, Osaka-shi, Osaka 554-0022 (JP); NAKAMURA, Kei, Osaka-shi, Osaka 554-0022 (JP); TOJO, Shingo, Osaka-shi, Osaka 554-0022 (JP); KURIMOTO, Ayumu, Osaka-shi, Osaka 554-0022 (JP); ISOBE, Yoshiaki, Osaka-shi, Osaka 554-0022 (JP); WADA, Hiroki, Leicestershire LE115RH (GB); BONNERT, Roger, Leicestershire LE115RH (GB)
(74) Representative: Vossius & Partner
(86) International application number: PCT/JP2006/318758
(87) International publication number: WO 2007/034882

(57) **Abstract**

An adenine compound useful as a medicine represented by the following formula (1): 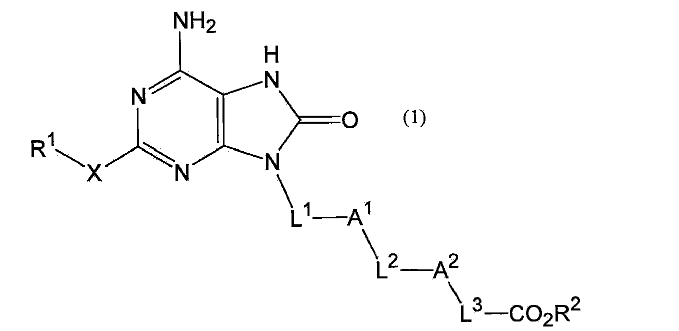 [wherein R¹ is halogen atom, optionally substituted alkyl group, optionally substituted aryl group, etc.;
X is oxygen atom, sulfur atom, a single bond, etc.;
A¹ is optionally substituted and optionally saturated 4 to 8 membered
heterocyclic group containing 1 to 2 hetero atoms selected from 1 to 3 nitrogen atoms, 0 to 1 oxygen atom, and 0 to 1 sulfur atom;
A² is optionally substituted 6 to 10 cyclic aromatic hydrocarbon group or optionally substituted 5 to 10 membered heterocyclic aromatic group;
L¹ and L² are independently, substituted straight or branched alkylene or a single bond, etc.;
L³ is optionally substituted straight or branched alkylene, etc.;
R² is hydrogen atom, optionally substituted alkyl group.]
or its pharmaceutically acceptable salt.

## Description

### TECHNICAL FIELD

The present invention relates to a novel adenine compound useful as a prophylactic or therapeutic agent for allergic diseases, viral diseases, cancers, etc.

### BACKGROUND ART

When a foreign substance such as bacteria, virus or parasite invades into a living body, immune system works to defend from the foreign substance. In acquired immune system, once a foreign substance invades, antigen is processed by antigen presenting cells such as dendritic cells (DC), and naive cells, via mutual action of DC/Th cells, functionally differentiate into Th1 cells or Th2 cells which contribute the main role to immune response in a living body. In this processing, when immune balance deviates to either one of Th1 cells or Th2 cells, it is considered that immune diseases develop.

Namely, in a body of a patient suffering from an allergic disease, cytokines such as interleukin-4 (IL-4) and interleukin-5 (IL-5) secreted from Th2 cells are excessively secreted. Therefore, compounds suppressing an immune response of Th2 cell can be expected as an agent for treating allergic diseases. On the other hand, compounds enhancing an immune response of Th1 cell can be expected as an agent for treating viral diseases, cancers, etc.

Natural immune system has been considered due to non specific phagocytosis. However, the presence of Toll-like receptor (TLR) is confirmed, and activation of the natural immune response is found to be mainly done via TLR. Once TLR recognizes ligands, it induces inflammatory cytokines such as IL-12, TNF, etc. As IL-12 induces naive T cells into Th1 cells, ligands of TLR have a function as a Th1/Th2 differentiation controlling agent, the ligands are expected as a prophylaxis or therapeutic agent for immune diseases. In fact it is known that Th2-cells are dominant in the patients suffering from asthma or atopic dermatitis, and asthma-targeted clinical trials are carried out for DNA (CpG DNA) derived from microorganism, TLR9 agonist. It is also known that imidazoquinoline derivatives, TLR7/8 agonist (See Patent Document 1) show an activity suppressing the production of Th2 cytokines, i.e. interleukin 4 (IL-4) and interleukin 5 (IL-5), and in fact are effective for treatment of allergic diseases in animal model.

On the other hand, compounds having an adenine structure and effective for treatment of immune diseases such as viral diseases or allergic diseases are disclosed in following patent documents 2 to 4.
[Patent Document 1] US Patent 4,689,338
[Patent Document 2] WO 98/01448
[Patent Document 3] WO 99/28321
[Patent Document 4] WO 04/029054

### DISCLOSURE OF INVENTION

The problem to be solved by the present invention is to provide TLR activating agents, in more detail, the novel adenine compounds having TLR7 activating effect, an immune modulator containing them, such as prophylactic or therapeutic agents for allergic diseases such as asthma, COPD, allergic rhinitis, allergic conjunctivitis and atopic dermatosis, viral diseases such as hepatitis B, hepatitis C, HIV and HPV, bacterial infectious diseases, cancers and dermatosis.

The present inventors earnestly investigated in order to find a therapeutic or prophylactic agent for allergic diseases, viral diseases or cancers, having excellent TLR activating effect and succeeded in finding a novel compound of the present invention. Namely the compound of the present invention is useful for therapeutic and prophylactic agent of allergic diseases, viral diseases and cancers.

Thus the present invention has been completed based on the above findings.

Namely, the present invention relates to the following invention.
[1] An adenine compound represented by the following formula (1): 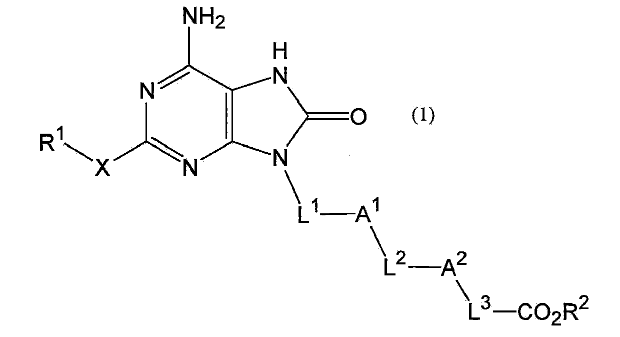 [wherein
   R¹ is halogen atom, optionally substituted alkyl group, optionally substituted alkenyl group, optionally substituted alkynyl group, optionally substituted cycloalkyl group, optionally substituted aryl group or optionally substituted heteroaryl group;
   R² is hydrogen atom, optionally substituted alkyl group, optionally substituted alkenyl group, optionally substituted alkynyl group or optionally substituted cycloalkyl group;
   X is oxygen atom, sulfur atom, NR⁴ (wherein R⁴ is hydrogen atom or C₁₋₆ alkyl group), SO, SO₂ or a single bond, provided that X is a single bond when R¹ is halogen atom;
   A¹ is optionally substituted and saturated or unsaturated 4 to 8 membered heterocyclic group containing 1 to 2 hetero atoms selected from 1 to 2 nitrogen atoms, 0 to 1 oxygen atom, and 0 to 1 sulfur atom;
   A² is optionally substituted 6 to 10 membered aromatic carbocyclic group or optionally substituted 5 to 10 membered aromatic heterocyclic group;
   L³ is optionally substituted straight or branched alkylene or a single bond; and
   L¹ and L² are independently, straight or branched alkylene or a single bond and any 1 to 3 methylene groups in said alkylene group may be replaced by oxygen atom, sulfur atom, NR⁵ (wherein R⁵ is hydrogen atom, optionally substituted alkyl group, optionally substituted cycloalkyl group, optionally substituted aryl group or optionally substituted heteroaryl group), SO, SO₂, C=NR⁶ (wherein R⁶ is optionally substituted alkyl group, optionally substituted aryl group or optionally substituted heteroaryl group), or carbonyl group.]
   or its pharmaceutically acceptable salt.
[2] The adenine compound or its pharmaceutically acceptable salt described in the above [1], wherein
   substituted alkyl group, substituted alkenyl group or substituted alkynyl group in R¹ and R², and substituted alkyl group in R⁵ and R⁶ are substituted by one or more substituents independently selected from the group consisting of groups (a) to (c) below;
   (a) halogen atom, hydroxy group, carboxy group, mercapto group and C₁₋₆ haloalkoxy group;
   (b) C₁₋₆ alkoxy group, C₂₋₆ alkylcarbonyl group, C₂₋₆ alkoxycarbonyl group, C₁₋₆ alkylsulfonyl group, C₁₋₆ alkylsulfinyl group, C₂₋₆ alkylcarbonyloxy group, and C₁₋₆ alkylthio group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
   (c) optionally substituted amino group, optionally substituted carbamoyl group and optionally substituted sulfamoyl group (wherein the group of this group may be substituted by 1 or 2 substituents selected from the group consisting of groups (k), (1) and (m) below), optionally substituted 3 to 8 membered cycloalkyl group and optionally substituted 4 to 8 membered saturated heterocyclic group (wherein the group of this group may be substituted by one or more substituents selected from the group consisting of groups (d), (e) and (f) below), and optionally substituted 6 to 10 membered aryl group, optionally substituted 5 to 10 membered heteroaryl group, optionally substituted 6 to 10 membered aryloxy group and optionally substituted 5 to 10 membered heteroaryloxy group (wherein the group of this group may be substituted by one or more substituents selected from the group consisting of groups (g), (h) (i) and (j) below);
      substituted cycloalkyl group in R¹, R² and R⁵ is substituted by one or more substituents independently selected from the group consisting of groups (d) to (f) below;
   (d) halogen atom, hydroxy group, carboxy group, mercapto group, cyano group, nitro group, C₁₋₆ haloalkyl group and C₁₋₆ haloalkoxy group;
   (e) C₁₋₆ alkyl group, C₁₋₆ alkoxy group, C₂₋₆ alkenyl group, C₂₋₆ alkynyl group, C₂₋₆ alkoxycarbonyl group, and C₁₋₆ alkylthio group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
   (f) optionally substituted amino group, optionally substituted carbamoyl group and optionally substituted sulfamoyl group (wherein the group of this group may be substituted by one or two substituents selected groups (k), (l) and (m) below), optionally substituted 6 to 10 membered aryl group and optionally substituted 5 to 10 membered heteroaryl group (the group of this group may be substituted by one or more substituents selected from the group consisting of groups (g), (h), (i) and (j) below);
      substituted aryl group and substituted heteroaryl group in R¹, R⁵ and R⁶ are substituted by one or more substituents independently selected from the group consisting of groups (g) to (j) below;
   (g) halogen atom, hydroxy group, mercapto group, cyano group, nitro group, C₁₋₆ haloalkyl group, and C₁₋₆ haloalkoxy group;
   (h) C₁₋₆ alkyl group, C₁₋₆ alkoxy group, C₂₋₆ alkenyl group, C₂₋₆ alkynyl group, and C₁₋₆ alkylthio group (wherein the group of this group may be substituted by one or more substituents independently selected from a group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
   (i) 3 to 8 membered cycloalkyl group and 4 to 8 membered saturated heterocyclic group (the group of this group may be substituted by one or more substituents independently selected from group consisting of halogen atom, hydroxy group, carboxy group, C₁₋₆ alkyl group and C₁₋₆ alkoxy group);
   (j) optionally substituted amino group, optionally substituted carbamoyl group, and optionally substituted sulfamoyl group (the group of this group may be substituted by one or two substituents selected from group consisting of groups (k), (l) and (m) below);
      the substituted amino group, substituted carbamoyl group and substituted sulfamoyl group mentioned above are substituted by one or two substituents independently selected from the group consisting of groups (k) to (m) below;
   (k) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, and 3 to 8 membered cycloalkylsulfinyl group (wherein the group of this group may be substituted by one or more substituents independently selected from the group consisting of halogen atom, hydroxy group, carboxy group, C₁₋₆ alkoxy group, and C₂₋₆ alkoxycarbonyl group);
   (l) 6 to 10 membered aryl group, 6 to 10 membered arylcarbonyl group, 6 to 10 membered aryloxycarbonyl group, 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, mercapto group, cyano group, nitro group, C₁₋₆ alkyl group, C₁₋₆ alkoxy group or C₁₋₆ alkylthio group);
   (m) 4 to 7 membered saturated heterocyclic group containing 1 to 4 hetero atoms selected from 1 to 2 nitrogen atoms, 0 to 1 oxygen atom, and 0 to 1 sulfur atom which is formed by combining two substituents with the nitrogen atom (said saturated heterocyclic group may be substituted on any carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group);
      substituted 4 to 8 membered heterocyclic group in A¹ may be substituted by one or more substituents independently selected from a group consisting of halogen atom, hydroxy group, oxo group, C₁₋₆ alkyl group, C₁₋₆ alkoxy group, C₂₋₆ alkylcarbonyl group and C₂₋₆ alkoxycarbonyl group; substituted 6 to 10 membered aromatic carbocyclic group or substituted 5 to 10 membered aromatic heterocyclic group in A² may be substituted by one or more substituents independently selected from a group consisting of halogen atom, C₁₋₆ alkyl group, C₁₋₆ alkoxy group, C₁₋₆ haloalkyl group, C₁₋₆ haloalkoxy group, amino group optionally substituted by the same or different and one or two C₁₋₆ alkyl groups, 4 to 8 membered saturated heterocyclic group containing 1 to 2 hetero atoms selected from 1 to 2 nitrogen atoms, 0 to 1 oxygen atom, and 0 to 1 sulfur atom (said saturated heterocyclic group may be substituted by one or more substituents independently selected from a group consisting of halogen atom, hydroxy group, oxo group, C₁₋₆ alkyl group, C₁₋₆ alkoxy group, C₂₋₆ alkylcarbonyl group and C₂₋₆ alkoxycarbonyl group);
      L³ is straight or branched C₁₋₆ alkylene, or a single bond;
[3] The adenine compound or its pharmaceutically acceptable salt described in the above [1] or [2], wherein in the formula (1), A¹ is pyrrolidine, piperidine, azetidine, piperazine, morpholine, thiomorpholine, thiomorpholine-1-oxide, thiomorpholine-1,1-dioxi.de, 2,6-dimethylpiperidine, 3,5-dimethylpiperidine, 2,6-dimethylpiperazine, 2,6-dimethylmorpholine, 3,5-dimethylmorpholine, 2,6-dimethylthiomorpholine, or 3,5-dimethylthiomorpholine.
[4] The adenine compound or its pharmaceutically acceptable salt described in any one of the above [1] to [3], wherein in the formula (1), A² is benzene, pyridine, furan, imidazole or thiophene.
[5] The adenine compound or its pharmaceutically acceptable salt described in any one of the above [1] to [4], wherein in the formula (1), R² is C₁₋₄ alkyl group.
[6] The adenine compound or its pharmaceutically acceptable salt described in the above [5], wherein in the formula (1), R² is methyl group.
[7] The adenine compound or its pharmaceutically acceptable salt described in any one of the above [1] to [4], wherein in the formula (1), R² is C₂₋₈ alkyl group substituted by optionally substituted amino group.
[8] The adenine compound or its pharmaceutically acceptable salt described in any one of the above [1] to [7], wherein in the formula (1), L¹ is the following formula:

   (CH₂)ₙ-(Y⁴)ₘ-(CH₂)₁ₐ

   [wherein, n and 1a are independently an integer of 0 to 5, m is 0 or 1, Y⁴ is oxygen atom or NR⁵ (wherein R⁵ is the same as defined in the above [1])], L² is a single bond, oxygen atom, C₁₋₁₀ straight alkylene or the following formula:

   (CH₂)ₐ-(Y¹)ₚ-(CH₂)_{q}-(Y²)ᵣ-(CH₂)ₜ-(Y³)ᵤ

   [wherein Y¹ is carbonyl group, Y² is NR⁵' (R⁵' is the same as the definition of R⁵), Y³ is oxygen atom, a, t and q are independently, an integer of 0 to 4, p, r and u are independently 0 or 1, provided that t is 2 or more when r and u are 1], and L³ is a single bond or C₁₋₄ straight alkylene.
[9] The adenine compound or its pharmaceutically acceptable salt described in the above [8], wherein R⁵ is hydrogen atom, C₁₋₆ alkyl group, C₁₋₆ alkylcarbonyl group or C₁₋₆ alkylsulfonyl group (these groups may be substituted by one or more substituents independently selected from a group consisting of halogen atom, hydroxy group, alkoxy group, 3 to 8 membered cycloalkyl group, 6 to 10 membered aryl group, 6 to 10 membered arylcarbonyl group and 5 to 10 membered heteroaryl group (this group may be substituted by one or more substituents independently selected from a group consisting of halogen atom, hydroxy group, nitro group, cyano group, C₁₋₆ alkyl group, C₁₋₆ alkoxy group, C₁₋₆ haloalkyl group and C₁₋₆ haloalkoxy group).
[10] The adenine compound or its pharmaceutically acceptable salt described in the above [1] selected from the group of the following compounds:
   2-Butoxy-7,8-dihydro-9-{[2-(3-{N-[(3-methoxycarbonylmethylphenyl-1-yl)methyl]-N-methylamino}propyl)piperidin-4-yl]-methyl}-8-oxoadenine;
   2-Butoxy-7,8-dihydro-9-{[2-(3-{N-[(3-methoxycarbonylmethylphenyl-1-yl)methyl]amino}propyl)piperidin-4-yl]methyl}-8-oxoadenine;
   7,8-Dihydro-9-(1-{2-[3-(methoxycarbonylmethyl)phenoxy]ethyl}piperidin-4-ylmethyl)-2-(2-methoxyehoxy)-8-oxoadenine;
   7,8-Dihydro-9-{1-[3-(N-{2-[3-(methoxycarbonylmethyl)phenoxy]ethyl}-N-methylamino)propyl]piperidin-4-ylmethyl}-2-(2-methoxyehoxy)-8-oxoadenine;
   7,8-Dihydro-9-[1-(3-{N-[3-(methoxycarbonylmethyl)benzyl]-N-methylamino}propyl)piperidin-4-ylmethyl]-8-oxoadenine;
   2-Butoxy-7,8-dihydro-9-(1-{2-[3-(methoxycarbonylmethyl)phenoxy]ethyl}piperidin-4-ylmethyl)-8-oxoadenine;
   2-Butoxy-7,8-dihydro-9-{1-[3-(N-{2-[3-(methoxycarbonylmethyl)phenoxy]ethyl}-N-methylamino)propyl]piperidin-4-ylmethyl}-8-oxoadenine;
   7,8-Dihydro-9-(1-{2-[3-(methoxycarbonylmethyl)phenoxy]ethyl}piperidin-4-ylethyl)-2-(2-methoxyehoxy)-8-oxoadenine;
   7,8-Dihydro-9-[1-(3-[{N-methyl-N-[3-(methoxycarbonylmethyl)benzyl]}amino]propyl)piperidin-4-ylethyl]-2-(2-methoxyehoxy)-8-oxoadenine;
   7,8-Dihydro-9-{1-[3-([N-methyl-N-{2-[3-(methoxycarbonylmethyl)phenoxy]ethyl}]amino)propyl]piperidin-4-ylethyl}-2-(2-methoxyehoxy)-8-oxoadenine;
   2-Butoxy-7,8-dihydro-9-(1-{2-[3-(methoxycarbonylmethyl)phenoxy]ethyl}piperidin-4-ylethyl)-8-oxoadenine;
   2-Butoxy-7,8-dihydro-9-{1-[3-([N-methyl-N-{2-[3-(methoxycarbonylmethyl) phenoxy] ethyl}] amino) propyl] piperidin-4-ylethyl}-8-oxoadenine;
   7,8-Dihydro-9-(1-{[3-(methoxycarbonylmethyl)phenyl]aminocarbonylmethyl)piperidin-4-ylethyl)-2-(2-methoxyehoxy)-8-oxoadenine;
   2-Butoxy-7,8-dihydro-9-(1-{[3-(methoxycarbonylmethyl)phenyl]aminocarbonylmethyl}piperidin-4-ylmethyl)-8-oxoadenine;
   2-Butoxy-7,8-dihydro-9-{1-[(N-{2-[3-(methoxycarbonylmethyl)phenoxy]ethyl}-N-methyl)aminomethylcarbonyl]piperidin-4-ylmethyl}-8-oxoadenine;
   7,8-Dihydro-2-(2-methoxyehoxy)-9-{1-[(N-{2-[3-(methoxycarbonylmethyl)phenoxy]ethyl}-N-methyl)aminomethylcarbonyl]piperidin-4-yhmethyl}-8-oxoadenine;
   7,8-Dihydro-2-(2-methoxyehoxy)-9-[1-(N-{2-[3-(methoxycarbonylmethyl)phenoxy]ethyl}-N-methylaminomethylcarbonyl)piperidin-4-ylmethyl]-8-oxoadenine;
   2-Butoxy-7,8-dihydro-9-(2-{1-[(N-{2-[3-(methoxycarbonylmethyl)phenoxy]ethyl}-N-methyl)aminocarbonylmethyl]piperidin-4-yl}ethyl)-8-oxoadenine;
   2-Butoxy-7,8-dihydro-9-{1-[(N-{2-[2-methoxy-5-(methoxycarbonylmethyl)phenoxy]ethyl}-N-methyl)aminomethylcarbonyl]piperidin-4-ylmethyl}-8-oxoadenine;
   2-Butoxy-7,8-dihydro-9-[5-(4-{2-[N-methyl-N-(3-methoxycarbonylmethyl)benzyl] aminoethyl}piperazin-1-yl)pentyl]-8-oxoadenine;
   2-Butoxy-7, 8-dihydro-9-[7-(4-{2-[N-methyl-N-(3-methoxycarbonylmethyl)benzyl]aminoethyl}piperazin-1-yl)heptyl]-8-oxoadenine;
   2-Butoxy-7,8-dihydro-9-(5-{4-[2-(3-methoxycarbonylmethylphenyloxy)ethyl]piperazin-1-yl}pentyl)-8-oxoadenine;
   2-Butoxy-7,8-dihydro-9-(7-{4-[2-(3-methoxycarbonylmethylphenyloxy)ethyl]piperazin-1-yl}heptyl)-8-oxoadenine;
   2-Butoxy-7,8-dihydro-9-[2-{4-(3-methoxycarbonylmethylphenoxy)piperidin-1-yl}ethyl]-8-oxoadenine;
   2-Butoxy-7,8-dihydro-9-[2-{4-(3-methoxycarbonylmethylphenyl)piperidin-1-yl}ethyl]-8-oxoadenine;
   2-Butoxy-7,8-dihydro-9-[2-{4-(3-methoxycarbonylmethylbenzyl)piperidin-1-yl}ethyl]-8-oxoadenine;
   2-Butoxy-7,8-dihydro-9-[2-{4-(4-methoxycarbonylmethylpyridin-2-yl)piperazin-1-yl}ethyl] -8-oxoadenine;
   2-Butoxy-7,8-dihydro-9-(3-{4-[3-(2-methoxy-2-oxoethyl)phenoxy]piperidin-1-yl}propyl)-8-oxoadenine;
   2-Butoxy-7,8-dihydro-9-{4-[4-(3-methoxycarbonylmethyl)benzylpiperazin-1-yl] butyl}-8-oxoadenine;
   2-Butoxy-7,8-dihydro-9-{4-[4-(3-methoxycarbonylmethylbenzyl)piperazin-1-yl] butyl}-8-oxoadenine;
   2-Butoxy-7,8-dihydro-9-{4-[4-(4-methoxycarbonylbenzylcarbonylbenzyl)piperidin-1-yl]butyl}-8-oxoadenine;
   2-Butoxy-7,8-dihydro-9-[1-(5-methoxycarbonylfuran-2-ylmethyl)piperidin-4-ylmethyl]-8-oxoadenine;
   2-Butoxy-7,8-dihydro-9-[5-{4-(3-methoxycarbonylmethylphenyl)piperidin-1-yl}pentyl] -8-oxoadenine;
   7,8-Dihydro-2-(2-methoxyehoxy)-9-[2-{4-(3-methoxycarbonylmethylbenzyl)piperidin-1-yl}ethyl]-8-oxoadenine;
   2-Butoxy-7,8-dihydro-9-{[2-(3-{N-[(3-hydroxycarbonylmethylphenyl-1-yl)methyl]-N-methylamino}-propyl)-piperidin-4-yl]-methyl}-8-oxoadenine;
   2-Butoxy-7,8-dihydro-9-(1-{2-[3-(hydroxycarbonylmethyl)phenoxy]ethyl}piperidin-4-ylmethyl)-8-oxoadenine;
   2-Butoxy-7,8-dihydro-9-{1-[3-(N-{2-[3-(hydroxycarbonylmethyl)phenoxy]ethyl}-N-methylamino)propyl]piperidin-4-ylmethyl}-8-oxoadenine;
   9-(1-(2-[3-(carboxymethyl)phenoxy]ethyl)piperidin-4-ylethyl)-7,8-dihydro-2-(2-methoxyehoxy)-8-oxoadenine;
   2-Butoxy-7,8-dihydro-9-(1-{2-[3-(carboxymethyl)phenoxy]ethyl}piperidin-4-ylethyl)-8-oxoadenine;
   2-Butoxy-7,8-dihydro-9-{1-[3-([N-methyl-N-{2-[3-(carboxymethyl)phenoxy]ethyl}]amino)propyl]piperidin-4-ylethyl}-8-oxoadenine;
   7,8-Dihydro-9-(1-{[3-(hydroxycarbonylmethyl)phenyl]aminocarbonylmethyl}piperidin-4-ylethyl)-2-(2-methoxyehoxy)-8-oxoadenine;
   2-Butoxy-7, 8-dihydro-9-(1-{[3-(hydroxycarbonylmethyl)phenyl]aminocarbonylmethyl}piperidin-4-ylmethyl)-8-oxoadenine;
   2-Butoxy-7,8-dihydro-9-{1-[(N-{2-[3-(hydroxycarbonylmethyl)phenoxy] ethyl}-N-methyl)aminomethylcarbonyl]piperidin-4-ylmethyl}-8-oxoadenine;
   7,8-Dihydro-9-{1-[(N-{2-[3-(hydroxycarbonylmethyl)phenoxy]ethyl}-N-methyl)aminomethylcarbonyl]piperidin-4-ylmethyl}-2-(2-methoxyehoxy)-8-oxoadenine;
   7,8-Dihydro-9-[1-(N-{2-[3-(hydroxycarbonylmethyl)phenoxy]ethyl}-N-methylaminomethylcarbonyl)piperidin-4-ylmethyl]-2-(2-methoxyehoxy)-8-oxoadenine;
   2-Butoxy-7,8-dihydro-9-(2-{1-[(N-{2-[3-(hydroxycarbonylmethyl)phenoxy]ethyl}-N-methyl)aminocarbonylmethyl]piperidin-4-yl}ethyl)-8-oxoadenine;
   2-Butoxy-7,8-dihydro-9-[5-(4-{2-[N-methyl-N-(3-hydroxycarbonylmethyl)benzyl]aminoethyl}piperazin-1-yl)pentyl]-8-oxoadenine;
   2-Butoxy-7,8-dihydro-9-[7-(4-{2-[N-methyl-N-(3-hydroxycarbonylmethyl)benzyl]aminoethyl}piperazin-1-yl)heptyl]-8-oxoadenine;
   2-Butoxy-7,8-dihydro-9-(5-{4-[2-(3-hydroxycarbonylmethylphenyloxy)ethyl]piperazin-1-yl}pentyl)-8-oxoadenine;
   2-Butoxy-7,8-dihydro-9-(7-{4-[2-(3-hydroxycarbonylmethylphenyloxy) thyl]piperazin-1-yl}heptyl)-8-oxoadenine;
   2-Butoxy-7,8-dihydro-9-[2-{4-(3-hydroxycarbonylmethylphenoxy)piperidin-1-yl}ethyl]-8-oxoadenine;
   2-Butoxy-7,8-dihydro-9-[2-{4-(3-hydroxycarbonylmethylphenyl)piperidin-1-yl}ethyl]-8-oxoadenine;
   2-Butoxy-7,8-dihydro-9-[2-{4-(3-hydroxycarbonylmethylbenzyl)piperidin-1-yl}ethyl]-8-oxoadenine;
   2-Butoxy-7,8-dihydro-9-[5-{4-(3-hydroxycarbonylmethylphenyl)piperidin-1-yl}pentyl]-8-oxoadenine;
   2-Butoxy-7,8-dihydro-9-(3-{4-[3-(2-hydroxy-2-oxoethyl)phenoxy]piperidin-1-yl}propyl)-8-oxoadenine;
   2-Butoxy-7,8-dihydro-9-{4-[(3-hydroxycarbonylmethyl)benzylpiperazin-1-yl]butyl}-8-oxoadenine;
   2-Butoxy-7,8-dihydro-9-{4-[4-(3-hydroxycarbonylmethylbenzyl) piperazin-1-yl]butyl}-8-oxoadenine;
   2-Butoxy-7,8-dihydro-9-{4-[4-(4-hydroxycarbonylbenzylcarbonylbenzyl)piperidin-1-yl]butyl}-8-oxoadenine;
   2-Butoxy-7,8-dihydro-9-[5-{4-(3-hydroxycarbonylmethylphenyl)piperidin-1-yl}pentyl]-8-oxoadenine;
   Methyl {3-[({1-[2-(6-amino-2-butoxy-8-oxo-7,8-dihydro-9H-purin-9-yl)ethyl]piperazin-4-yl}amino)methyl]phenyl}acetate;
   {3-[({1-[2-(6-amino-2-butoxy-8-oxo-7,8-dihydro-9H-purin-9-yl)ethyl]piperidin-4-yl}amino)methyl]phenyl}acetic acid;
   Methyl {3-[2-(4-{[3-(6-amino-2-butoxy-8-oxo-7,8-dihydro-purin-9-yl) propyl] isobutylamino}piperidin-1-yl) ethoxy] phenyl}acetate;
   Methyl [4-({1-[3-(6-amino-2-butoxy-8-oxo-7,8-dihydro-purin-9-yl)propyl]piperidin-4-ylamino}methyl)phenyl]acetate;
   Methyl [4-({1-[3-(6-amino-2-butoxy-8-oxo-7,8-dihydro-9H-purin-9-yl)propyl]-4-methylpiperazin-2-yl}methyl)phenyl]acetate;
   Methyl [4-({3-[(6-amino-2-butoxy-8-oxo-7,8-dihydro-9H-purin-9-yl)methyl] piperidin-1-yl}methyl)-3-(dimethylamino)phenyl] acetate;
   2-Butoxy-7,8-dihydro-9-[2-(1-[2-({3-[3-(methoxycarbonylmethyl) phenoxy] propyl}-N-methylamino) ethyl] piperidin-2-yl)ethyl]-8-oxoadenine;
   2-Butoxy-7, 8-dihydro-9-{[3-{N-[2-(3-methoxycarbonylmethylphenoxy-1-yl)ethyl])piperidin-4-yl]aminopropyl}-8-oxoadenine;
   2-Butoxy-7,8-dihydro-9-{3-(N-{N-[2-(3-methoxycarbonylmethylphenoxy)ethyl]piperidin-4-yl}-N-(2H-imidazol-4-ylmethyl)amino)propyl}-8-oxoadenine;
   {3-[2-(4-{[3-(6-Amino-2-butoxy-8-oxo-7,8-dihydropurin -9-yl)propyl]isobutylamino}piperidin-1-yl)ethoxy]phenyl}acetic acid;
   [4-({1-[3-(6-Amino-2-butoxy-8-oxo-7,8-dihydropurin-9-yl)propyl]piperidin-4-ylamino}methyl)phenyl]acetic acid 2 hydrochloride;
   2-Butoxy-7,8-dihydro-9-{[3-{N-[2-(3-hydroxycarbonylmethylphenoxy-1-yl)ethyl])piperidin-4-yl]aminopropyl}-8-oxoadenine; and
   2-Butoxy-7,8-dihydro-9-{3-(N-{N-[2-(3-methoxycarbonylmethylphenoxy)ethyl]piperidin-4-yl}-N-(2H-imidazol-4-ylmethyl)amino)propyl}-8-oxoadenine.
[11] A pharmaceutical composition containing the adenine compound or a pharmaceutically acceptable salt thereof as described in any one of the above [1] to [10] as an active ingredient.
[12] A TLR7 activator containing the adenine compound or a pharmaceutically acceptable salt thereof as described in any one of the above [1] to [10] as an active ingredient.
[13] An immuno-modifier containing the adenine compound or a pharmaceutically acceptable salt thereof as described in any one of the above [1] to [10] as an active ingredient.
[14] A therapeutic or prophylactic agent for allergic diseases, viral diseases or cancers containing the adenine compound or a pharmaceutically acceptable salt thereof as described in any one of the above [1] to [10] as an active ingredient.
[15] A therapeutic or prophylactic agent for asthma, COPD, allergic rhinitis, allergic conjunctivitis, atopic dermatosis, cancer, hepatitis B virus, hepatitis C virus, HIV, HPV, a bacterial infectious disease, or dermatosis containing the adenine compound or a pharmaceutically acceptable salt thereof as described in any one of the above [1] to [10] as an active ingredient.
[16] A medicament for topical administration containing the adenine compound or a pharmaceutically acceptable salt thereof as described in any one of the above [1] to [10] as an active ingredient.

### Effect of the invention

According to the present invention it is possible to provide a novel adenine compound useful as a prophylactic or therapeutic agent for allergic diseases, viral diseases, cancers, etc.

### THE BEST MODE FOR CARRYING OUT THE INVENTION

The present invention is described in detail below.

"Halogen atom" in the present specification includes fluorine atom, chlorine atom, bromine atom, or iodine atom, preferably fluorine atom or chlorine atom.

"Alkyl group" includes C₁₋₁₂ straight or branched chain alkyl group, such as methyl group, ethyl group, propyl group, 1-methylethyl group, butyl group, 2-methylpropyl group, 1-methylpropyl group, 1,1-dimethylethyl group, pentyl group, 3-methylbutyl group, 2-methylbutyl group, 2,2-dimethylpropyl group, 1-ethylpropyl group, 1,1-dimethylpropyl group, hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, heptyl group, 1-methylhexyl group, 1-ethylpentyl group, octyl group, 1-methylheptyl group, 2-ethylhexyl group, nonyl group, decyl group, etc., preferably C₁₋₆ alkyl group, more preferably C₁₋₄ alkyl group.

"Alkenyl group" includes C₂₋₁₀ straight or branched chain alkenyl group, such as ethenyl group, propenyl group, 1-methylethenyl group, butenyl group, 2-methylpropenyl group, 1-methylpropenyl group, pentenyl group, 3-methylbutenyl group, 2-methylbutenyl group, 1-ethylpropenyl group, hexenyl group, 4-methylpentenyl group, 3-methylpentenyl group, 2-methylpentenyl group, 1-methylpentenyl group, 3,3-dimethylbutenyl group, 1,2-dimethylbutenyl group, heptenyl group, 1-methylhexenyl group, 1-ethylpentenyl group, octenyl group, 1-methylheptenyl group, 2-ethylhexenyl group, nonenyl group, decenyl group, etc., preferably C₂₋₆ alkenyl group, more preferably C₂₋₄ alkenyl group.

"Alkynyl group" includes C₂₋₁₀ straight or branched chain alkynyl group, such as ethynyl group, propynyl group, butynyl group, pentynyl group, 3-methylbutynyl group, hexynyl group, 4-methylpentynyl group, 3-methylpentynyl group, 3,3-dimethylbutynyl group, heptynyl group, octynyl group, 3-methylheptynyl group, 3-ethylhexynyl group, nonynyl group, decynyl group, etc., preferably C₂₋₆ alkynyl group, more preferably, C₂₋₄ alkynyl group.

"Cycloalkyl group" includes 3 to 8 membered monocyclic cycloalkyl group, such as cyclopropyl group, cyclobutyl group, cyclopentyl group, cyclohexyl group, cycloheptyl group, or cyclooctyl group.

"Cycloalkoxy group" includes 3 to 8 membered monocyclic cycloalkoxy group, such as cyclopropyloxy group, cyclobutyloxy group, cyclopentyloxy group, cyclohexyloxy group, cycloheptyloxy group, or cyclooctyloxy group.

"Aryl group" includes 6 to 10 membered aryl group, such as phenyl group, 1-naphthyl group, or 2-naphthyl group.

"Heteroaryl group" includes 5 to 10 membered monocyclic or bicyclic heteroaryl group containing 1 to 4 hetero atoms selected from 0 to 3 nitrogen atoms, 0 to 1 oxygen atom and 0 to 1 sulfur atom, such as furyl group, thienyl group, pyrrolyl group, pyridyl group, indolyl group, isoindolyl group, quinolyl group, isoquinolyl group, pyrazolyl group, imidazolyl group, pyrimidinyl group, pyrazinyl group, pyridazinyl group, thiazolyl group, oxazolyl group, etc. The binding position is not specifically limited, and it may be on any carbon atom or nitrogen atom, if chemically stable.

"Saturated heterocyclic group" includes 4 to 10 membered mono or bicyclic saturated heterocyclic group containing 1 to 3 hetero atoms selected from 0 to 3 nitrogen atoms, 0 to 1 oxygen atom and 0 to 1 sulfur atom and said sulfur atom may be submitted by one or two oxygen atoms, such as pyrrolidinyl group, piperidinyl group, piperazinyl group, morpholinyl group, thiomorpholinyl group, 1-oxothiomorpholinyl group, 1,1-dioxothiomorpholinyl group, tetrahydrofuranyl group, oxazolydinyl group, etc. The binding position on the heterocyclic group is not specifically limited and it may be on any of nitrogen or carbon atoms, if chemically stable. The 4 to 8 membered monocyclic saturated heterocyclic group is preferablly illustrated.

"Alkylene" includes straight or branched chain C₁₋₁₂ alkylene, such as methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, 3-methylpentamethylene, etc., preferably C₁₋₁₀, more preferably C₁₋₈, further more preferably C₁₋₆ straight or branched chain alkylene.

"Haloalkyl group" includes alkyl group substituted by the same or different and 1 to 5 halogen atoms, such as trifluoromethyl group, 2,2,2-trifluoroethyl group, 2,2-difluoroethyl group, pentafluoroethyl group, etc.

"Alkoxy group" includes C₁₋₁₀ straight or branched chain alkoxy group, for example methoxy group, ethoxy group, propoxy group, 1-methylethoxy group, butoxy group, 2-methylpropoxy group, 1-methylpropoxy group, 1,1-dimethylethoxy group, pentoxy group, 3-methylbutoxy group, 2-methylbutoxy group, 2,2-dimethylpropoxy group, 1-ethylpropoxy group, 1,1-dimethylpropoxy group, hexyloxy group, 4-methylpentyloxy group, 3-methylpentyloxy group, 2-methylpentyloxy group, 1-methylpentyloxy group, 3,3-dimethylbutoxy group, 2,2-dimethylbutoxy group, 1,1-dimethylbutoxy group, 1,2-dimethylbutoxy group, heptyloxy group, 1-methylhexyloxy group, 1-ethylpentyloxy group, octyloxy group, 1-methylheptyloxy group, 2-ethylhexyloxy group, nonyloxy group, decyloxy group, etc, preferably C₁₋₆ alkoxy group, more preferably C₁₋₄ alkoxy group.

"Haloalkoxy group" includes alkoxy group substituted by the same or different and 1 to 5 halogen atoms, such as trifluoromethoxy group, 2,2,2-trifluoroethoxy group, 2,2-difluoroethoxy group, 2-fluoroethoxy, pentafluoroethoxy group, etc.

"Alkylthio group" includes straight or branched chain C₁₋₁₀ alkylthio group, such as methylthio group, ethylthio group, propylthio group, 1-methylethylthio group, butylthio group, 2-methylpropylthio group, 1-methylpropylthio group, 1,1-dimethylethylthio group, pentylthio group, 3-methylbutylthio group, 2-methylbutylthio group, 2,2-dimethylpropylthio group, 1-ethylpropylthio group, 1,1-dimethylpropylthio group, hexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, heptylthio group, 1-methylhexylthio group, 1-ethylpentylthio group, octylthio group, 1-methylheptylthio group, 2-ethylhexylthio group, nonylthio group, decylthio group, etc., preferably C₁₋₆ alkylthio group, more preferably C₁₋₄ alkylthio group.

"Alkyl moiety" in "alkylcarbonyl group", "alkylcarbonyloxy group", "alkylsulfonyl group" or "alkylsulfinyl group" includes the same as the alkyl group as mentioned above.

"Alkylcarbonyl group" includes for example, acetyl group, propanoyl group, butanoyl group, 2-methylpropanoyl group, pentanoyl group, 3-methylbutanoyl group, 2-methylbutanoyl group, 2,2-dimethylpropanoyl (pivaloyl) group, hexanoyl group, 4-methylpentanoyl group, 3-methylpentanoyl group, 2-methylpentanoyl group, 3,3-dimethylbutanoyl group, 2,2-dimethylbutanoyl group, heptanoyl group, octanoyl group, 2-ethylhexanoyl group, nonanoyl group, decanoyl group, etc., preferably C₂₋₆ alkylcarbonyl group, more preferably, C₂₋₅ straight or branched chain alkylcarbonyl group.

"Alkylcarbonyloxy group" includes for example, acetoxy group, propanoyloxy group, butanoyloxy group, 2-methylpropanoyloxy group, pentanoyloxy group, 3-methylbutanoyloxy group, 2-methylbutanoyloxy group, 2,2-dimethylpropanoyloxy (pivaloyloxy) group, hexanoyloxy group, 4-methylpentanoyloxy group, 3-methylpentanoyloxy group, 2-methylpentanoyloxy group, 3,3-dimethylbutanoyloxy group, 2,2-dimethylbutanoyloxy group, heptanoyloxy group, octanoyloxy group, 2-ethylhexanoyloxy group, nonanoyloxy group, decanoyloxy group, etc., preferably C₂₋₆ alkylcarbonyloxy group, more preferably C₂₋₅ straight or branched chain alkylcarbonyloxy group.

"Alkylsulfonyl group" includes for example, as methanesulfonyl group, ethanesulfonyl group, propylsulfonyl group, 1-methylethylsulfonyl group, butylsulfonyl group, 2-methylpropylsulfonyl group, 1-methylpropylsulfonyl group, 1,1-dimethylethylsulfonyl group, pentylsulfonyl group, 3-methylbutylsulfonyl group, 2-methylbutylsulfonyl group, 2,2-dimethylpropylsulfonyl group, 1-ethylpropylsulfonyl group, 1,1-dimethylpropylsulfonyl group, hexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group, 1,2-dimethylbutylsulfonyl group, heptylsulfonyl group, 1-methylhexylsulfonyl group, 1-ethylpentylsulfonyl group, octylsulfonyl group, 1-methylheptylsulfonyl group, 2-ethylhexylsulfonyl group, nonylsulfonyl group, decylsulfonyl group, etc., preferably C₁₋₆ alkylsulfonyl group, more preferably C₁₋₄ straight or branched chain alkylsulfonyl group.

"Alkylsulfinyl group" includes such as methylsulfinyl group, ethylsulfinyl group, propylsulfinyl group, 1-methylethylsulfinyl group, butylsulfinyl group, 2-methylpropylsulfinyl group, 1-methylpropylsulfinyl group, 1,1-dimethylethylsulfinyl group, pentylsulfinyl group, 3-methylbutylsulfinyl group, 2-methylbutylsulfinyl group, 2,2-dimethylpropylsulfinyl group, 1-ethylpropylsulfinyl group, 1,1-dimethylpropylsulfinyl group, hexylsulfinyl group, 4-methylpentylsulfinyl group, 3-methylpentylsulfinyl group, 2-methylpentylsulfinyl group, 1-methylpentylsulfinyl group, 3,3-dimethylbutylsulfinyl group, 2,2-dimethylbutylsulfinyl group, 1,1-dimethylbutylsulfinyl group, 1,2-dimethylbutylsulfinyl group, heptylsulfinyl group, 1-methylhexylsufinyl group, 1-ethylpentylsulfinyl group, octylsulfinyl group, 1-methylheptylsulfinyl group, 2-ethylhexylsulfinyl group, nonylsulfinyl group, decylsulfinyl group, etc., preferably C₁₋₆alkylsulfinyl group, more preferably C₁₋₄ straight or branched chain alkylsulfinyl group.

"Alkoxy moiety" in "alkoxycarbonyl group" is the same as the alkoxy group mentioned above. Examples of "alkoxycarbonyl group" are methoxycarbonyl group, ethoxycarbonyl group, propoxycarbonyl group, 1-methylethoxycarbonyl group, butoxycarbonyl group, 2-methylpropoxycarbonyl group, 1-methylpropoxycarbonyl group, 1,1-dimethylethoxycarbonyl group, pentoxycarbonyl group, 3-methylbutoxycarbonyl group, 2-methylbutoxycarbonyl group, 2,2-dimethylpropoxycarbonyl group, 1-ethylpropoxycarbonyl group, 1,1-dimethylpropoxycarbonyl group, hexyloxycarbonyl group, 4-methylpentyloxycarbonyl group, 3-methylpentyloxycarbonyl group, 2-methylpentyloxycarbonyl group, 1-methylpentyloxycarbonyl group, 3,3-dimethylbutoxycarbonyl group, 2,2-dimethylbutoxycarbonyl group, 1,1-dimethylbutoxycarbonyl group, 1,2-dimethylbutoxycarbonyl group, heptyloxycarbonyl group, 1-methylhexyloxycarbonyl group, 1-ethylpentyloxycarbonyl group, octyloxycarbonyl group, 1-methylheptyloxycarbonyl group, 2-ethylhexyloxycarbonyl group, nonyloxycarbonyl group, decyloxycarbonyl group, etc., preferably C₂₋₆ alkoxycarbonyl group, more preferably C₂₋₄ straight or branched chain alkoxycarbonyl group.

"Alkenyl moiety" in "alkenyloxy group", "alkenyloxycarbonyl group", "alkenylcarbonyl group", "alkenylcarbonyloxy group", "alkenylsulfonyl group", and "alkenylsulfinyl group" is the same as the alkenyl group mentioned above.

"Alkenyloxy group" includes for example, ethenyloxy group, propenyloxy group, 1-methylethenyloxy group, butenyloxy group, 2-methylpropenyloxy group, 1-methylpropenyloxy group, pentenyloxy group, 3-methylbutenyloxy group, 2-methylbutenyloxy group, 1-ethylpropenyloxy group, hexenyloxy group, 4-methylpentenyloxy group, 3-methylpentenyloxy group, 2-methylpentenyloxy group, 1-methylpentenyloxy group, 3,3-dimethylbutenyloxy group, 1,2-dimethylbutenyloxy group, heptenyloxy group, 1-methylhexenyloxy group, 1-ethylpentenyloxy group, octenyloxy group, 1-methylheptenyloxy group, 2-ethylhexenyloxy group, nonenyloxy group, decenyloxy group, etc., preferably C₂₋₆, more preferably C₂₋₅ alkenyloxy group.

"Alkenylcarbonyl group" includes such as, ethenylcarbonyl group, propenylcarbonyl group, 1-methylethenylcarbonyl group, butenylcarbonyl group, 2-methylpropenylcarbonyl group, 1-methylpropenylcarbonyl group, pentenylcarbonyl group, 3-methylbutenylcarbonyl group, 2-methylbutenylcarbonyl group, 1-ethylpropenylcarbonyl group, hexenylcarbonyl group, 4-methylpentenylcarbonyl group, 3-methylpentenylcarbonyl group, 2-methylpentenylcarbonyl group, 1-methylpentenylcarbonyl group, 3,3-dimethylbutenylcarbonyl group, 1,2-dimethylbutenylcarbonyl group, heptenylcarbonyl group, 1-methylhexenylcarbonyl group, 1-ethylpentenylcarbonyl group, octenylcarbonyl group, 1-methylheptenylcarbonyl group, 2-ethylhexenylcarbonyl group, nonenylcarbonyl group, decenylcarbonyl group, etc., preferably C₃₋₆, and more preferably C₃₋₅ alkenylcarbonyl group.

"Alkenylcarbonyloxy group" includes one constituted by binding an oxygen atom to carbonyl moiety of alkenylcarbonyl group, preferably, C₃₋₆, and more preferably C₃₋₅ alkenylcarbonyloxy group.

"Alkenyloxycarbonyl group" includes for example, ethenyloxycarbonyl group, propenyloxycarbonyl group, 1-methylethenyloxycarbonyl group, butenyloxycarbonyl group, 2-methylpropenyloxycarbonyl group, 1-methylpropenyloxycarbonyl group, pentenyloxycarbonyl group, 3-methylbutenyloxycarbonyl group, 2-methylbutenyloxycarbonyl group, 1-ethylpropenyloxycarbonyl group, hexenyloxycarbonyl group, 4-methylpentenyloxycarbonyl group, 3-methylpentenyloxycarbonyl group, 2-methylpentenyloxycarbonyl group, 1-methylpentenyloxycarbonyl group, 3,3-dimethylbutenyloxycarbonyl group, 1,2-dimethylbutenyloxycarbonyl group, heptenyloxycarbonyl group, 1-methylhexenyloxycarbonyl group, 1-ethylpentenyloxycarbonyl group, octenyloxycarbonyl group, 1-methylheptenyloxycarbonyl group, 2-ethylhexenyloxycarbonyl group, nonenyloxycarbonyl group, decenyloxycarbonyl group, etc., preferably C₃₋₆, and more preferably C₃₋₅ alkenyloxycarbonyl group.

"Alkenylsulfonyl group" includes for example, ethenylsulfonyl group, propenylsulfonyl group, 1-methylethenylsulfonyl group, butenylsulfonyl group, 2-methylpropenylsulfonyl group, 1-methylpropenylsulfonyl group, pentenylsulfonyl group, 3-methylbutenylsulfonyl group, 2-methylbutenylsulfonyl group, 1-ethylpropenylsulfonyl group, hexenylsulfonyl group, 4-methylpentenylsulfonyl group, 3-methylpentenylsulfonyl group, 2-methylpentenylsulfonyl group, 1-methylpentenylsulfonyl group, 3,3-dimethylbutenylsulfonyl group, 1,2-dimethylbutenylsulfonyl group, heptenylsulfonyl group, 1-methylhexenylsulfonyl group, 1-ethylpentenylsulfonyl group, octenylsulfonyl group, 1-methylheptenylsulfonyl group, 2-ethylhexenylsulfonyl group, nonenylsulfonyl group, decenylsulfonyl group, etc., more preferably C₂₋₆, more preferably C₂₋₅ alkenylsulfonyl group.

"Alkenylsulfinyl group" includes such as ethenylsulfinyl group, propenylsulfinyl group, 1-methylethenylsulfinyl group, butenylsulfinyl group, 2-methylpropenylsulfinyl group, 1-methylpropenylsulfinyl group, pentenylsulfinyl group, 3-methylbutenylsulfinyl group, 2-methylbutenylsulfinyl group, 1-ethylpropenylsulfinyl group, hexenylsulfinyl group, 4-methylpentenylsulfinyl group, 3-methylpentenylsulfinyl group, 2-methylpentenylsulfinyl group, 1-methylpentenylsulfinyl group, 3,3-dimethylbutenylsulfinyl group, 1,2-dimethylbutenylsulfinyl group, heptenylsulfinyl group, 1-methylhexenylsulfinyl group, 1-ethylpentenylsulfinyl group, octenylsulfinyl group, 1-methylheptenylsulfinyl group, 2-ethylhexenylsulfinyl group, nonenylsulfinyl group, decenylsulfinyl group, etc., preferably C₂₋₆, more preferably C₂₋₅ alkenylsulfinyl group.

"Alkynyl moiety" in "alkynyloxy group", "alkynylcarbonyl group", "alkylcarbonyloxy group", "alkynylsulfonyl group", "alkynylsulfinyl group" and "alkynyloxycarbonyl group" is the same as the alkynyl group as mentioned above.

"Alkynyloxy group" includes for example, ethynyloxy group, propynyloxy group, butynyloxy group, pentynyloxy group, 3-methylbutynyloxy group, hexynyloxy group, 4-methylpentynyloxy group, 3-methylpentynyloxy group, 3,3-dimethylbutynyloxy group, heptynyloxy group, octynyloxy group, 3-methylheptynyloxy group, 3-ethylhexynyloxy group, nonynyloxy group, decynyloxy group, etc., preferably C₂₋₆ and more preferably C₂₋₅ alkynyloxy group.

"Alkynylcarbonyl group" includes for example, ethynylcarbonyl group, propynylcarbonyl group, butynylcarbonyl group, pentynylcarbonyl group, 3-methylbutynylcarbonyl group, hexynylcarbonyl group, 4-methylpentynylcarbonyl group, 3-methylpentynylcarbonyl group, 3,3-dimethylbutynylcarbonyl group, heptynylcarbonyl group, octynylcarbonyl group, 3-methylheptynylcarbonyl group, 3-ethylhexynylcarbonyl group, nonynylcarbonyl group, decynylcarbonyl group, etc., preferably C₃₋₆, more preferably C₃₋₅ alkynylcarbonyl group.

"Alkynylcarbonyloxy group" includes for example, one constituted by combining an oxygen atom to carbonyl moiety of the above "alkynylcarbonyl group". Preferably C₃₋₆, and more preferably C₃₋₅ alkynylcarbonyloxy groups are illustrated.

"Alkynylsulfonyl group", includes for example, ethynylsulfonyl group, propynylsulfonyl group, butynylsulfonyl group, pentynylsulfonyl group, 3-methylbutynylsulfonyl group, hexynylsulfonyl group, 4-methylpentynylsulfonyl group, 3-methylpentynylsulfonyl group, 3,3-dimethylbutynylsulfonyl group, heptynylsulfonyl group, octynylsulfonyl group, 3-methylheptynylsulfonyl group, 3-ethylhexynylsulfonyl group, nonynylsulfonyl group, or decynylsulfonyl group, preferably C₂₋₆, more preferably C₂₋₅ alkynylsulfonyl group.

"Alkynylsulfinyl group" includes for example, ethynylsulfinyl group, propynylsulfinyl group, butynylsulfinyl group, pentynylsulfinyl group, 3-methylbutynylsulfinyl group, hexynylsulfinyl group, 4-methylpentynylsulfinyl group, 3-methylpentynylsulfinyl group, 3,3-dimethylbutynylsulfinyl group, heptynylsulfinyl group, octynylsulfinyl group, 3-methylheptynylsulfinyl group, 3-ethylhexynylsulfinyl group, nonynylsulfinyl group, or decynylsulfinyl group, preferably C₂₋₆, more preferably C₂₋₅ alkynylsulfinyl group.

"Alkynyloxycarbonyl group" includes for example, ethynyloxycarbonyl group, propynyloxycarbonyl group, butynyloxycarbonyl group, pentynyloxycarbonyl group, 3-methylbutynyloxycarbonyl group, hexynyloxycarbonyl group, 4-methylpentynyloxycarbonyl group, 3-methylpentynyloxycarbonyl group, 3,3-dimethylbutynyloxycarbonyl group, heptynyloxycarbonyl group, octynyloxycarbonyl group, 3-methylheptynyloxycarbonyl group, 3-ethylhexynyloxycarbonyl group, nonynyloxycarbonyl group, or decynyloxycarbonyl group, preferably C₃₋₆, more preferably C₃₋₅ alkynyloxycarbonyl group.

As "cycloalkyl moiety" in "cycloalkylcarbonyl group", "cycloalkylcarbonyloxy group", "cycloalkylsulfonyl group" and "cycloalkylsulfinyl group", the same groups as the above cycloalkyl groups are illustrated.

As "cycloalkylcarbonyl group", the following groups are illustrated; cyclopropylcarbonyl group, cyclobutylcarbonyl group, cyclopentylcarbonyl group, cyclohexylcarbonyl group, cycloheptylcarbonyl group, or cyclooctylcarbonyl group.

As "cycloalkylcarbonyloxy group", one constituted by binding an oxygen atom to carbonyl moiety of "cloalkylcarbonyl group" are illustrated. For example, cyclopropylcarbonyloxy group, cyclobutylcarbonyloxy group, cyclopentylcarbonyloxy group, cyclohexylcarbonyloxy group, cycloheptylcarbonyloxy group, and cyclooctylcarbonyloxy group are illustrated.

As "cycloalkylsulfonyl group", the following groups are illustrated; cyclopropylsulfonyl group, cyclobutylsulfonyl group, cyclopentylsulfonyl group, cyclohexylsulfonyl group, cycloheptylsulfonyl group, and cyclooctylsulfonyl group.

As "cycloalkylsulfinyl group", the following groups are illustrated; cyclopropylsulfinyl group, cyclobutylsulfinyl group, cyclopentylsulfinyl group, cyclohexylsulfinyl group, cycloheptylsulfinyl group, and cyclooctylsulfinyl group.

As "cycloalkoxy" in "cycloalkoxycarbonyl group", the same as the above cycloalkoxy group is illustrated. For example, cyclopropyloxycarbonyl group, cyclobutyloxycarbonyl group, cyclopentyloxycarbonyl group, cyclohexyloxycarbonyl group, cycloheptyloxycarbonyl group, or cyclooctyloxycarbonyl group is illustrated.

As aryl in "aryloxy group", "arylcarbonyl group", "aryloxycarbonyl group", "arylcarbonyloxy group", "arylsulfonyl group" and "arylsulfinyl group", the same as the above aryl group is illustrated. As "aryloxy group" is illustrated phenoxy group, 1-naphthoxy group or 2-naphthoxy group. As "arylcarbonyl group" is illustrated benzoyl group, 1-naphthaloyl group or 2-naphthaloyl group. As "aryloxycarbonyl group" is illustrated phenoxycarbonyl group, 1-naphthoxycarbonyl group or 2-naphthoxycarbonyl group. As "arylcarbonyloxy group" is illustrated benzoyloxy group, 1-naphthoyloxy group or 2-naphthoyloxy group. As "arylsulfonyl group" is illustrated phenylsulfonyl group, 1-naphthylsulfonyl group, or 2-naphthylsulfonyl group. As "arylsulfinyl group" is illustrated phenylsulfinyl group, 1-naphthylsulfinyl group, or 2-naphthylsulfinyl group.

As heteroaryl group in "heteroaryloxy group", "heteroarylcarbonyl group", "heteroaryloxycarbonyl group", "heteroarylcarbonyloxy group", "heteroarylsulfonyl group" and "heteroarylsulfinyl group" is illustrated the same as the above heteroaryl groups. As "heteroaryloxy group" is illustrated pyrrolyloxy group, pyridyloxy group, pyrazinyloxy group, pyrimidinyloxy group, pyridazynyloxy group, furyloxy group, or thienyloxy group. As "heteroarylcarbonyl group" is illustrated pyrrolylcarbonyl group, pyridylcarbonyl group, pyrazinylcarbonyl group, pyrimidinylcarbonyl group, pyridazinylcarbonyl group, furylcarbonyl group, thienylcarbonyl group, etc. As "heteroaryloxycarbonyl group" is illustrated pyrrolyloxycarbonyl group, pyridyloxycarbonyl group, pyrazinyloxycarbonyl group, pyrimidinyloxycarbonyl group, pyridazinyloxycarbonyl group, furyloxycarbonyl group, or thienyloxycarbonyl group. As "heteroarylcarbonyloxy group" is illustrated pyrrolylcarbonyloxy group, pyridylcarbonyloxy group, pyrazinylcarbonyloxy group, pyrimidinylcarbonyloxy group, pyridazinylcarbonyloxy group, furylcarbonyloxy group, or thienylcarbonyloxy group. As "heteroarylsulfonyl group" is illustrated pyrrolylsulfonyl group, pyridylsulfonyl group, pyrazinylsulfonyl group, pyrimidinylsulfonyl group, pyridazinylsulfonyl group, furylsulfonyl group, or thienylsulfonyl group. As "heteroarylsulfinyl group" is illustrated pyrrolylsulfinyl group, pyridylsulfinyl group, pyrazinylsulfinyl group, pyrimidinylsulfinyl group, pyridazinylsulfinyl group, furylsulfinyl group, or thienylsulfinyl group.

As "saturated heterocyclic group" in A¹ is illustrated saturated 4 to 8 membered heterocyclic ring containing 1 to 2 hetero atoms selected from 1 to 2 nitrogen atoms, 0 to 1 oxygen atom and 0 to 1 sulfur atom (containing at least one nitrogen atom) and the sulfur atom may be substituted by 1 or 2 oxygen atoms, for example, azetidine, pyrrolidine, piperidine, piperazine, morpholine, thiomorpholine, thiomorpholine-1-oxide, thiomorpholine-1,1-dioxide, perhydroazepine, 2,6-dimethylpiperidine, 3,5-dimethylpiperidine, 2,6-dimethylpiperazine, 2,6-dimethylmorpholine, 3,5-dimethylmorpholine, 2,6-dimethylthiomorpholine, 3,5-dimethylthiomorpholine, etc.

As "unsaturated heterocyclic group" in A¹ is illustrated unsaturated 5 to 7 membered unsaturated heterocyclic ring containing 1 or 2 double bonds therein and containing 1 to 2 hetero atoms selected from 1 to 2 nitrogen atoms, 0 to 1 oxygen atom and 0 to 1 sulfur atom (containing at least one nitrogen atom) and the sulfur atom may be substituted by 1 or 2 oxygen atoms, for example, 5 membered nonaromatic unsaturated heterocyclic ring and containing a double bond therein, or 6 to 7 membered nonaromatic unsaturated heterocyclic ring and containing 1 or 2 double bonds therein

As "saturated or unsaturated heterocyclic ring group" in A¹ is illustrated bivalent group of a saturated heterocyclic ring selected from the following formulas (2) to (14): 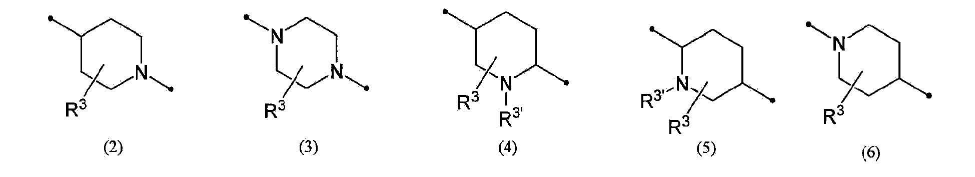 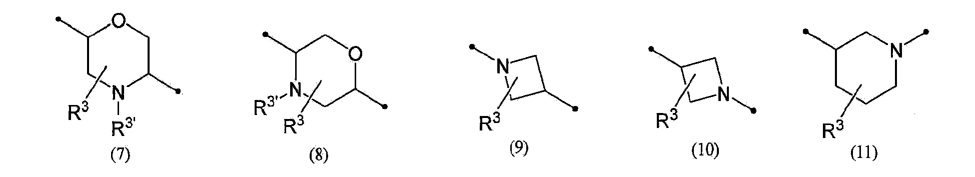 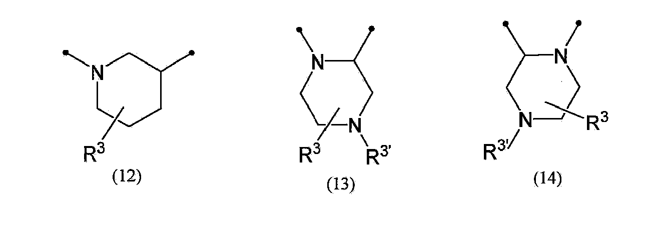 (wherein R³ and R³' are independently hydrogen atom or optionally substituted alkyl group, and the substitution-position is not limited, if chemically stable.)
or bivalent group of an unsaturated nonaromatic hetero cyclic, wherein double bond is formed between one or two C-C bonds or C-N bonds in the above ring structure.

A¹ is preferably selected from bivalent group of saturated heterocyclic group in the above formulas (2) to (14).

Aromatic carbocyclic group in A² includes benzene ring and naphthalene ring and its binding position is not limited.

In A² aromatic heterocyclic group includes 5 to 10 membered monocyclic or bicyclic heteroaromatic ring containing 1 to 4 hetero atoms selected from 0 to 3 nitrogen atoms, 0 to 1 oxygen atom and 0 to 1 sulfur atom, such as furan, thiophene, pyrrole, pyridine, indole, isoindole, quinoline, isoquinoline, pyrazole, imidazole, pyrimidine, pyrazine, pyridazine, thiazole, oxazole, etc. The binding position in the heterocyclic aromatic group is not specifically limited, if chemically stable.

"Substituent" in substituted alkyl group, substituted alkenyl group and substituted alkynyl group is selected from the group consisting of the following groups (a) to (c):
(a) halogen atom, hydroxy group, carboxy group, mercapto group and haloalkoxy group;
(b) alkoxy group, alkylcarbonyl group, alkoxycarbonyl group, alkylsulfonyl group, alkylsulfinyl group, alkylcarbonyloxy group, and alkylthio group (the group of this group may be substituted by halogen atom, hydroxy group, carboxyl group, alkoxy group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, or alkylsulfonyl group.);
(c) optionally substituted amino group, optionally substituted carbamoyl group, optionally substituted sulfamoyl group, optionally substituted cycloalkyl group, optionally substituted aryl group, optionally substituted heteroaryl group, optionally substituted saturated heterocyclic group, optionally substituted aryloxy group, and optionally substituted heteroaryloxy group, and said group can be substituted by one or more, and the same or different groups, preferably 1 to 5, more preferably 1 to 3 substituents.
   "Substituent" in optionally substituted cycloalkyl group and optionally substituted saturated heterocyclic group is selected from the group consisting of the following groups (d) to (f):
(d) halogen atom, hydroxy group, carboxy group, mercapto group, cyano group, nitro group,haloalkyl group, and haloalkoxy group;
(e) alkyl group, alkoxy group, alkenyl group, alkynyl group, alkoxycarbonyl group, and alkylthio group (the group of this group may be substituted by halogen atom, hydroxy group, carboxyl group, alkoxy group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, or alkylsulfonyl group.);
(f) optionally substituted amino group, optionally substituted carbamoyl group or optionally substituted sulfamoyl group, optionally substituted aryl group, optionally substituted heteroaryl group, optionally substituted aryloxy group, and optionally substituted heteroaryloxy group,
   and this group can be substituted by one or more, and the same or different groups, preferably 1 to 5, more preferably 1 to 3 substituents.
   "Substituent" in substituted aryl group, substituted heteroaryl group, substituted aryloxy group and substituted heteroaryloxy group is selected from the group consisting of the following groups (g) to (j);
(g) halogen atom, hydroxy group, carboxy group, mercapto group, cyano group, nitro group, haloalkyl group, and haloalkoxy group;
(h) alkyl group, alkoxy group, alkenyl group, alkynyl group, alkoxycarbonyl group, and alkylthio group (the group of this group may be substituted by halogen atom, hydroxy group, carboxyl group, alkoxy group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, or alkylsulfonyl group.);
(i) cycloalkyl group and saturated heterocyclic group (wherein the group of this group may be substituted by halogen atom, hydroxy group, carboxy group, alkyl group or alkoxy group.);
(j) optionally substituted amino group, optionally substituted carbamoyl group and optionally substituted sulfamoyl group; And this group can be substituted by one or more, and the same or different groups, preferably 1 to 5, more preferably 1 to 3 substituents.
   "Substituent" in substituted amino group, substituted carbamoyl group and substituted sulfamoyl group is selected from the group consisting of the following groups (k) to (m);
(k) alkyl group, alkenyl group, alkynyl group, alkylcarbonyl group, alkoxycarbonyl group, alkylsulfonyl group, alkylsulfinyl group, cycloalkyl group, cycloalkylcarbonyl group, cycloalkyloxycarbonyl group, cycloalklsulfonyl group, and cycloalkylsulfinyl group (the group of this group may be substituted by halogen atom, hydroxy group, carboxyl group, alkoxy group or alkoxycarbonyl group.);
(l) aryl group, arylcarbonyl group, aryloxycarbonyl group, arylsulfonyl group, arylsulfinyl group, heteroaryl group, heteroarylcarbonyl group, heteroaryloxycarbonyl group, heteroarylsulfonyl group, and heteroarylsulfinyl group (the group of this group may be substituted halogen atom, hydroxy group, carboxy group, mercapto group, cyano group, nitro group, alkyl group, alkoxy group, alkoxycarbonyl group or alkylthio group.);
(m) 4 to 7 membered saturated heterocyclic group containing 1 to 4 hetero atoms selected from 1 to 2 nitrogen atoms, 0 to 1 oxygen atom and 0 to 1 sulfur atom which is formed by combining two substituents (this saturated heterocyclic group containing nitrogen atom may be substituted, if chemically stable, on any carbon atom or nitrogen atom, by halogen atom, hydroxy group, carboxyl group, alkyl group, alkoxy group, alkoxycarbonyl group or alkylcarbonyl group); And said group may be substituted by one or two substituents, if chemically stable.
   "Saturated heterocyclic ring" mentioned above includes azetidine, pyrrolidine, piperidine, piperazine, morpholine, thiomorpholine, thiomorpholine-1-oxide, thiomorpholine-1,1-dioxide, perhydroazepine, etc.

In the present specification, "substituent" when alkylene is substituted includes halogen atom, hydroxy group, alkoxy group, etc., and said group may be substituted by the same or different and one or more substituents, preferably 1 to 5, more preferably 1 to 3 substituents.

A² of the formula (1) is preferably benzene ring or 5 to 6 membered heteroaromatic ring containing at least one hetero atom selected from 0 to 2 nitrogen atoms, 0 to 1 oxygen atom and 0 to 1 sulfur atom, more preferably benzene, pyridine and furan, and its binding sites are not limited, if chemically stable.

L³ of the formula (1) is preferably a single bond or C₁₋₄, preferably C₁₋₃ straight or branched alkylene, more preferably a single bond, methylene, ethylene, 1-methylmethylene, or 1,1-dimethylmethylene.

Preferable mode of "-A²-L³-CO₂R²" of the formula (1) is selected from following formulas (15) to (26): 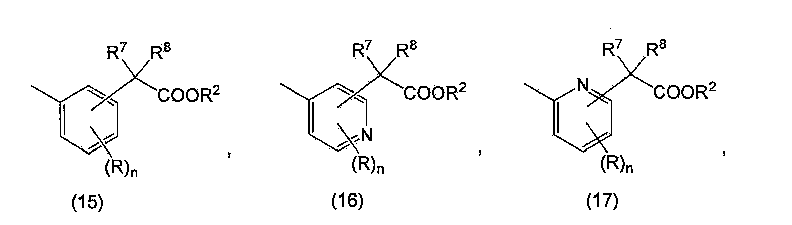 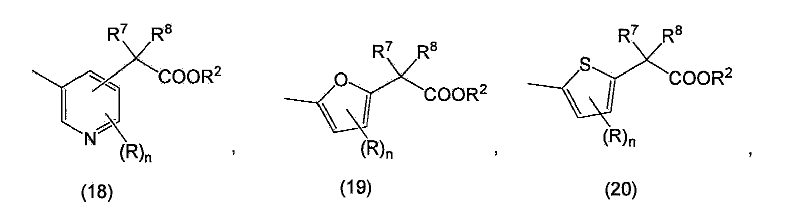 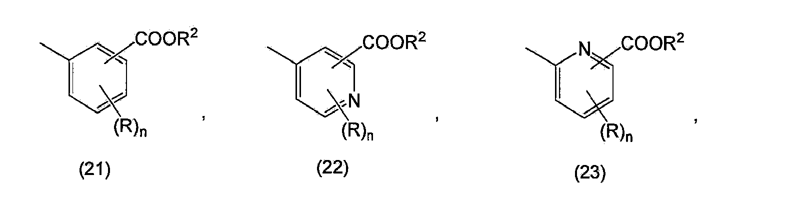 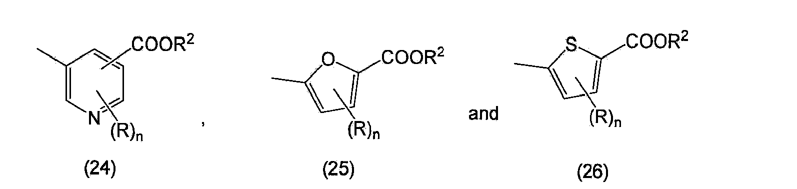 (wherein R² is the same as defined above, R⁷ and R⁸ are independently, hydrogen atom, or C₁₋₃ alkyl group, R is hydrogen atom, halogen atom, haloalkyl group, C₁₋₆ alkyl group, C₁₋₆ alkoxy group, C₁₋₆ haloalkyl group, C₁₋₆ haloalkoxy group, amino group optionally sybstituted by the same or different and 1 or 2 C₁₋₆ alkyl groups, or 4 to 8 membered saturated heterocyclic group containing 1 to 2 hetero atoms selected from 1 to 2 nitrogen atoms, 0 to 1 oxygen atom and 0 to 1 sulfur atom (this saturated heterocyclic group may be substituted by one or more substituents selected from halogen atom, hydroxy group, oxo group, C₁₋₆ alkyl group, C₁₋₆ alkoxy group, C₂₋₆ alkylcarbonyl group and C₂₋₆ alkoxycarbonyl group), n is an integer 0~2 and when n is 2, R may be the same or different. The binding position thereof is not limited, if chemically stable.)

More preferable ones are the formulas (15) to (20) wherein R⁷ and R⁸ are hydrogen atom.

Preferable examples on "-L¹-A¹-L²" in the formula (1) are illustrated below.

When nitrogen atom in the ring in A¹ is bound to L¹, preferable L¹ includes the group represented by the following formula (I) (wherein L¹ is bound at its left side with the adenine structure.):

(I) -(CH₂)_{2~8}-.

When carbon atom in the ring in A¹ is bound to L¹, preferable L¹ includes the group represented by the following formulas (II) or (III)(wherein L¹ is bound in its left side to the adenine structure.),

(II) -(CH₂)_{0~8}-_{,}

(III) -(CH₂)_{0~8}-NR⁵-

(wherein R⁵ is the same as defined above.).

When nitrogen atom in the ring in A¹ is bound to L², preferable L² includes the group represented by the following formulas (IV) to (IX) (wherein L² is bound at its left side with the adenine structure.),

(IV) -(CH₂)_{0~5}-,

(V) -(CH₂)_{2~5}-O-,

(VI) -(CH₂)_{2~5}-NR⁵'-(CH₂)_{0~3}-,

(VII) -(CH₂)_{2∼5}-NR⁵'-(CH₂)_{2~3}-O-,

(VIII) -(CH₂)_{0~5}-CO-(CH₂)_{0~2}-NR⁵'-(CH₂)_{0~3}-,

(IX) -(CH₂)_{0~5}-CO-(CH₂)_{0~2}-NR⁵'-(CH₂)_{2~3}-O-.

When carbon atom in the ring in A¹ is bound to L², preferable L² includes the group represented by the following formulas (X) to (XII) (wherein L² is bound at its left side with the adenine structure.):

(X) -(CH₂)_{0~5}-NR⁵'-(CH₂)_{0~3}-,

(XI) -(CH₂)_{0~5}-NR⁵'-(CH₂)_{0~3}-O-,

(XII) -(CH₂)_{0~5},

(XIII) -(CH₂)_{0~5}-O-.

(wherein R⁵' in the above (IV) to (XIII) is hydrogen atom or C₁₋₃ alkyl group.)

R² of the formula (1) is preferably C₁₋₄ alkyl group, C₃₋₈ alkylcarbonyloxyalkyl group, 6 to 10 membered arylcarbonyloxyalkyl group, 5 to 10 membered heteroarylcarbonyloxyalkyl group or alkyl group substituted by optionally substituted amino group. The alkyl group substituted by optionally substuted amino group preferably includes dialkylaminoaminoalkyl group, or alkyl group substituted by morpholino group, 1-piperidinyl group, piperazino group or 1-pyrrolidinyl, for example 4-dimethylaminobutyl group, 4-morpholinobutyl group, etc.
As the above alkylcarbonyloxyalkyl group are illustrated acetoxymethyl group, 1-acetoxyethyl group, etc. As the above arylcarbonyloxyalkyl group is illustrated benzoyloxymethyl group. R² is further preferably methyl group.

In the formula (1), X is preferably oxygen atom, or a single bond. When X is NR⁴, R⁴ is preferably hydrogen atom, or C₁₋₃ alkyl group, preferably hydrogen atom or methyl group.

In the formula (1), R¹ is preferably, optionally substituted C₁₋₆ straight or branched alkyl group such as methyl group, ethyl group, propyl group, butyl group, pentyl group, 1-methylethyl group, 1-methylpropyl group, 2-methylbutyl group respectively optionally substituted, more preferably straight chained C₁₋₄ alkyl group.

The substituent wherein R¹ is substituted alkyl group includes the above substituent of alkyl group, preferably fluorine atom, hydroxy group, C₁₋₄ straight or branched alkoxy group, or C₁₋₄ straight or branched alkylthio group, more preferably hydroxy group, or C₁₋₃ straight or branched alkoxy group, which may be substituted by one to three substituents.

The adenine compound of the present invention includes all tautomers, geometrical isomers and stereoisomers which are formed in accordance with the kind of the substituent, and a mixture thereof.

Namely, in a case where there are one or more asymmetrical carbon atoms in the compound of the formula (1), there exist diastereomers and optical isomers, and mixtures of those diastereomers and optical isomers and separated ones are also included in the present invention.

Additionally, the adenine compound shown by the formula (1) and its tautomer is chemically equivalent, and the adenine compound of the present invention includes such a tautomer. The tautomer is specifically a hydroxy compound shown by the formula (1'): 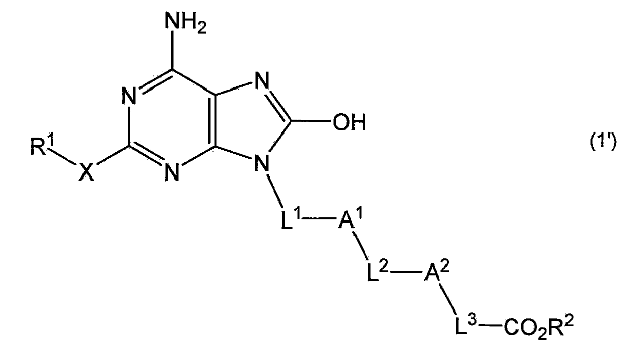 (wherein R¹, R², R³, A¹, A², X, L¹, L² and L³ are the same as defined above.)

The pharmaceutically acceptable salt is exemplified by an acid salt and a base addition salt. The acid salt is, for example, an inorganic acid salt such as hydrochloride, hydrobromide, sulfate, hydroiodide, nitrate and phosphate, and an organic acid salt such as citrate, oxalate, acetate, formate, propionate, benzoate, trifluoroacetate, fumarate, maleate, succinate, tartrate, lactate, pyruvate, methanesulfonate, benzenesulfonate and p-toluenesulfonate, and the base salt is exemplified by an inorganic base salt such as sodium salt, potassium salt, calcium salt, magnesium salt and ammonium salt, and an organic base salt such as triethylammonium salt, triethanolammonium salt, pyridinium salt and diisopropylammonium salt, and further a basic or acidic amino acid salt such as arginine salt, aspartic acid salt and glutamic acid salt. The compound shown by the formula (1) may be hydrate and a solvate such as ethanolate.

The compound of the generic formula (1) can be prepared by the following method. The starting materials which are not described can be prepared in accordance with the following method or by known methods or in accordance with the known methods.

### Preparation method 1

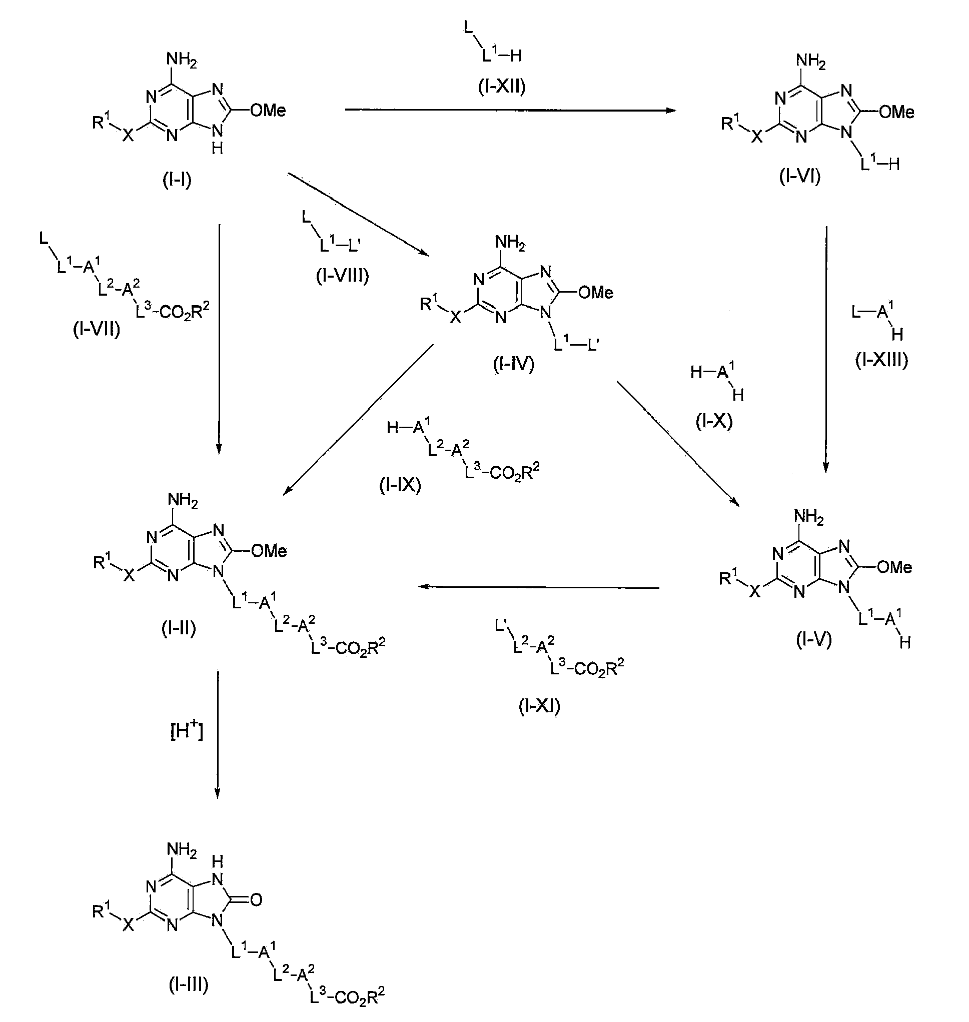

(wherein L and L' are the same or different an d a leaving group, A¹, A², R¹, R², X, L¹, L² and L³ are the same as defined above.)

The leaving group included halogen atom in alkylation or acylation, hydroxy group in dehydrative condensation, oxo group in reductive alkylation of amine, etc.

### [Step 1]

Compound (I-II) can be prepared by reacting compound (I-I) and compound (I-VII) in the presence of base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., metal hydride such as sodium hydride, etc, or metal alkoxide such as t-butoxy potassium, etc.

The solvent includes an aprotic solvent such as dimethylformamide, dimethyl sulfoxide, acetonitrile, etc., a halogenated hydrocarbon such as carbon tetrachloride, chloroform, methylene chloride, etc., an ether such as diethyl ether, tetrahydrofuran, 1,4-dioxne, etc. The reaction temperature is selected from the range of about 0°C to around boiling point of the solvent.

### [Step 2]

Compound (I-III) can be prepared by treating compound (I-II) under acidic condition.

The acid includes an inorganic acid such as hydrochloric acid, hydrobromic acid, sulfuric acid, etc., or an organic acid such as trifluoroacetic acid, etc. The solvent includes water or a mixture of water and an organic solvent. The above organic solvent includes an ether such as diethyl ether, tetrahydrofuran etc., an aprotic solvent such as dimethylformamide, acetonitrile, etc., and an alcohol such as methanol, ethanol, etc. The reaction temperature is selected from the range of room temperature to around boiling point of the solvent.

In the step for preparing compound (I-II) from compound (I-I), the compound (I-II) can be prepared by reacting compound (I-I) and compound (I-VIII) in the same manner as the above step 1 to give compound (I-IV) and then reacting the product and compound (I-IX) by the well known method in the art as dehydrative condensation or reductive alkylation in the same manner as the above step 1.

In the step to compound (I-II) from compound (I-IV), compound (I-II) can be also prepared by reacting compound (I-IV) and compound (I-X) by a well known method in the art such as dehydrative condensation or reductive alkylation in the same manner as step 1 to obtain compound (I-V) and then reacting the compound (I-V) and compound (I-XI) in the same manner as the above step 1.

In the step to compound (I-V) from compound (I-I), compound (I-V) can be also prepared by reacting compound (I-I) and compound (I-XII) in the same manner as step 1 to obtain compound (I-VI) and then reacting the compound (I-VI) and compound (I-XIII) by the well known method in the art such as dehydrative condensation or reductive alkylation.

### [Step 3]

Compound (I-VII) and compound (I-IX) can be also prepared by the following methods. 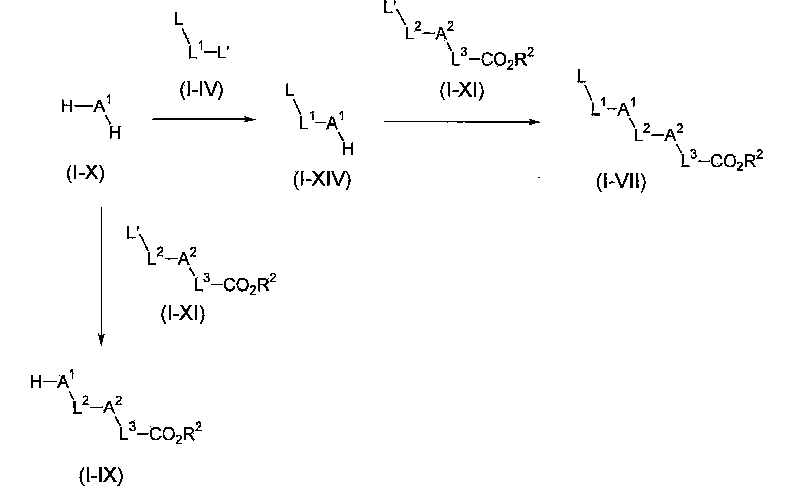 (wherein L and L' are the same or different and a leaving group, A¹, A², R², L¹, L² and L³ are the same as defined above.)

Compound (I-XIV) can be prepared by reacting compound (I-X) and compound (I-IV) in the presence of a base. Then, compound (I-VII) can be prepared by reacting compound (I-IV) and compound (I-XI) in the presence of a base.

Compound (I-IX) can be prepared by reacting compound (I-X) and compound (I-XI) in the presence of a base.

The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., an organic base such as triethylamine, diisopropylethylamine, pyridine, 4-dimethylaminopyridine, etc., or metal alkoxide such as sodium methoxide, etc.

The solvent includes a halogenated hydrocarbon such as carbon tetrachloride, chloroform, methylene chloride, etc., an ether such as diethyl ether, tetrahydrofuran, 1,4-dioxane, etc., an alcohol such as methanol, ethanol etc., an aprotic solvent such as dimethylformamide, dimethyl sulfoxide, acetonitrile, etc. The reaction temperature is selected from the range of about 0°C to around boiling point of the solvent.

When the compound of the present invention or its intermediate has a functional group such as amino group, carboxy group, hydroxy group, or oxo group etc., the compound can be protected or deprotected, if necessary. The preferable protecting group, the protecting method and deprotecting method are described in detail in "Protective Groups in Organic Synthesis 2nd Edition (John Wiley & Sons, Inc.; 1990)" and so on.

### [Step 4]

Compound (I-I) can be also prepared by the following methods. 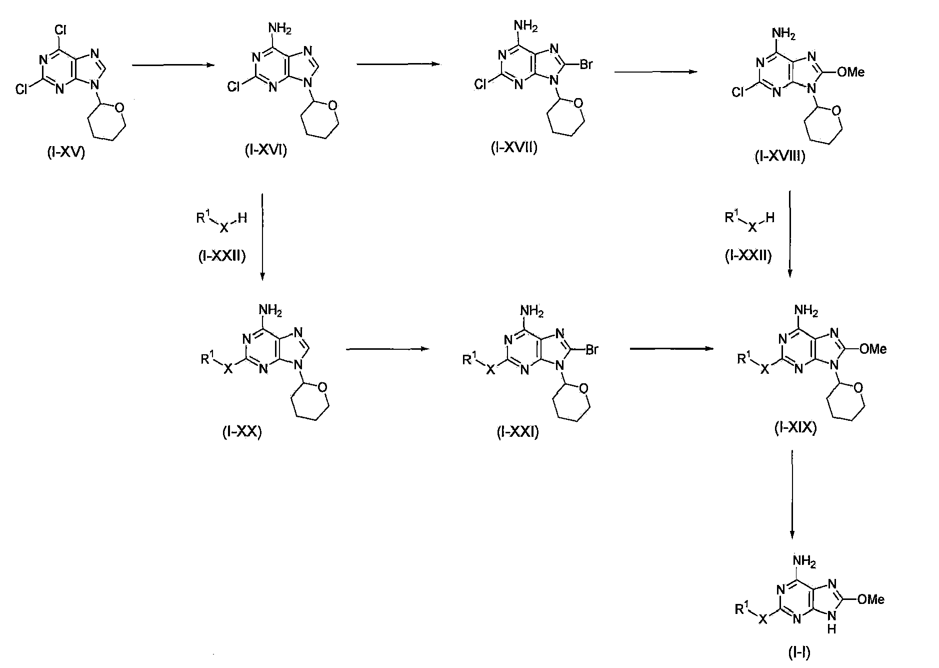 (wherein R¹ and X are the same as defined above.)

Compound (I-XVI) can be prepared by reacting compound (I-XV) and ammonia in an aqueous solution, an organic solvent or a mixture thereof.

The solvent includes an alcohol such methanol, ethanol, propanol, butanol, etc., an ether such as tetrahydrofuran, 1,4-dioxane, diglyme, etc., an aprotic solvent such as acetonitrile, etc. The reaction temperature is selected from the range of about room temperature to 200°C. The reaction may be carried out in an autoclave, if necessary.

Compound (I-XVII) can be prepared by brominating compound (I-XVI). The brominating agent includes for example, bromine, hydrobromic acid perbromide, N-bromosuccinimide, etc. In this reaction, a reaction additive such as sodium acetate, etc., may be added. The solvent includes a halogenated hydrocarbon such as carbon tetrachloride, methylene chloride, dichloroethane, etc., an ether such as diethyl ether, etc., acetic acid, and carbon disulfide. The reaction temperature is selected from the range of about 0°C to around boiling point of the solvent.

Compound (I-XVIII) can be prepared by reacting compound (I-XVII) and sodium methoxide.

The organic solvent used in the reaction includes an ether such as diethyl ether, tetrahydrofuran, 1,4-dioxane, etc., an aprotic solvent such as dimethylformamide, etc., an alcohol such as methanol, etc. The reaction temperature is selected from room temperature to around boiling point of the solvent.

Compound (I-XVIII) can be prepared by treating compound (I-XVII) in an aqueous alkaline solution containing methanol.

The aqueous alkaline solution is an aqueous solution containing alkali metal hydroxide such as sodium hydroxide or potassium hydroxide. The reaction temperature was selected from the range of room temperature to boiling point of the solvent.

Compound (I-XIX) can be prepared by reacting compound (I-XVIII) and compound (I-XXII).

The compound wherein X is NR⁴ (R⁴ is the same as defined above) can be reacted in the presence or absence of a base.

The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., an organic base such as triethylamine, diisopropylethylamine, 4-dimethylaminopyridine, etc.

The solvent includes an ether such as tetrahydrofuran, 1,4-dioxane, diglyme, etc., an alcohol such as propanol, butanol, etc., an aprotic solvent such as dimethylformamide, etc. The reaction may be carried out without a solvent. The reaction temperature is selected from the range of about 50°C to 200°C.

The compound wherein X is oxygen atom or sulfur atom is reacted in the presence of a base. The base includes, for example alkali metal such as metalic sodium, metalic potassium, etc., or an alkali metal hydride such as sodium hydride, etc. The solvent includes an ether such as tetrahydrofuran, 1,4-dioxane, diglyme, etc., an aprotic solvent such as dimethylformamide, dimethyl sulfoxide, etc. The reaction may be carried out without a solvent. The reaction temperature is selected from the range of about 50°C to 200°C.

The compound wherein X is SO₂, can be prepared by oxidizing the intermediate wherein X is sulfur atom with Oxone® or m-chloroperbenzoic acid (mCPBA).

In the step to compound (I-XIX) from compound (I-XVI), the compound (I-XIX) can be also prepared by preparing compound (I-XX) in the same manner as above method and then after converting the compound into compound (I-XXI) to prepare compound (I-XIX).

Compound (I-I) can be prepared by treating compound (I-XIX) with trifluoroacetic acid in an organic solvent such as methanol solvent.

The acid includes an inorganic acid such as hydrochloric acid, hydrobromic acid, sulfuric acid, etc., or an organic acid such as trifluoroacetic acid, etc.

The solvent includes water or a mixture of water and an organic solvent. The above organic solvent includes an ether such as diethyl ether, tetrahydrofuran etc., an aprotic solvent such as dimethylformamide, acetonitrile, etc., and an alcohol such as methanol, ethanol, etc. The reaction temperature is selected from the range of room temperature to around boiling point of the solvent.

### Preparation method 2

When L² is a group represented by the following formula: 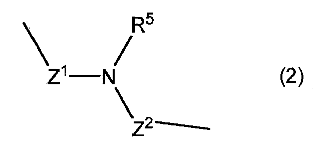 (wherein Z¹ and Z² are alkylene, and R⁵ is the same as defined.)
the compound can be prepared by the following methods. 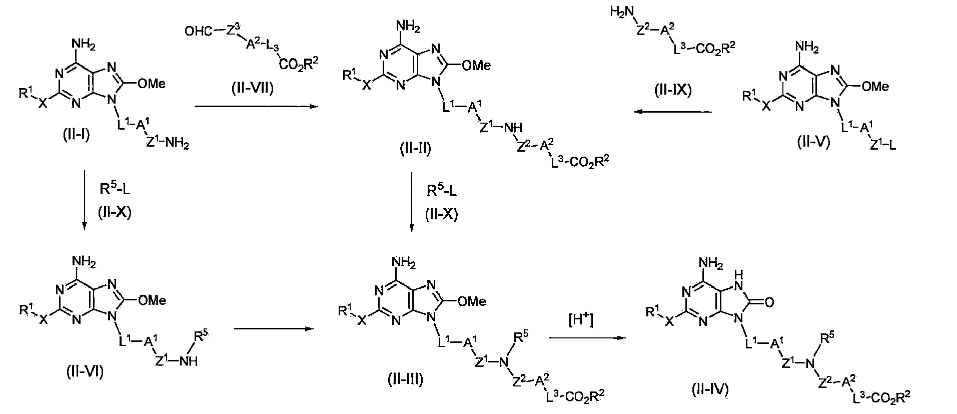 (wherein L, L¹, A¹, A², R¹, R², R⁵, X, Z¹, Z² and L³ are the same as defined above, Z³ is alkylene which corresponds to the alkylene prepared by deleting the terminal alkylene from Z².)

Namely compound (II-II) can be prepared by reacting compound (II-I) with aldehyde compound of compound (II-VII) in the presence of a reductive agent such as sodium borohydride (NaBH₄) in a solvent such as methanol, etc.

Compound (II-II) can be also prepared by reacting compound (II-V) with compound (II-IX) in the presence or absence of a base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., an organic base such as triethylamine, diisopropylethylamine, 4-dimethylaminopyridine, etc.

The solvent includes an ether such as tetrahydrofuran, 1,4-dioxane, diglyme, etc., an alcohol such as propanol, butanol, etc., an aprotic solvent such as dimethylformamide, dimethyl sulfoxide, acetonitrile, etc. The reaction may be carried out without a solvent. The reaction temperature is selected from the range of room temperature to boiling point of the solvent.

In case of compound wherein R⁵ is other group except hydrogen atom, compound (II-III) can be prepared by reacting compound (II-II) and a alkyl halide reagent represented by compound (II-X) in the presence of a base such as potassium carbonate, etc., in a solvent such as acetonitrile, dimethylformamide, etc.

Compound (II-III) can be prepared by converting compound (II-I)into compound (II-VI) in the same manner as the process of compound (II-III) from compound (II-II) in the preparation method 2, and then applying the same method as the process for preparing compound (II-II).

Compound (II-IV) being the same as compound (I-III) can be prepared in the same manner as the process of compound (I-III) from compound (I-II) in the preparation method 1.

### Preparation method 3

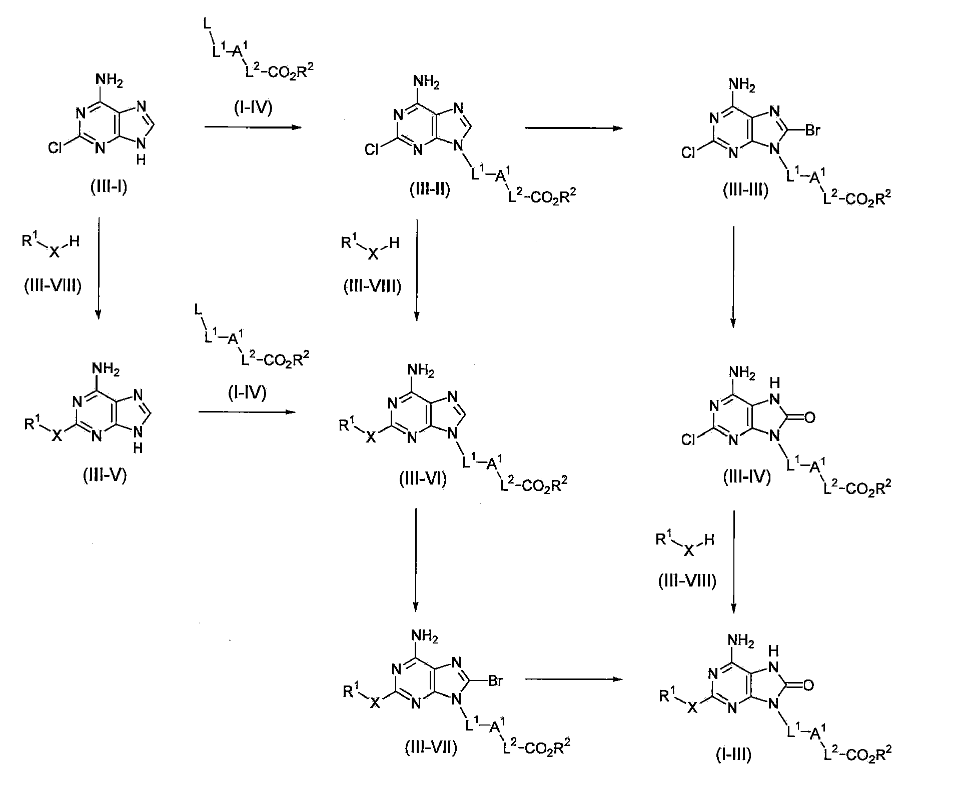

(wherein L is a leaving group, A¹, R¹, R², X, L¹ and L² are the same as defined above.)

Compound (III-II) can be prepared by reacting compound (III-I) and compound (I-IV) in the presence of a base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., metal hydride such as sodium hydride, etc, or metal alkoxide such as potassium t-butoxide, etc. The solvent includes a halogenated hydrocarbon such as carbon tetrachloride, chloroform, methylene chloride, etc., an ether such as diethyl ether, tetrahydrofuran, 1,4-dioxane, etc., an aprotic solvent such as dimethylformamide, dimethyl sulfoxide, acetonitrile, etc. The reaction temperature is selected from the range of about 0°C to around boiling point of the solvent.

Compound (III-III) can be prepared by brominating compound (III-II). The brominating agent includes for example, bromine, hydrobromic acid perbromide, N-bromosuccinimide, etc. In this reaction, a reaction auxiliary such as sodium acetate, etc., may be added. The solvent includes a halogenated hydrocarbon such as carbon tetrachloride, methylene chloride, dichloroethane, etc., an ether such as diethyl ether, etc., acetic acid, or carbon disulfide. The reaction temperature is selected from the range of about 0°C to around boiling point of the solvent.

Compound (III-IV) can be prepared by reacting compound (III-III) an metal alkoxide such as sodium methoxide and treating them in an acidic condition.

In case reacting the metal alkoxide, there can be used the solvent such as an ether such as diethyl ether, tetrahydrofuran, 1,4-dioxne, etc., an aprotic solvent such as dimethyfoemamide, etc. or an alcohol corresponding to the metal alkoxide such as methanol, etc. The reaction temperature is selected from the range of, for example, room temperature to boiling point of the solvent.

The acid includes an inorganic acid such as hydrochloric acid, hydrobromic acid, sulfuric acid, etc., or an organic acid such as trifluoroacetic acid, etc.

The solvent includes water or a mixture of water and an organic solvent. The above organic solvent includes an ether such as diethyl ether, tetrahydrofuran etc., an aprotic solvent such as dimethylformamide, acetonitrile, etc., and an alcohol such as methanol, ethanol, etc. The reaction temperature is selected from the range of room temperature to around boiling point of the solvent.

Compound (I-III) can be prepared by reacting compound (III-IV) and compound (III-VIII).

The compound wherein X is NR⁴ (R⁴ is the same as defined above) is reacted in the presence or absence of a base. The base includes, for example alkali metal carbonate such as sodium carbonate, potassium carbonate, etc., alkaline earth metal carbonate such as calcium carbonate, etc., metal hydroxide such as sodium hydroxide, potassium hydroxide, etc., an organic base such as triethylamine, diisopropylethylamine, 4-dimethylaminopyridine, etc.

The solvent includes an ether such as tetrahydrofuran, 1,4-dioxane, diglyme, etc., an alcohol such as propanol, butanol, etc., an aprotic solvent such as dimethylformamide, etc. The reaction may be carried out without a solvent. The reaction temperature is selected from the range of about 50°C to 200°C.

The compound wherein X is oxygen atom or sulfur atom is reacted in the presence of a base. The base includes, for example alkali metal such as metalic sodium, metalic potassium, etc., or an alkali metal hydride such as sodium hydride, etc. The solvent includes an ether such as tetrahydrofuran, 1,4-dioxane, diglyme, etc., an aprotic solvent such as dimethylformamide, dimethyl sulfoxide, etc. The reaction may be carried out without a solvent. The reaction temperature is selected from the range of about 50°C to 200°C.

The compound wherein X is SO₂ can be prepared by oxidizing the intermediate wherein X is sulfur atom with Oxone® or m-chloroperbenzoic acid (mCPBA).

In the step to compound (I-III) from compound (III-I), compound (I-III) is prepared by preparing compound (III-VI) from compound (III-II) in the same manner as the above method or by preparing compound (III-VI) via compound (III-V) from compound (III-I), and then converting compound (III-VI) to compound (I-III) via compound (III-VII).

In case of reaction with benzoyl isocyanate, the base includes, for example alkali metal carbonate such as sodium carbonate, potassium carbonate, etc., alkaline earth metal carbonate such as calcium carbonate, etc., an organic base such as triethylamine, diisopropylethylamine, pyridine, 4-dimethylaminopyridine, etc.

The solvent includes a hologenated hydrocarbon such as methylene chloride, an ether such as tetrahydrofuran, 1,4-dioxane, etc., an aprotic solvent such as dimethylformamide, dimethyl sulfoxide, etc. The reaction temperature is selected from the range of about 0°C to around boiling point of the solvent.

As the base used in the cyclization reaction, can be illustrated the base such as an alkali metal hydroxide, like sodium hydroxide or potassium hydroxide, or metal alkoxide, like sodium methoxide or potassium t-butoxide. Can be used the solvent such as an ether, like tetrahydrofuran, an alcohol, like ethanol or 2-propanol or an aprotic solvent, like dimethylformamide or dimethyl sulfoxide. The reaction temperature is selected from a range of around room temperature to around the boiling point of the solvent.

In a case where the compound of the present invention or its intermediate or the starting compound has a functional group, a reaction for increasing a carbon atom, a reaction for introducing a substituent or a reaction for conversion of the functional group can be conducted optionally according to a manner conventional to the skilled artisan in an appropriate step, namely in an intermittent step in each of the preparation methods described in the preparation method 1 or 2. For this purpose, the methods described in "JIKKEN KAGAKU-KOZA (edited by NIHON KAGAKU-KAI, MARUZEN)", or "Comprehensive Organic Transformation, R. C. Lalock (VCH Publishers, Inc. 1989)" can be used. The reaction for increasing a carbon atom includes a method comprising converting an ester group to hydroxymethyl group using a reducing agent such as aluminum lithium hydride, introducing a leaving group and then introducing a cyano group. The reacting for conversion of a functional group includes a reaction for conducting acylation or sulfonylation using an acid halide, a sulfonyl halide, etc., a reaction for reacting an alkylation agent such as an alkyl halide, a hydrolysis reaction, a reaction for C-C bond formation such as Friedel-Crafts reaction and Wittig reaction, and oxidizing or reducing reaction, etc.

In a case where the compound of the present invention or its intermediate contains a functional group such as amino group, carboxy group, hydroxy group and oxo group, a technology of protection and deprotection can optionally be used. A preferable protective group, a protection method and a deprotection method are described in details in "Protective Groups in Organic Synthesis 2nd Edition (John Wiley & Sons, Inc.; 1990)", etc.

The compound of the formula (1) of the present invention and the intermediate compound for production thereof can be purified by a method known to the skilled artisan. For instance, purification can be conducted by column chromatography (e.g. silica gel column chromatography or ion exchange chromatography) or recrystallization. As a recrystallization solvent, for instance, can be used an alcohol such as methanol, ethanol and 2-propanol, an ether such as diethyl ether, an ester such as ethyl acetate, an aromatic hydrocarbon such as benzene and toluene, a ketone such as acetone, a hydrocarbon such as hexane, an aprotic solvent such as dimethylformamide and acetonitrile, water and a mixture of two or more thereof. As other purification method, can be used those described in "JIKKEN KAGAKU-KOZA (edited by NIHON KAGAKU-KAI, MARUZEN) Vol. 1", etc.

In a case where the compound of the formula (1) of the present invention contains one or more asymmetric carbon, its production can be conducted by using the starting material containing those asymmetric carbons or by the asymmetric induction during the production steps. For instance, in a case of an optical isomer, the object can be obtained by using an optically active starting material or by conducting an optical resolution at a suitable stage of the production steps. The optical resolution method can be conducted by a diastereomer method comprising allowing the compound of the formula (1) or its intermediate to form a salt with an optically active acid (e.g. a monocarboxylic acid such as mandelic acid, N-benzyloxyalanine and lactic acid, a dicarboxylic acid such as tartaric acid, o-diisopropylidene tartrate and malic acid, a sulfonic acid such as camphor sulfonic acid and bromocamphor sulfonic acid) in an inert solvent (e.g. an alcohol such as methanol, ethanol, and 2-propanol, an ether such as diethyl ether, an ester such as ethyl acetate, a hydrocarbon such as toluene, an aprotic solvent such as acetonitrile and a mixture thereof).

In a case where the compound of the formula (1) or its intermediate contains an acidic functional group such as carboxylic group, the object can be attained also by forming a salt with an optically active amine (e.g. an organic amine such as α-phenethylamine, quinine, quinidine, cinchonidine, cinchonine and strychnine).

The temperature for salt formation is selected from room temperature to the boiling point of the solvent. In order to increase optical purity, the temperature is preferably once increased up to the boiling point of the solvent. Upon recovering the salt formed by filtration, the yield can be increased optionally by cooling. An amount of the optical active acid or amine is about 0.5 to about 2.0 equivalent, preferably around 1 equivalent, relative to the substrate. An optically active salt with highly optical purity can be obtained optionally by recrystallization from an inert solvent (e.g. an alcohol such as methanol, ethanol and 2-propanol, an ether such as diethyl ether, an ester such as ethyl acetate, a hydrocarbon such as toluene, an aprotic solvent such as acetonitrile and a mixture thereof). If necessary, the optically resoluted salt can be converted into a free form by treating with an acid or a base by the conventional method.

The adenine compound or its pharmaceutically acceptable salt of the present invention activates Toll-like receptor (TLR), concretely TLR7 and is useful as an immuno-modulator and thus useful as a therapeutic and prophylactic agent for diseases associated with an abnormal immune response (e.g. autoimmune diseases and allergic diseases) and various infectious diseases and cancers which are required for activation of an immune response. For instance, the adenine compound or its pharmaceutically acceptable salt of the present invention is useful as a therapeutic and prophylactic agent for the diseases mentioned in the following (1)-(8).
(1) (Respiratory diseases): asthma, including bronchial, allergic, intrinsic, extrinsic, exercise-induced, drug-induced (including NSAID such as aspirin and indomethacin) and dust-induced asthma both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
(2) (Skin) psoriasis, atopic dermatitis, contact dermatitis or other eczematous dermatoses, and delayed-type hypersensitivity reactions; phyto-and photodermatitis; seborrheic dermatitis, dermatitis herpetiformis, lichen planus, lichen sclerosus, lichen sclerosus et atrophicus, pyoderma gangrenosum, skin sarcoidosis, discoid lupus erythematosus, pemphigus, pemphigoid, epidermolysis bullosa, urticaria, angioedema, vasculitides, toxic erythemas, cutaneous eosinophilias, alopecia areata, male-pa.ttern baldness, Sweet's syndrome, Weber-Christian syndrome, erythema multiforme; cellulitis, both infective and non-infective; panniculitis; cutaneous lymphomas, non-melanoma skin cancer and other dysplastic lesions; drug-induced disorders including fixed drug eruptions;
(3) (Eyes) blepharitis; conjunctivitis, including perennial and vernal allergic conjunctivitis; iritis; anterior and posterior uveitis; choroiditis; autoimmune, degenerative or inflammatory disorders affecting the retina; ophthalmitis including sympathetic ophthalmitis; sarcoidosis; infections including viral, fungal, and bacterial;
(4) (Genitourinary) nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
(5) (Allograft rejection) acute and chronic following, for example, transplantation of kidney, heart, liver, lung, bone marrow, skin or cornea or following blood transfusion; or chronic graft versus host disease;
(6) (Auto-immune diseases) other auto-immune and allergic disorders including rheumatoid arthritis, irritable bowel syndrome, systemic lupus erythematosus, multiple sclerosis, Hashimoto's thyroiditis, Graves' disease, Addison's disease, diabetes, idiopathic thrombocytopaenic purpura, eosinophilic fasciitis, hyper-IgE syndrome, antiphospholipid syndrome;
(7) (Oncology) treatment of common cancers including prostate, breast, lung, ovarian, pancreatic, liver bowel and colon, stomach, skin and brain tumors and malignant bone marrow neoplasm (including the leukaemias) and lymphoproliferative systems neoplasm, such as Hodgkin's and non-Hodgkin's lymphoma; including the prevention and treatment of metastasis and tumor recurrences, and paraneoplastic syndromes; and
(8) (Infectious diseases) viral diseases such as genital warts, common warts, plantar warts, hepatitis B, hepatitis C, herpes simplex virus, molluscum contagiosum, variola, acquired immunodeficiency syndrome (HIV), or infectious diseases due to human papilloma virus (HPV), cytomegalo virus (CMV), varicella zoster virus (VZV), rhinovirus, adenovirus, coronavirus, influenza, or para-influenza; bacterial diseases such as tuberculosis, mycobacterium avium, or leprosy; other infectious diseases, such as fungal diseases, candida, chlamydia, or aspergillus, cryptococcal meningitis, pneumocystis carnii, cryptosporidiosis, histoplasmosis, toxoplasmosis, trypanosome infection, or leishmaniasis.

The adenine compounds or pharmaceutically acceptable salt thereof can also be used as vaccine adjuvant.

The adenine compound of the present invention, or its pharmaceutically acceptable salt has an activating effect of TLR, concretely TLR7. The adenine compound of the present invention, or its pharmaceutically acceptable salt shows an interferon-α or interferon-y inducing activity and a suppressing activity of the production of IL-4 or IL-5, and thus shows an effect as a medicament having an immunomodulating activity specific against type 1 helper T-cell (Thlcell)/type 2 helper T-cell (Th2cell), namely, preferably useful as a prophylactic or therapeutic agent for allergic diseases such as asthma, COPD, allergic rhinitis, allergic conjunctivitis and atopic dermatosis due to the cell selective immuno-suppressive action. On the other hand, due to its immune activating effect, it is useful as a prophylactic or therapeutic agent for cancer, hepatitis B, hepatitis C, acquired immunodeficiency syndrome (HIV) and viral disease caused by infection with human papilloma virus (HPV), a bacterial infectious disease and dermatosis such psoriasis.

The adenine compound of the present invention, or its pharmaceutically acceptable salt is useful as a prophylactic or therapeutic agent for airway obstruction such as asthma or COPD, or for reduction of the risk thereof.

The adenine compound of the present invention or its pharmaceutically acceptable salt has no limitation as to its administration formulation and is administered orally or parenterally. The preparation for oral administration can be exemplified by capsules, powders, tablets, granules, fine-grain, syrups, solutions, suspensions, etc., and the preparation for parenteral administration can be exemplified by injections, drips, eye-drops, intrarectal preparations, inhalations, sprays (e.g. liquids/suspensions for sprays, aerosols, or cartridge spray for inhalators or insufflators), lotions, gels, ointments, creams, transdermal preparations, transmucosa preparations, nasal drops, ear drops, tapes, transdermal patches, cataplasms, powders for external application, and the like. Those preparations can be prepared by known manners, and acceptable conventional carriers, fillers, binders, lubricants, stabilizers, disintegrants, buffering agents, solubilizing agents, isotonic agents, surfactants, antiseptics, perfumes, and so on can be used. Two or more pharmaceutical carriers can be appropriately used.

The compound of the present invention, or its pharmaceutically acceptable salt is admixed with a pharmaceutically acceptable carrier by the conventional method for the person in the art to prepare the pharmaceutical composition suitable for administration. For example, the pharmaceutical composition containing the compound of the present invention or its pharmaceutically acceptable salt 0.05-99 weight %, preferably 0.05 - 80 weight %, more preferably 0.1 - 70 weight %, and further more preferably 0.1 - 50 weight % as an active ingredient can be prepared.

The liquid preparation such as emulsions and syrups, among the preparations for oral administration, can be prepared by using additives for a pharmaceutical preparation including water; a sugar such as sucrose, sorbitol and fructose; ethanol; a glycol such as polyethylene glycol and propylene glycol; an oil such as sesame oil, olive oil and soybean oil; an preservative such as p-hydroxybenzoate; a sweetening such as saccharin, a thickening agent such as carboxymethyl cellulose, a flavor such as strawberry flavor and peppermint flavor, a coloring agent and so on.

The solid preparation such as capsules, tablets, powders and granules can be prepared by appropriately using following filleres: a carrier such as lactose, glucose, sucrose sorbitol, mannitol, mannite and a cellulose derivative; a disintegrant such as starch (potato starch, corn starch, amylopectin, etc.) and sodium alginate; a lubricant such as magnesium stearate, calcium stearate, polyethylene glycol, wax, paraffin and talc; a binder such as polyvinyl alcohol, polyvinyl pyrrolidone, hydroxypropyl cellulose and gelatin; a surfactant such as a fatty acid ester; or a plasticizer such as glycerin.

In case of preparation of sugar coated tablets, a condensed sugar solution, which may contain gum arabic, gelatin, talc, or titanium oxide is coated on the core of tables prepared by using fillers as described above. There can be also prepared a film tablet, which is coated by a suitable polymer dissolved in an easily removable organic solvent.

In case of preparation of soft gelatin capsules, the capsules can be prepared by mixing the compound of the present invention with for example, vegetable oil or polyethylene glycol. In case of preparation of hard gelatin capsules, the capsules can be prepared by using granules of the compound of the present invention which are prepared by mixing it with a suitable filler as described above.

The liquid preparation such as injections, drips, eye-drops and ear drops, among the preparations for parenteral administration, can be prepared preferably as a sterilized isotonic liquid preparation. For instance, injections can be prepared by using an aqueous medium such as a salt solution, a glucose solution or a mixture of a salt solution and a glucose solution. The preparation for intrarectal administration can be prepared by using a carrier such as cacao butter usually in the form of suppository.

The ointments, creams and gels contain the compound of the present invention usually in an amount of 0.01-10 w/w%, and there may be incorporated a thickener suitable to an aqueous or oily base and/or a gelling agent and/or a solvent. The base is exemplified by water and/or an oil such as liquid paraffin, a vegetable oil such as arachis oil and castor oil, a solvent such as polyethylene glycol, and so on. The thickener and gelling agent are exemplified by soft paraffin, aluminum stearate, cetostearic alcohol, polyethylene glycol, sheep fat, beeswax, carboxypolymethylene and cellulose derivatives and/or glyceryl monostearate and/or nonionic emulsifiers.

The lotions contain the compound of the present invention usually in an amount of 0.01-10 w/w%, and it may be prepared with the use of an aqueous or oily base, it may contain generally emulsifiers, stabilizers, dispersing agents, precipitation inhibitors and also thickeners.

Powders for external use contain the compound of the present invention usually an amount of 0.01-10 w/w%, and it may be formulated using a suitable powdery base such as talc, lactose and starch.

The drips may be formulated by using an aqueous or non-aqueous base, and may contain dispersing agents, solubilizing agents, precipitation inhibitors or preservatives.

The sprays (sprays, aerosols, dry-powders, etc.) may be formulated into an aqueous solution or suspension using a suitable liquid propellant, or into an aerosol distributed from a pressured package such as a metered-dose inhaler. Dry-powders preparations can be used.

The aerosols suitable to inhalation may be a suspension or aqueous solution, and they contain generally the compound of the present invention and a suitable propellant such as fluorocarbon, hydrogencontaining chlorofluorocarbon and a mixture thereof, particularly hydrofluoroalkane, specifically 1,1,1,2-tetrafluoroethane, heptafluoroalkane (HFA) such as 1,1,1,2,3,3,3-heptafluoro-n-propane or a mixture thereof. The aerosols may contain optionally additional excipients well known in the art such as a surfactant, (e.g., oleic acid or lecithin) and a co-solvent such as ethanol. For example, an inhaler known as Turubuhaler® is illustrated.

The gelatin capsules or cartridges used for inhalator or insufflator may be formulated by using a powdery mixture of the compounds used in the present invention and a powdery base such as lactose and starch. They contain the compound of the present invention usually in an amount of 20µg- 10mg. The compound of the present invention may be administered without using excipients such as lactose as an alternative method.

In case of being orally or nasally inhalated in the form of pressured HFA aerosols or dry-powders preparations, the adenine compound of the present invention, or its pharmaceutically acceptable salt is pulverized in a size of less than 10µm and it is dispersed in a dispersing agent such as C₈₋₂₀ fatty acid or its salt (e.g., oleic acid), bile salt, phospholipid, an alkyl saccharide, a completely fluorinated or polyethoxylated surfactant, or a pharmaceutically acceptable dispersing agent.

The adenine compound of the present invention is preferably parenterally administered as a preparation for topical administration. The suitable preparation is exemplified by ointments, lotions (solutions or suspensions), creams, gels, tapes, transdermal patches, cataplasms, sprays, aerosols, dry-powders, aqueous solutions/suspensions for cartridge spray for inhalators or insufflators, eye-drops, ear drops, nasal drops, transdermal agents, pulmonary absorbent, air-way absorbent, powders for external administrations and so on.

A ratio of the active compound of the present invention in the preparation for topical administration of the present invention is, though depending upon the formulation, generally 0.001-10 wt%, preferably 0.005-1%. The ratio used in powders for inhalation or insufflation is 0.1-5%.

In a case of aerosols, the compound of the present invention is preferably contained in an amount of 20-2000µg, more preferably about 20µg-500µg per each a measured amount or one sprayed amount. The dosage is once or several times per day, for instance, 2, 3, 4 or 8 times, and one to three units are administered per each time.

The pharmacological activity can be measured by any of conventional evaluation methods, preferably by an in vitro evaluation method. An example of the methods is a method described in examples of the present specification.

The invention further relates to combination therapies wherein a compound of the formula (1) or its pharmaceutically acceptable salt or a pharmaceutical composition comprising a compound of the formula (1) or its pharmaceutically acceptable salt is administered concurrently or sequentially or as a combined preparation with other therapeutic agent(s), for the treatment of one or more of the conditions listed in the specification.

In particular, for the treatment of the inflammatory diseases, COPD, asthma and allergic rhinitis, the compounds of the invention may be combined with agents such as tumour necrosis factor alpha (TNF-α) inhibitors such as anti-TNF monoclonal antibodies (for example Remicade, CDP-870 and adalimumab) and TNF receptor immunoglobulin molecules (such as Enbrel); non-selective cyclo-oxygenase COX-1/COX-2 inhibitors whether applied topically or systemically (such as piroxicam, diclofenac, propionic acids such as naproxen, flubiprofen, fenoprofen, ketoprofen and ibuprofen, fenamates such as mefenamic acid, indomethacin, sulindac, azapropazone, pyrazolones such as phenylbutazone, salicylates such as aspirin), COX-2 inhibitors (such as meloxicam, celecoxib, rofecoxib, valdecoxib, lumarocoxib, parecoxib and etoricoxib); glucocorticosteroids (whether administered by topical, oral, intramuscular, intravenous, or intra-articular routes); methotrexate, leflunomide; hydroxychloroquine, d-penicillamine, auranofin or other parenteral or oral gold preparations.

The present invention still further relates to combination therapies of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; SB-210661; pyridinyl-substituted 2-cyanonaphthalene compounds such as L-739,010; 2-cyanoquinoline compounds such as L-746,530; MK-591, MK-886, and BAY-x-1005.

The present invention still further relates to combination therapies of a compound of the invention together with a receptor antagonist for leukotrienes (LT)B4, LTC4, LTD4 and LTE4 selected from the group consisting of phenothiazin compound such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY-x-7195.

The present invention still further relates to combination therapies of a compound of the invention together with a phosphodiesterase (PDE) inhibitor such as the methylxanthanines including theophylline and aminophylline; and selective PDE isoenzyme inhibitors including PDE4 inhibitors and inhibitors of isoform PDE4D, and inhibitors of PDE5.

The present invention still further relates to combination therapies of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, or mizolastine, which is applied orally, topically or parenterally.

The present invention still further relates to combination therapies of a compound of the invention together with a gastroprotective histamine type 2 receptor antagonist.

The present invention still further relates to combination therapies of a compound of the invention with an antagonist of the histamine type 4 receptor.

The present invention still further relates to combination therapies of a compound of the invention together with an alpha-1/ alpha-2 adrenoceptor agonist, vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, or ethylnorepinephrine hydrochloride.

The present invention still further relates to combination therapies of a compound of the invention together with an anticholinergic agent including muscarinic receptor (M1, M2 and M3) antagonists such as atropine, hyoscine, glycopyrrolate, ipratropium bromide; tiotropium bromide; oxitropium bromide; pirenzepine; or telenzepine.

The present invention still further relates to combination therapies of a compound of the invention together with a beta-adrenoceptor agonist (including beta receptor subtypes 1-4) such as isoprenaline, salbutamol, formoterol, salmeterol, terbutaline, orciprenaline, bitolterol mesylate, or pirbuterol.

The present invention still further relates to combination therapies of a compound of the invention together with a chromone, such as sodium cromoglycate or nedocromil sodium.

The present invention still further relates to combination therapies of a compound of the invention together with an insulin-like growth factor type I (IGF-1) mimetic.

The present invention still further relates to combination therapies of a compound of the invention together with an inhaled glucocorticoid, such as flunisolide, triamcinolone acetonide, beclomethasone dipropionate, budesonide, fluticasone propionate, ciclesonide, or mometasone furoate.

The present invention still further relates to combination therapies of a compound of the invention together with an inhibitor of matrix metalloproteases, i.e., an inhibitor of stromelysin, collagenase, gelatinase, aggrecanase; especially collagenase-1 (MMF-1), collagenase-2 (MMP-8), collagenase-3 (MMP-13), stromelysin-1 (MMP-3), stromelysin-2 (MMP-10), stromelysin-3 (MMF-11), MMP-9 or MMP-12.

The present invention still further relates to combination therapies of a compound of the invention together with modulators of chemokine receptor function such as antagonists of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX3CR1 (for the C-X3-C family).

The present invention still further relates to combination therapies of a compound of the invention together with a cytokine or a modulator of cytokine function including agents which act on cytokine signalling pathways, such as alpha-, beta-, and gamma-interferon; interleukins (IL) including IL-1 to IL-15, and interleukin antagonists or inhibitors.

The present invention still further relates to combination therapies of a compound of the invention together with an immunoglobulin (Ig), an Ig preparation, or an antagonist or antibody modulating Ig function such as anti-IgE (omalizumab).

The present invention still further relates to combination therapies of a compound of the invention together with systemic or topically-applied anti-inflammatory agents such as thalidomide or its derivatives, retinoids, dithranol, or calcipotriol.

The present invention still further relates to combination therapies of a compound of the invention together with an antibacterial agent including penicillin derivatives, tetracyclines, macrolides, beta-lactams, flouroquinolones, metronidazole and inhaled aminoglycosides; antiviral agent including acyclovir, famciclovir, valaciclovir, ganciclovir, cidofovir, amantadine, rimantadine, ribavirin; zanamavir and oseltamavir; enzyme inhibitors such as indinavir, nelfinavir, ritonavir, and saquinavir; nucleoside reverse transcriptase inhibitors such as didanosine, lamivudine, stavudine, zalcitabine and zidovudine; or non-nucleoside reverse transcriptase inhibitors such as nevirapine or efavirenz.

The present invention still further relates to combination therapies of a compound of the invention together with agents used for treatment of cancers. Suitable agents to be used in the combination therapies include:
(i) antiproliferative/antineoplastic drugs and combinations thereof, which are used as an anticancer agent, such as alkylating agents (for example cisplatin, carboplatin, cyclophosphamide, nitrogen mustard, melphalan, chlorambucil, busulphan or nitrosoureas); antimetabolites (for example fluoropyrimidines, like 5-fluorouracil and tegafur, antifolates such as raltitrexed, methotrexate, cytosine arabinoside, hydroxyurea, gemcitabine or paclitaxel); antitumour antibiotics (for example anthracyclines, like adriamycin, bleomycin, doxorubicin, daunomycin, epirubicin, idarubicin, mitomycin-C, dactinomycin or mithramycin); antimitotic agents (for example vinca alkaloids, like vincristine, vinblastine, vindesine and vinorelbine and taxoids, such as taxol and taxotere); or topoisomerase inhibitors (for example epipodophyllotoxins, such as etoposide, teniposide, amsacrine, topotecan or camptothecins);
(ii) cytostatic agents such as antiestrogens (for example tamoxifen, toremifene, raloxifene, droloxifene or iodoxyfene), estrogen receptor down regulators (for example fulvestrant), antiandrogens (for example bicalutamide, flutamide, nilutamide and cyproterone acetate), LHRH antagonists or LHRH agonists (for example goserelin, leuprorelin or buserelin), progestogens (for example megestrol acetate), aromatase inhibitors (for example as anastrozole, letrozole, vorazole or exemestane) and inhibitors of 5α-reductase such as finasteride;
(iii) agents which inhibit cancer cell invasion (for example metalloproteinase inhibitors, such as marimastat or inhibitors of urokinase plasminogen activator receptor function);
(iv) inhibitors of growth factor function, for example such inhibitors include growth factor antibodies, growth factor receptor antibodies (for example the anti erbb2 antibody trastuzumab or the anti erbb1 antibody cetuximab [C225]), farnesyl transferase inhibitors, tyrosine kinase inhibitors or serine/threonine kinase inhibitors; for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI 774) or 6-acrylamido-N-(3-chloro-4-fluorophenyl)-7-(3-morpholinopropoxy)quinazolin-4-amine (CI 1033)); for example inhibitors of the platelet-derived growth factor family; or for example inhibitors of the hepatocyte growth factor family;
(v) antiangiogenic agents such as those which inhibit the effects of vascular endothelial growth factor, (for example the anti vascular endothelial cell growth factor antibody bevacizumab, compounds disclosed in WO 97/22596, WO 97/30035, WO 97/32856 or WO 98/ 13354), or compounds that work by other mechanisms (for example linomide, inhibitors of integrin αvβ3 function or angiostatin);
(vi) vascular damaging agents such as combretastatin A4 or compounds disclosed in WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene directed enzyme pro-drug therapy) approaches such as those using cytosine deaminase, thymidine kinase or a bacterial nitroreductase enzyme and approaches to increase patient tolerance to chemotherapy or radiotherapy such as multi drug resistance gene therapy; or
(ix) immunotherapy approaches, including for example ex vivo and in vivo approaches to increase the immunogenicity of patient tumour cells, such as exposure of cytokines such as interleukin 2, interleukin 4 or granulocyte macrophage colony stimulating factor (GM-CSF), approaches to decrease T-cell anergy, approaches using transplanted immune cells such as cytokine exposed dendritic cells, approaches using cytokine exposed tumour cell lines and approaches using anti idiotypic antibodies.

### Example

The following compounds were prepared in accordance with the method described in the present specification. The abbreviations used in the present specification are as follows:
EtOAc: Ethyl acetate
DCM: Dichloromethane
NBS: N-Bromosuccinimide
DMF: N,N-Dimethylformamide
DMSO: dimethyl sulfoxide
THF: tetrahydrofuran
TFA: trifluoroacetic acid
MS: Mass spectrometry
APCI: Atmospheric Chemical Ionization Method
HCl: Hydrochloric acid

In the present specification, in case of reverse-phase HPLC, "Waters Symmetry C8, Xterra or Phenomenex Gemini columns" was used, and the solvent such as acetonitrile and buffer (aqueous ammonium acetate, aqueous ammonia, aqueous formic acid or aqueous trifluoroacetic acid were used as an eluent. Silica gel was used as column chromatography. SCX means solid phase extraction using sulfonic acid absorbent and a mixture was absorbed in sulfonic acid absorbent and eluted into a solvent such as, methanol, acetonitrile, etc. and then, free basic substance is eluted into a solvent such as aqueous ammonia /methanol·acetonitrile, etc.

The present invention is concretely explained by the following examples, but should not be limited by the examples.

### Example 1

2-Butoxy-7,8-dihydro-9-{[2-(3-{N-[(3-methoxycarbonylmethylphenyl-1-yl)methyl]-N-methylamino}propyl)piperidin-4-yl]methyl}-8-oxoadenine

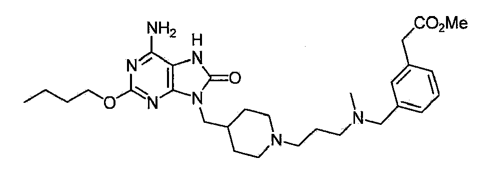

### Step (i)

### 4-(Methanesulufonyloxymethyl)piperidine-1-carboxylic acid tert-butyl ester

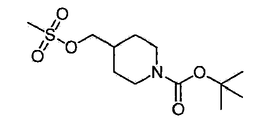

To 4-hydroxylmethylpiperidine-1-carboxylic acid tert-butyl ester) 15.4g (71.5mmol) in THF (300ml) were added triethylamine 25ml (179mmol), 4-dimethylaminopyridine 181mg (1.48mmol) and methanesulfonyl chloride 6.5m1 (84.0mmol) at 0°C, and then the mixture was stirred at room temperature for 30 minutes. Thereto was added saturated brine 400ml and the mixture was extracted with ethyl acetate (800ml). The organic layer was dried over anhydrous magnesium sulfate and concentrated in vacuo. To the residue was added chloroform-hexane and the resulting solid was filtered to give the subtitled compound 19.4g as a white solid. Yield 92%
¹H NMR (CDCl₃) δ 4.15 (2H, brd J= 13.4 Hz), 4.07 (2H, d J= 6.5 Hz), 3.02 (3H, s), 2.71 (2H, brt, J= 13.4 Hz), 1.96-1.84 (1H, m), 1.74 (2H, brd J= 13.4 Hz), 1.46 (9H, s), 1.27-1.16 (2H, m).

### Step (ii)

### 2-Butoxy-9-{[2-(1-tert-butoxycarbonyloxy)piperidin-4-yl]methyl}-8-methoxyadenine

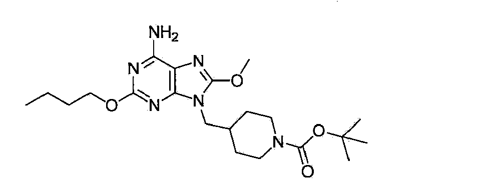

To 2-butoxy-8-methoxyadenine 3.03g (9.08mmol) in DMF (90ml)were added potassium carbonate 3.19g (23.0mmol) and 4-(methanesulufonyloxymethyl)-piperidine-1-carboxylic acid tert-butyl ester 3.19g (10.9mmol), and the mixture was stirred at 60°C for 10 hours, at 80°C for 2.5 hours and then at 100°C for 2 hours. After removal of the solvent by distillation, thereto was added saturated brine 50ml and the mixture was extracted three times with chloroform (50ml). The organic layer was dried over anhydrous magnesium sulfate and concentrated in vacuo. To the residue was added diethyl ether-hexane and the resulting solid was filtered. The residue was repulp-purified with diethyl ether-hexane (1:2) to give the subtitled compound as a white solid 2.74g. Yield 70%
¹H NMR (CDCl₃) δ 5.79 (2H, bs), 4.30 (2H, t, J= 6.7 Hz), 4.12 (3H, s), 3.81 (2H, d, J= 7.3 Hz), 2.70-2.60 (2H, m), 2.06-1.70 (4H, m), 1.58-1.40 (5H, m), 1.45 (9H, s), 1.28-1.14 (2H, m), 0.97 (3H, t, J= 7.4 Hz).

### Step (iii)

### 2-Butoxy-9-[(2-{3-[(N-tert-butoxycarbonyloxy)-N-methylamino]propyl}piperidin-4-yl)methyl]-8-methoxyadenine

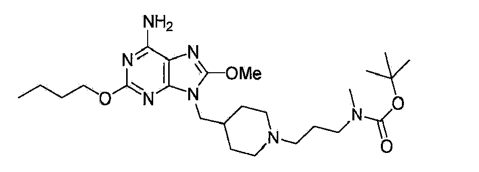

To the compound 305 mg (0.70mmol) obtained in step (ii) was added trifluoroacetic acid 10ml and the mixture was stirred at room temperature for 30 minutes. After removal of the solvent by distillation, to the residue was added DMF 8ml. Thereto were added tert-butyl 3-chloropropylmethylcarboxylate 298mg (1.43mmol), potassium carbonate 488mg (3.53mmol) and potassium iodide 119mg (0.715mmol) at room temperature, and the mixture was stirred at room temperature for 18 hours, at 60°C for 4 hours and then at 80°C for 5.5 hours. After removal of the solvent by distillation, to the residue was added water 20ml and the mixture was extracted with chloroform (60ml). The organic layer was dried over anhydrous magnesium sulfate, concentrated in vacuo and purified by silica gel column chromatography to give the subtitled compound 217mg as a colorless oil. Yield 61% ¹H NMR (CDCl₃) δ 5.14 (2H, bs), 4.27 (2H, t, J= 6.7 Hz), 4.11 (3H, s), 3.81 (2H, d, J= 7.3 Hz), 3.24-3.18 (2H, m), 2.89-2.84 (2H, m), 2.83 (3H, s), 2.32-2.27 (2H, m), 1.93-1.66 (10H, m), 1.60-1.28 (6H, m), 1.44 (9H, s), 0.96 (3H, t, J= 7.4 Hz).

### Step (iv)

### 2-Butoxy-9-{[2-(3-{N-[(3-methoxycarbonylmethylphenyl-1-yl)methyl]-N-methylamino}propyl)piperidin-4-yl]methyl}-8-methoxyadenine

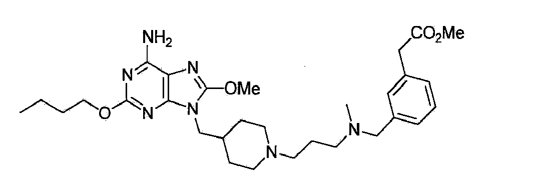

To the compound 181mg (0.357mmol) obtained in step (iii) was added trifluoroacetic acid 5ml and the mixture was stirred at room temperature for 35 minutes. After removal of the solvent by distillation, to the residue was added aqueous saturated sodium bicarbonate 20ml and the mixture was extracted with 33% ethanol-chloroform (50ml x 3). The organic layer was dried over anhydrous magnesium sulfate and concentrated in vacuo. To the resulting crude product in methanol (5ml) were added sodium cyanoborohydride 96.7mg (1.46mmol) and methyl 3-formyl-phenyl-acetate 77.1mg (0.432mmol) at room temperature, and the mixture was stirred at room temperature for 4 days. After removal of the solvent by distillation, to the residue was added saturated brine 5ml and the mixture was extracted with 33% ethanol-chloroform (10ml x 3). The organic layer was dried over anhydrous magnesium sulfate, concentrated in vacuo and purified by silica gel column chromatography to give the subtitled compound 138mg as a colorless oil. Yield 68% ¹H NMR (CDCl₃) δ7.30-7.14 (4H, m), 5.22 (2H, bs), 4.27 (2H, t, J= 6.7 Hz), 4.10 (3H, s), 3.81 (2H, d, J= 7.3 Hz), 3.69 (3H, s), 3.61 (3H, s), 3.45 (2H,s), 2.93-2.86 (2H, m), 2.39-2.30 (4H, m), 2.17 (3H, s), 1.93-1.68 (10H, m), 1.60-1.30 (6H, m), 0.96 (3H, t, J= 7.4 Hz).

### Step (v)

### 2-Butoxy-7,8-dihydro-9-{[2-(3-{N-[(3-methoxycarbonylmethylphenyl-1-yl)methyl]-N-methylamino}propyl)piperidin-4-yl]methyl}-8-oxoadenine

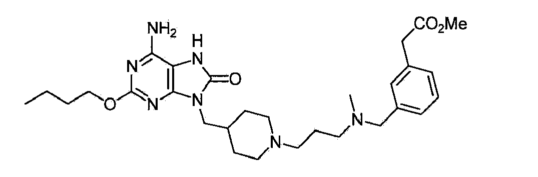

To the compound 135mg (0.238mmol) obtained in step (iv) in methanol (10ml) was added concentrated sulfuric acid (200µl) and the mixture was refluxed for 3 hours. After neutralizing with aqueous saturated sodium bicarbonate, the resulting solid was filtered to give the titled compound 107mg as a white solid. Yield 81%
¹H NMR (DMSO-d₆) δ 7.35-7.10 (4H, m), 6.64 (2H, bs), 4.13 (2H, t, J= 6.6 Hz), 3.65 (2H, s), 3.60 (3H, s), 3.53 (2H, d, J= 7.1 Hz), 3.40 (2H, s), 2.79-2.75 (2H, m), 2.30 (2H, t, J= 6.9 Hz), 2.22 (2H, t, J= 7.4 Hz), 2.08 (3H, s), 1.80-1.70 (3H, m), 1.68-1.52 (4H, m), 1.50-1.33 (4H, m), 1.20-1.10 (2H, m), 0.92 (3H, t, J= 7.3 Hz).

### Example 2

### 2-Butoxy-7,8-dihydro-9-{[2-(3-{N-[(3-methoxycarbonylmethylphenyl-1-yl)methyl]amino}propyl)piperidin-4-yl]methyl}-8-oxoadenine

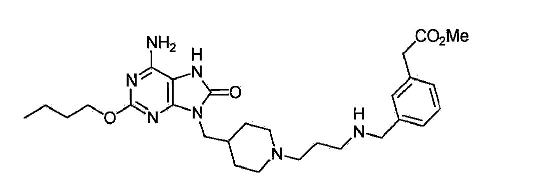

### Step (i)

### 2-Butoxy-9-({2-[3-(N-phthaloyl)propyl]piperidin-4-yl}methyl)-8-oxoadenine

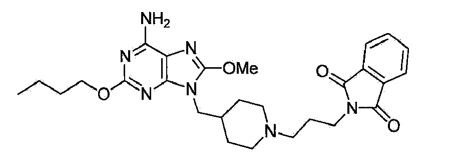

To the compound 296mg (0.682mmol) obtained by example 1 step (i) was added trifluoroacetic acid 8ml and the mixture was stirred for 40 minutes. After removal of the solvent by distillation, to the residue was added DMF 8ml. Thereto were added 3-bromopropylphthalimide 311mg (1.16mmol), potassium carbonate 474mg (3.43mmol) and potassium iodide 113mg (0.683mmol) at room temperature, and the mixture was stirred at 80°C for 5.5 hours. After removal of the solvent by distillation, to the residue was added water 20ml and the mixture was extracted with chloroform (60ml). The organic layer was dried over anhydrous magnesium sulfate, concentrated in vacuo and purified by silica gel column chromatography to give the subtitled compound 327mg as a colorless oil. Yield 92%
¹H NMR (CDCl₃) δ 7.85-7.82 (2H, m), 7.75-7.71 (2H, m), 5.13 (2H, bs), 4.26 (2H, t, J= 6.7 Hz), 4.09 (3H, s), 3.80-3.73 (4H, m), 1.79-1.58 (10H, m), 1.53-1.43 (3H, m), 0.96 (3H, t, J= 7.3 Hz).

### Step (ii)

### 2-Butoxy-9-{[2-(3-{N-[(3-methoxycarbonylmethylphenyl-1 - yl)methyl]amino}propyl)piperidin-4-yl]methyl}-8-methoxyadenine

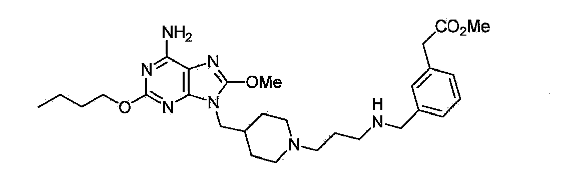

To the compound 292mg (0.559mmol) obtained in step (i) in ethanol (10ml) was added hydrazine monohydrate (1ml) and the mixture was refluxed for 30 minutes. After being cooled to room temperature, the resulting solid was filtered off. After removal of the solvent by distillation, to the residue, was added aqueous saturated sodium bicarbonate 30ml and the mixture was extracted with 33% ethanol-chloroform (150ml). The organic layer was dried over anhydrous magnesium sulfate and concentrated in vacuo. To the obtained crude product in methanol (6ml) were added sodium cyanoborohydride 178mg (2.69mmol) and methyl (3-formylphenyl)acetate 105mg (0.588mol) at room temperature, and the mixture was stirred at room temperature for 45 hours. After removal of the solvent by distillation, to the residue was added saturated brine 6ml and the mixture was extracted with 33% ethanol-chloroform (45ml). The organic layer was dried over anhydrous magnesium sulfate, concentrated in vacuo and purified by silica gel column chromatography to give the subtitled compound 97.7mg as a colorless oil. Yield 33% ¹H NMR (CDCl₃) δ 7.36-7.18 (4H, m), 5.14 (2H, brs), 4.26 (2H, t, J = 6.7 Hz), 4.10 (3H, s), 3.87 (2H, brs), 3.76 (2H, d, J= 7.3 Hz), 3.69 (3H, s), 3.64 (2H, s), 3.00-2.94 (2H, m), 2.84-2.78 (2H, m), 2.47-2.41 (2H, m), 1.93-1.70 (7H, m), 1.60-1.42 (4H, m), 1.34-1.23 (2H, m), 0.96 (3H, t, J = 7.4 Hz).

### Step (iii)

### 2-Butoxy-7,8-dihydro-9-{[2-(3-{N-[(3-methoxycarbonylmethylphenyl-1-yl)methyl]amino}propyl)piperidin-4-yl]methyl}-8-oxoadenine

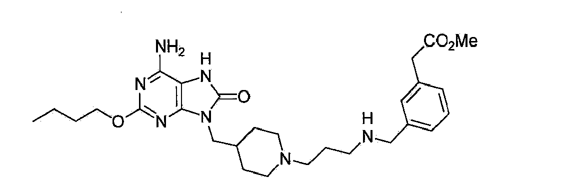

To the compound 97.2 mg (0. 176mmol) obtained in step (ii) in methanol (10ml) was added concentrated sulfuric acid (200µl) and the mixture was refluxed for 2.5 hours. After neutralizing with aqueous saturated sodium bicarbonate, the solvent was removed by distillation. To the residue was added water 20ml and the mixture was extracted with 33% ethanol-chloroform (200ml). The organic layer was dried over anhydrous magnesium sulfate and concentrated in vacuo. To the residue was added chloroform-hexane and the resulting solid was filtered to give the titled compound 62.1mg as a white solid. Yield 66%
¹H NMR (DMSO-d₆) δ 9.85 (1H, bs), 7.34-7.14 (4H, m), 6.45 (2H, brs), 4.14 (2H, t, J= 6.6 Hz), 3.81 (1H, brs), 3.68 (2H, s), 3.61 (3H, s), 3.53 (2H, d, J= 7.2 Hz), 3.42 (2H, s), 2.89-2.80 (2H, m), 2.70-2.60 (2H, m), 2.36-2.28 (2H, m), 1.89-1.72 (3H, m), 1.68-1.58 (4H, m), 1.42-1.32 (2H, m), 1.20-1.11 (2H, m), 0.92 (3H, t, J= 7.3 Hz).

### Example 3

### 7,8-Dihydro-9-(1-{2-[3-(methoxycarbonylmethyl)phenoxy]ethyl}piperidin-4-ylmethyl)-2-(2-methoxyethoxy)-8-oxoadenine

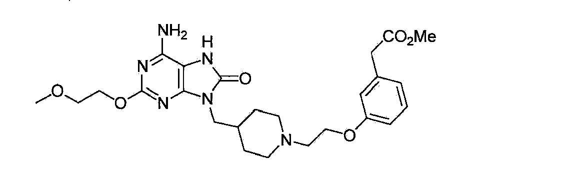

### Step (i)

### 9-[1-(tert-Butoxycarbonyl)piperidin-4-ylmethyl]-2-(2-methoxyethoxy)-8-methoxyadenine

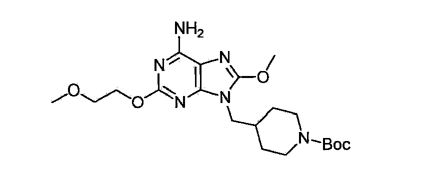

To 2-{(2-Methoxyethoxy)}-8-methoxyadenine 10.0g (41.8mmol) in DMF (350ml) were added potassium carbonate 7.52g (54.4mmol) and 4-(methanesulufonyloxymethyl)-piperidine-1-carboxylic acid tert-butyl ester 14.7g (50.2mmol), and the mixture was stirred at 80°C for 11 hours. After removal of the solvent by distillation, to the residue was added saturated brine 300ml and the mixture was extracted with chloroform (750ml). The organic layer was dried over anhydrous magnesium sulfate and concentrated in vacuo. The solid residue was repulp-purified with diethyl ether to give the subtitled compound 12.5 g as a white solid. Yield 69% ¹H NMR (CDCl₃) δ 5.24 (2H, bs), 4.44 (2H, t, J= 5.0 Hz), 4.11 (3H, s), 3.81 (2H, d, J= 7.3 Hz), 3.76 (2H, t, J= 5.0 Hz), 3.41 (3H, s), 2.70-2.58 (2H, m), 2.07-1.95 (1H, m), 1.84-1.72 (2H, m), 1.56-1.48 (2H, m), 1.45 (9H, s), 1.25-1.12 (2H, m).

### Step (ii)

### 8-Methoxy-9-(1-(2-[3-(methoxycarbonylmethyl)phenoxy]ethyl)piperidin-4-ylmethyl)-2-(2-methoxyethoxy)adenine

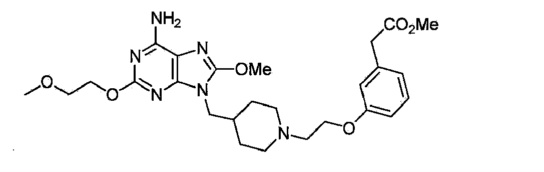

To the compound 356mg (0.816mmol) obtained in step (i) was added trifluoroacetic acid 5ml, and the mixture was stirred at room temperature for 35 minutes. After removal of the solvent by distillation, to the residue was added DMF 8ml. Thereto were added 3-(2-bromoethoxy)phenyl]acetic acid methyl ester 356mg (1.30mmol) and potassium carbonate 702 mg (5.08mmol) at room temperature, and the mixture was stirred at 60°C for 2 hours. After removal of the solvent by distillation, to the residue was added saturated brine 20ml, and the mixture was extracted with chloroform (60ml). The organic layer was dried over anhydrous magnesium sulfate, concentrated in vacuo and purified by silica gel column chromatography to give the subtitled compound 340mg as colorless oil. Yield 79%
¹H NMR (CDCl₃) δ 7.22-7.21 (1H, m), 6.91-6.88 (1H, m), 6.83-6.77 (2H, m), 5.17 (2H, brs), 4.43 (4H, t, J= 5.0 Hz), 4.10 (2H, s), 3.87 (2H, d, J= 6.3 Hz), 3.75 (2H, t J= 5.0 Hz), 3.69 (3H, s), 3.59 (2H, s), 3.47 (3H, s), 2.20-2.05 (2H, m), 1.80-1.50 (7H, m).

### Step (iii)

### 7, 8-Dihydro-9-(1-{2-[3-(methoxycarbonylmethyl)phenoxy]ethyl}piperidin-4-ylmethyl)-2-(2-methoxyethoxy)-8-oxoadenine

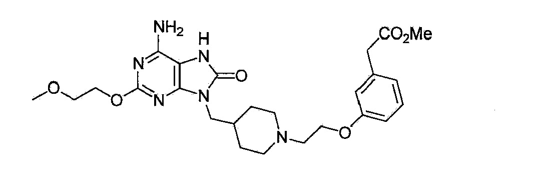

To the compound 314mg (0.593mmol) obtained in step (ii) in methanol (10ml) was added concentrated sulfuric acid (200µl) and the mixture was refluxed for 3 hours. The mixture was neutralized with aqueous saturated sodium bicarbonate and the resulting solid was filtered to give the titled compound 313mg as a white solid. Yield 100%
¹H NMR (DMSO-d₆) δ 7.23-7.18 (1H, m), 6.84-6.76 (3H, m), 4.25 (2H, t, J= 4.6 Hz), 4.02 (2H, t J= 5.9 Hz), 3.67-3.59 (2H, m), 3.63 (2H, s), 3.61 (3H, s), 3.54 (2H, d, J= 7.2 Hz), 3.29 (3H, s), 2.91-2.87 (2H, m), 2.64 (2H, t, J= 5.9 Hz), 1.99-1.93 (2H, m), 1.86-1.70 (1H, m), 1.56-1.46 (2H, m), 1.28-1.14 (2H, m).

### Example 4

### 7,8-Dihydro-9-{1-[3-(N-(2-[3-(methoxycarbonylmethyl)phenoxy]ethyl)-N-methylamino)propyl]piperidin-4-ylmethyl}-2-(2-methoxyethoxy)-8-oxoadenine

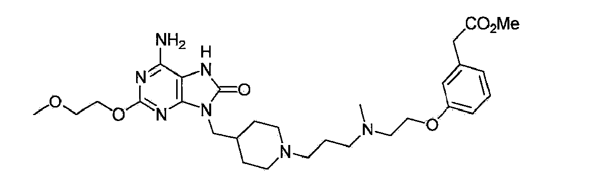

### Step (i)

### 9-(1-{3-[N-(tert-Butoxycarbonyl)-N-methylamino]propyl}piperidin-4-ylmethyl)-2-(2-methoxyethoxy)-8-methoxyadenine

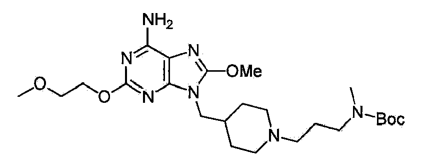

To the compound 1.01g (2.30mmol) obtained by example 3 step (i) was added trifluoroacetic acid 20ml and the mixture was stirred at room temperature for 55 minutes. After removal of the solvent by distillation, to the residue was added DMF 25ml. Thereto were added N-tert-butoxycarbonyl-N-(3-chloropropyl)-N-methylamine 964mg (4.64mmol) and potassium carbonate 2.93g (21.2mmol) at room temperature, and the mixture was stirred at 80°C for 9 hours. After removal of the solvent by distillation, to the residue was added saturated brine 50ml, and the mixture was extracted with 25% ethanol-chloroform (150ml). The organic layer was dried over anhydrous magnesium sulfate and concentrated in vacuo and purified by silica gel column chromatography to give the subtitled compound 983mg as a pale yellow oil. Yield 84%
¹H NMR (CDCl₃) δ 5.40 (2H, bs), 4.43 (2H, t, J= 5.0 Hz), 4.10 (3H, s), 3.81 (2H, d, J= 7.3 Hz), 3.75 (2H, t, J= 5.0 Hz), 3.43 (3H, s), 3.26-3.18 (2H, m), 2.95-2.86 (2H, m), 2.84 (3H, s), 2.34-2.26 (2H, m), 1.94-1.82 (3H, m), 1.75-1.66 (2H, m), 1.61-1.53 (2H, m), 1.44 (9H, s), 1.46-1.33 (2H, m).

### Step (ii)

### 8-Methoxy-9-{1-[3-(N-{2-[3-(methoxycarbonylmethyl)phenoxy]ethyl}-N-methylamino)propyl]piperidin-4-ylmethyl}-2-(2-methoxyethoxy)adenine

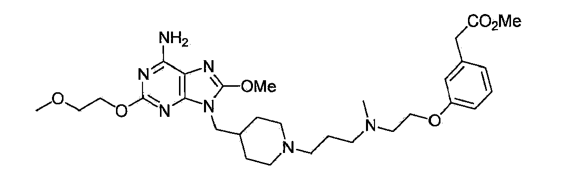

To the compound 295g (0.581mmol) obtained in step (i) was added trifluoroacetic acid 5ml and the mixture was stirred at room temperature for 40 minutes. After removal of the solvent by distillation, to the residue was added DMF 8ml and thereto were added methyl [3-(2-bromoethoxy)phenyl]acetate 225mg (0.825mmol) and potassium carbonate 640g (4.63mmol) at room temperature, and the mixture was stirred at 60°C for 4 hours. After removal of the solvent by distillation, thereto was added saturated brine 30ml and the mixture was extracted with 33% ethanol-chloroform (150ml). The organic layer was dried over anhydrous magnesium sulfate, concentrated in vacuo and purified by silica gel column chromatography to give the subtitled compound 246mg as a colorless oil. Yield 71%
¹H NMR (CDCl₃) δ 7.22-7.20 (1H, m), 6.85-6.78 (3H, m), 5.19 (2H, brs), 4.43 (2H, t, J= 4.9 Hz), 4.10 (3H, s), 4.07 (2H, t, J= 6.4 Hz), 3.83 (2H, d J= 7.3 Hz), 3.75 (2H, t, J= 4.9 Hz), 3.68 (3H, s), 3.58 (2H, s), 3.42 (3H, s), 2.80 (2H, t, J= 5.6 Hz), 2.58-2.46 (2H, m), 2.17 (3H, m), 2.24-1.56 (11H, m).

### Step (iii)

### 7,8-Dihydro-9-{1-[3-(N-{2-[3-(methoxycarbonylmethyl)phenoxy]ethyl}-N-methylamino)propyl]piperidin-4-ylmethyl}-2-(2-methoxyethoxy)-8-oxoadenine

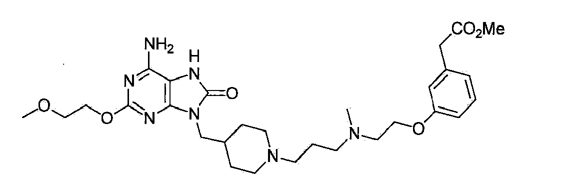

To the compound 244 mg (0.407mmol) obtained in step (ii) in methanol (7.5ml) was added concentrated sulfuric acid (150µl) and the mixture was refluxed for 3.5 hours. After neutralized with aqueous saturated sodium bicarbonate, the solvent was removed by distillation. To the residue was added saturated brine 10ml and the mixture was extracted with 33% ethanol-chloroform (60ml). The organic layer was dried over anhydrous magnesium sulfate and concentrated in vacuo. To the residue was added chloroform-hexane and the resulting solid was filtered to give the titled compound 152mg as a white solid. Yield 64%
¹H NMR (DMSO-d₆) δ 9.87 (1H, brs), 7.23-7.19 (1H, m), 6.83-6.80 (3H, m), 6.43 (2H, brs), 4.26 (2H, t, J= 4.6 Hz), 4.01 (2H, t, J= 5.8 Hz), 3.70-3.58 (2H, m), 3.63 (2H, s), 3.60 (3H, s), 3.54 (2H, d, J= 7.2 Hz), 3.27 (3H, s), 2.83-2.74 (2H, m), 2.68 (2H, t, J= 5.8 Hz), 2.38 (2H, t, J= 5.8 Hz), 2.30-2.16 (2H, m), 2.22 (3H, s), 1.84-1.70 (3H, m), 1.58-1.44 (4H, m), 1.27-1.10 (3H, m).

### Example 5

### 7,8-Dihydro-9-[1-(3-{N-[3-(methoxylcarbonylmethyl)benzyl]-N-methylamino}propyl)piperidin-4-ylmethyl]-2-(2-methoxyethoxy)-8-oxoadenine

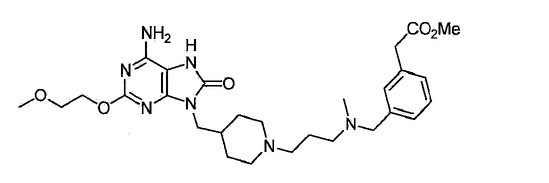

### Step (i)

### 8-Methoxy-9-[1-(3-{N-[3-(methoxylcarbonylmethyl)benzyl]-N-methylamino}propyl)piperidin-4-ylmethyl]-2-(2-rnethoxyethoxy)adenine

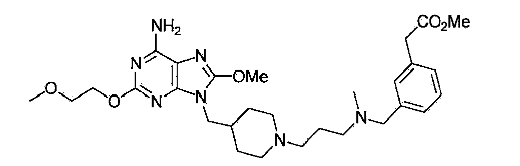

To the compound 423g (0.836mmol) obtained by example 4 step (i) was added trifluoroacetic acid 15ml and the mixture was stirred at room temperature for 45 minutes. To the obtained crude substance in methanol (10ml) were added sodium cyanoborohydride 280mg (4.24mmol) and methyl 3-(formylphenyl)-acetate 228mg (1.28mmol) at room temperature, and the mixture was stirred at room temperature for 21 hours. After removal of the solvent by distillation, thereto was added saturated brine 50ml and the mixture was extracted with 33% ethanol-chloroform (150ml). The organic layer was dried over anhydrous magnesium sulfate, concentrated in vacuo and purified by silica gel column chromatography to give the subtitled compound 316mg as a colorless oil. Yield 67%
¹H NMR (CDCl₃) δ 7.32-7.14 (4H, m), 5.19 (2H, brs), 4.43 (2H, t, J= 4.9 Hz), 4.10 (3H, s), 3.84 (2H, d, J= 7.2 Hz), 3.75 (2H, t, J= 4.9 Hz), 3.68 (3H, s), 3.63 (2H, s), 3.38 (3H, s), 2.44-2.40 (2H, m), 2.24 (3H, s), 2.30-1.80 (11H, m), 1.70-1.60 (2H, m).

### Step (ii)

### 7,8-Dihydro-9-{1-[3-(N-{2-[3-(methoxycarbonylmethyl)phenoxy] ethyl}-N-methylamino)propyl]piperidin-4-ylmethyl}-2-(2-methoxyethoxy)-8-oxoadenine

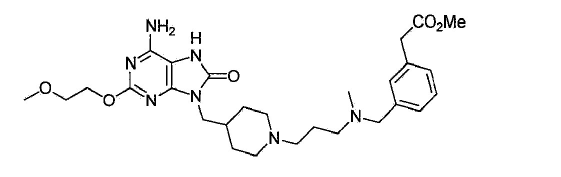

To the compound 312mg (0.548mmol) obtained in step (i) in methanol (10ml) was added concentrated sulfuric acid (200µl) and the mixture was refluxed for 7 hours. After neutralized with aqueous saturated sodium bicarbonate, the solvent was removed by distillation. To the residue was added aqueous saturated sodium bicarbonate 15ml and the mixture was extracted with 33% ethanol-chloroform (75ml). The organic layer was dried over anhydrous magnesium sulfate and concentrated in vacuo. To the residue was added chloroform-hexane and the resulting solid was filtered to give the titled compound 251mg as a white solid. Yield 82%
¹H NMR (DMSO-d₆) δ 9.88 (1H, brs), 7.28-7.25 (1H, m), 7.23-7.06 (3H, m), 6.49 (2H, brs), 4.26 (2H, t, J= 4.7 Hz), 3.65 (2H, s), 3.60 (3H, s), 3.54 (2H, d, J= 7.1 Hz), 3.39 (2H, s), 3.37 (2H, s), 3.29 (3H, s), 2.80-2.72 (2H, m), 2.32-2.20 (4H, m), 2.09 (3H, s), 1.82-1.68 (3H, m), 1.60-1.42 (4H, m), 1.24-1.10 (3H, m).

### Example 6

### 2-Butoxy-7,8-dihydro-9-(1-{2-[3-(methoxycarbonylmethyl)phenoxy]ethyl}piperidin-4-ylmethyl)-8-oxoadenine

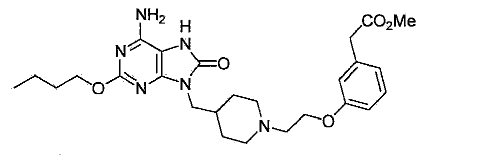

### Step (i)

### 2-Butoxy-8-methoxy-9-(1-{2-[3-(methoxycarbonylmethyl)phenoxy]ethyl}piperidin-4-ylmethyl)adenine

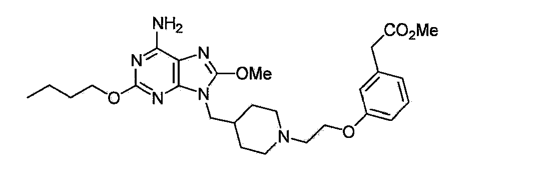

To the compound 260mg (0.5976mmol) obtained by example 1 step (i) was added trifluoroacetic acid 5ml and the mixture was stirred at room temperature for 40 minutes. After removal of the solvent by distillation, to the residue was added DMF 8ml. Thereto were added methyl [3-(2-bromoethoxy)-phenyl]-acetate 249mg (0.911mmol) and potassium carbonate 661mg (4.78mmol) at room temperature, and the mixture was stirred at 60°C for 3 hours. After removal of the solvent by distillation, thereto added saturated brine 15ml and the mixture was extracted with chloroform (90ml). The organic layer was dried over anhydrous magnesium sulfate, concentrated in vacuo and purified by silica gel column chromatography to give the subtitled compound 259mg as a colorless oil. Yield 82%
¹H NMR (CDCl₃) δ 7.25-7.20 (1H, m), 6.88-6.79 (3H, m), 5.19 (2H, brs), 4.27 (2H, t, J= 6.7 Hz), 4.21-4.12 (2H, m), 4.11 (3H, s), 3.90 (2H, t, J= 7.2 Hz), 3.69 (3H, s), 3.59 (2H, s), 3.14-3.C14 (2H, m), 2.90-2.83 (2H, m), 2.28-2.15 (2H, m), 2.00-1.66 (8H, m), 1.54-1.44 (2H, m), 1.42-1.22 (5H, m), 0.96 (3H, t, J= 7.4 Hz).

### Step (ii)

### 2-Butoxy-9-(1-{2-[3-(methoxycarbonylmethyl)phenoxy]ethyl}piperidin-4-ylmethyl)-8-oxoadenine

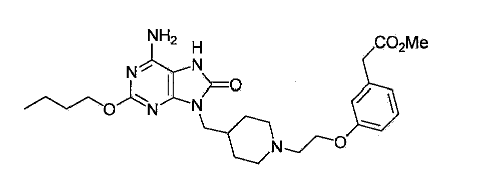

To the compound 259mg (0.491mmol) obtained in step (i) in methanol (10ml) was added concentrated sulfuric acid (200µl) and the mixture was refluxed for 3 hours. After neutralized with aqueous saturated sodium bicarbonate, the resulting solid was filtered to give the titled compound 199mg as a white solid. Yield 79%
¹H NMR (DMSO-d₆) δ 9.87 (1H, brs), 7.23-7.19 (1H, m), 6.83-6.80 (3H, m), 6.41 (2H, brs), 4.14 (2H, t, J= 6.8 Hz), 4.02 (2H, t, J= 5.8 Hz), 3.61 (2H, s), 3.56 (3H, s), 3.55 (2H, d, J= 6.6 Hz), 3.34 (3H, s), 2.94-2.86 (2H, m), 2.64 (2H, t, J= 5.8 Hz), 1.96 (2H, brt, J= 7.0 Hz), 1.85-1.72 (1H, m), 1.68-1.58 (2H, m), 1.54-1.46 (2H, m), 1.41-1.30 (2H, m), 1.26-1.12 (2H, m), 0.92 (3H, t, J= 7.3 Hz).

### Example 7

### 2-Butoxy-9-{1-[3-(N-{2-[3-(methoxycarbonylmethyl)phenoxy]ethyl}-N-methylamino)propyl]piperidin-4-ylmethyl}-8-oxoadenine

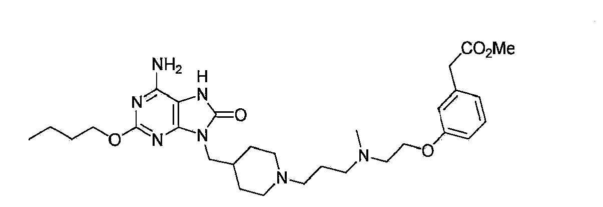

### Step (i)

### 2-Butoxy-8-methoxy-9-{1-[3-(N-{2-[3-(methoxycarbonylmethyl)phenoxy]ethyl}-N-methylamino)propyl]piperidin-4-ylmethyl}adenine

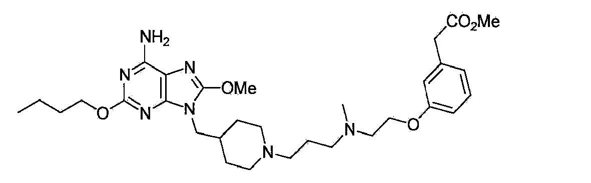

To the compound 239mg (0.473mmol) obtained by example 1, step (ii) was added trifluoroacetic acid 5ml and the mixture was stirred at room temperature for 35 minutes. After removal of the solvent by distillation, to the residue was added DMF 5ml. Thereto were added methyl [3-(2-bromoethoxy)-phenyl]-acetate 196mg (0.718mmol) and potassium carbonate 526mg (3.81mmol) at room temperature, and the mixture was stirred at 60°C for 5 hours and at 80°C for 1.5 hours. After removal of the solvent by distillation, thereto was added saturated brine 20ml and the mixture was extracted with 33% ethanol-chloroform (90ml). The organic layer was dried over anhydrous magnesium sulfate, concentrated in vacuo and purified by silica gel column chromatography to give the subtitled compound 234mg as a colorless oil. Yield 83%
¹H NMR (CDCl₃) δ 7.27-7.20 (1H, m), 6.85-6.78 (3H, m), 5.19 (2H, brs), 4.26 (2H, t, J= 6.6 Hz), 4.09 (3H, s), 4.07 (2H, t, J= 5.6 Hz), 3.84 (2H, d, J= 7.3 Hz), 3.68 (3H, s), 3.58 (2H, s), 2.80 (2H, t, J= 5.6 Hz), 2.58-2.48 (2H, m), 2.35 (3H, s), 2.24-1.60 (15H, m), 1.54-1.43 (2H, m), 0.96 (3H, t, J= 7.4 Hz).

### Step (ii)

### 2-Butoxy-7,8-dihydro-9-{1[3-(N-{2-[3-(methoxycarbonylmethyl)phenoxy]ethyl}-N-methylamino)propyl]piperidin-4-ylmethyl}-8-oxoadenine

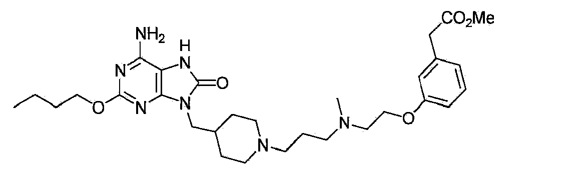

To the compound 230mg (0.385mmol) obtained in step (i) in methanol (10ml) was added concentrated sulfuric acid (200µl) and the mixture was refluxed for 6.5 hours. After neutralized with aqueous saturated sodium bicarbonate, the resulting solid was filtered to give the titled compound 113mg as a white solid. Yield 50%
¹H NMR (DMSO-d₆) δ 9.85 (1H, brs), 7.26-7.19 (1H, m), 6.88-6.63 (3H, m), 6.41 (2H, brs), 4.14 (2H, t, J= 6.6 Hz), 4.01 (2H, t, J= 5.8 Hz), 3.63 (2H, s), 3.60 (2H, s), 3.53 (2H, d, J= 7.2 Hz), 2.84-2.76 (2H, m), 2.67 (2H, t, J= 5.8 Hz), 2.50 (2H, t, J= 5.8 Hz), 2.28-2.15 (2H, m), 2.21 (3H, s), 1.81-1.70 (3H, m), 1.68-1.57 (2H, m), 1.56-1.30 (6H, m), 1.22-1.10 (2H, m), 0.92 (3H, t, J= 7.3 Hz).

### Example 8

### 7, 8-Dihydro-9-(1-{2-[3-(methoxycarbonylmethyl)phenoxy]ethyl}piperidin-4-ylethyl)-2-(2-methoxyethoxy)-8-oxoadenine

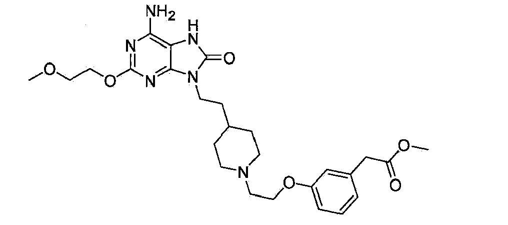

### Step (i)

### 9-(1-tert-Butoxycarbonylpiperidin-4-ylethyl)-8-methoxy-2-(2-methoxyethoxy)adenine

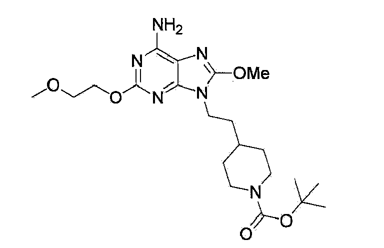

Using 8-methoxy-2-(2-methoxyethoxy)adenine 3.27g (13.7mmol) and 4-[2-(methanesulufonyloxy)ethyl]piperidine-1-carboxylic acid tert-butyl ester 4.21g (13.7mmol), in the same manner as example 3 step (i), there was obtained the subtitled compound 3.62g as a white solid. Yield 59%
¹H NMR (CDCl₃) δ 5.44 (2H, brs), 4.44 (2H, t, J = 5.0 Hz), 4.12 (3H, s), 3.96 (2H, t, J = 5.4 Hz), 3.75 (2H, t, J = 5.0 Hz), 3.43 (3H, s), 2.67-2.06 (2H, m), 1.83-1.65 (6H, m), 1.45 (9H, s), 1.40-1.33 (1H, m), 1.18-1.11 (2H, m).

### Step (ii)

### 8-Mehoxy-9-(1-{2-[3-(methoxycarbonylmethyl)phenoxy]ethyl}piperidin-4-ylethyl)-2-(2-methoxyethoxy)adenine

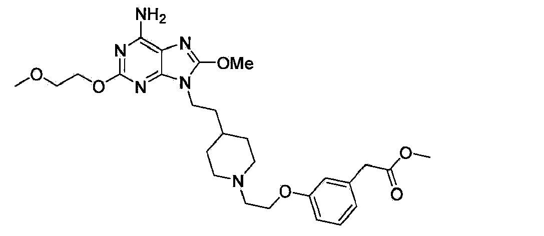

Using the compound 0.35g (0.78mmol) obtained in step (i), in the same manner as example 3 step (ii) there was obtained the subtitled compound 0.36g as colorless oil. Yield 86%
¹H NMR (DMSO-d₆) δ 7.20 (1H, dd, J = 8.4 Hz, 7.6 Hz), 6.83-6.77 (5H, m), 4.28 (2H, t, J = 4.8 Hz), 4.03 (3H, s), 4.01 (2H, t, J = 6.0 Hz), 3.86 (2H, t, J = 6.9 Hz), 3.63 (2H, s), 3.63-3.59 (2H, m), 3.59 (3H, s), 3.29 (3H, s), 2.90-2.86 (2H, m), 2.62 (2H, t, J = 5.8 Hz), 1.96-1.88 (2H, m), 1.72-1.66 (2H, m), 1.61-1.57 (2H, m), 1.18-1.11 (3H, m).

### Step (iii)

### 7,8-Dihydro-9-(1-{2-[3-(methoxycarbonylmethyl)phenoxy]ethyl}piperidin-4-ylethyl)-2-(2-methoxyethoxy)-8-oxoadenine

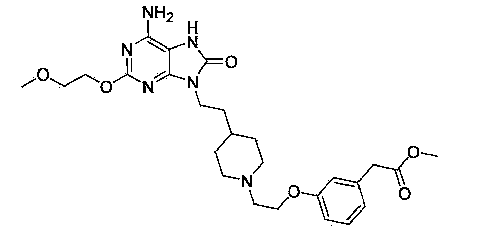

Using the compound 0.36g (0.66mmol) obtained in step (ii), in the same manner as example 1 step (v) there was obtained the titled compound 0.31g as a white solid. Yield 90%
¹H NMR (DMSO-d₆) δ 9.96 (1H, brs), 7.20 (1H, dd, J = 8.4 Hz, 7.6 Hz), 6.83-6.77 (3H, m), 6.53 (2H, brs), 4.26 (2H, t, J = 4.8 Hz), 4.02 (2H, t, J = 5.9 Hz), 3.69 (2H, t, J = 6.9 Hz), 3.63 (2H, s), 3.63-3.58 (2H, m), 3.60 (3H, s), 3.28 (3H, s), 2.90-2.86 (2H, m), 2.63 (2H, t, J = 5.9 Hz), 1.96-1.88 (2H, m), 1.72-1.67 (2H, m), 1.58-1.55 (2H, m), 1.16-1.11 (3H, m).

### Example 9

### 7,8-Dihydro-9-[1-(3-[{N-methyl-N-[3-(methoxycarbonylmethyl)benzyl]}amino]propyl)piperidin-4-ylethyl]-2-(2-methoxyethoxy)-8-oxoadenine

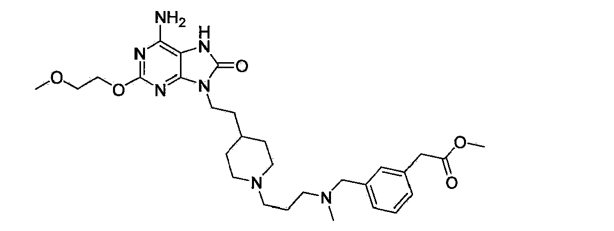

### Step (i)

### 9-(1-[3-{[N-(tert-Butoxycarbonyl)-N-methyl]amino}propyl]piperidin-4-ylethyl)-8-methoxy-2-(2-methoxyethoxy)adenine

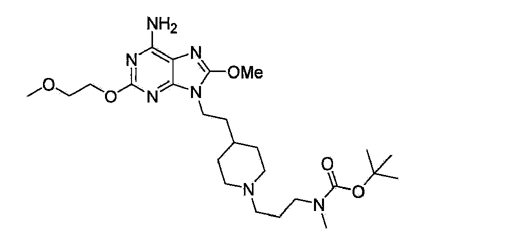

Using the compound 1.02g (2.25mmol) obtained by example 8 step (i), in the same manner as example 1 step (iii), there was obtained the subtitled compound 0.81g as a pale yellow oil. Yield 69%
¹H NMR (CDCl₃) 6 5.30 (2H, brs), 4.42 (2H, t, J = 5.0 Hz), 4.11 (3H, s), 3.96 (2H, t, J = 7.0 Hz), 3.75 (2H, t, J = 5.0 Hz), 3.43 (3H, s), 3.25-2.20 (2H, m), 3.05-2.89 (2H, m), 2.84 (3H, s), 2.50-2.30 (2H, m), 2.10-1.65 (9H, m), 1.45 (9H, s), 1.45-1.19 (2H, m).

### Step (ii)

### 9-[1-(3-[{N-Methyl-N-[3-(methoxycarbonylmethyl)benzyl]}amino]propyl)piperidin-4-ylethyl]-8-methoxy-2-(2-methoxyethoxy)adenine

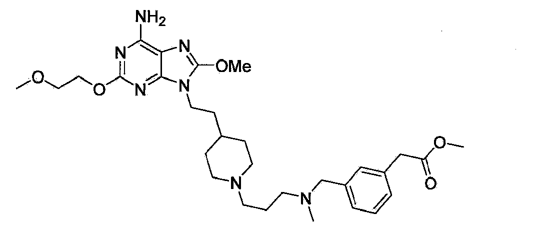

Using the compound 0.41 g (0.79mmol) obtained in step (i) in the same manner as example 1 step (iv) there was obtained the subtitled compound 0.28g as a colorless oil. Yield 60%
¹H NMR (DMSO-d₆) δ 7.23 (1H, dd, J = 7.9 Hz, 7.4 Hz), 7.17 (1H, s), 7.15 (1H, d, J = 7.9 Hz), 7.11 (1H, d, J = 7.4 Hz), 6.81 (2H, brs), 4.28 (2H, t, J = 4.8 Hz), 4.04 (3H, s), 3.86 (2H, t, J = 6.9 Hz), 3.65 (2H, s), 3.62-3.59 (2H, m), 3.60 (3H, s), 3.40 (2H, s), 3.29 (3H, s), 2.80-2.75 (2H, m), 2.29 (2H, t, J = 7.1 Hz), 2.22-2.05 (2H, m), 2.08 (3H, s), 1.75-1.63 (4H, m), 1.60-1.54 (4H, m), 1.10-1.05 (3H, m).

### Step (iii)

### 7,8-Dihydro-9-[1-(3-[{N-methyl-N-[3-(methoxycarbonylmethyl)benzyl]}amino]propyl)piperidin-4-ylethyl]-2-(2-methoxyethoxy)-8-oxoadenine

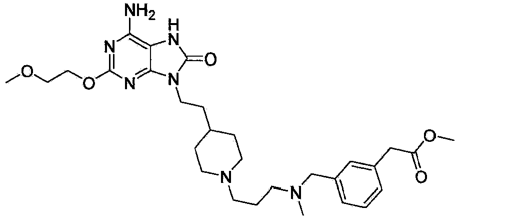

Using the compound 0.26g (0.45mmol) obtained in step (ii), in the same as example 1 step (v) there was obtained the titled compound 0.22g as a white solid. Yield 87%
¹H NMR (DMSO-d₆) δ 9.86 (1H, s), 7.25 (1H, dd, J = 8.0 Hz, 7.5 Hz), 7.17 (1H, s), 7.15 (1H, d, J = 8.0 Hz), 7.11 (1H, d, J = 7.5 Hz), 6.43 (2H, brs), 4.25 (2H, t, J = 4.7 Hz), 3.68 (2H, t, J = 7.0 Hz), 3.65 (2H, s), 3.62-3.59 (2H, m), 3.60 (3H, s), 3.30 (2H, s), 3.28 (3H, s), 2.78-2.73 (2H, m), 2.29 (2H, t, J = 7.1 Hz), 2.22-2.05 (2H, m), 2.08 (3H, s), 1.75-1.65 (4H, m), 1.58-1.54 (4H, m), 1.13-1.05 (3H, m).

### Example 10

### 7,8-Dihydro-9-{1-[3-([N-methyl-N-{2-[3-(methoxycarbonylmethyl)phenoxy]ethyl}]amino)propyl]piperidin-4-ylethyl}-2-(2-methoxyethoxy)-8-oxoadenine

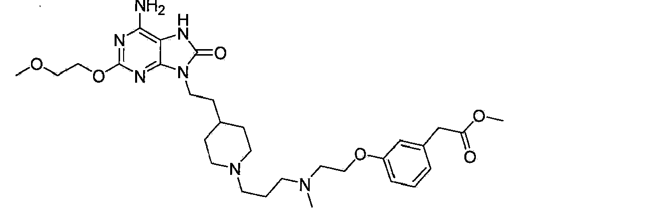

### Step (i)

### 9-{1-[3-([N-Methyl-N-{2-[3-(methoxycarbonylmethyl) phenoxy] ethyl}] amino) propyl] piperidin-4-ylethyl}-8-methoxy-2-(2-methoxyethoxy)adenine

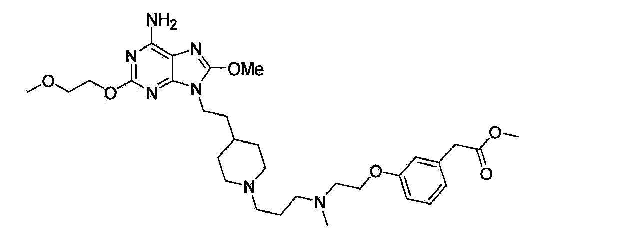

Using compound 0.27g (0.53mmol) obtained by example 8 step (i), in the same manner as example 3 step (ii) there was obtained the subtitled compound 0.21g as colorless oil. Yield 64%
¹H NMR (DMSO-d₆) δ 7.23-7.18 (1H, m), 6.87-6.77 (5H, m), 4.27 (2H, t, J = 4.8 Hz), 4.04 (3H, s), 4.00 (2H, t, J = 5.8 Hz), 3.85 (2H, t, J = 6.9 Hz), 3.65 (2H, s), 3.62-3.58 (2H, m), 3.60 (3H, s), 3.28 (3H, s), 2.80-2.74 (2H, m), 2.66 (2H, t, J = 5.8 Hz), 2.36 (2H, t, J = 7.1 Hz), 2.23-2.17 (2H, m), 2.21 (3H, s), 1.68-1.63 (4H, m), 1.60-1.54 (4H, m), 1.12-1.05 (3H, m).

### Step (ii)

### 7,8-Dihydro-9-{1-[3-([N-methyl-N-{2-[3-(methoxycarbonylmethyl)phenoxy]ethyl}]amino)propyl]piperidin-4-ylethyl}-2-(2-methoxyethoxy)-8-oxoadenine

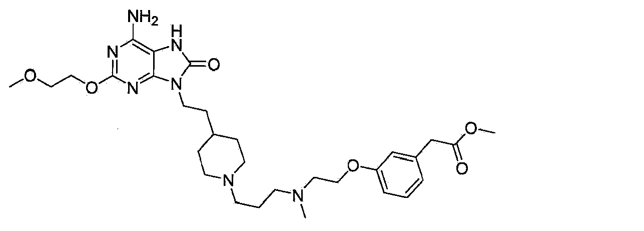

Using the compound 0.21g (0.33mmol) obtained in step (i), in the same manner as example 1 step (v) there was obtained the titled compound 0.17g as a white solid. Yield 84%
¹H NMR (DMSO-d₆) δ 7.23-7.18 (1H, m), 6.83-6.78 (3H, m), 6.54 (2H, brs), 4.26 (2H, t, J = 4.8 Hz), 4.00 (2H, t, J = 5.9 Hz), 3.68 (2H, t, J = 6.9 Hz), 3.63 (2H, s), 3.61-3.58 (2H, m), 3.60 (3H, s), 3.28 (3H, s), 2.80-2.74 (2H, m), 2.66 (2H, t, J = 5.8 Hz), 2.36 (2H, t, J = 7.1 Hz), 2.23-2.18 (2H, m), 2.21 (3H, s), 1.80-1.68 (4H, m), 1.58-1.51 (4H, m), 1.13-1.06 (3H, m).

### Example 11

### 2-Butoxy-7,8-dihydro-9-(1-{2-[3-(methoxycarbonylmethyl)phenoxy]ethyl}piperidin-4-ylethyl)-8-oxoadenine

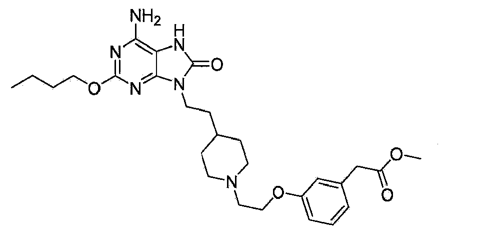

### Step (i)

### 2-Butoxy-8-methoxy-9-(1-{2-[3-(methoxycarbonylmethyl)phenoxy]ethyl}piperidin-4-ylethyl)adenine

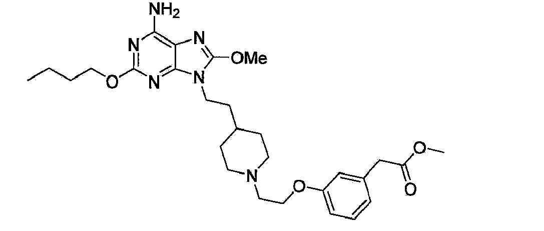

Using 2-butoxy-9-(1-tert-butoxycarbonylpiperidin-4-ylethyl)-8-methoxyadenine 0.25g (0.72mmol), in the same manner as example 3 step (ii) there was obtained the subtitled compound 0.27g as a pale yellow oil. Yield 69%
¹H NMR (DMSO-d₆) δ 7.20 (1H, dd, J = 7.8 Hz, 7.4 Hz), 6.83-6.75 (5H, m), 4.15 (2H, t, J = 6.6 Hz), 4.04 (3H, s), 4.01 (2H, t, J = 5.8 Hz), 3.87 (2H, t, J = 6.8 Hz), 3.63 (2H, s), 3.60 (3H, s), 2.90-2.86 (2H, m), 2.62 (2H, t, J = 5.8 Hz), 1.96-1.88 (2H, m), 1.73-1.58 (6H, m), 1.43-1.37 (2H, m), 1.18-1.11 (3H, m), 0.91 (3H, t, J = 7.4 Hz).

### Step (ii)

### 2-Butoxy-7,8-dihydro-9-(1-{2-[3-(methoxycarbonylmethyl)phenoxy]ethyl}piperidin-4-ylethyl)-8-oxoadenine

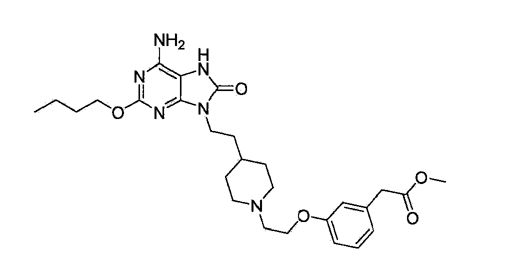

Using compound 0.25g (0.47mmol) obtained in step (i), in the same manner as example 1 step (v) there was obtained the titled compound 0.18g as a white solid. Yield 73%
¹H NMR (DMSO-d₆) δ 9.87 (1H, brs), 7.20 (1H, dd, J = 7.7 Hz, 7.5 Hz), 6.83-6.79 (3H, m), 6.40 (2H, brs), 4.14 (2H, t, J = 6.6 Hz), 4.01 (2H, t, J = 5.9 Hz), 3.69 (2H, t, J = 6.8 Hz), 3.63 (2H, s), 3.60 (3H, s), 2.90-2.86 (2H, m), 2.62 (2H, t, J = 5.8 Hz), 1.96-1.88 (2H, m), 1.73-1.55 (6H, m), 1.43-1.37 (2H, m), 1.18-1.11 (3H, m), 0.91 (3H, t, J = 7.4 Hz).

### Example 12

### 2-Butoxy-7,8-dihydro-9-{1-[3-([N-methyl-N-{2-[3-(methoxycarbonylmethyl)phenoxy]ethyl}]amino)propyl]piperidin-4-ylethyl}-8-oxoadenine

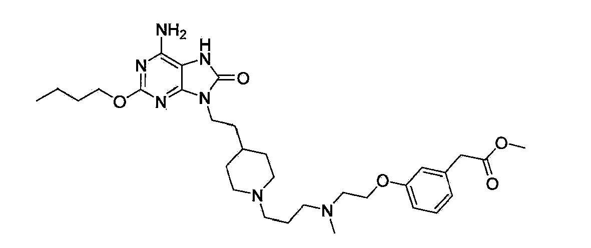

### Step (i)

### 2-Butoxy-9-(1-[3-{[N-(tert-butoxycarbonyl)-N-methyl]amino}propyl]piperidin-4-ylethyl)-8-methoxyadenine

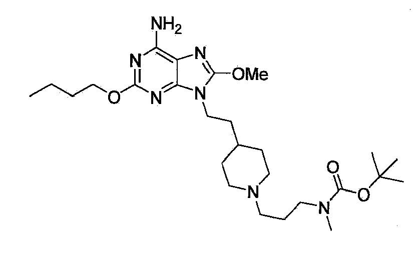

Using 2-butoxy-9-(1-tert-butoxycarbonylpiperidin-4-ylethyl)-8-methoxyadenine 0.71g (2.02mmol), in the same manner as example 1 step (iii) there was obtained the subtitled compound 0.62g as a pale yellow oil. Yield 59%
¹H NMR (CDCl₃) δ 6.77 (2H, brs), 4.15 (2H, t, J = 6.6 Hz), 4.04 (3H, s), 3.86 (2H, t, J = 6.8 Hz), 3.13 (2H, t, J = 7.2 Hz), 2.83-2.75 (2H, m), 2.74 (3H, s), 2.22-2.18 (2H, m), 1.80-1.55 (10H, m), 1.42-1.37 (2H, m), 1.37 (9H, s), 1.15-1.08 (3H, m), 0.92 (3H, t, J = 7.4 Hz).

### Step (ii)

### 2-Butoxy-9-{1-[3-([N-methyl-N-{2-[3-(methoxycarbonylmethyl)phenoxy]ethyl}]amino)propyl]piperidin-4-ylethyl}-8-methoxyadenine

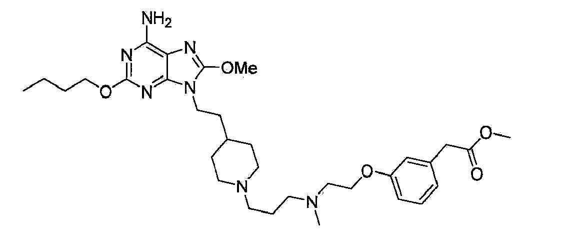

Using the compound 0.29g (0.55mmol) obtained in step (i), in the same manner as example 3 step (ii) there was obtained the subtitled compound 0.22g as a pale yellow oil. Yield 66%
¹H NMR (DMSO-d₆) δ 7.22-7.18 (1H, m), 6.87-6.70 (5H, m), 4.15 (2H, t, J = 6.6 Hz), 4.04 (3H, s), 4.00 (2H, t, J = 5.9 Hz), 3.85 (2H, t, J = 6.8 Hz), 3.63 (2H, s), 3.60 (3H, s), 2.80-2.74 (2H, m), 2.66 (2H, t, J = 5.8 Hz), 2.36 (2H, t, J = 7.1 Hz), 2.23-2.17 (2H, m), 2.20 (3H, s), 1.75-1.60 (6H, m), 1.59-1.47 (4H, m), 1.44-1.34 (2H, m), 1.13-1.06 (3H, m), 0.91 (3H, t, J = 7.4 Hz).

### Step (iii)

### 2-Butoxy-7,8-dihydro-9-{1-[3-([N-methyl-N-{2-[3-(methoxycarbonylmethyl)phenoxy]ethyl}]amino)propyl]piperidin-4-ylethyl}-8-oxoadenine

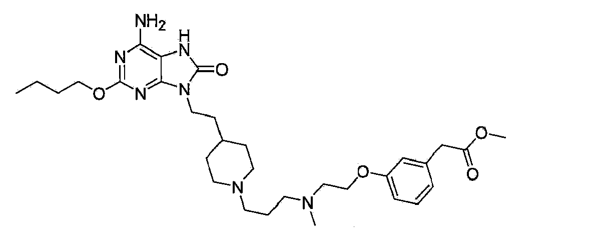

Using the compound 0.22g (0.37mmol) obtained in step (ii), in the same manner as example 1 step (v) there was obtained the titled compound 0.20g as a white solid. Yield 92%
¹H NMR (DMSO-d₆) δ 9.88 (1H, brs), 7.20 (1H, dd, J = 7.3 Hz, 7.2 Hz), 6.82-6.79 (3H, m), 6.41 (2H, brs), 4.13 (2H, t, J = 6.6 Hz), 4.00 (2H, t, J = 5.8 Hz), 3.68 (2H, t, J = 6.9 Hz), 3.62 (2H, s), 3.60 (3H, s), 2.79-2.74 (2H, m), 2.66 (2H, t, J = 5.8 Hz), 2.36 (2H, t, J = 7.1 Hz), 2.23-2.17 (2H, m), 2.21 (3H, s), 1.75-1.57 (6H, m), 1.56-1.47 (4H, m), 1.42-1.34 (2H, m), 1.13-1.06 (3H, m), 0.91 (3H, t, J = 7.4 Hz).

### Example 13

### 7,8-Dihydro-9-(1-{[3-(methoxycarbonylmethyl)phenyl]aminocarbonylmethyl}piperidin-4-ylethyl)-2-(2-methoxyethoxy)-8-oxoadenine

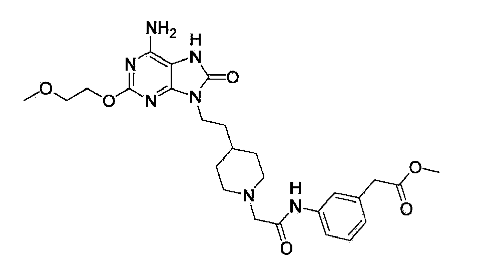

### Step (i)

### 8-Methoxy-9-(1-{[3-(methoxycarbonylmethyl)phenyl]aminocarbonylmethyl}piperidin-4-ylethyl)-2-(2-methoxyethoxy)adenine

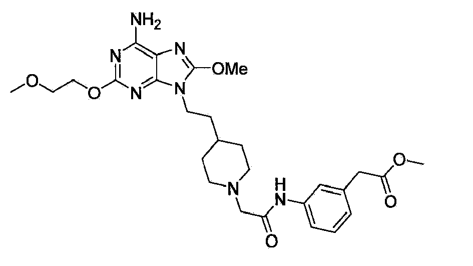

To the compound 0.15g (0.33mmol) obtained by example 8 step (i) was added trifluoroacetic acid 2ml and the mixture was stirred at room temperature for 20 minutes. After removal of trifluoroacetic acid by distillation, thereto were added DMF 10ml, potassium carbonate 0.28g (2.0mmol) and 2-chloro-N-{3-(methoxycarbonylmethyl)phenyl}acetamide 84mg (0.35mmol), and the mixture was stirred at room temperature for 18 hours. After removal of the solvent by distillation, thereto was added water, and the mixture was extracted with chloroform/ethanol (3/1). The organic layer was dried over anhydrous magnesium sulfate, concentrated in vacuo and purified by silica gel column chromatography to give the subtitled compound 0.18g as a colorless oil. Yield 99%
¹H NMR (DMSO-d₆) δ 9.63 (1H, s), 7.54-7.52 (2H, m), 7.24 (1H, dd, J = 8.6 Hz, 7.6 Hz), 6.94 (1H, d, J = 7.6 Hz), 6.81 (2H, brs), 4.27 (2H, t, J = 4.8 Hz), 4.05 (3H, s), 3.88 (2H, t, J = 7.0 Hz), 3.64 (2H, s), 3.63-3.57 (2H, m), 3.61 (3H, s), 3.28 (3H, s), 3.04 (2H, s), 2.85-2.80 (2H, m), 2.10-2.01 (2H, m), 1.74-1.69 (2H, m), 1.66-1.60 (2H, m), 1.30-1.25 (2H, m), 1.14-1.11 (1H, m).

### Step (ii)

### 7,8-Dihydro-9-(1-{[3-(methoxycarbonylmethyl)phenyl]aminocarbonylmethyl}piperidin-4-ylethyl)-2-(2-methoxyethoxy)-8-oxoadenine

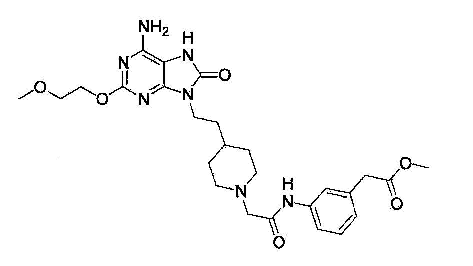

Using compound 0.17g (0.31mmol) obtained in step (ii), in the same manner as example 1 step (v) there was obtained the titled compound 0.27g as a white solid. Yield 86%
¹H NMR (DMSO-d₆) δ 9.93 (1H, brs), 9.63 (1H, s), 7.55-7.52 (2H, m), 7.24 (1H, dd, J = 8.6 Hz, 7.6 Hz), 6.94 (1H, d, J = 7.6 Hz), 6.46 (2H, brs), 4.26 (2H, t, J = 4.8 Hz), 3.71 (2H, t, J = 7.1 Hz), 3.64 (2H, s), 3.63-3.57 (2H, m), 3.61 (3H, s), 3.28 (3H, s), 3.05 (2H, s), 2.85-2.80 (2H, m), 2.10-2.01 (2H, m), 1.74-1.69 (2H, m), 1.64-1.57 (2H, m), 1.30-1.10 (3H, m).

### Example 14

### 2-Butoxy-7,8-dihydro-9-(1-{[3-(methoxycarbonylmethyl)phenyl] aminocarbonylmethyl}piperidin-4-ylethyl)-8-oxoadenine

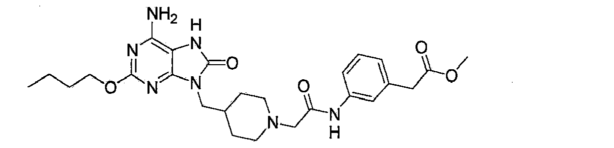

Using 2-butoxy-9-(1-tert-butoxycarbonylpiperidin-4-ylmethyl)-8-methoxyadenine 0.19g (0.44mmol) and 2-chloro-N-{3-(methoxycarbonylmethyl)phenyl} acetamide 0.12g (0.49mmol), in the same manner as example 13 step (i) and then step (ii), there was obtained the titled compound as a white solid 64mg. Yield 28%
¹H NMR (DMSO-d₆) δ 9.95 (1H, brs), 9.63 (1H, s), 7.55 (1H, d, J = 8.8 Hz), 7.54 (1H, s), 7.24 (1H, dd, J = 8.8 Hz, 7.6 Hz), 6.94 (1H, d, J = 7.6 Hz), 6.44 (2H, brs), 4.14 (2H, t, J = 6.6 Hz), 3.64 (2H, s), 3.61 (3H, s), 3.57 (2H, d, J = 7.3 Hz), 3.05 (2H, s), 2.85-2.80 (2H, m), 2.10-2.04 (2H, m), 1.85-1.74 (1H, m), 1.68-1.60 (2H, m), 1.54-1.50 (2H, m), 1.40-1.33 (4H, m), 0.91 (3H, t, J = 7.4 Hz).

### Example 15

### 2-Butoxy-7,8-dihydro-9-{1-[(N-{2-[3-(methoxylcarbonylmethyl)phenoxy]ethyl}-N-methyl)aminomethylacarbonyl]piperidin-4-ylmethyl}-8-oxoadenine

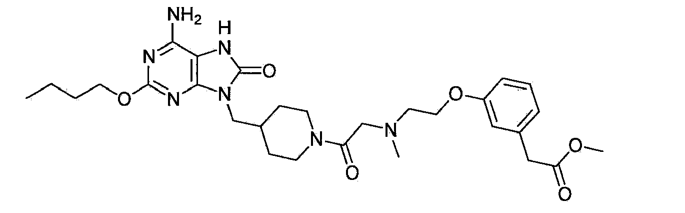

### Step (i)

### 2-Butoxy-8-methoxy-9-(piperidin-4-ylmethyl)adenine

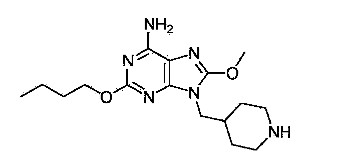

To the compound 2.0g (4.6mmol) obtained by example 1 step (ii) was added trifluoroacetic acid 5ml and the mixture was stirred at room temperature for 1 hour. After removal of trifluoroacetic acid by distillation, thereto was added aqueous sodium hydrogencarbonate and the mixture was extracted with chloroform-ethanol. The organic layer was dried over sodium sulfate, concentrated and dried in vacuo to give the titled compound 1.54g (4.6mmol) as a yellow white solid.
1H NMR (DMSO-d₆) δ 6.81 (2H, brs), 4.16 (2H, t, J = 6.6 Hz), 4.05 (3H, s), 3.73 (2H, d, J = 7.1 Hz), 3.09 (2H, m), 2.68-2.59 (2H, m), 1.99-1.92 (1H, m), 1.68-1.66 (2H, m), 1.58-1.53 (2H, m), 1.44-1.35 (2H, m), 1.23-1.19 (2H, m), 0.92 (3H, t, J = 7.4 Hz).

### Step (ii)

### 2-Butoxy-7,8-dihydro-9-{1-[(N-{2-[3-(methoxylcarbonylmethyl)phenoxy]ethyl}-N-methyl)aminomethylacarbonyl]piperidin-4-ylmethyl}-8-oxoadenine

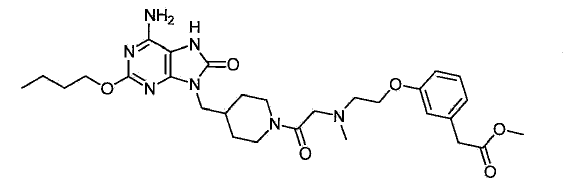

To 2-butoxy-8-methoxy-9-(piperidin-4-ylmethyl)adenine 500mg (1.15mmol) obtained in step (ii) in DMF (10ml) were added triethylamine 236µl (1.73mmol) and then chloroacetyl chloride 110µl (1.38mmol), and the mixture was stirred at room temperature for 25 minutes. After quenching the reaction with water, the solvent was removed by distillation. To the residue was added water and the mixture was extracted with chloroform-methanol. The organic layer was washed with water and saturated brine, concentrated and dried in vacuo. After addition of DMF 5ml to the obtained residue, thereto were added diisopropylethylamine 772µl (4.49mmol), and then N-{2-[3-(methoxycarbonyl)phenoxy]ethyl}-N-methylamine hydrochloride 388mg (1.15mmol), and the mixture was stirred at 60°C for 3 hours. After adding water, the mixture was extracted with chloroform-methanol. The organic layer was washed with water, saturated brine, dried over sodium sulfate, concentrated, purified by gel column chromatography and dried in vacuo.

To the residue were added methanol 10ml and concentrated sulfuric acid 500µl, and the mixture was stirred at 80°C for 90 minutes. After neutralized with aqueous ammonia, methanol is removal by distillation. After adding water, the mixture was extracted with chloroform-methanol. The organic layer was washed with water, saturated brine, dried over sodium sulfate and concentrated. The residue was purified by silica gel column chromatography and dried in vacuo to give the subtitled compound 160mg (0.27mmol) as a pale pink solid. Yield 37%
¹H NMR (DMSO-d₆) δ 9.86 (1H, brs), 7.20 (1H, t, J = 7.6 Hz), 6.82-6.79 (3H, m), 6.42 (2H, brs), 4.28 (1H, d, J = 12.9 Hz), 4.13 (2H, t, J = 6.6 Hz), 4.05-3.78 (3H, m), 3.62 (2H, s), 3.59 (3H, s), 3.53 (2H, d, J = 7.2 Hz), 3.29 (1H, d, J = 13.7 Hz), 3.15 (1H, d, J = 13.7 Hz), 2.88 (1H, t, J = 12.9 Hz), 2.80-2.71 (2H, m), 2.81-2.45 (1H, m), 2.27 (3H, s), 2.03-1.96 (1H, s), 1.66-1.59 (2H, s), 1.56-1.49 (2H, s), 1.42-1.33 (2H, s), 1.21-1.11 (2H, s), 1.02-0.97 (2H, s), 0.90 (3H, t, J = 7.4 Hz).

### Example 16

### 7,8-Dihydro-2-(2-methoxyethoxy)-9-{1-[(N-{2-[3-(methoxylcarbonylmethyl)phenoxy]ethyl}-N-methyl)aminomethylcarbonyl]piperidin-4-ylmethyl}-8-oxoadenine

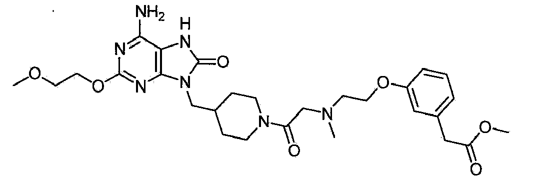

In the same manner as example 3 step (i) and example 15, there was obtained the subtitled compound as a white solid. Yield 19%
¹H NMR (DMSO-d₆) δ 9.89 (1H, brs), 7.21 (1H, t, J = 7.6 Hz), 6.84-6.77 (3H, m), 6.44 (2H, brs), 4.00-4.23 (3H, m), 4.07-3.98 (3H, m), 3.64-3.60 (2H, m), 3.62 (2H, s), 3.59 (3H, s), 3.53 (2H, d, J = 7.2 Hz), 3.32-3.30 (1H, m), 3.27 (3H, s), 3.16 (1H, d, J = 13.6 Hz), 2.97-2.85 (2H, m), 2.80-2.72 (2H, m), 2.27 (3H, s), 2.06-1.98 (1H, m), 1.55-1.50 (2H, m), 1.22-1.10 (2H, m), 1.06-0.95 (3H, t, J = 7.4 Hz).

### Example 17

### 7,8-Dihydro-2-(2-methoxyethoxy)-9-{1-[(N-{2-[3-(methoxylcarbonylmethyl)phenoxy]ethyl}-N-methyl)aminocarbonylmethyl]piperidin-4-ylmethyl}-8-oxoadenine

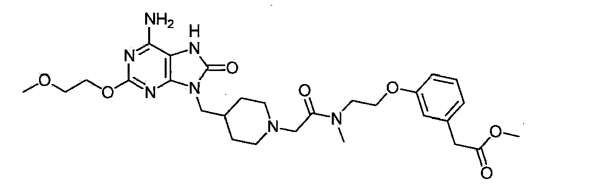

### Step (i)

### 2-Mmethoxyethoxy)-9-(piperidin-4-ylmethyl)adenine

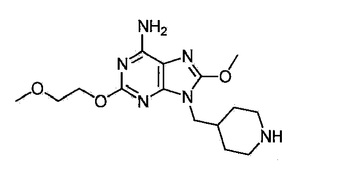

In the same manner as example 15 step (i) there was obtained the subtitled compound as a yellowish white solid. Yield 99%
¹H NMR (DMSO-d₆) δ 6.83 (2H, brs), 4.27 (2H, dd, J = 4.6, 4.6 Hz), 4.05 (3H, s), 3.72 (2H, d, J = 6.0 Hz), 3.46-3.41 (1H, m), 3.29 (3H, s), 3.08-3.02 (2H, m), 2.61-2.54 (2H, m), 1.98-1.89 (1H, m), 1.55-1.49 (2H, m), 1.25-1.14 (2H, m),

### Step (ii)

### ((N-(2-[3-(Methoxycarbonylmetyl)phenoxy]ethyl)-N-methyl)aminocarbonylmethyl chloride

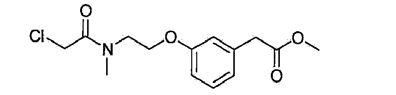

To N-{2-[3-(methoxycarbonylmetyl)phenoxy]ethyl}-N-methylamine 300mg (1.16mmol) in DMF (10ml) were added triethylamine 474µl (3.46mmol) and then chloroacethyl chloride 110µl (1.39mmol) and the mixture was stirred at room temperature for 2 hours. The solvent is removed by distillation and the residue was dried in vacuo to give the subtitled compound as orange liquid.

### Step (iii)

### 7,8-Dihydro-2-(2-methoxyethoxy)-9-{1-[(N-{2-[3-(methoxylcarbonylmethyl)phenoxy]ethyl}-N-methyl)aminocarbonylmethyl]piperidin-4-ylmethyl}-8-oxoadenine

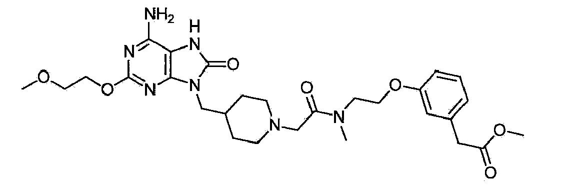

To the compound obtained in step (i) in DMF 7ml were added diisopropylethylamine 597µl (3.47mmol) and then the compound 300mg (1.16mmol) obtained in step (ii), and the mixture was heated at 60°C for 3 hours. After removal of the solvent by distillation, to the residue were added methanol 10ml and then concentrated sulfuric acid 300 µl and the mixture was stirred at 80°C for 2 hours. After neutralized with aqueous ammonia, the solvent was removed by distillation. After adding water, the mixture was extracted with chloroform-methanol. The organic layer was dried over sodium sulfate. After concentration, the residue was purified by column chromatography and dried in vacuo to give the titled compound 240mg (0.41mmol) as a white solid.
¹H NMR (DMSO-d₆) δ 9.85 (1H, brs), 7.26-7.18 (1H, m), 6.85-6.81 (3H, m), 6.43 (2H, brs), 4.25 (2H, dd, J = 3.7, 4.6 Hz), 4.18-4.13 (1H, m), 4.05-4.01 (1H, m), 3.82-3.79 (1H, m), 3.65-3.60 (5H, m), 3.59 (3H, s), 3.52 (2H, d, J = 7.2 Hz), 3.28 (3H, s), 3.14-3.08 (2H, m), 2.80-2.71 (2H, m), 2.51 (3H, s), 1.96-1.87 (2H, s), 1.80-1.69 (1H, m), 1.52-1.42 (2H, m), 1.24-1.11 (2H, m).

### Example 18

### 2-Butoxy-7,8-dihydro-9-(2-{1-[(N-{2-[3-(methoxylcarbonylmethyl)phenoxy]ethyl}-N-methyl)aminocarbonylmethyl]piperidin-4-yl}ethyl)-8-oxoadenine

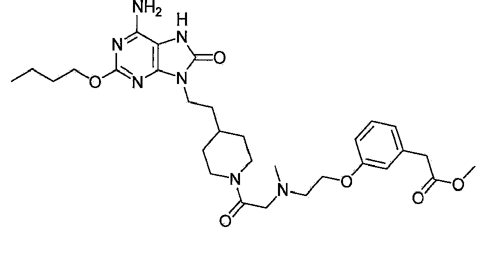

### Step (i)

### 8-Methoxy-2-(2-methoxyethoxy)-9-[2-(piperidin-4-yl)ethyl]adenine

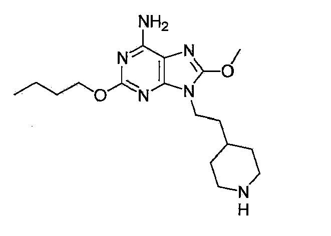

In the same manner as example 15 step (i) there was obtained the subtitled compound as whitish yellow liquid.
¹H NMR (DMSO-d₆) δ 6.79 (2H, brs), 4.14 (2H, t, J = 6.6 Hz), 4.05 (3H, s), 3.87 (2H, t, J = 6.8 Hz), 3.22-3.15 (2H, m), 2.77-2.69 (2H, m), 1.90-1.84 (2H, m), 1.68-1.59 (4H, m), 1.45-1.35 (3H, m), 1.30-1.18 (2H, m), 0.92 (3H, t,J=7.4Hz).

### Step (ii)

### 2-Butoxy-7,8-dihydro-9-(2-{1-[{N-{2-[3-(methoxylcarbonylmethyl)phenoxy]ethyl}-N-methyl)aminocarbonylmethyl]piperidin-4-yl)ethyl)-8-oxoadenine

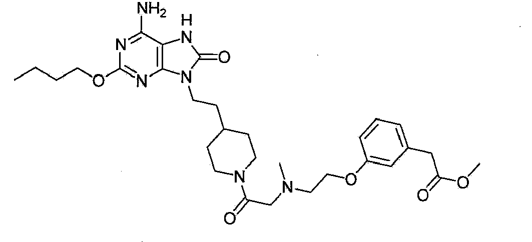

In the same manner as example 15 step (ii), there was obtained the subtitled compound as whitish yellow liquid.
¹H NMR (DMSO-d₆) δ 9.84 (1H, brs), 7.23-7.18 (1H, m), 6.89-6.79 (3H, m), 6.40 (2H, brs), 4.29 (1H, d, J = 12.6 Hz), 4.13 (2H, t, J = 6.6 Hz), 4.02 (2H, t, J = 5.6 Hz), 4.02-3.98 (1H, m), 3.69 (2H, t, J = 6.9 Hz), 3.61 (2H, s), 3.59 (3H, s), 3.32-3.29 (1H, m), 3.18-3.13 (1H, m), 2.89-2.81 (1H, m), 2.80-2.74 (2H, m), 2.47-2.40 (1H, m), 2.28 (3H, s), 1.77-1.70 (2H, m), 1.67-1.59 (2H, m), 1.58-1.52 (2H, m), 1.46-1.32 (3H, m), 1.15-1.00 (1H, m), 0.97-0.93 (1H, m), 0.90 (3H, t, J = 7.4 Hz).

### Example 19

### 2-Butoxy-7,8-dihydro-9-{1-[(N-{2-[2-methoxy-5-(methoxylcarbonylmethyl)phenoxy]ethyl}-N-methyl)aminomethylacarbonyl]piperidin-4-ylmethyl}-8-oxoadenine

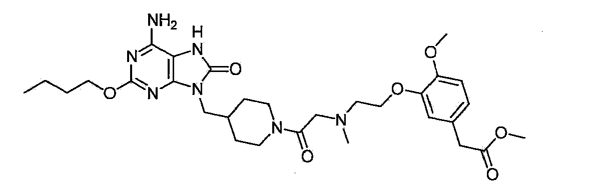

To the compound 0350mg (1.05mmol) obtained by example 15 step (i) in DMF 10ml were added triethylamine 215µl (1.57mmol) and then chloroacethyl chloride 100µl, and the mixture was stirred at room temperature for 1 hour. After removal the solvent by distillation, to the residue was added water, and the mixture was extracted with chloroform-methanol. The extracted organic layer was washed with water and saturated brine, and dried. The residue was dissolved in DMF 10ml and thereto were added diisopropylethylamine 360µl (2.09mmol) and then N-[2-methoxy-5-(methoxylcarbonylmethyl)phenoxy]-N-methyl]amine) 398µl (1.57mmol). The mixture was stirred at 50°C for 6 hours. After removal of the solvent by distillation, to the residue were added methanol 10ml and then concentrated sulfuric acid 300µl, and the mixture was stirred at 80°C for 3 hours. After neutralized with aqueous ammonia, the solvent was removed by distillation. To the residue was added water and the mixture was extracted with chloroform-methanol. The organic layer was washed with water and saturated brine and dried. After concentration, the residue was purified by column chromatography. Thereto was added diethyl ether, the resulting white solid was filtered, and dried to give the titled compound 182mg as a white solid. Yield 28%
¹H NMR (DMSO-d₆) δ 9.87 (1H, brs), 6.86-6.84 (2H, m), 6.75 (1H, d, J = 8.2 Hz), 6.41 (2H, brs), 4.31-4.25 (1H, m), 4.13 (2H, t, J = 6.6 Hz), 4.03-4.00 (1H, m), 4.01 (2H, t, J = 5.7 Hz), 3.70 (3H, s), 3.59 (3H, s), 3.56 (2H, s), 3.56-3.52 (2H, m), 3.27-3.23 (2H, m), 2.91-2.86 (1H, m), 2.78-2.71 (2H, m), 2.49-2.42 (1H, m), 2.28 (3H, s), 2.09-1.98 (1H, m), 1.66-1.58 (2H, m), 1.57-1.46 (2H, m), 1.42-1.32 (2H, m), 1.18-0.95 (1H, m), 0.90 (3H, t, J = 7.4 Hz).

### Example 20

### 2-Butoxy-7,8-dihydro-9-[5-(4-{2-[N-methyl-N-(3-methoxycarbonylmethyl)benzyl]aminomethyl}piperazin-1-yl)pentyl]-8-oxoadenine

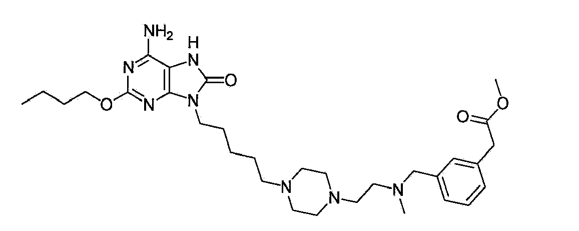

### Step (i)

### Methyl-3-{[N-(2-hydroxyethyl)-N-methyl]aminomethyl}phenylacetate

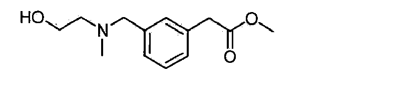

To bromide 1.5g (6.17mmol) in acetonitrile (20ml) was added methylaminoethanol (1ml) and the mixture was refluxed for 3 hours. After concentration of the solvent, the residue was extracted with ethyl acetate and dried over anhydrous sodium sulfate. The solvent was concentrated to give the titled compound 0.98g as a colorless oil. Yield 67%
¹H NMR (CDCl₃) δ 7.31-7.20 (4H, m), 3.69 (3H, s), 3.67 (2H, t, J= 5.3 Hz), 3.64 (2H, s), 3.63 (2H, s), 2.67 (2H, t, J= 5.3 Hz), 2.31 (3H, s).

### Step (ii)

### Methyl-3-{[N-(2-chroroethyl)-N-methyl] aminomethyl}phenylacetate

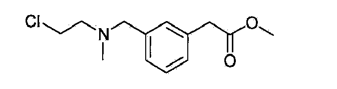

To the compound 0.90g (3.79mmol) obtained in step (i) in chloroform was added thionyl chloride 2.26g (18.9mmol) and the mixture was refluxed for 30 minutes. The solvent was concentrated to give the titled compound 0.97g as a colorless oil. Quantitatively
¹H NMR (CDCl₃) δ 7.33-7.20 (4H, m), 3.75-3.59 (9H, m), 2.83 (2H, brs), 2.35 (3H, brs).

### Step (iii)

### Methyl-3-({N-[2-(4-butoxycarbonylpiperazin-1-yl)ethyl]-N-methyl}aminomethyl)phenylacetate

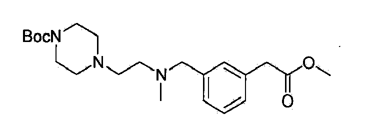

To the compound 0.97g (3.79mmol) obtained in step (ii) and potassium carbonate 1.05g (7.59mmol) in acetonitrile was added N-butoxycarbonylpiperazine, and the mixture was refluxed for 5 hours. The reaction mixture was filtered, and the filtrate was concentrated in vacuo. The residue was purified by column chromatography to give the titled compound 0.74g as colorless oil. Yield 48%
¹H NMR (CDCl₃) δ 7.31-7.18 (4H, m), 3.69 (3H, s), 3.64 (2H, s), 3.62 (2H, brs), 3.43 (4H, t, J= 4.9 Hz), 2.59 (4H, brs), 2.41 (2H, t, J= 4.59 Hz), 2.11 (3H, s), 1.45 (9H, s).

### Step (iv)

### Methyl-3-({N-[2-(piperazin-1-yl)ethyl]-N-methyl}aminomethyl)phenylacetate.hydrochloride

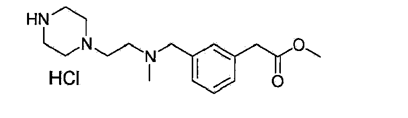

The compound obtained in step (iii) in 4N-hydrochloric acid-dioxane was stirred at room temperature for 3 hours. The solvent was concentrated to give the titled compound 743mg as a white solid. Yield 57%
¹H NMR (DMSO-d₆) δ 9.38 (1H, brs), 7.55 (H, d, J= 7.5 Hz), 7.50 (1H, s), 7.44 (1H, t, J= 7.5 Hz), 7.38 (1H, d, J= 7.5 Hz),4.90 (2H, brs), 4.35 (2H, brs), 3.74 (3H, s), 3.64 (3H, s), 3.33 (2H, brs), 3.26 (4H, brs), 2.98 (4H, brs), 2.69 (3H, s).

### Step (v)

### 9-(5-Bromopentyl)-2-butoxy-8-methoxyadenine

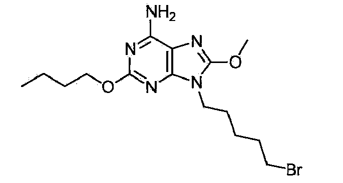

To 2-butoxy-8-methoxyadenine 2.00g (8.43mmol) in DMF (30ml) were added potassium carbonate 1.40g (10.1mmol) and 1,5-dibromopentane 3.87g (16.9mmol), and the mixture was stirred at room temperature for 6 hours. After removal of the solvent by distillation, thereto was added water 80ml and the mixture was extracted with 5% methanol-chloroform (100ml). The organic layer was washed with water and saturated brine, successively, dried over sodium sulfate, concentrated in vacuo and purified by silica gel column chromatography to give the subtitled compound 1.69g as a pale pink solid. Yield 52%
¹H NMR (DMSO-d₆) δ 6.78 (2H, bs), 4.16 (2H, t, J= 6.6 Hz), 4.03 (3H, s), 3.84 (2H, t, J= 6.8 Hz), 1.86-1.78 (2H, m), 1.74-1.60 (4H, m), 1.45-1.35 (2H, m), 1.35-1.28 (2H, m), 0.92 (3H, t, J= 7.4 Hz).

### Step (vi)

### 2-Butoxy-8-methoxy-9-[5-(4-{2-[N-methyl-N-{3-methoxycarbonylmethyl}benzyl]aminomethyl}piperazin-1-yl) pentyl]adenine

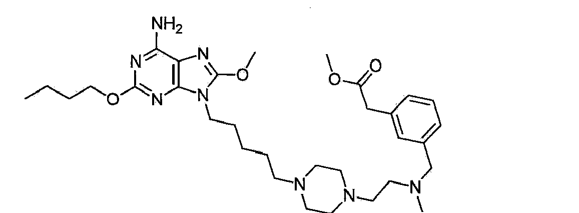

To the compound 212mg (0.62mmol) obtained in step (iv) in DMF (10ml) were added N-diisopropylethylamine 335mg (2.59mmol), compound 200mg (0.52mmol) obtained in step (v) and dimethylaminopyridine 64mg (0.62mmol), and the mixture was stirred at room temperature for 12 hours. After removal of the solvent by distillation, thereto was added water 80ml and the mixture was extracted with 5% methanol-chloroform (100ml). The organic layer was washed with water, and saturated brine, successively, dried over sodium sulfate, concentrated in vacuo and the residue was purified by silica gel column chromatography to give the subtitled compound 149 mg as colorless oil. Yield 47%
¹H NMR (CDCl₃) δ 7.32-7.16 (4H, m), 5.17 (2H, s), 4.27 (2H, t, J= 6.6 Hz), 4.10 (3H, s), 3.92 (2H, t, J= 7.2 Hz), 3.69 (3H, s), 3.62 (2H, s), 5.12 (2H, s), 2.55-2.50 (4H, m), 2.33 (2H, brs), 2.24 (3H, s), 1.81-1.73 (12H, m), 1.54-1.46 (4H, m), 1.33-1.28 (2H, m), 0.96 (3H, t, J= 7.4 Hz).

### Step (vii)

### 2-Butoxy-7,8-dihydro-9-[5-(4-{2-[N-methyl-N-(3-methoxycarbonylmethyl)benzyl]aminomethyl}piperazin-1-yl)pentyl]-8-oxoadenine

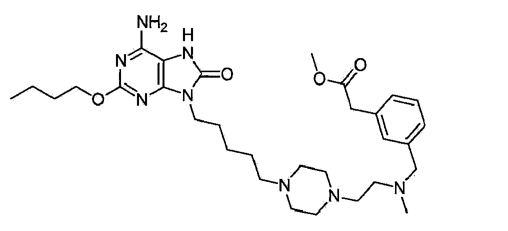

To the compound 148mg (0.24mmol) obtained in step (vi) in methanol (10ml) was added concentrated sulfuric acid (500µl) and the mixture was refluxed for 4 hours. After neutralized with 28% aqueous ammonia, the solvent was removed by distillation. To the residue was added water and the resulting solid was filtered to give the titled compound 119 mg as a white solid. Yield 82%
¹H NMR (DMSO-d₆) δ 9.84 (1H, s), 7.33-7.12 (4H, m), 6.40 (2H, s), 4.14 (2H, t, J= 6.6 Hz), 3.66-3.63 (4H, m), 3.61 (3H, s), 3.45 (2H, s), 2.41-2.18 (14H, m), 2.13 (3H, s), 1.66-1.62 (4H, m), 1.43-1.36 (4H, m), 1.23-1.20 (2H, m), 0.92 (3H, t, J= 7.4 Hz).

### Example 21

### 2-Butoxy-7,8-dihydro-9-[7-(4-{2-[N-methyl-N-(3-methoxycarbonylmethyl)benzyl] aminomethyl}piperazin-1-yl)heptyl]-8-oxoadenine

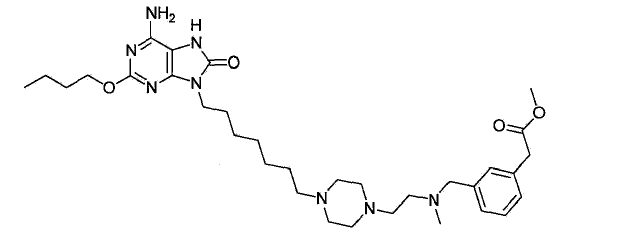

### Step (i)

### 9-(5-Bromopentyl)-2-butoxy-8-methoxyadenine

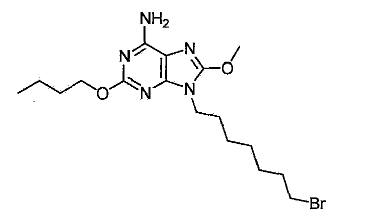

Using 2-butoxy-8-methoxyadenine 3.00g (8.54mmol) and 1,7-dibromoheptane 4.4mg (17.1mmol), in the same manner as example 20 step (v) there was obtained the titled compound 1.75g as a white solid.
Yield 49%
¹H NMR (CDCl₃) δ 5.30 (2H, bs), 4.28 (2H, t, J= 6.7 Hz), 4.11 (3H, s), 3.92 (2H, t, J= 7.2 Hz), 3.39 (2H, t, J= 6.8 Hz), 1.85-1.73 (8H, m), 1.52-1.47 (2H, m), 1.45-1.32 (4H, m), 0.96 (3H, t, J= 7.4 Hz).

### Step (ii)

### 2-Butoxy-8-oxo-9-[7-(4-{2-[N-methyl-N-(3-methoxycarbonylmethyl)benzyl]aminomethyl}piperazin-1-yl)heptyl]adenine

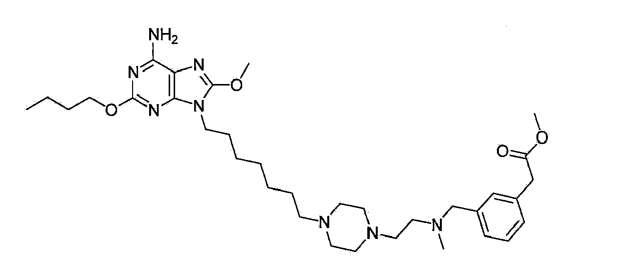

Using the compound 266mg (0.78mmol) obtained by example 20 step (iv) and the compound 200mg (0.52mmol) obtained in step (i), in the same manner as example 20 step (vi), there was obtained the titled compound 195mg as colorless oil. Yield 59%
¹H NMR (CDCl₃) δ 7.31-7.16 (4H, m), 5.15 (2H, s), 4.27 (2H, t, J= 6.7 Hz), 4.11 (3H, s), 3.90 (2H, t, J= 7.2 Hz), 3.69 (3H, s), 3.62 (2H, s), 3.51 (2H, s), 2.61-2.32 (6H, m), 2.24 (3H, s), 1.78-1.71 (12H, m), 1.52-1.46 (4H, m), 1.30-1.25 (6H, m), 0.96 (3H, t, J= 7.4 Hz).

### Step (iii)

### 2-Butoxy-7,8-dihydro-9-[7-(4-{2-[N-methyl-N-(3-methoxycarbonylmethyl)benzyl]aminomethyl}piperazin-1-yl)heptyl]-8-oxoadenine

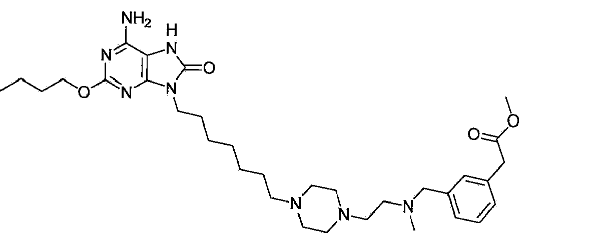

Using the compound 195mg (0.31mmol) obtained in step (vi) in the same manner as example 20 step (vii), there was obtained the titled compound 138mg as a white solid. Yield 73%
¹H NMR (DMSO-d₆) δ 9.84 (1H, s), 7.43-7.22 (4H, m), 6.41 (2H, s), 4.14 (2H, t, J= 6.6 Hz), 3.69-3.62 (7H, m), 3.36-3.32 (4H, m), 2.92 (8H, bs), 2.42-2.11 (5H, m), 1.66-1.62 (4H, m), 1.42-1.36 (4H, m), 1.28-1.25 (6H, m), 0.94 (3H, t, J= 7.4 Hz).

### Example 22

### 2-Butoxy-7,8-dihydro-9-(5-{4-[2-(3-methoxycarbonylmethylphenyloxy)ethyl]piperazin-1-yl}pentyl)-8-oxoadenine

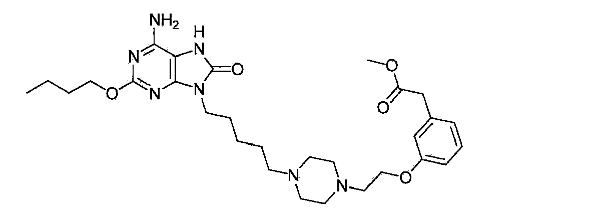

### Step (i)

### 2-Butoxy-9-{5-[4-(t-butoxycarbonyl)piperazin-1-yl]pentyl}-8-methoxyadenine

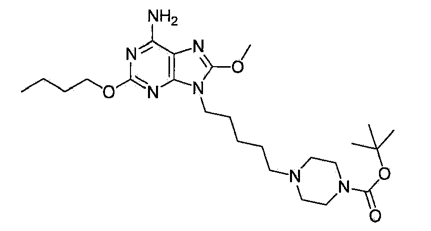

To N-Boc-piperazine 579mg (3.11mmol) and potassium carbonate 715 mg (5.18mmol) in dimethylformamide 20ml was added the compound 1.0g (2.59mmol) obtained by example 20 step (v) and the mixture was stirred under an atmosphere of nitrogen at room temperature for 48 hours. After removal of the solvent by distillation, thereto was added water 20ml and the mixture was extracted with chloroform. The organic layer was washed with saturated brine, dried over magnesium sulfate and the solvent was removed by distillation. The residue was purified by silica gel column chromatography to give the titled compound 1.25g as colorless oil. Yield 98%
¹H NMR (CDCl₃) δ 5.14 (2H, bs), 4.27 (2H, t, J= 6.7 Hz), 4.11 (3H, s), 3.92 (2H, t, J= 7.1 Hz), 3.46 (4H, bs), 2.38 (4H, bs), 1.80-1.73 (4H, m), 1.62-1.57 (2H, m), 1.52-1.48 (4H, m), 1.46 (9H, s), 1.33-1.29 (2H, m), 0.96 (3H, t, J= 7.4 Hz).

### Step (ii)

### 2-Butoxy-8-methoxy-9-(5-{4-[2-(3-methoxycarbonylmethylphenyloxy)ethyl]piperazin-1-yl}pentyl)adenine

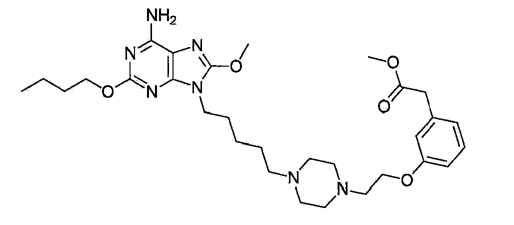

The compound 200mg (0.41mmol) obtained in step (i) in trifluoroacetic acid 2ml was stirred at room temperature for 30 minutes. After removal of the solvent by distillation, the residue was dried in vacuo for 2 hours. Thereto were added potassium carbonate 281mg (2.1mmol), dimethylformamide 7ml and methyl [3-(2-bromoethoxy)phenyl] acetate 167mg (0.611mmol), and the mixture was stirred under an atmosphere of nitrogen at 60°C. After removal of the solvent by distillation, thereto was added water 20ml, and the mixture was extracted with chloroform. The organic layer washed with saturated brine, dried over magnesium sulfate and the solvent was removed by distillation. The residue was purified by silica gel column chromatography to give the titled compound 165 mg as a colorless oil. Yield 69%
¹H NMR (CDCl₃) δ 7.22 (1H, t, J= 7.9 Hz), 6.87-6.79 (3H, m), 5.16 (2H, s), 4.27 (2H, t, J= 6.7 Hz), 4.11 (3H, s), 4.10 (2H, t, J= 5.8 Hz), 3.92 (2H, t, J= 7.1 Hz), 3.69 (3H, s), 3.59 (2H, s), 2.82 (2H, t, J= 5.8 Hz), 2.65 (2H, bs), 2.52 (2H, bs), 2.36 (2H, bs), 1.79-1.73 (8H, m), 1.53-1.46 (4H, m), 1.33-1.29 (2H, m), 0.96 (3H, t, J= 7.4 Hz).

### Step (iii)

### 2-Butoxy-7,8-dihydro-9-(5-{4-[2-(3-methoxycarbonylmethylphenyloxy)ethyl]piperazin-1-yl}pentyl)-8-oxoadenine

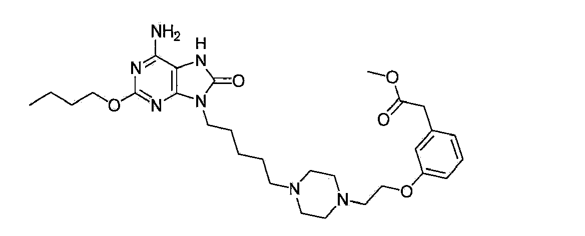

Using the compound 164mg (0.28mmol) obtained in step (ii), in the same manner as example 20 step (vii), there was obtained the titled compound 140mg as a white solid. Yield 90%
¹H NMR (DMSO-d₆) δ 9.76 (1H, bs), 7.14 (1H, t, J= 7.4 Hz), 6.76-6.73 (3H, m), 6.32 (2H, s), 4.07 (2H, t, J= 6.6 Hz), 3.94 (2H, t, J= 5.8 Hz), 3.58-3.55 (4H, m), 3.53 (3H, s), 2.57 (2H, t, J= 5.8 Hz), 2.41 (4H, bs), 2.38 (4H, bs), 2.12 (2H, bs), 1.58-1.54 (4H, m), 1.34-1.30 (4H, m), 1.16-1.12 (2H, m), 0.96 (3H, t, J= 7.4 Hz).

### Example 23

### 2-Butoxy-7,8-dihydro-9-(7-{4-[2-(3-methoxycarbonylmethylphenyloxy)ethyl]piperazin-1-yl}heptyl)-8-oxoadenine

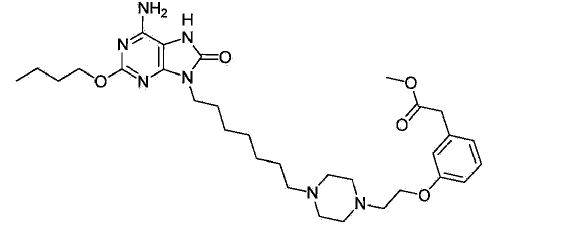

### Step (i)

### 2-Butoxy-9-{7-[4-(t-butoxycarbonyl)piperazin-1-yl]heptyl}-8-methoxyadenine

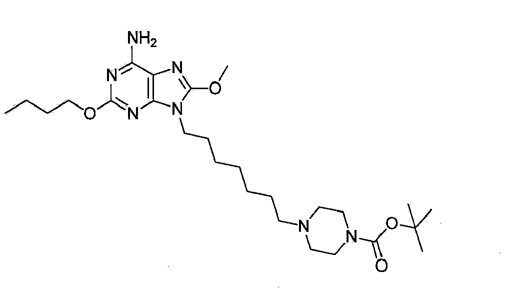

Using the compound 1.0g (2.41mmol) obtained by example 21 step (i), in the same manner as example 22 step (i), there was obtained the titled compound 1.16g as a colorless oil. Yield 92%
¹H NMR (CDCl₃) δ 5.16 (2H, bs), 4.27 (2H, t, J= 6.7 Hz), 4.11 (3H, s), 3.91 (2H, t, J= 7.2 Hz), 3.45 (2H, bs), 2.39 (2H, bs), 2.33 (2H, bs), 1.78-1.68 (8H, m), 1.52-1.48 (4H, m), 1.46 (9H, s), 1.31-1.27 (6H, m), 0.96 (3H, t, J= 7.4 Hz).

### Step (ii)

### 2-Butoxy-8-methoxy-9-(7-{4-[2-(3-methoxycarbonylimethylphenyloxy)ethyl]piperazin-1-yl}heptyl)adenine

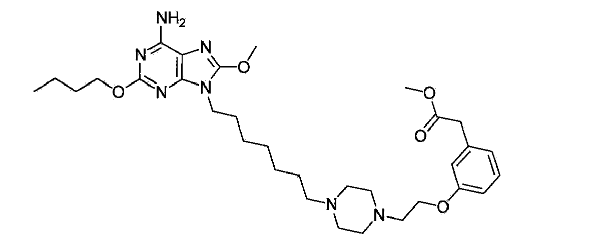

Using the compount 200mg (0.39mmol) obtained in step (i), in the same manner as example 22 step (ii), there was obtained the titled compound 123mg as a colorless oil. Yield 52%.
¹H NMR (CDCl₃) δ 7.23 (1H, t, J=7.8 Hz), 6.87-679 (3H, m), 5.16 (2H, s), 4.27 (2H, t, J= 6.7Hz), 4.11 (3H, s), 4.10 (2H, t, J=5.8 Hz), 3.91 (2H, t, J= 7.2 Hz), 3.69 (3H, s) 3.64 (2H, s), 2.83 (2H, t, J= 5.8 Hz), 2.72 (2H, bs), 2.57 (4H, bs), 2.38 (2H, bs), 1.78-1.72 (6H, m), 1.54-1.46 (4H, m), 1.35-1.25 (6H, m), 0.96 (3H, t, J=7.4 Hz).

### Step (iii)

2-Butoxy-7,8-dihydro-9-(7-{4-[2-(3-methoxycarbonylmethylphenyloxy)ethyl]piperazin-1-yl}heptyl)-8-oxoadenine

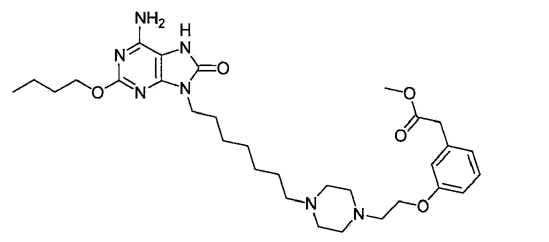

Using the compound 123mg (0.20mmol) obtained in step (ii), in the same manner as example 20 step (vii), there was obtained the titled compound 112mg as a white solid. Yield 93%
¹H NMR (DMSO-d₆) δ 7.22 (1H, t, J= 8.0 Hz), 6.86-6.79 (3H, m), 6.44 (2H, bs), 4.14 (2H, t, J= 6.6 Hz), 4.03 (2H, t, J= 5.8 Hz), 3.66 (2H, t, J= 7.1 Hz), 3.64 (2H, s), 3.61 (3H, s), 2.67 (2H, bs), 2.34 (6H, bs), 2.22 (4H, bs), 1.66-1.62 (4H, m), 1.42-1.36 (4H, m), 1.28-1.24 (6H, m), 0.92 (3H, t, J= 7.4 Hz).

### Example 24

### 2-Butoxy-7,8-dihydro-9-[2-{4-(3-methoxycarbonylmethylphenoxy)piperidin-1-yl}ethyl]-8-oxoadenine

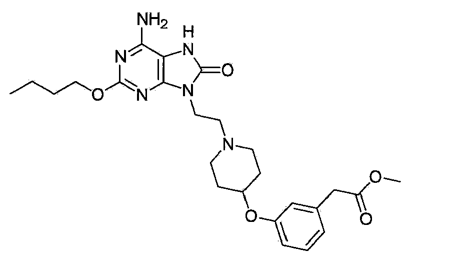

### Step (i)

### 9-(2-Bromoethyl)-2-butoxy-8-methoxyadenine

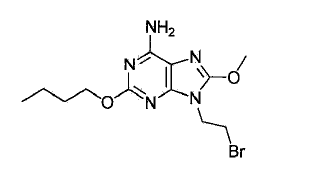

Using 2-butoxy-8-methoxyadenine 500mg (2.11mmol), in the same manner as example 20 step (v), there was obtained the subtitled compound 573 mg as a white solid. Yield 79%
¹H NMR (CDCl₃) δ 5.31 (2H, brs), 4.32 (2H, t, J= 7.0 Hz), 4.27 (2H, t, J= 6.7 Hz), 4.12 (3H, s), 3.66 (2H, t, J= 7.0 Hz), 1.79-1.72 (2H, m), 1.52-1.46 (2H, m), 0.95 (3H, t, J= 7.4 Hz).

### Step (ii)

### 2-Butoxy-8-methoxy-9-[2-{4-(3-methoxycarbonylmethylphenoxy)piperidin-1-yl}ethyl]adenine

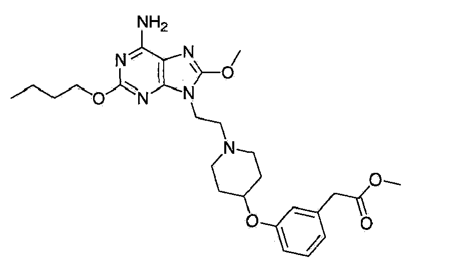

Using the compound 200mg (0.58mmol) obtained in step (i), in the same manner as example 20 step (vi), there was obtained the subtitled compound 150mg as a colorless oil. Yield 50%
¹H NMR (DMSO-d₆) δ 7.19 (1H, dd, J= 7.6, 7.6 Hz), 6.82 (1H, s), 6.79 (2H, d, J= 7.6 Hz), 6.76 (2H, bs),4.36-4.28 (1H, m), 4.15 (2H, t, J= 6.6 Hz), 4.04 (2H, s), 3.94 (2H, t, J= 6.4 Hz), 3.60 (3H, s), 2.77-2.68 (2H, m), 2.62 (2H, t, J= 6.4 Hz), 2.31-2.22 (2H, m), 1.90-1.80 (2H, m), 1.67-1.60 (2H, m), 1.56-1.45 (2H, m), 1.43-1.33 (2H, m), 0.90 (3H, t, J= 7.4 Hz).

### Step (iii)

### 2-Butoxy-7, 8-dihydro-9-[2-{4-(3-methoxycarbonylmethylphenoxy)piperidin-1-yl}ethyl]-8-oxoadenine

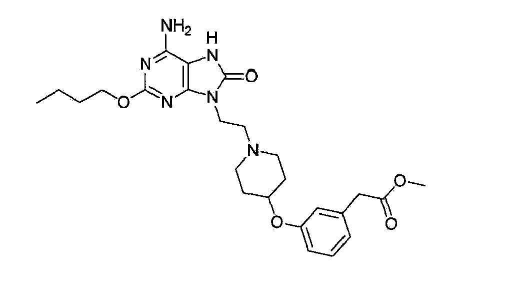

Using the compound 150mg (0.29mmol) obtained in step (ii), in the same manner as example 20 step (vii), there was obtained the subtitled compound 95mg as a white solid. Yield 65%
¹H NMR (DMSO-d₆) δ 9.83 (1H, bs), 7.19 (1H, dd, J= 7.6, 7.6 Hz), 6.82 (1H, s), 6.79 (2H, d, J= 7.6 Hz), 6.39 (2H, bs), 4.35-4.28 (1H, m), 4.13 (1H, t, J= 6.6 Hz), 3.78 (1H, t, J= 6.4 Hz), 3.62 (2H, s), 3.60 (3H, s), 2.78-2.72 (2H, m), 2.59 (2H, t, J= 6.4 Hz), 2.31-2.25 (2H, m), 1.89-1.82 (2H, m), 1.67-1.59 (2H, m), 1.55-1.46 (2H, m), 1.42-1.32 (2H, m), 0.89 (3H, t, J= 7.6 Hz).

### Example 25

### 2-Butoxy-7,8-dihydro-9-[2-{4-(3-methoxycarbonylmethylphenyl)piperazin-1-yl}ethyl] -8-oxoadenine

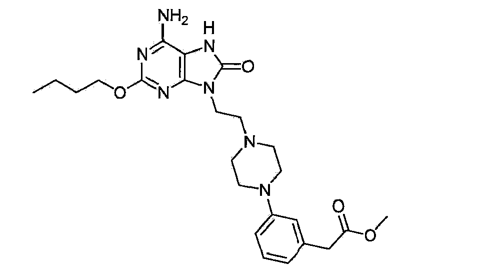

### Step (i)

### N-(3-Methoxycarbonylmethylphenyl)piperazine

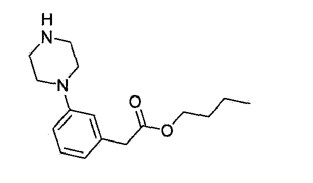

3-Methoxycarbonylmethylaniline 100mg (0.61mmol) was dissolved in butanol 6ml. To the solution was added bis(2-chloroethyl)amine hydrochloride 324mg (1.82mmol) and the mixture was stirred at 140°C for 22 hours. After removal of the solvent by distillation, thereto was added aqueous saturated sodium hydrogencarbonate, and the mixture was extracted with ethyl acetate. The organic layer was washed with water, dried over sodium sulfate and concentrated. The residue was purified by silica gel column chromatography to give the subtitled compound 65mg as a pink oil. Yield 46%
¹H NMR (DMSO-d₆) δ 8.43 (1H, bs), 7.18 (1H, dd, J= 7.6 Hz), 6.87 (1H, s), 6.86 (1H, d, J= 7.6, 7.6 Hz), 6.73 (1H, d, J= 7.6 Hz), 4.02 (2H, t, J= 6.6 Hz), 3.59 (2H, s), 3.30-3.25 (4H, m), 3.15-3.11 (4H, m), 1.57-1.49 (2H, m), 1.33-1.24 (2H, m), 0.86 (3H, t, J= 7.4 Hz).

### Step (ii)

### 2-Butoxy-9-[2-{4-(3-butoxycarbonylmethylphenyl)piperazin-1-yl}ethyl]-8-methoxyadenine

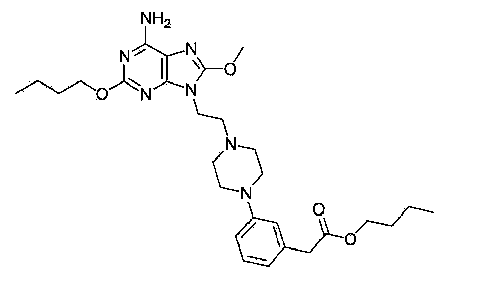

Using the compound 300mg (0.87mmol) obtained by example 25 step (i) and compound 289mg (1.05mmol) obtained in step (i), in the same manner as example 20 step (vi), there was obtained the subtitled compound 200mg as a yellow oil. Yield 43%
¹H NMR (DMSO-d₆) δ 7.12 (1H, t, J= 7.6 Hz), 6.79 (1H, s), 6.79 (1H, d, J= 7.6 Hz), 6.78 (2H, bs), 6.64 (1H, d, J= 7.6 Hz), 4.16 (2H, t, J= 6.6 Hz), 4.25 (3H, s), 4.01 (2H, t, J= 6.6 Hz), 3.98 (2H, t, J= 6.3 Hz), 3.55 (2H, s), 3.06-3.00 (4H, m), 2.65 (2H, t, J= 6.3 Hz), 2.59-2.54 (4H, m), 1.69-1.61 (2H, m), 1.56-1.48 (2H, m), 1.44-1.33 (2H, m), 1.33-1.23 (2H, m), 0.92 (3H, t, J= 7.4 Hz), 0.85 (3H, t, J= 7.4 Hz).

### Step (iii)

### 2-Butoxy-7,8-dihydro-9-[2-{4-(3-methoxycarbonylmethylphenyl)piperazin-1-yl}ethyl]-8-oxoadenine

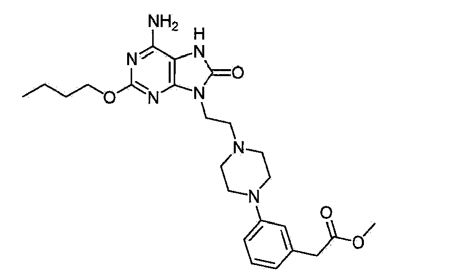

Using the compound 200mg (0.37mmol) obtained in step (ii), in the same manner as example 20 step (vii), there was obtained the subtitled compound 160mg as a white solid. Yield 89%
¹H NMR (DMSO-d₆) δ 9.85 (1H, bs), 7.12 (1H, dd, J= 7.6 Hz), 6.79 (1H, s), 6.78 (1H, d, J= 7.6 Hz), 6.65 (1H, d, J= 7.6 Hz), 6.41 (2H, bs), 4.14 (2H, t, J= 6.6 Hz), 3.82 (2H, t, J= 6.4 Hz), 3.59 (3H, s), 3.57 (2H, s), 3.06-3.01 (4H, m), 2.63 (2H, t, J= 6.4 Hz), 2.59-2.54 (4H, m), 1.68-1.60 (2H, m), 1.44-1.33 (2H, m), 0.91 (3H, t, J= 7.4 Hz).

### Example 26

### 2-Butoxy-7,8-dihydro-9-[2-{4-(3-methoxycarbonylmethylbenzyl)piperazin-1-yl}ethyl] -8-oxoadenine

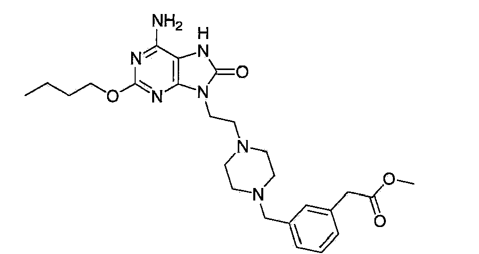

### Step (i)

### 2-Butoxy-8-methoxy-9-[2-{4-(3-methoxycarbonylmethylbenzyl)piperazin-1-yl}ethyl]adenine

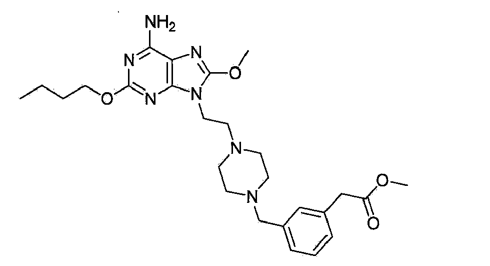

Using the compound 300 mg (0.87mmol) obtained by example 24 step (i), in the same manner as example 20 step (vi), there was obtained the subtitled compound 220mg as a colorless oil. Yield 50%
¹H NMR (DMSO-d₆) δ 7.25 (1H, dd, J= 7.6, 7.6 Hz), 7.16 (1H, s), 7.14 (1H, d, J= 7.6 Hz), 7.12 (1H, d, J= 7.6 Hz), 6.76 (2H, bs), 4.13 (2H, t, J= 6.6 Hz), 4.02 (3H, s), 3.92 (2H, t, J= 6.4 Hz), 3.65 (2H, s), 3.60 (3H, s), 3.38 (2H, s), 2.58 (2H, t, J= 6.4 Hz), 2.48-2.37 (4H, m), 2.33-2.22 (4H, m), 1.67-1.58 (2H, m), 1.42-1.33 (2H, m), 0.90 (3H, t, J= 7.4 Hz).

### Step (i)

### 2-Butoxy-7,8-dihydro-9-[2-{4-(3-methoxycarbonylmethylbenzyl)piperazin-1-yl}ethyl]-8-oxoadenine

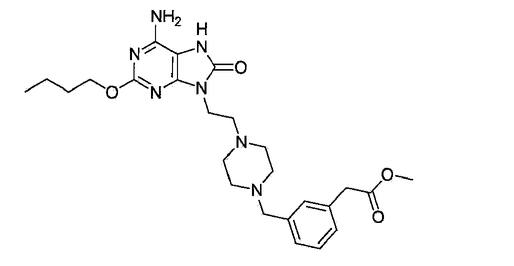

Using compound 215mg (0.42mmol) obtained in step (i), in the same manner as example 20 step (vii), there was obtained the subtitled compound 140mg as a colorless oil. Yield 67%
¹H NMR (DMSO-d₆) δ 9.84 (1H, bs), 7.25 (1H, dd, J= 7.6, 7.6 Hz), 7.16 (1H, s), 7.14 (1H, d, J= 7.6 Hz), 7.12 (1H, d, J= 7.6 Hz), 6.43 (2H, bs), 4.13 (2H, t, J= 6.6 Hz), 4.02 (3H, s), 3.92 (2H, t, J= 6.4 Hz), 3.65 (2H, s), 3.60 (3H, s), 3.38 (2H, s), 2.58 (2H, t, J= 6.4 Hz), 2.48-2.37 (4H, m), 2.33-2.22 (4H, m), 1.67-1.58 (2H, m), 1.42-1.33 (2H, m), 0.90 (3H, t, J= 7.4 Hz).

### Example 27

### 2-Butoxy-7, 8-dihydro-9-[2-{4-(4-methoxycarbonylmethylpyridin-2-yl)piperazin-1-yl}ethyl]-8-oxoadenine

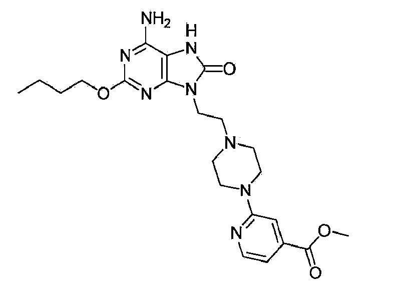

### Step (i)

### N-(4-Methoxycarbonylmethylpyridin-2-yl)piperazine

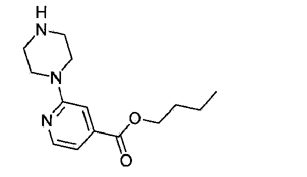

To 4-butoxycarbonyl-2-chloropiridine 1.87g (10.9mmol) in n-butanol were added diisopropylethylamine 5.7ml (32.7mmol) and piperazine 9.38g (109mmol), and the mixture was heated at 110°C for 5 hours. After removal of the solvent by distillation, thereto was added water and the mixture was extracted with ethyl acetate. The organic layer was washed with water, dried and concentrated to give the subtitled compound 1.70g as an orange oil. Yield 59%
¹H NMR (CDCl₃) δ 8.28 (1H, d, J= 5.1 Hz), 7.22 (1H, s), 7.12 (1H, d, J= 5.1 Hz), 3.59-3.54 (4H, m), 3.10-2.97 (4H, m), 1.79-1.71 (2H, m), 1.50-1.42 (2H, m), 0.98 (3H, t, J= 7.4 Hz).

### Step (ii)

### 2-Butoxy-9-[2-{4-(4-butoxycarbonymethylpyridin-2-yl)piperazin-1-yl}ethyl]-8-methoxyadenine

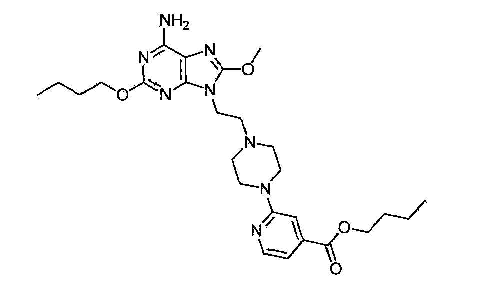

Using the compound 300mg (0.87mmol) obtained by example 24 step (i) and the compound 344mg (1.31mmol) obtained in step (i), in the same manner as example 20 step (vi), there was obtained the subtitled compound 215mg as a yellow oil. Yield 45%
¹H NMR (DMSO-d₆) δ 8.26 (1H, s), 7.16 (1H, s), 7.02 (1H, d, J= 5.1 Hz), 6.76 (2H, bs), 4.27 (2H, t, J= 6.6 Hz), 4.16 (2H, t, J= 6.6 Hz), 4.05 (3H, s), 3.99 (2H, t, J= 6.3 Hz), 3.47-3.42 (4H, m), 2.66 (2H, t, J= 6.3 Hz), 2.56-2.51 (4H, m), 1.72-1.61 (4H, m), 1.45-1.35 (4H, m), 0.92 (6H, t, J= 7.4 Hz).

### Step (iii)

### 2-Butoxy-7,8-dihydro-9-[2-{4-(4-methoxycarbonylmethylpyridin-2-yl) piperazin-1-yl}ethyl]-8-oxoadenine

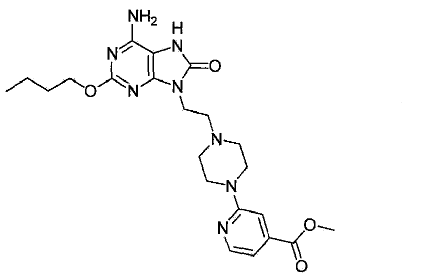

Using the compound 200mg (0.38mmol) obtained in step (ii), in the same manner as example 20 step (vii), there was obtained the subtitled compound 154mg as a white solid. Yield 86%
¹H NMR (DMSO-d₆) δ 9.84 (1H, bs), 8.26 (1H, d, J= 5.1 Hz), 7.18 (1H, s), 7.03 (1H, d, J= 5.1 Hz), 6.40 (2H, s), 4.15 (2H, t, J= 6.6 Hz), 3.86 (3H, s), 3.83 (2H, t, J= 6.4 Hz), 3.48-3.42 (4H, m), 2.64 (2H, t, J= 6.4 Hz), 2.57-2.52 (4H, m), 1.68-1.60 (2H, m), 1.44-1.34 (2H, m), 0.91 (3H, t, J= 7.4 Hz).

### Example 28

### 2-Butoxy-7,8-dihydro-9-(3-{4-[3-(2-methoxy-2-oxoethyl)phenoxy]piperidin-1-yl}propyl)-8-oxoadenine

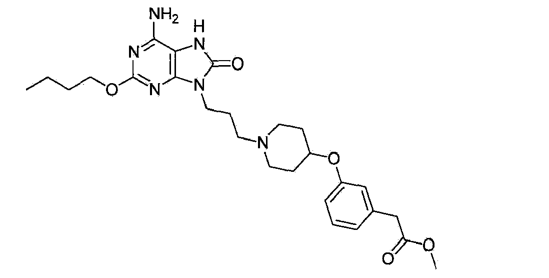

### Step (i)

### 9-(3-Bromopropyl)-2-butoxy-8-methoxyadenine

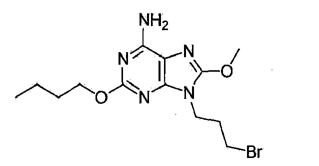

Using 2-butoxy-8-methoxyadenine 2.00g (8.43mmol), in the same manner as example 20 step (v), there was obtained the subtitled compound 0.75g as a white solid. Yield 25%
¹H NMR (CDCl₃) δ 5.21 (2H, brs), 4.28 (2H, t, J= 6.6 Hz), 4.12 (3H, s), 4.09 (2H, t, J= 6.6 Hz), 3.38 (2H, t, J= 6.6 Hz), 2.36-2.32 (2H, m), 1.79-1.73 (2H, m), 1.52-1.46 (2H, m), 0.96 (3H, t, J= 7.4 Hz).

### Step (ii)

### 2-Butoxy-8-methoxy-9-(3-{4-[3-(2-methoxy-2-oxoethyl)phenoxy]piperidin-1-yl}propyl) adenine

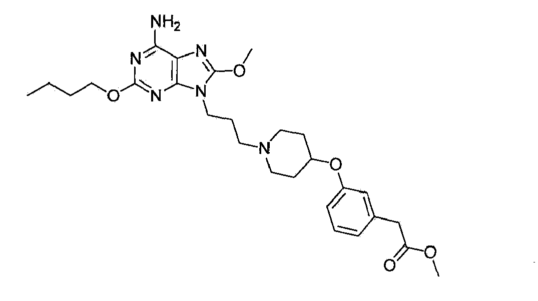

Using the compound 150mg (0.42mmol) obtained in step (i), in the same manner as example 20 step (vi), there was obtained the subtitled compound 98 mg as a white solid. Yield 44%
¹H NMR (CDCl₃) δ 7.21 (1H, t, J= 6.8 Hz), 6.84-6.78 (3H, m), 5.14 (2H, brs), 4.29 (1H, m), 4.27 (2H, t, J= 6.7 Hz), 4.11 (3H, s), 3.99 (2H, t, J= 7.0 Hz), 3.69 (3H, s), 3.58 (2H, s), 2.72-2.64 (2H, m), 2.39 (2H, t, J= 7.4 Hz), 2.28-2.20 (2H, m), 2.05-1.91 (4H, m), 1.80-1.72 (4H, m), 1.51-1.45 (2H, m), 0.96 (3H, t, J= 7.4 Hz).

### Step (iii)

### 2-Butoxy-7, 8-dihydro-9-(3-{4-[3-(2-methoxy-2-oxoethyl)phenoxy]piperidin-1-yl}propyl)-8-oxoadenine

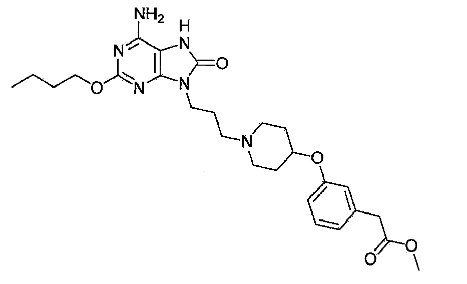

Using the compound 98mg (0.19mmol) obtained in step (ii), in the same manner as example 20 step (vii), there was obtained the titled compound 72mg as a white solid. Yield 76%
¹H NMR (DMSO-d₆) δ 9.84 (1H, brs), 7.20 (1H, t, J= 6.8 Hz), 6.83-6.78 (3H, m), 6.39 (2H, brs), 4.31 (1H, m), 4.15 (2H, t, J= 6.6 Hz), 3.73 (2H, t, J= 6.9 Hz), 3.63 (2H, s), 3.60 (3H, s), 2.67-2.59 (2H, m), 2.31 (2H, t, J= 6.8 Hz), 2.18-2.10 (2H, m), 1.89-1.77 (4H, m), 1.65-1.48 (4H, m), 1.41-1.34 (2H, m), 0.91 (3H, t, J= 7.4 Hz).

### Example 29

### 2-Butoxy-7,8-dihydro-9-{4-[4-(3-methoxycarbonylmethylphenoxy)piperazin-1-yl]butyl}-8-oxoadenine

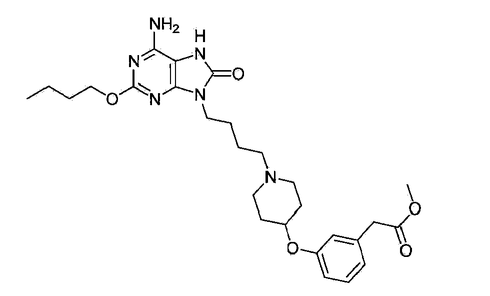

### Step (i)

### 9-(4-Bromobutyl)-2-butoxy-8-methoxyadenine

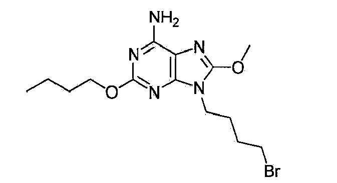

Using 2-butoxy-8-methoxyadenine 300mg (1.26mmol), in the same as example 20 step (v), there was obtained the titled compound 378mg as a white solid. Yield 81%
¹H NMR (CDCl₃) δ5.18 (2H, brs), 4.27 (2H, t, J= 6.6 Hz), 4.12 (3H, s),3.97 (2H, t, J= 6.7 Hz), 3.44 (2H, t, J= 6.5 Hz), 1.94-1.85 (4H, m), 1.78-1.75 (2H, m), 1.52-1.47 (2H, m), 0.97 (3H, t, J= 7.4 Hz).

### Step (ii)

### 2-Butoxy-8-methoxy-9-{4-[4-(3-methoxycarbonylmethylphenoxy)piperidin-1-yl]butyl}adenine

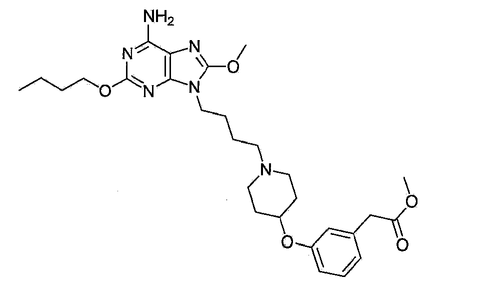

Using 9-(4-bromobutyl)-2-butoxy-8-methoxyadenine 200mg (0.54mmol), in the same manner as example 20 step (vi), there was obtained the subtitled compound 150mg as a pale yellow oil. Yield 52% ¹H NMR (CDCl₃) δ 7.22 (1H, t, J= 7.8Hz), 6.85-6.79 (3H, m), 5.12 (2H, brs), 4.32-4.26 (3H, m), 4.11 (3H, s), 3.95 (2H, t, J= 7.1 Hz), 3.69 (3H, s), 3.59 (2H, s), 2.70 (2H, m), 2.37 (2H, m), 2.62 (2H, m), 1.97 (2H, m), 1.82-1.73 (6H, m), 1.54-1.44 (4H, m), 0.96 (3H, t, J= 7.4 Hz).

### Step (iii)

### 2-Butoxy-7,8-dihydro-9-{-4-[4-(3-methoxycarbonylmethylphenoxy)piperazin-1-yl]butyl}-8-oxoadenine

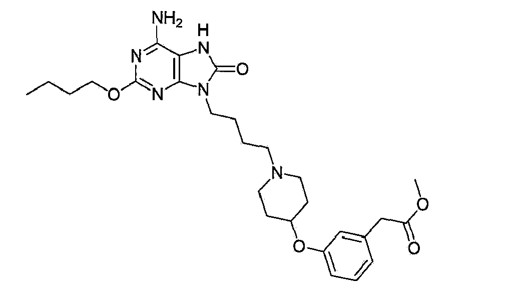

Using the compound 150mg (0.28mmol) obtained in step (ii), in the same manner as example 20 step (vii), there was obtained the titled compound 126mg as a white solid. Yield 86%
¹H NMR (DMSO-d₆) δ 9.84 (1H, brs), 7.20 (1H, t, J= 7.5Hz), 6.83-6.80 (3H, m), 6.40 (2H, brs), 4.32 (1H, m), 4.14 (2H, t, J= 6.6 Hz), 3.68 (2H, t, J= 7.0 Hz), 3.63 (2H, s), 3.60 (3H, s), 2.64 (2H, m), 2.29 (2H, m), 2.15 (2H, m), 1.88 (2H, m), 1.68-1.55 (6H, m), 1.42-1.36 (4H, m), 0.91 (3H, t, J= 7.4 Hz).

### Example 30

### 2-Butoxy-7, 8-dihydro-9-{4-[4-(3-methoxycarbonylmethylbenzyl)piperazin-1-yl]butyl}-8-oxoadenine

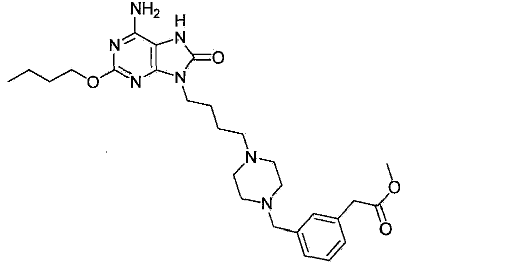

### Step (i)

### 2-Butoxy-8-methoxy-9-{4-[4-(3-methoxycarbonylmethylbenzyl)piperazin-1-yl]butyl}adenine

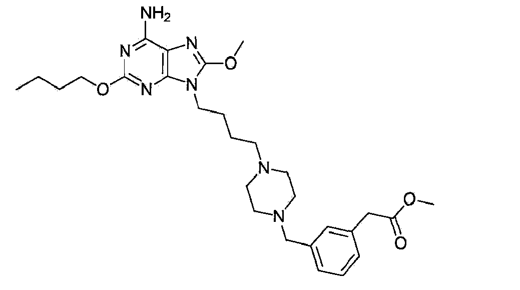

Using 1-(3-methoxycarbonylmethylbenzyl)piperazine hydrochloride 306mg (1.08mmol) and 9-(4-bromobutyl)-2-butoxy-8-methoxyadenine 200mg (0.54mmol), as the same manner as example 20 step (vi), there was obtained the subtitled compound 175mg as a pale yellow oil. Yield 60%
¹H NMR (CDCl₃) δ 7.29 (4H, m), 5.12 (2H, s), 4.27 (2H, t, J= 6.7 Hz), 4.10 (3H, s), 3.93 (2H, t, J= 7.1 Hz), 3.69 (3H, s), 3.62 (2H, s), 3.48 (2H, s), 2.56-2.39 (8H, m), 2.34 (2H, t, 7.6Hz), 1.79-1.72 (4H, m), 1.51-1.44 (4H, m), 0.96 (3H, t, J= 7.4 Hz).

### Step (ii)

### 2-Butoxy-7,8-dihydro-9-{4-[4-(3-methoxycarbonylmethylbenzyl)piperazin-1-yl] butyl}-8-oxoadenine

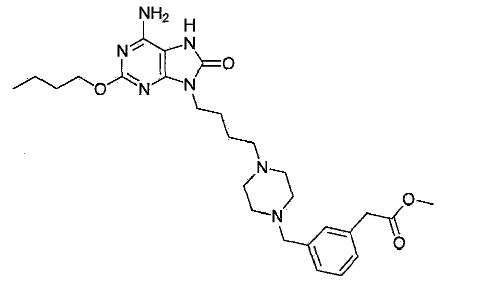

Using the compound 175mg (0.35mmol) obtained in step (i), in the same manner as example 20 step (vii), there was obtained the titled compound 153mg as a white solid. Yield 90%
¹H NMR (DMSO-d₆) δ 9.84 (1H, s), 7.28 (1H, t, J= 6.6Hz), 7.17-7.12 (3H, m), 6.40 (2H, brs), 4.14 (2H, t, J= 6.6 Hz), 3.68-3.65 (4H, m), 3.61 (3H, s), 3.41 (2H, s), 2.41-2.20 (10H, m), 1.68-1.60 (4H, m), 1.43-1.34 (4H, m), 0.91 (3H, t, J= 7.4 Hz).

### Example 31

### 2-Butoxy-7,8-dihydro-9-{4-[4-(4-methoxycarbonylbenzyl)piperidin-1-yl] butyl}-8-oxoadenine

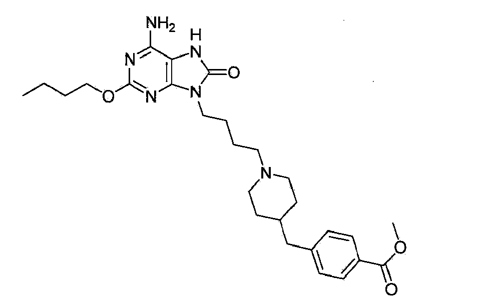

### Step (i)

### 2-Butoxy-8-methoxy-9-{4-[4-(4-methoxycarbonylbenzyl)piperidin-1-yl]butyl})butyl)adenine

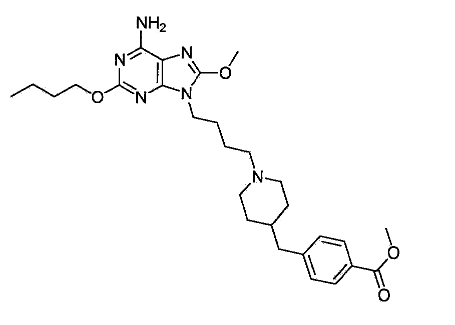

Using 4-(4-Methoxycarbonylbenzyl)piperadine hydrochloride 217mg (0.81mmol) and 9-(4-bromobutyl)-2-butoxy-8-methoxyadenine 200mg (0.54mmol), in the same manner as example 20 step (vi), there was obtained the subtitled compound 257mg as a pale yellow oil. Yield 91% ¹H NMR (CDCl₃) δ 7.98 (2H, d, J= 8.2Hz), 7.22 (2H, d, J= 8.2Hz), 5.13 (2H, brs), 4.26 (2H, t, J= 6.6 Hz), 4.13 (3H, s), 3.96 (2H, t, J= 6.8 Hz), 3.92 (3H, s), 3.45 (2H, m), 2.95 (2H, m), 2.66 (2H, m), 2.52 (2H, m), 2.10 (2H, m), 1.77-170 (7H, m), 1.53-1.44 (2H, m), 0.97 (3H, t, J= 7.4 Hz).

### Step (ii)

### 2-Butoxy-7,8-dihydro-9-{4-[4-(4-methoxycarbonylbenzyl)piperidin-1-yl]-8-oxoadenine}

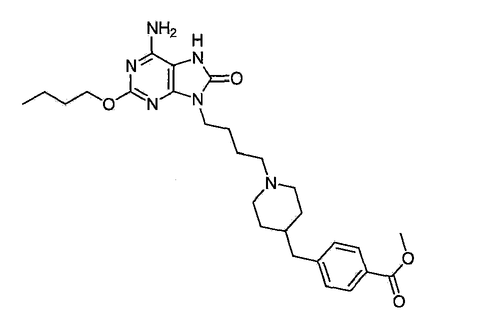

Using the compound 257mg (0.49mmol) obtained in step (i), in the same manner as example 20 step (vii), there was obtained the titled compound 214mg as a white solid. Yield 85%
¹H NMR (DMSO-d₆) δ9.83 (1H, s), 7.87 (2H, d, J= 8.2Hz), 7.30 (2H, d, J= 8.2Hz), 6.41 (2H, brs), 4.14 (2H, t, J= 6.6 Hz), 3.83 (3H, s), 3.65 (2H, t, J= 6.8 Hz), 2.75 (2H, m), 2.55 (2H, d, J= 6.6Hz), 2.22 (2H, m), 1.75 (2H, m), 1.66-1.60 (4H, m), 1.47 (2H, m), 1.41-1.35 (4H, m), 1.49 (2H, m), 0.91 (3H, t, J= 7.4 Hz).

### Example 32

### 2-Butoxy-7,8-dihydro-9-[1-(5-methoxycarbonylfuran-2-ylmethyl)piperidin-4-ylmethyl]-8-oxoadenine

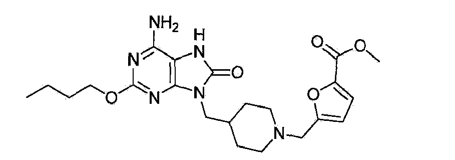

### Step (i)

### 8-Bromo-2-butoxy-9-(1-tert-butoxycarbonylpiperidin-4-ylmethyl)adenine

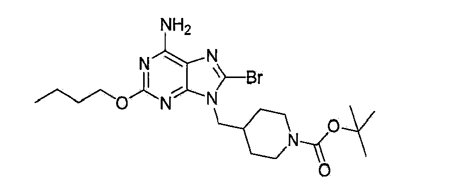

To 8-bromo-2-butoxyadenine 0.30g (1.05mmol) in DMF 30ml were added potassium carbonate 0.19g (1.40mmol) and 4-(methanesulufonyloxymethyl)-piperidine-1-carboxylic acid tert-butyl ester 0.92g (3.15mmol) obtained by example 1 step (i), and the mixture was stirred at 120°C for 5 hours. After removal of the solvent by distillation, the residue was extracted with chloroform. The organic layer was concentrated and purified by silica gel column chromatography to give the subtitled compound 0.37g as a pale yellow solid. Yield 74%
¹H NMR (CDCl₃) δ 5.95 (2H, brs), 4.30 (2H, t, J = 6.6 Hz), 4.11 (2H, m), 4.00 (2H, d, J = 7.4 Hz), 2.66 (2H, m), 2.12 (1H, m), 1.78 (2H, m), 1.54 (4H, m), 1.46 (9H, s), 1.29 (2H, m), 0.97 (3H, t, J = 7.3 Hz).

### Step (ii)

### 2-Butoxy-8-chloro-9-[1-(5-ethoxycarbonylfuran-2-ylmethyl)piperidin-4-ylmethyl] adenine

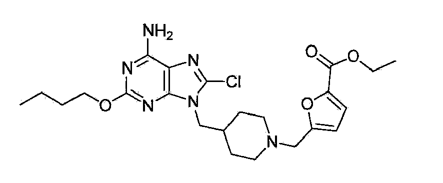

To 8-bromo-2-butoxy-9-(1-tert-butoxycarbonylpiperidin-4-ylmethyl)adenine 0.15g (0.30mmol) obtained in step (i) was added 4N hydrochloric acid-dioxane 5ml and the mixture was stirred for 30 minutes. After removal of the solvent by distillation, thereto were added potassium carbonate 68mg (0.49mmol) and 2-ethoxycarbonyl-5-bromomethylfuran 0.14g (0.74mmol) in DMF 6ml and the mixture was stirred at room temperature for 2 hours. After removal of the solvent in vacuo, the residue was extracted with chloroform. The extract was dried over magnesium sulfate and the solvent was removed in vacuo. The residue was purified by silica gel column chromatography to give the subtitled compound 0.11g as a colorless liquid. Yield 67%
¹H NMR (DMSO-d₆) δ 7.35 (2H, brs), 7.21 (1H, d, J = 3.4 Hz), 6.48 (1H, d, J = 3.4 Hz), 4.26 (2H, q, J = 7.1 Hz), 4.20 (2H, t, J = 6.6 Hz), 3.92 (2H, d, J = 7.3 Hz), 3.53 (2H, s), 2.80 (2H, m), 1.93 (2H, m), 1.83 (1H, m), 1.67 (2H, m), 1.48 (2H, m), 1.39 (2H, m), 1.30 (2H, m), 1.28 (3H, t, J = 7.1 Hz), 0.91 (3H, t, J = 7.3 Hz).

### Step (iii)

### 2-Butoxy-7,8-dihydro-9-[1-(5-methoxycarbonylfuran-2-ylmethyl)piperidin-4-ylmethyl] -8-oxoadenine

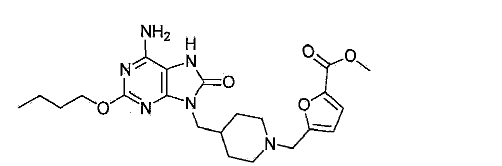

2-Butoxy-8-chloro-9-[1-(5-ethoxycarbonylfuran-2-ylmethyl)piperidin-4-ylmethyl]adenine 0.11g obtained in step (ii) was suspended in methanol 10ml and thereto was added 5M aqueous sodium hydroxide 10ml, followed by stirring under reflux for 7 hours. After neutralized with concentrated hydrochloric acid, the solvent was evaporated to dryness. Thereto were added methanol 20ml and concentrated sulfuric acid 0.5 ml, and the mixture was stirred under reflux for 4 hours. After being cooled to 0°C, the mixture was neutralized with aqueous saturated sodium bicarbonate. The resulting solid was filtered and washed with water to give the titled compound 0.72g as a white solid.
Yield 77%
¹H NMR (DMSO-d₆) δ 9.86 (1H, s), 7.23 (1H, d, J = 3.4 Hz), 6.48 (1H, d, J = 3.4 Hz), 6.41 (2H, brs), 4.13 (2H, t, J = 6.6 Hz), 3.78 (3H, s), 3.53 (2H, d, J = 7.3 Hz), 3.52 (2H, s), 2.77 (2H, m), 1.91 (2H, m), 1.76 (1H, m), 1.63 (2H, m), 1.50 (2H, m), 1.39 (2H, m), 1.23 (2H, m), 0.90 (3H, t, J = 7.3 Hz).

### Example 33

### 2-Butoxy-7,8-dihydro-9-[5-{4-(3-methoxylcarbonylmethylphenyl)piperazin-1-yl}pentyl]-8-oxoadenine

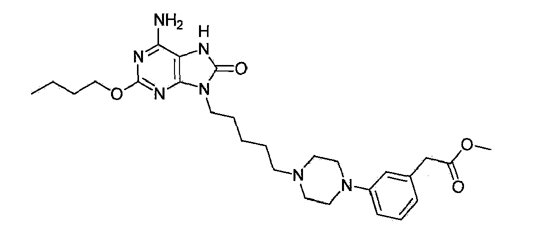

### Step (i)

### 9-(5-Bromopentyl)-2-butoxy-8-methoxyadenine

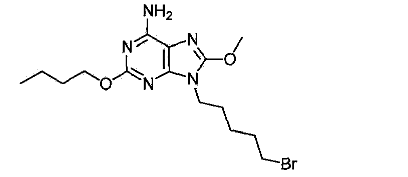

To 2-butoxy-8-methoxyadenine 2.00g (8.43mmol) in DMF (30ml) were added potassium carbonate 1.40g (10.1mmol) and 1,5-dibromopentane 3.87g (16.9mmol), and the mixture was stirred at room temperature for 6 hours. After removal of the solvent by distillation, thereto was added water 80ml and the mixture was extracted with 5% methanol-chloroform (100ml). The organic layer was washed with water and saturated brine, successively and dried over sodium sulfate, followed by concentration in vacuo. The residue was purified by silica gel column chromatography to give the subtitled compound 1.69g as a pale pink solid.
Yield 52%
¹H NMR (DMSO-d₆) δ 6.78 (2H, bs), 4.16 (2H, t, J= 6.6 Hz), 4.03 (3H, s), 3.84 (2H, t, J= 6.8 Hz), 1.86-1.78 (2H, m), 1.74-1.60 (4H, m), 1.45-1.35 (2H, m), 1.35-1.28 (2H, m), 0.92 (3H, t, J= 7.4 Hz).

### Step (ii)

### 2-Butoxy-9-[5-{4-(3-butoxylcarbonylmethylphenyl)piperazin-1-yl}pentyl]-8-methoxyadenine

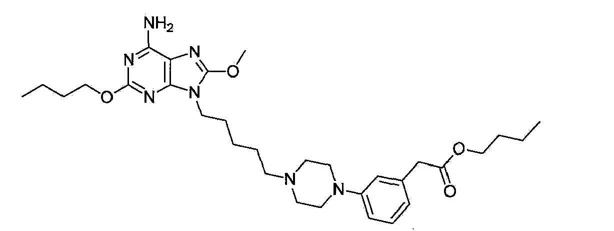

Using the compound 200mg (0.52mmol) obtained in step (i) and 1-[3-(butoxycarbonylmethyl)phenyl]piperazine) 215mg (0.78mmol), in the same manner as example 20 step (vi), there was obtained the subtitled compound 215mg as a yellow oil. Yield 71%
¹H NMR (DMSO-d₆) δ 7.13 (1H, t, J= 7.6 Hz), 6.80 (1H, s), 6.79 (1H, d, J= 7.6 Hz), 6.78 (2H, bs), 6.65 (1H, d, J= 7.6 Hz), 4.16 (1H, d, J= 6.6 Hz), 4.05 (3H, s), 4.01 (2H, t, J= 6.6 Hz), 3.85 (2H, t, J= 6.8 Hz), 3.57 (2H, s), 3.09-3.02 (4H, m), 2.46-2.40 (4H, m), 2.28-2.20 (2H, t, J= 7.2 Hz), 1.76-1.67 (2H, m), 1.67-1.60 (2H, m), 1.56-1.49 (2H, m), 1.50-1.41 (2H, m), 1.42-1.33 (2H, m), 1.33-1.22 (2H, m), 1.25-1.19 (2H, m), 0.91 (3H, t, J= 7.4 Hz), 0.85 (3H, t, J= 7.4 Hz).

### Step (ii)

### 2-Butoxy-7, 8-dihydro-9-[5-{4-(3-methoxylcarbonylmethylphenyl)piperazin-1-yl}pentyl]-8-oxoadenine

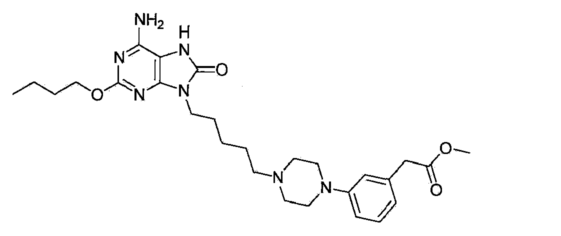

Using the compound 210mg (0.36mmol) obtained in step (i), in the same manner as example 20 step (vii), there was obtained the subtitled compound 180mg as a yellow oil. Yield 95%
¹H NMR (DMSO-d₆) δ 9.85 (1H, bs), 7.13 (1H, dd, J= 7.6 Hz), 6.81 (1H, s), 6.79 (1H, d, J= 7.6 Hz), 6.65 (1H, t, J= 7.6 Hz), 6.41 (2H, bs), 4.14 (2H, t, J= 6.6 Hz), 3.66 (2H, t, J= 6.8 Hz), 3.60 (3H, s), 3.58 (2H, s), 3.09-3.04 (4H, m), 2.47-2.41 (4H, m), 2.26 (2H, t, J= 7.3 Hz), 1.71-1.59 (4H, m), 1.52-1.42 (2H, m), 1.42-1.32 (2H, m), 1.30-1.20 (2H, m), 0.91 (3H, t, J= 7.4 Hz).

### Example 34

### 7,8-Dihydro-2-(2-methoxyethoxy)-9-[2-{4-(3-methoxylcarbonylmethylbenzyl)piperazin-1-yl}ethyl]-8-oxoadenine

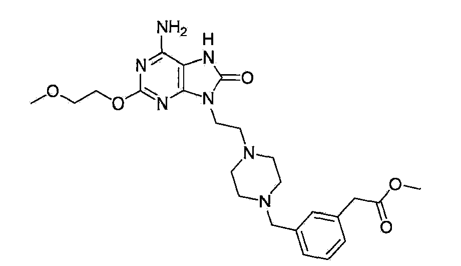

In the same manner as example 26, there was obtained the titled compound as a white solid.
¹H NMR (DMSO-d₆) δ 9.84 (1H, brs), 7.25 (1H, dd, J = 7.6, 7.6 Hz), 7.16 (1H, s), 7.14 (1H, d, J = 7.6 Hz), 7.12 (1H, d, J = 7.6 Hz), 6.43 (2H, brs), 4.24 (2H, t, J = 6.6 Hz), 3.76 (2H, t, J = 6.4 Hz), 3.65 (2H, s), 3.60 (3H, s), 3.59-3.57 (2H, m), 3.39 (2H, s), 3.27 (3H, s), 2.59-2.54 (2H, m), 2.49-2.37 (4H, m), 2.33-2.22 (4H, m).

### Example 35

### 2-Butoxy-7,8-dihydro-9-{[2-(3-{N-[(3-hydroxylcarbonylmethylphenyl-1-yl)methyl]-N-methylamino}propyl)piperidin-4-yl]methyl}-8-oxoadenine

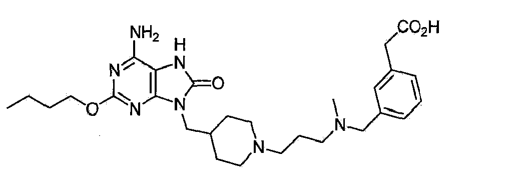

To the compound 53.6mg (0.0097mmol) obtained by example 1 was added 2N aqueous sodium hydroxide (5ml) and the mixture was refluxed for 1.5 hours. After neutralized with 1N hydrochloric acid, the resulting solid was filtered to give the titled compound 32mg as a white solid. Yield 61%
¹H NMR (DMSO-d₆) δ 10.96 (1H, bs), 7.21-7.04 (4H, m), 6.90 (2H, brs), 4.13 (2H, t, J= 6.6 Hz), 3.53 (2H, s), 3.51 (2H, s), 3.50-3.30 (2H, m), 3.37 (3H, s), 2.79-2.75 (2H, m), 2.29 (2H, t, J= 7.0 Hz), 2.22 (2H, t, J= 7.1 Hz), 2.07 (3H, s), 1.76-1.34 (10H, m), 1.20-1.04 (2H, m), 0.91 (3H, t, J= 7.3 Hz).

### Example 36

### 2-Butoxy-7,8-dihydro-9-(1-{2-[3-(hydoroxycarbonylmethyl)phenoxy]ethyl}piperidin-4-ylmethyl)-8-oxoadenine

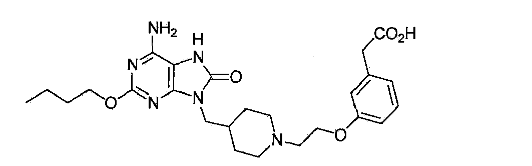

To the compound 76.7mg (0.150mmol) obtained by example 6 was added 2N aqueous sodium hydroxide (5 ml) and the mixture was refluxed for 2 hours. After neutralized with 1N hydrochloric acid, the resulting solid was filtered to give the titled compound 57.4mg as a white solid.
Yield 77%
¹H NMR (DMSO-d₆) δ 9.96 (1H, bs), 7.22 (1H, t, J= 7.8 Hz), 6.85-6.82 (3H, m), 6.47 (2H, bs), 4.18-4.04 (2H, m), 4.14 (2H, t, J= 6.6 Hz), 3.57 (2H, d, J= 6.8 Hz), 3.53 (2H, s), 3.40-3.22 (7H, m), 1.96-1.80 (1H, m), 1.68-1.54 (4H, m), 1.40-1.24 (4H, m), 0.92 (3H, t, J= 7.4 Hz).

### Example 37

### 2-Butoxy-7,8-dihydro-9-{1-[3-(N-{2-[3-(hydroxycarbonylmethyl)phenoxy]ethyl}-N-methylamino)propyl]piperidin-4-ylmethyl}-8-oxoadenine

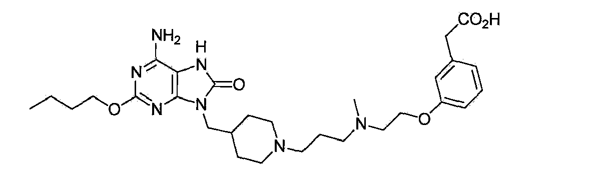

To the compound 31.4mg (0.0538mmol) obtained by example 7 was added 2N aqueous sodium hydroxide (4ml) and the mixture was refluxed for an hour. After neutralized with 1N hydrochloric acid, the solvent was removed by distillation. After adding a small amount of water, the resulting suspension was centrifuged to give the titled compound 34.4mg as a white solid. Yield 100%
¹H NMR (DMSO-d₆) δ11.35 (1H, brs), 7.15-7.07 (1H, m), 6.92-6.60 (5H, m), 4.13 (2H, t, J= 6.5 Hz), 3.97 (2H, t, J= 5.7 Hz), 3.53 (2H, d, J= 6.6 Hz), 3.52-3.21 (5H, m), 2.80-2.76 (2H, m), 2.66 (2H, t, J= 5.7 Hz), 2.36 (2H, t, J= 7.2 Hz), 2.28-2.13 (1H, m), 2.20 (3H, s), 1.86-1.34 (9H, m), 1.23-1.08 (2H, m), 0.91 (3H, t, J= 7.3 Hz).

### Example 38

### 9-(1-{2-[3-(Carboxymethyl)phenoxy]ethyl}piperielin-4-ylethyl)-7,8-dihydro-2-(2-methoxyethoxy)-8-oxoadenine

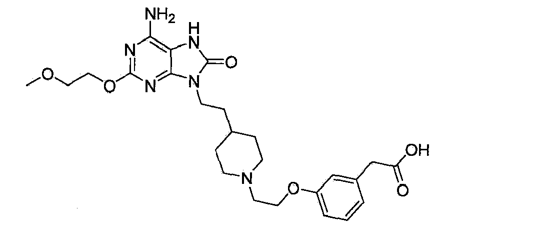

Using the compound 88mg (0.17mmol) obtained by example 8, in the same manner as example 35, there was obtained the titled compound 66mg as a white solid. Yield 77%
¹H NMR (DMSO-d₆) δ 11.79 (1H, brs), 7.09 (1H, dd, J = 7.9 Hz, 7.4 Hz), 7.00 (2H, brs), 6.84 (1H, s), 6.75 (1H, d, J = 7.4 Hz), 6.66 (1H, d, J = 7.9 Hz), 4.24 (2H, t, J = 4.6 Hz), 3.98 (2H, t, J = 5.6 Hz), 3.68 (2H, t, J = 6.5 Hz), 3.59 (2H, t, J = 4.6 Hz), 3.27 (3H, s), 3.21 (2H, s), 2.87-2.83 (2H, m), 2.62 (2H, t, J = 5.5 Hz), 1.96-1.88 (2H, m), 1.70-1.65 (2H, m), 1.56-1.53 (2H, m), 1.14-1.09 (3H, m).

### Example 39

### 2-Butoxy-7,8-dihydro-9-(1-(2-[3-(carboxymethyl)phenoxy]ethyl)piperidin-4-ylethyl)-8-oxoadenine

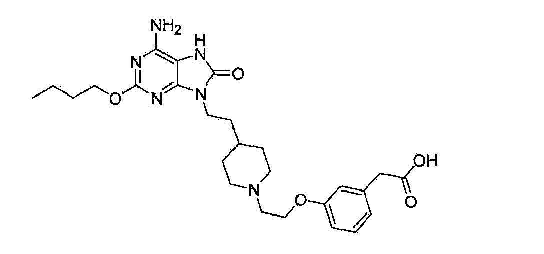

Using compound 0.17g (0.31mmol) obtained by example 11, in the same manner as example 35, there was obtained the titled compound 0.15g as a white solid. Yield 86%
¹H NMR (DMSO-d₆) δ 10.05 (1H, s), 7.20 (1H, dd, J = 7.9 Hz, 7.7 Hz), 6.82-6.79 (3H, m), 6.50 (2H, brs), 4.14 (2H, t, J = 6.6 Hz), 4.04 (2H, t, J = 5.8 Hz), 3.69 (2H, t, J = 6.8 Hz), 3.51 (2H, s), 2.94-2.90 (2H, m), 2.70-2.66 (2H, m), 2.10-1.98 (2H, m), 1.75-1.55 (6H, m), 1.42-1.32 (2H, m), 1.18-1.13 (3H, m), 0.91 (3H, t, J = 7.4 Hz).

### Example 40

### 2-Butoxy-7,8-dihydro-9-{1-[3-([N-methyl-N-{2-[3-(carboxymethyl)phenoxy]ethyl}]amino)propyl]piperidin-4-ylethyl}-8-oxoadenine

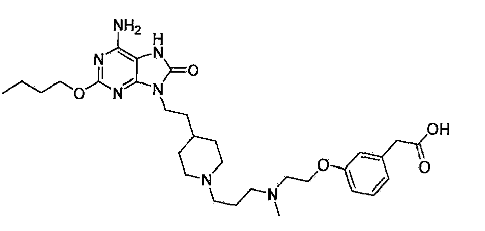

Using the compound 83 mg (0.14mmol) obtained by example 12, in the same manner as example 35, there was obtained the titled compound 43mg as a white solid. Yield 53%
¹H NMR (DMSO-d₆) δ 10.17 (1H, brs), 7.21 (1H, dd, J = 8.0 Hz, 7.6 Hz), 6.85-6.79 (3H, m), 6.57 (2H, brs), 4.13 (2H, t, J = 6.6 Hz), 4.06 (2H, t, J = 5.8 Hz), 3.70 (2H, t, J = 6.7 Hz), 3.51 (2H, s), 3.27-3.23 (2H, m), 2.85-2.80 (2H, m), 2.78 (2H, t, J = 5.6 Hz), 2.60-2.50 (4H, m), 2.30 (3H, s), 1.90-1.86 (2H, m), 1.79-1.73 (2H, m), 1.66-1.57 (4H, m), 1.42-1.33 (5H, m), 0.91 (3H, t, J = 7.4 Hz).

### Example 41

### 7,8-Dihydro-(9-(1-{[3-(hydroxycarbonylmethyl)phenyl]aminocarbonylmethyl}piperidin-4-ylethyl)-2-(2-methoxyethoxy)-8-oxoadenine

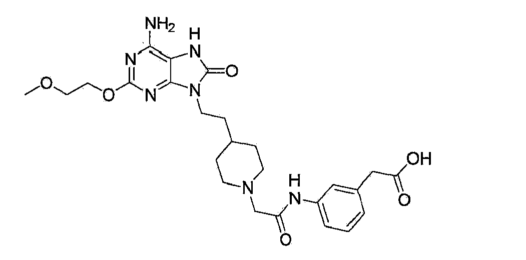

Using the compound 61mg (0. 11mmol) obtained by example 13, in the same manner as example 35, there was obtained the titled compound 25mg as a white solid. Yield 43%
¹H NMR (DMSO-d₆) δ 12.42 (1H, brs), 9.90 (1H, brs), 9.65 (1H, s), 7.54-7.52 (2H, m), 7.23 (1H, dd, J = 8.6 Hz, 7.6 Hz), 6.94 (1H, d, J = 7.6 Hz), 6.45 (2H, brs), 4.26 (2H, t, J = 4.7 Hz), 3.71 (2H, t, J = 6.9 Hz), 3.61-3.57 (2H, m), 3.52 (2H, s), 3.28 (3H, s), 3.08 (2H, brs), 2.86-2.82 (2H, m), 2.12-2.06 (2H, m), 1.75-1.70 (2H, m), 1.63-1.57 (2H, m), 1.30-1.10 (3H, m).

### Example 42

### 2-Butoxy-7,8-dihydro-9-(1-{[3-(hydroxycarbonylmethyl)phenyl]aminocarbonylmethyl}piperidin-4-ylethyl)-8-oxoadenine

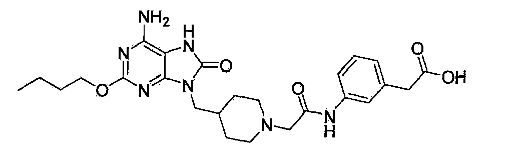

Using compound 26mg (0.050mmol) obtained by example 14, in the same manner as example 35, there was obtained the titled compound 19mg as a white solid. Yield 73%
¹H NMR (DMSO-d₆) δ 10.33 (1H, brs), 9.61 (1H, s), 7.52 (1H, d, J = 8.3 Hz), 7.49 (1H, s), 7.21 (1H, dd, J = 8.3 Hz, 7.6 Hz), 6.93 (1H, d, J = 7.6 Hz), 6.56 (2H, brs), 4.14 (2H, t, J = 6.6 Hz), 3.57 (2H, d, J = 7.1 Hz), 3.46 (2H, s), 3.05 (2H, s), 2.85-2.80 (2H, m), 2.10-2.04 (2H, m), 1.85-1.74 (1H, m), 1.66-1.58 (2H, m), 1.54-1.50 (2H, m), 1.41-1.33 (4H, m), 0.91 (3H, t, J = 7.4 Hz).

### Example 43

### 2-Butoxy-7,8-dihydro-9-{1-[(N-{2-[3-(hydroxylcarbonylmethyl)phenoxy]ethyl}-N-methyl)aminomethylacarbonyl]piperidin-4-ylmethyl}-8-oxoadenine

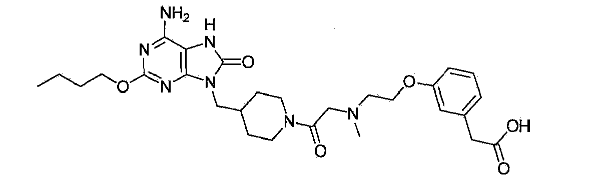

To the compound 100mg (0.17mmol) obtained by example 15 was added 1N aqueous sodium hydroxide (3ml) and the mixture was refluxed for 1.5 hours. After neutralized with concentrated hydrochloric acid, the resulting solid was filtered to give the titled compound 80mg as a white solid. Yield 82%
¹H NMR (DMSO-d₆) δ 10.06 (1H, brs), 9.68 (1H, brs), 7.24 (1H, t, J = 7.6 Hz), 6.87-6.83 (3H, m), 6.51 (2H, brs), 4.25-4.21 (3H, m), 4.13 (2H, t, J = 6.6 Hz), 3.78-3.65 (1H, m), 3.56 (2H, d, J = 7.2 Hz), 3.54 (2H, s), 2.96 (1H, t, J = 12.9 Hz), 2.78-2.65 (2H, m), 2.62-2.55 (1H, m), 2.50 (3H, s), 2.10-2.02 (1H, m), 1.67-1.56 (4H, m), 1.43-1.33 (2H, m), 1.25-1.12 (1H, m), 1.12-0.97 (1H, m), 0.91 (3H, t, J = 7.4 Hz).

### Example 44

### 7, 8-Dihydro-9-{1-[(N-{2-[3-(hydroxylcarbonylmethyl)phenoxy]ethyl}-N-methyl)aminomethylacarbonyl]piperidin-4-ylmethyl}-2-(2-methoxyethoxy)-8-oxoadenine

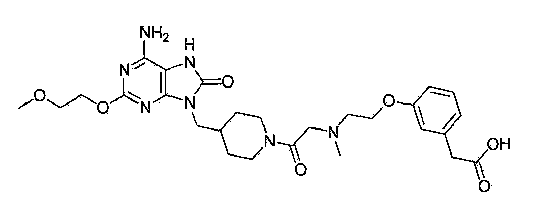

In the same manner as example 35, there was obtained the titled compound as a white solid. Yield 61 %
¹H NMR (DMSO-d₆) δ 9.94 (1H, brs), 9.66 (1H, brs), 7.29-7.23 (1H, m), 6.90-6.83 (3H, m), 6.43 (2H, brs), 4.48-4.41 (2H, m), 4.41-4.28 (4H, m), 4.28-4.22 (2H, m), 3.61-3.52 (3H, m), 3.29 (3H, s), 3.06-2.95 (1H, m), 2.95-2.87 (3H, m), 2.69-2.52 (2H, m), 2.50 (3H, s), 2.13-2.04 (1H, m), 1.65-1.59 (2H, m), 1.26-1.16 (1H, m), 1.11-1.00 (1H, m).

### Example 45

### 7,8-Dihydro-9-{1-[(N-{2-[3-(hydroxylcarbonylmethyl)phenoxy]ethyl}-N-methyl)aminocarbonylmethyl]piperidin-4-ylmethyl}-2-(2-methoxyethoxy)-8-oxoadenine

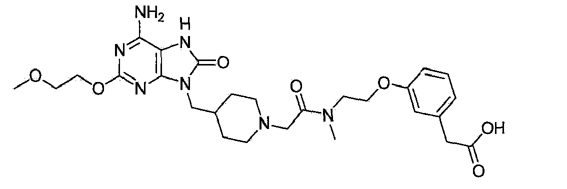

In the same manner as example 35, there was obtained the titled compound as a white solid. Yield 40%

### Example 46

### 2-Butoxy-7,8-dihydro-9-(2-{1-[(N-{2-[3-(hydroxylcarbonylmethyl)phenoxy]ethyl}-N-methyl)aminocarbonylmethyl]piperidin-4-yl}ethyl)-8-oxoadenine

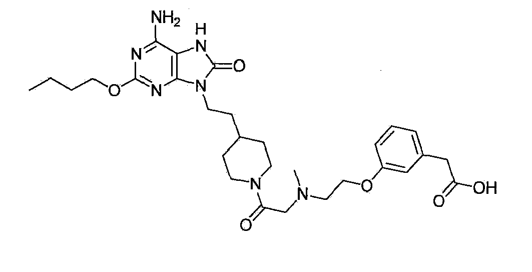

In the same manner as example 35, there was obtained the titled compound as whitish yellow liquid.
¹H NMR (DMSO-d₆) δ 12.27 (1H, brs), 9.86 (1H, brs), 7.20 (1H, t, J = 7.6 Hz), 6.82-6.79 (3H, m), 6.41 (2H, brs), 4.30 (1H, d, J = 12.4 Hz), 4.13 (2H, t, J = 6.6 Hz), 4.09-4.03 (2H, m), 3.96 (1H, d, J = 10.5 Hz), 3.69 (2H, t, J = 6.7 Hz), 3.51 (2H, s), 3.40-3.25 (2H, m), 2.90-2.82 (3H, m), 2.49-2.41 (1H, m), 2.28 (3H, m), 1.78-1.71 (2H, m), 1.67-1.59 (2H, m), 1.58-1.53 (2H, m), 1.45-1.33 (3H, m), 1.13-1.03 (1H, m), 0.96-0.94 (1H, m), 0.91 (3H, t, J = 7.4 Hz).

### Example 47

### 2-Butoxy-7,8-dihydro-9-[5-(4-{2-[N-methyl-N-(3-hydroxycarbonylmethyl)benzyl]aminomethyl}piperazin-1-yl)pentyl]-8-oxoadenine

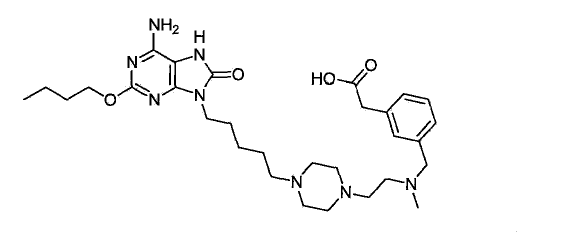

To the compound 55mg (0.13mmol) obtained by example 20 was added 1N aqueous sodium hydroxide (4ml) and the mixture was refluxed for 1.5 hours. After neutralized with hydrochloric acid, the solvent was removed by distillation. To the residue was added water and the resulting solid was filtered to give the titled compound 30mg as a white solid. Yield 55%
¹H NMR (DMSO-d₆) δ 9.32 (1H, bs), 7.24-7.06 (4H, m), 6.86 (2H, bs), 4.13 (2H, t, J= 6.6 Hz), 3.66 (2H, t, J= 6.4 Hz), 3.43 (4H, s), 2.34-2.13 (17H, m), 1.68-1.62 (4H, m), 1.40-1.36 (4H, m), 1.22-1.18 (2H, m), 0.92 (3H, t, J= 7.4 Hz).

### Example 48

### 2-Butoxy-7,8-dihydro-9-[7-(4-{2-[N-methyl-N-(3-hydroxycarbonylmethyl)benzyl]aminomethyl}piprazin-1-yl)heptyl]-8-oxoadenine

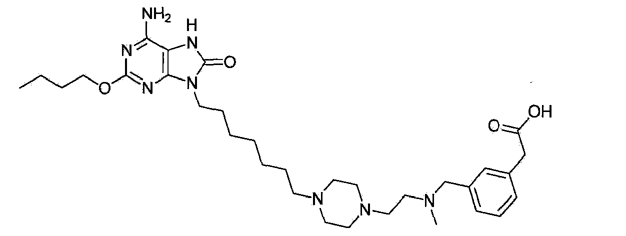

Using the compound 72mg (0.12mmol) obtained by example 21 step (iii), in the same manner as example 35, there was obtained the titled compound 26mg as a white solid. Yield 37%
¹H NMR (DMSO-d₆) δ 10.83 (1H, bs), 7.14-7.03 (4H, m), 6.69 (2H, bs), 4.07 (2H, t, J= 6.6 Hz), 3.58 (2H, t, J= 6.6 Hz), 3.37 (4H, s), 2.34-2.23 (12H, m), 2.15-2,09 (5H, m), 1.59-1.55 (4H, m), 1.33-1.14 (10H, m), 0.85 (3H, t, J= 7.4 Hz).

### Example 49

### 2-Butoxy-7,8-dihydro-9-(5-{4-[2-(3-hydroxycarbonylmethylphenyloxy)ethyl]piperazin-1-yl}pentyl)-8-oxoadenine

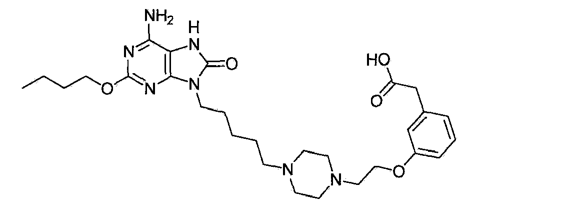

Using the compound 50mg (0.09mmol) obtained in example 22, in the same manner as example 35, there was obtained the titled compound 35mg as a white solid. Yield 72%
¹H NMR (DMSO-d₆) δ 12.09 (1H, bs), 7.12 (1H, t, J= 7.8 Hz), 7.07 (2H, bs), 6.97 (1H, s), 6.75 (1H, d, J= 7.8 Hz), 6.69 (1H, d, J= 7.8 Hz), 4.13 (2H, t, J= 6.6 Hz), 4.05 (2H, t, J= 5.8 Hz), 3.67 (2H, t, J= 7.1 Hz), 3.17 (2H, s), 2.65 (2H, t, J= 5.8 Hz), 2.39 (4H, bs), 2.20 (4H, bs), 2.17 (2H, t, J= 6.5 Hz), 1.70-1.61 (4H, m), 1.41-1.36 (4H, m), 1.21-1.18 (2H, m), 0.92 (3H, t, J= 7.4 Hz).

### Example 50

### 2-Butoxy-7, 8-dihydro-9-(7-{4-[2-(3-hydroxycarbonylmethylphenyloxy)ethyl]piperazin-1-yl}heptyl)-8-oxoadenine

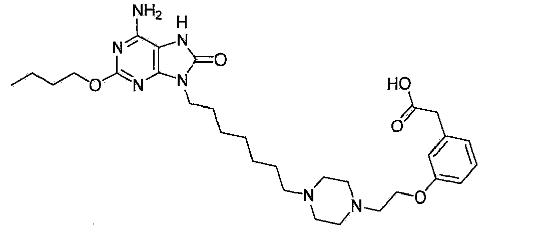

Using the compound 50mg (0.08mmol) obtained by example 23, in the same manner as example 35, there was obtained the titled compound 38mg as a white solid. Yield 78%
¹H NMR (DMSO-d₆) δ 11.66 (1H, bs), 7.13 (1H, t, J= 7.8 Hz), 7.07 (2H, bs), 6.87 (1H, s), 6.77 (1H, d, J= 7.8 Hz), 6.71 (1H, d, J= 7.8 Hz), 4.13 (2H, t, J= 6.6 Hz), 4.03 (2H, t, J= 5.5 Hz), 3.64 (2H, t, J= 7.1 Hz), 3.17 (2H, s), 2.70 (2H, t, J= 5.5 Hz), 2.47 (4H, bs), 2.28 (4H, bs), 2.16 (2H, t, J= 6.9 Hz), 1.65-1.63 (4H, m), 1.40-1.22 (10H, m), 0.92 (3H, t, J= 7.4 Hz).

### Example 51

### 2-Butoxy-7,8-dihydro-9-[2-{4-(3-hydroxylcarbonylmethylphenoxy)piperizin-1-yl}ethyl]-8-oxoadenine

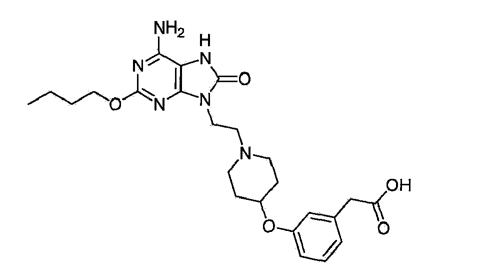

Using the compound 30mg (0.06mmol) obtained in example 24, in the same manner as example 35, there was obtained the subtitled compound 15mg as a white solid. Yield 51%
¹H NMR (DMSO-d₆) δ 12.30 (1H, bs), 9.84 (1H, bs), 7.19 (1H, dd, J= 7.6, 7.6 Hz), 6.82 (1H, s), 6.79 (2H, d, J= 7.6 Hz), 6.41 (2H, bs), 4.35-4.26 (1H, m), 4.13 (1H, t, J= 6.6 Hz), 3.78 (1H, t, J= 6.4 Hz), 3.51 (2H, s), 2.78-2.72 (2H, m), 2.59 (2H, t, J= 6.4 Hz), 2.31-2.25 (2H, m), 1.89-1.82 (2H, m), 1.67-1.59 (2H, m), 1.55-1.46 (2H, m), 1.42-1.32 (2H, m), 0.90 (3H, t, J= 7.6 Hz).

### Example 52

### 2-Butoxy-7,8-dihydro-9-[2-{4-(3-hydroxylcarbonylmethylphenyl)piperazin-1-yl}ethyl] -8-oxoadenine

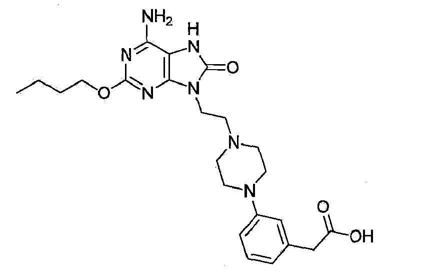

Using compound 80mg (0.17mmol) obtained by example 25, in the same manner as example 35, there was obtained the titled compound 50mg as a white solid. Yield 64%
¹H NMR (DMSO-d₆) δ 12.42 (1H, bs), 10.40 (1H, bs), 7.09 (1H, dd, J= 7.6, 7.6 Hz), 6.79 (1H, s), 6.74 (1H, d, J= 7.6 Hz), 6.65 (1H, d, J= 7.6 Hz), 6.57 (2H, bs), 4.14 (2H, t, J= 6.6 Hz), 3.82 (2H, t, J= 6.4 Hz), 3.39 (2H, s), 3.06-3.00 (4H, m), 2.62 (2H, t, J= 6.4 Hz), 2.58-2.55 (4H, m), 1.68-1.60 (2H, m), 1.44-1.33 (2H, m), 0.91 (3H, t, J= 7.4 Hz).

### Example 53

### 2-Butoxy-7,8-dihydro-9-[2-{4-(3-hydroxylcarbonylmethylbenzyl)piperazin-1-yl}ethyl] -8-oxoadenine

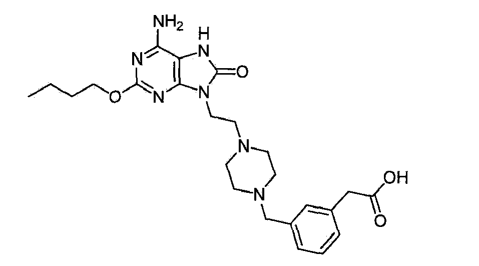

Using the compound 30mg (0.06mmol) obtained by example 26, in the same manner as example 35, there was obtained the titled compound 26mg as a colorless oil. Yield 89%
¹H NMR (DMSO-d₆) δ 11.22 (1H, bs), 7.14 (1H, s), 7.13 (1H, dd, J= 7.6 Hz, 7.6 Hz), 7.07 (1H, d, J= 7.6 Hz), 7.00 (1H, d, J= 7.6 Hz), 6.90 (2H, bs), 4.10 (2H, t, J= 6.6 Hz), 3.73 (2H, t, J= 6.4 Hz), 3.34 (2H, s), 3.24 (2H, s), 2.52 (2H, t, J= 6.4 Hz), 2.47-2.33 (4H, m), 2.33-2.23 (4H, m), 1.65-1.56 (2H, m), 1.41-1.31 (2H, m), 0.89 (3H, t, J= 7.4 Hz).

### Example 54

### 2-Butoxy-7,8-dihydro-9-[5-{4-(3-hydroxylcarbonylmethylphenyl)piperazin-1-yl}penyl] -8-oxoadenine

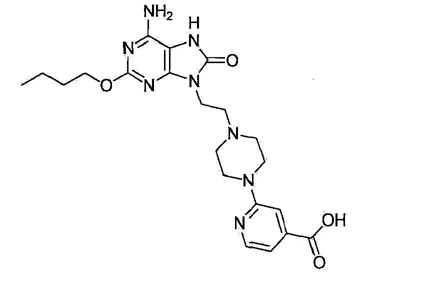

Using the compound 50 mg (0.10mmol) obtained by example 27, in the example manner as 35, there was obtained the titled compound 49mg as a yellow oil. Yield 99%
¹H NMR (DMSO-d₆) δ 13.40 (1H, bs), 9.93 (1H, bs), 8.22 (1H, d, J= 5.1 Hz), 7.17 (1H, s), 7.02 (1H, d, J= 5.1 Hz), 6.43 (2H, bs), 4.05 (2H, t, J= 6.6 Hz), 3.83 (2H, t, J= 6.4 Hz), 3.47-3.42 (4H, m), 2.64 (2H, t, J= 6.4 Hz), 2.58-2.51 (4H, m), 1.68-1.60 (2H, m), 1.44-1.34 (2H, m), 0.91 (3H, t, J= 7.4 Hz).

### Example 55

### 2-Butoxy-7,8-dihydro-9-(3-{4-[3-(2-hydroxy-2-oxoethyl)phenoxy]piperidin-1-yl}propyl)-8-oxoadenine

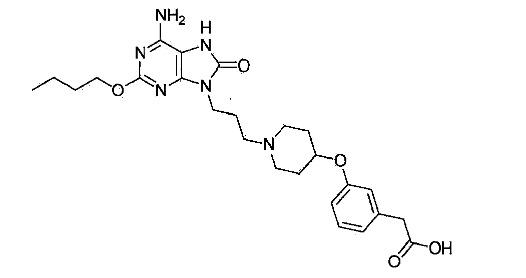

Using the compound 37mg (0.072mmol) obtained by example 28, in the same manner as example 35, there was obtained the titled compound 23mg as a white solid. Yield 64%
¹H NMR (DMSO-d₆) δ 7.12 (1H, t, J= 7.8 Hz), 6.83-6.71 (3H, m), 6.69 (2H, brs), 4.28 (1H, m), 4.15 (2H, t, J= 6.6 Hz), 3.70 (2H, t, J= 6.9 Hz), 3.34 (2H, s), 2.68-2.58 (2H, m), 2.30 (2H, t, J= 6.8 Hz), 2.16-2.08 (2H, m), 1.86-1.76 (4H, m), 1.65-1.48 (4H, m), 1.41-1.34 (2H, m), 0.90 (3H, t, J= 7.4 Hz).

### Example 56

### 2-Butoxy-7,8-dihydro-9-{4-[4-(3-hydroxycarbonylmethylphenoxy)piperazin-1-yl]butyl}-8-oxoadenine

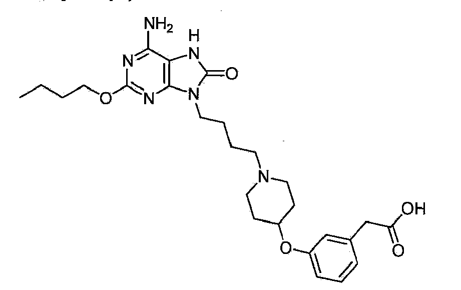

Using the compound 50mg (0.10mmol) obtained by example 29, in the same manner as example 35, there was obtained the titled compound 13mg as a white solid. Yield 27%
¹H NMR (DMSO-d₆) δ 11.81 (1H, brs), 7.08 (1H, t, J= 7.8Hz), 6.98 (2H, brs), 6.88 (1H, s), 6.75-6.66 (2H, m), 4.25 (1H, m), 4.13 (2H, t, J= 6.6 Hz), 3.66 (2H, t, J= 6.7Hz), 3.23 (2H, s), 2.56 (2H, m), 2.25 (2H, m), 2.09 (2H, m), 1.81 (2H, m), 1.67-1.53 (6H, m), 1.41-1.36 (4H, m), 0.92 (3H, t, J= 7.4 Hz).

### Example 57

### 2-Butoxy-7,8-dihydro-9-{4-[4-(3-hydroxycarbonylmethylbenzyl)piperazine-1-yl]butyl}-8-oxoadenine

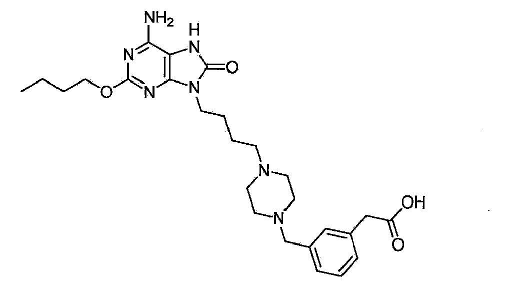

Using the compound 50mg (0.10mmol) obtained by example 30, in the same manner as example 35, there was obtained the titled compound 31mg as a white solid. Yield 64%
¹H NMR (DMSO-d₆) δ 9.87 (1H, s), 7.25 (1H, t, J= 7.5Hz), 7.16-7.12 (3H, m), 6.41 (2H, brs), 4.14 (2H, t, J= 6.6 Hz), 3.66 (2H, t, J= 6.7Hz), 3.54 (2H, s), 3.41 (2H, s), 2.33-2.26 (10H, m), 1.67-1.60 (4H, m), 1.41-1.34 (4H, m), 0.92 (3H, t, J= 7.4 Hz).

### Example 58

### 2-Butoxy-7,8-dihydro-9-{4-[4-(4-hydrooxycarbonylbenzyl)piperidin-1-yl] butyl}-8-oxoadenine

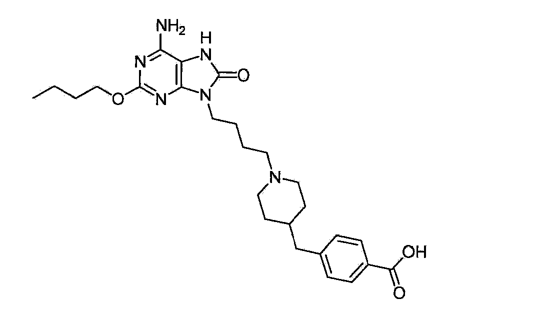

Using the compound 50mg (0.10mmol) obtained by example 31, in the same manner as example 35there was obtained the titled compound 32mg as a white solid. Yield 65%
¹H NMR (DMSO-d₆) δ 11.69 (1H, brs), 7.78 (2H, d, J= 8.1Hz), 7.05 (2H, d, J= 8.1Hz), 7.01 (2H, brs), 4.14 (2H, t, J= 6.6 Hz), 3.66 (2H, t, J= 6.7Hz), 2.74 (2H, m), 2.46 (2H, d, J= 6.5Hz), 2.21 (2H, m), 1.72 (2H, m), 1.65-1.62 (4H, m), 1.46 (2H, m), 1.41-1.35 (5H, m), 1.15 (2H, m), 0.92 (3H, t, J= 7.4 Hz).

### Example 59

### 2-Butoxy-7,8-dihydro-9-[5-{4-(3-hydroxylcarbonylmethylphenyl)piperazin-1-yl}pentyl]-8-oxoadenine

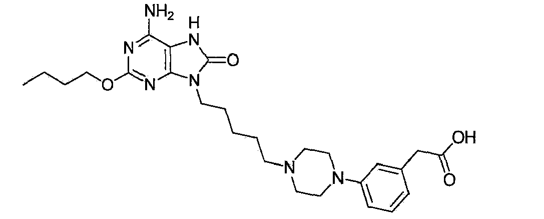

Using the compound 45mg (0.09mmol) obtained by example 33, in the same manner as example 35, there was obtained the titled compound 41mg as a yellow oil. Yield 94%
¹H NMR (DMSO-d₆) δ 12.29 (1H, bs), 10.25 (1H, bs), 7.19 (1H, dd, J= 7.6, 7.6 Hz), 6.89 (1H, s), 6.88 (1H, d, J= 7.6 Hz), 6.76 (1H, d, J= 7.6 Hz), 6.67 (2H, bs), 4.14 (2H, t, J= 6.6 Hz), 3.80-3.73 (2H, m), 3.69 (2H, t, J= 6.7 Hz), 3.57-3.50 (2H, m), 3.50 (2H, s), 3.14-3.00 (6H, m), 1.79-1.59 (6H, m), 1.44-1.33 (2H, m), 1.33-1.24 (2H, m), 0.91 (3H, t, J= 7.4 Hz).

### Example 60

### Methyl {3-[({1-[2-(6-amino-2-butoxy-8-oxo-7,8-dihydro-9H-purin-9-yl)ethyl]piperidin-4-yl}amino)methyl]phenyl}acetate

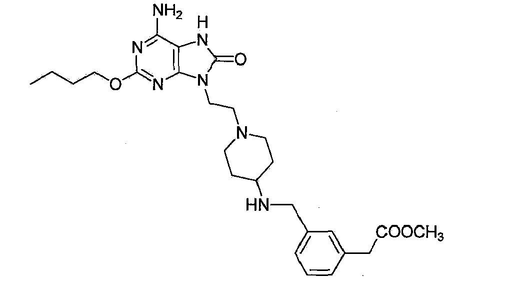

### Step (i)

### 2-Chloro-9-(tetrahydro-2H-pyran-2-yl)-9H-purin-6-amine

2,6-Dichloro-9-(tetrahydro-2H-pyran-2-yl)-9H-purine) (55g) was dissolved in 7N-aqueous ammonia-methanol (500ml) and the solution was heated at 100°C for 6 hours in a sealed flask. The reaction mixture was cooled to room temperature and left overnight. The mixture was filtered to give the titled compound. Yield 40g
¹H NMR^{™} (CDCl₃) 8.02 (1H, s), 5.94 (2H, brs), 5.71 (1H, dd), 4.15 - 4.22 (1H, m), 3.75 - 3.82 (1H, m), 1.27 - 2.12 (6H, m).

### Step (ii)

### 2-Butoxy-9-(tetrahydro-2H-pyran-2-yl)-9H-purin-6-amine

The compound (40g) obtained in step (i) was dissolved in 19% (W/W) sodium n-butoxide/butanol (250ml). The reaction mixture was stirred under reflux for 6 hours. The obtained suspension was cooled to room temperature, diluted with water and the mixture was extracted with diethyl ether. The combined organic layer was washed with water, dried over and concentrated in vacuo. The residue was crystallized from diethyl ether/isohexane and filtered to give the titled compound. Yield 19g
¹H NMR^{™} (CDCl₃) 7.87 (1H, s), 5.56 - 5.68 (3H, m), 4.31 - 4.35 (2H, t), 4.14 - 4.17 (1H, m), 3.76 - 3.80 (1H, m), 1.49 - 2.08 (10H, m), 0.98 (3H, t).

### Step (iii)

### 8-Bromo-2-butoxy-9-(tetrahydro-2H-pyran-2-yl)-9H-purin-6-amine

The product obtained in step (ii) was dissolved in dried dichloromethane (200ml) and the solution was stirred at room temperature. Thereto was added dropwise N-bromosuccinimide (NBS) (27g). The reaction mixture was stirred at room temperature overnight. Thereto was added 20% (w/w) sodium sulfate, and the mixture was separated by a separating funnel. The aqueous layer was extracted with dichloromethane. The combined organic layer was washed with aqueous saturated sodium hydrogencarbonate and saturated brine. After concentrated in vacuo, the residue was dissolved in ethyl acetate, washed with water and saturated brine and dried. The solution was filtered through silica gel and concentrated in vacuo. The residue was crushed in diethyl etherisohexane and the titled compound was filtered (26g). The filtrate was concentrated in vacuo, and the residue was purified by silica gel column chromatography (ethyl acetate/isohexane) to obtain further the titled compound 2.5g. These compounds were combined and obtained as a yellow solid. Yield 28.5g, m.p. 148-50°C
¹H NMR^{™}(CDCl₃) 5.59-5.64 (3H, m), 4.32 (2H, m), 4.17 (1H, m), 3.74 (1H, m), 3.08 (1H, m), 2.13 (1H, d), 1.48 - 1.83 (8H, m), 0.98 (3H, t).

### Step (iv)

### 2-Butoxy-8-methoxy-9-(tetrahydro-2H-pyran-2-yl)-9H-purin-6-amine

Sodium (3.7g) was added to anhydrous methanol (400ml) under an atomosphere of nitrogen. Thereto was added the compound (28.5g) obtained in step (iii) and the reaction mixture was stirred at 65°C for 9 hours. After concentrated in vacuo, thereto was added water. The aqueous layer was extracted with ethyl acetate, washed with saturated brine and dried. The residue was crystallized from diethyl ether to give the titled compound. Yield 14.2g
¹H NMR^{™} (CDCl₃) 5.51 (1H, dd), 5.28 (2H, brs), 4.29 (2H, t), 4.11 - 4.14 (4H, m), 3.70 (1H, m), 2.76 - 2.80 (1H, m), 2.05 (1H, d), 1.47 - 1.81 (8H, m), 0.97 (3H, t).

### Step (v)

### 2-Butoxy-8-methoxy-9H-purin-6-amine TFA salt

To the compound (24g) obtained in step (iv) in anhydrous methanol (300ml) was added TFA (30ml). The reaction mixture was stirred at room temperature for 3 days, concentrated in vacuo and crushed in methanol/ethyl acetate to give the titled compound as white crystals.
Yield 21g
¹H NMR^{™} (CD₃OD) 4.48 (2H, t), 4.15 (3H, s), 1.80 (2H, quintet), 1.50 (2H, sextet), 0.99 (3H, t).

### Step (vi)

### 9-(2-Bromoethyl)-2-butoxy-8-methoxy-9H-purin-6-amine

The compound (2g) obtained in step (v) in DMF (20ml)was dropped at the room temperature in a period of 10 minutes or more to the mixture of potassium carbonate (3.7g) and 1,2-dibromoethane (0.6ml) which was stirred in high speed, and then the mixture was stirred for 1.5 hours. The reaction mixture was diluted with water and extracted with ethyl acetate. The combined extract was washed with saturated brine, dried over and purified by column chromatography to give the titled compound as a white solid. Yield 1.2g
¹H NMR^{™} (CDCl₃) 5.15 (2H, s), 4.30 (4H, m), 4.13 (3H, s), 3.65 (2H, t), 1.82 - 1.72 (2H, m), 1.56 - 1.43 (2H, m), 0.97 (3H, t).

### Step (vii)

### tert-Butyl {1-[2-(6-amino-2-butoxy-8-methoxy-9H-purin-9-yl)ethyl]piperidin-4-yl}carbamate

To the compound (400mg) obtained in step (vi) in acetonitrile (10ml) was added tert-butylpiperidine-4-ylcarbamate (1.1g) and the reaction mixture was stirred at 50°C overnight. After cooled to room temperature, the reaction mixture was concentrated in vacuo and to the residue was added water. The suspension was stirred at room temperature overnight. The resulting solid was collected by filtration, left for 16 hours in vacuo, and dried to give the titled compound as a white solid. Yield 530mg
¹H NMR^{™} (DMSO-d₆) 6.75 (2H, brs), 4.17 (2H, J = 6.6 Hz, t), 4.06 (3H, s), 3.94 (2H, J = 5.7 Hz, t), 2.88 - 2.51 (5H, m), 2.01 - 1.64 (6H, m), 1.45 - 1.21 (13H, m), 0.92 (3H, J = 7.5 Hz, t).

### Step (viii)

### 6-Amino-9-[2-(4-aminopiperidin-1-yl)ethyl]-2-butoxy-7,9-dihydro-8H-purin-8-one

The compound (530mg) obtained in step (vii) in methanol (5ml) was treated with 4M hydrochloric acid /dioxane (1ml). The reaction mixture was stirred at room temperature overnight, concentrated in vacuo and the residue was purified by SCX to give the titled compound as a white solid. Yield 400mg
¹H NMR^{™} (DMSO-d₆) 6.40 (2H, brs), 4.17 (2H, J = 6.6 Hz, t), 3.91 (2H, m), 2.85 (2H, m), 2.69 - 2.48 (3H, m), 1.98 - 1.60 (6H, m), 1.43 - 1.16 (4H, m), 0.92 (3H, J = 7.5 Hz, t).
MS: APCI (+ve): 550 (M+H)

### Step (ix)

### Methyl {3-[({1-[2-(6-amino-2-butoxy-8-oxo-7,8-dihydro-9H-purin-9-yl)ethyl]piperidin-4-yl}amino)methyl]phenyl}acetate

The compound (400mg) obtained in step (viii), methyl (3-formylphenyl)-acetate (204mg) and sodium triacetoxyborohydride (730mg) were dissolved in methanol (10ml), and the solution was stirred at room temperature overnight. The reaction mixture was treated with SCX, and purified by reverse-phase HPLC to give the titled compound. Yield 96mg ¹H NMR δ (DMSO-d₆) 7.25 - 7.07 (4H, m), 6.37 (2H, brs), 4.14 (2H, J = 6.8 Hz, t), 3.75 (2H, J = 6.4 Hz, t), 3.66 (2H, s), 3.64 (2H, s), 3.60 (3H, s), 2.85 (2H, m), 2.55 - 2.32 (3H, m), 1.96 - 1.12 (10H, m), 0.92 (3H, J = 7.2 Hz, t). MS: APCI (+ve): 512 (M+H)

### Example 61

### {3-[({1-[2-(6-Amino-2-butoxy-8-oxo-7,8-dihydro-9H-purin-9-yl)ethyl]piperidin-4-yl}amino)methyl]phenyl}acetic acid

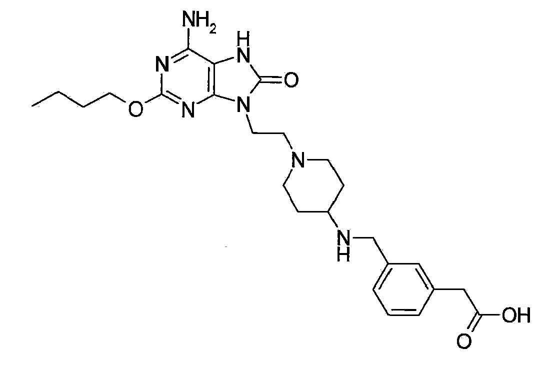

The compound (0.1g) obtained by example 60, methanol (2ml) and aqueous 2N lithium hydroxide were mixed and the mixture was stirred at room temperature overnight. After concentrated in vacuo, thereto was added water. The mixture was neutralized with acetic acid and purified by reverse-phase HPLC to give the titled compound. Yield 35mg
¹H NMR^{™} (DMSO-d₆) 7.23 - 7.08 (4H, m), 6.44 (2H, brs), 4.13 (2H, J = 6.8 Hz, t), 3.75 (2H, J = 6.8 Hz, t), 3.68 (2H, s), 3.35 (2H, s), 2.85 (2H, m), 2.55 - 2.32 (3H, m), 1.96 - 1.15 (10H, m), 0.91 (3H, J = 7.6 Hz, t).
MS: APCI (-ve): 496 (M-H)

### Example 62

### Human TLR7 reporter assay

HEK293 cells in which human TLR7 or rat TLR7 plasmid and reporter plasmid (NF-kB-SEAP) are stably introduced are dispersed in DMEM broth (10%FBS, 1%NEAA, 10ug/mL blastocidin S HCl, 100 ug/mL Zeocin), and were seeded to 96 well plate per 90µl/well (hTLR7/seap-293: 20000cells/well, rTLR7/seap-293:25000cells/well).

Test compound (DMSO stock solution (2µl) was diluted with the broth (200µl) by 100times) was added to the seeded cells to a 96well plate (10µl/well) (final concentration; InM - 10µM, common ratio). After stirring by tapping side of the plate, the cells were cultured in a CO₂ incubator for 20 hours. A substrate (50µl/well) for reporter assay (substrate for SEAP, pNPP) was added to cells stimulated by test sample. Ten minutes after adding the substrate, the reaction quenching solution (4N NaOH) was added by 50µl/well to cease enzymatic reaction. Sealing a top seal A on the plate, the absorbance was measured by a micro plate reader (405nm).

Human TLR7 binding activity (EC₅₀) of each compound is shown in Table 1.

**Table 1**

| compound | EC₅₀ (nM) |
|---|---|
| Example 1 | 1623.9 |
| Example 2 | 1864.4 |
| Example 11 | 187.5 |
| Example 12 | 579.1 |
| Example 17 | 5621.9 |
| Example 18 | 281.2 |
| Example 19 | 643.6 |
| Example 20 | 138.9 |
| Example 21 | 449.0 |
| Example 22 | 198.3 |
| Example 23 | 74.5 |
| Example 29 | 713.1 |

### Example 63

### {3-[2-(4-{[3-(6-Amino-2-butoxy-8-oxo-7, 8-dihydro-purin-9-yl)propyl]isobutylainino}piperidin-l-yl)ethoxy]phenyl}acetic acid methyl ester

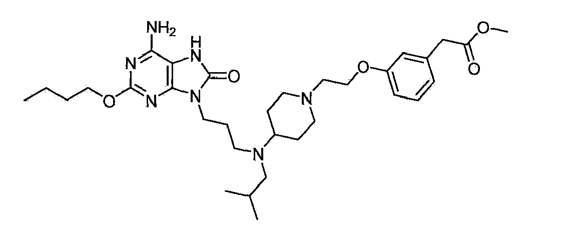

### Step (i)

### 9-(3-Bromo-propyl)-2-butoxy-8-methoxy-9H-purin-6-ylamine

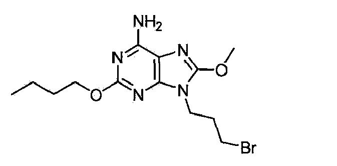

To 2-butoxy-8-methoxyadenine 5g (14.2mmol) in dimethylformamide (50ml) was added 1,3-dibromobutane (7.2ml) and potassium carbonate (9.2g) and the mixture was stirred at room temperature for 1.5 hours. Thereto was added water (200ml) and the mixture was extracted with ethyl acetate (75ml) and further extracted with ethyl acetate (75ml x 2). The organic layers were combined, dried over magnesium sulfate and filtered. The solvent was removed in vacuo and to the residue was added diethyl ether (25ml). The resulting crystals were filtered, washed with ether (5ml) and dried to give the subtitled compound 3.6g as a white solid. Yield 71%
¹H NMR (CDCl₃) δ 5.24 (2H, brs), 4.29 (2H, t, J= 6.7 Hz), 4.13 (3H, s), 4.09 (2H, t, J= 6.7 Hz), 3.38 (2H, t, J= 6.6 Hz), 2.34 (2H, q, J= 6.6 Hz), 1.80-1.73 (2H, m), 1.54-1.46 (2H, m), 0.96 (3H, t, J= 7.4 Hz).

### Step (ii)

### 6-Amino-9-(3-bromo-propyl)-2-butoxy-7,9-dihydro-purin-8-one

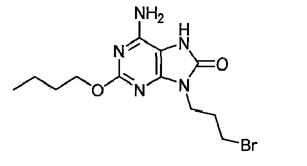

To the compound 1g (2.79mmol) obtained in step (i) in methanol (2ml) was added 4N-hydrochloric acid -dioxane (2ml) and the mixture was stirred at room temperature for 3.5 hours. After neutralized at 0°C with 28% aqueous ammonia, the mixture was stirred for 1 hour. The resulting crystals were filtered, washed with water (2ml x 2), and methanol (2ml x 2), and dried over to give the subtitled compound 882mg as a white solid.
Yield 92%
¹H NMR (CDCl₃) δ 9.89 (1H, brs), 6.43 (2H, s), 4.16 (2H, t, J= 6.6 Hz), 3.80 (2H, t, J= 6.6 Hz), 3.53 (2H, t, J= 6.6 Hz), 2.20 (2H, q, J= 6.6 Hz), 1.68-1.61 (2H, m), 1.42-1.36 (2H, m), 0.92 (3H, t, J= 7.4 Hz).

### Step (iii)

### 6-Amino-2-butoxy-9-(3-isobutylamino-propyl)-7,9-dihydro-purin-8-one

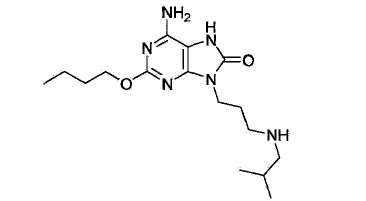

To the compound 600mg (1.74mmol) obtained in step (ii) in dimethyl sulfoxide (1ml) was added isobutylamine (2.55g) and the mixture was stirred at room temperature for 2.5 hours. After removal of isobutylamine in vacuo, thereto was added at 0°C acetonitrile (5ml) and the mixture was stirred 1 hour. The resulting crystals were filtered and dried to give the titled compound 573mg as a white solid. Yield 98%
¹H NMR (DMSO-d₆) δ 10.03 (1H, brs), 6.54 (2H, s), 4.15 (2H, t, J= 6.6 Hz), 3.74 (2H, t, J= 6.6 Hz), 2.72 (2H, brs), 2.54 (2H, d, J= 6.8 Hz), 1.92-1.88 (2H, m), 1.81-1.93 (1H, m), 1.66-1.61 (2H, m), 1.42-1.37 (2H, m), 0.91 (9H, m).

### Step (iv)

### 4-{[3-(6-Amino-2-butoxy-8-oxo-7,8-dihydro-purin-9-yl)propyl]isobutylamino}piperidin-1-carboxylic acid tert-butyl ester)

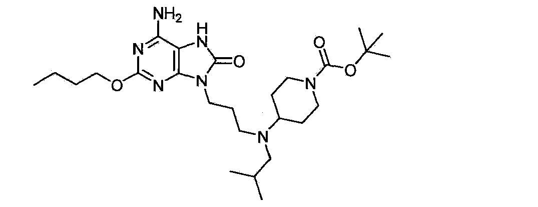

To the compound 573mg (1.70mmol) obtained in step (iii) in N-methylpyrrolidinone were added N-boc-4-pyrollidone (305mg) and sodium triacetoxyborohydride (469mg), and the mixture was stirred at 50°C for 12 hours. As the reaction was not completed, thereto were added additional N-Boc-4-pyrollidone (305mg), and sodium triacetoxyborohydride (469mg), and the mixture was stirred at 50°C for 12 hours. The mixture was cooled to room temperature and neutralized at 0°C with 1%-aqueous ammonia (30ml), followed by stirring for 1 hour. The resulting crystals was filtered, washed with 1%-aqueous ammonia (2ml) and dried to give the subtitled compound 659mg as a white solid. Yield 75%
¹H NMR (DMSO-d₆) δ 9.96 (1H, brs), 6.43 (2H, s), 4.14 (2H, t, J= 6.6 Hz), 3.97 (2H, brs), 3.67 (2H, t, J= 7.2 Hz), 2.69-2.35 (3H, m), 2.41 (2H, t, J= 6.8 Hz), 2.09 (2H, d, J= 7.2 Hz), 1.77-1.72 (2H, m), 1.65-1.55 (5H, m), 1.40-1.37 (11H, m), 1.35-1.26 (2H, m), 0.91 (3H, t, J= 7.4 Hz), 0.81 (6H, d, J= 6.6 Hz).

### Step (v)

### {3-[2-(4-{[3-(6-Amino-2-butoxy-8-oxo-7,8-dihydro-purin-9-yl)propyl]isobutylamino}piperidin-1-yl)ethoxy]phenyl}acetic acid methyl ester)

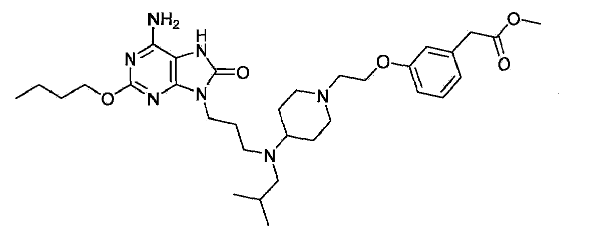

Using the compound 200mg (0.39mmol) obtained in step (iv) and methyl [3-(2-bromoehoxy)phenyl]acetate 137mg (0.50mmol), in the same manner as example 6 step (i), there was obtained the titled compound 59mg as a white solid. Yield 25%
¹H NMR (DMSO-d₆) δ 9.87 (1H, brs), 7.21 (1H, t, J= 8.0 Hz), 6.83-6.80 (3H, m), 6.39 (2H, s), 4.15 (2H, t, J= 6.6 Hz), 4.02 (2H, t, J= 5.9 Hz), 3.68 (2H, t, J= 7.3 Hz), 3.64 (2H, s), 3.61 (3H, s), 2.94 (2H, d, J= 11.8 Hz), 2.63 (2H, t, J= 5.8 Hz), 2.43 (2H, t, J= 6.9 Hz), 2.40-2.36 (1H, m), 2.10 (2H, d, J= 7.3 Hz), 1.97 (2H, t, J= 10.9 Hz), 1.78-1.73 (2H, m), 1.68-1.54 (5H, m), 1.44-1.34 (4H, m), 0.91 (3H, t, J= 7.4 Hz), 0.82 (6H, d, J= 6.5 Hz).

### Example 64

### [4-({1-[3-(6-Amino-2-butoxy-8-oxo-7, 8-dihydro-purin-9-yl)propyl]piperidin-4-ylamino}methyl)phenyl]acetic acid methyl ester

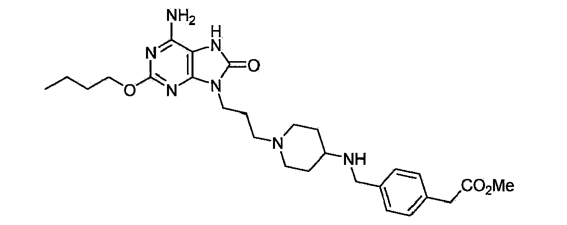

### Step (i)

### {1-[3-(6-Amino-2-butoxy-8-oxo-7,8-dihydro-purin-9-yl)propyl]piperidin-4-yl}carbamic acid tert-butyl ester

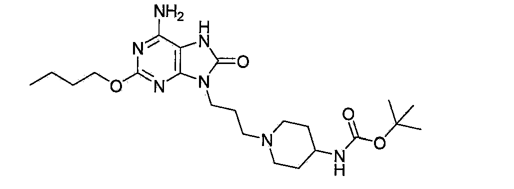

Using compound 500mg (1.45mmol) obtained by example 63 step (ii) and N-Boc-4-aminopiperidine 349mg (1.74mmol), in the same manner as example 63 step (iii), there was obtained the subtitled compound 574mg as a white solid. Yield 85%
¹H NMR (CDCl₃) δ 9.95 (1H, brs), 6.76 (1H, d, J= 7.7 Hz), 6.43 (2H, s), 4.14 (2H, t, J= 6.6 Hz), 3.68 (2H, t, J= 7.0 Hz), 5.29-3.18 (1H, m),2.77 (2H, d, J= 11.1 Hz), 2.25 (2H, t, J= 7.0 Hz), 1.83-1.74 (4H, m), 1.66-1.60 (4H, m), 1.42-1.32 (13H, m), 0.92 (3H, t, J= 7.4 Hz).

### Step (ii)

### 4-Bromomethylphenylacetic acid methyl ester

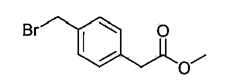

To 4-bromomethylphenylacetic acid 25g (109mmol) in methanol (120ml) was added thionyl chloride 120µl (1.64mmol) and the mixture was stirred at room temperature for 8 hours. After removal of the solvent in vacuo, the residue was neutralized with aqueous saturated sodium bicarbonate, and the mixture was extracted with ethyl acetate (300ml).
The organic layer was washed aqueous saturated sodium bicarbonate, (50ml) and saturated brine (20ml), successively and dried over magnesium sulfate. The solvent was removed in vacuo to give the subtitled compound 25g as colorless crystals. Yield 99%
¹H NMR (CDCl₃) δ 7.36 (2H, t, J= 8.1 Hz), 7.26 (2H, d, J= 8.1 Hz), 4.48 (2H, s), 3.69 (3H, s), 3.62 (2H, s).

### Step (iii)

### 4-Formylphenylacetic acid methyl ester

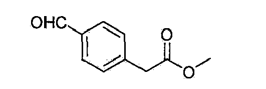

To the compound 5g (20.6mmol) obtained in step (i) in dimethyl sulfoxide (15ml) was added N-methylmorpholine-N-oxide 3.61g (30.9mmol) and the mixture was stirred at room temperature for 1.5 hours. Thereto was added water 50ml and the mixture was extracted with ethyl acetate (30ml x 3). The combined organic layer was washed with water (50ml) and saturated brine (30ml), successively, dried over magnesium sulfate. The solvent was removed in vacuo, and the residue was purified by column chromatography (silica gel 100g, hexane:ethyl acetate= 10:1) to give the subtitled compound 1.65g as colorless crystals. Yield 45%
¹H NMR (CDCl₃) δ 10.0 (1H, s), 7.86 (2H, t, J= 8.1 Hz), 7.46 (2H, d, J= 8.1 Hz), 3.72 (3H, s), 3.71 (2H, s).

### Step (iv)

### [4-({1-[3-(6-Amino-2-butoxy-8-oxo-7, 8-dihydro-purin-9-yl)propyl]piperidin-4-ylamino}methyl)phenyl]acetic acid methyl ester)

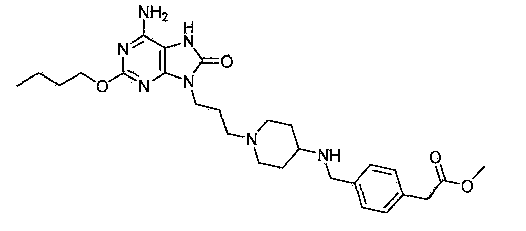

To a suspension of the compound 200mg (0.43mmol) obtained in step (i) in methanol (1ml) was added 6N-hydrochloric acid-methanol (1ml), and the mixture was stirred at room temperature for 2 hours. After removal of the solvent in vacuo, the residue was dried for 2 hours. Thereto were added methanol (5ml) and the compound 92mg (0.52mmol) obtained in step (iii), and the mixture was stirred at room temperature for 0.5 hours. Thereto was added sodium cyanoborohydride 43mg (0.69mmol) and the mixture was stirred at room temperature for 5 hours. After neutralized aqueous saturated sodium carbonate, the solution was extracted with chloroform (15ml x 2). The combined organic layer was dried over magnesium sulfate and the solvent was removed by distillation. The residue was purified by column chromatography (silica gel 6mg, chloroform:methanol = 50:1) to give the titled compound 185mg as white crystals. Yield 82%
¹H NMR (CDCl₃) δ 9.92 (1H, brs), 7.27 (2H, d, J= 8.0 Hz), 7.18 (2H, d, J= 8.0Hz), 6.41 (2H, s), 4.14 (2H, t, J= 6.6 Hz), 3.70-3.66 (2H, m), 3.64 (2H, s), 3.60 (3H, s), 2.73 (2H, brs), 2.33-2.27 (1H, m), 2.24 (2H, t, J= 6.9 Hz), 1.82-1.73 (6H, m), 1.68-1.60 (2H, m), 1.43-1.34 (2H, m), 1.24-1.15 (2H, m), 0.92 (3H, t, J= 7.4 Hz).

### Example 65

### [4-({1-[3-(6-amino-2-butoxy-8-oxo-7,8-dihydro-9H-purin-9-yl)propyl]-4-methylpiperazin-2-yl}methyl)phenyl]acetate methyl ester

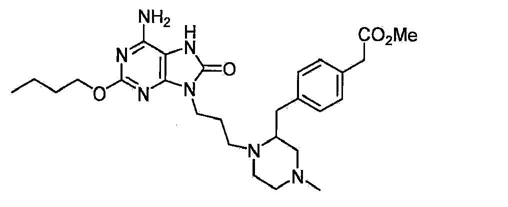

### Step (i)

### tert-Butyl N-benzylideneglycinate

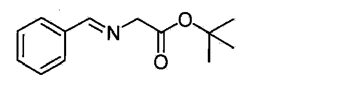

Benzaldehyde (5.52g, 52mmol) was dissolved in toluene (200ml), and thereto were added tert-butyl glycinate acetate (9.94g, 52mmol), triethylamine (7.25ml, 52mmol) and sodium sulfate (11.36g, 80ml), successively, followed by stirring overnight. The reaction mixture was washed with ice cooled-aqueous sodium bicarbonate and ice cooled saturated brine and was dried over sodium sulfate. The solvent was removed by distillation to give the subtitled compound 10.81 g as a yellow oil. Yield 94%
¹H NMR (CDCl₃) δ 8.26 (1H, s), 7.81-7.75 (2H, m), 7.47-7.36 (3H, m), 4.32 (2H, s), 1.49 (9H, s).

### Step (ii)

### N- (tert-butoxycarbonyl) -4- (methoxycarbonyl)phenylalanine

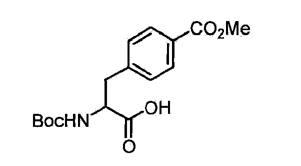

Diisopropylamine (8.91ml, 68mmol) was dissolved in tetrahydrofuran (136ml) under an atmosphere of nitrogen, and thereto was added dropwise under ice cooling n-butyl lithium (hexane 1.57M, 43.3ml, 68ml). After stirring for 15 minutes, the reaction mixture was cooled in a dry ice-acetone bath and thereto was added dropwise methyl 4-(bromomethyl)benzoate (15.58g, 68mmol) in tetrahydrofuran (68ml). After the reaction mixture was stirred for 30 minutes in a dry ice-acetone bath, the mixture was gradually warmed, and stirred at 0°C for additional one hour. After addition of cooled water, the mixture was stirred and then extracted with ethyl acetate. The organic layer was washed with water and saturated brine, dried over sodium sulfate and the solvent was removed by distillation. The residue was dissolved in tetrahydrofuran (25ml) and thereto was added ice cooled 6N aqueous hydrochloric acid (100ml). After reacting at room temperature for 1 hour, the volume of the reaction mixture was concentrated to half in vacuo. The mixture was washed with hexane-ethyl acetate (1:1), and the aqueous layer was concentrated in vacuo. The residue was made alkaline with 2N aqueous sodium hydroxide under ice cooling, and thereto was added dropwise di-tert-butyldicarbonate (27.06g, 124mmol) in tetrahydrofuran (62ml), followed by stirring under ice-cooling for 1 hour. Thereto was added hexane-toluene (1:1) and the mixture was stirred, followed by separating by a separating funnel. The aqueous layer was washed with hexane-toluene (1:1), adjusted to pH3-4 with aqueous 10% potassium hydrogensulfate under ice cooling and extracted with ethyl acetate. The organic layer was washed with saturated brine, and dried over sodium sulfate. The solvent was removed by distillation, and the residue was purified by silica gel column chromatogrophy to give the subtitled compound 4.85g as a yellow oil. Yield 24%
¹H NMR (CDCl₃) δ 7.98 (2H, d, J = 8.2 Hz), 7.29-7.23 (2H, m), 6.32 (1H x1/4, br), 4.98 (1H x3/4, d, J = 7.7 Hz), 4.69-4.60 (1H x3/4, m), 4.45-4.41 (1H x1/4, br), 3.91 (3H, s), 3.32-3.23 (1H, m), 3.17-3.08 (1H x3/4, m), 3.01-2.95 (1H x1/4, br), 1.42 (9H x3/4, s), 1.31 (9H x1/4, s).

### Step (iii)

### Methyl N-(tert-butoxycarbonyl)-4-(methoxycarbonyl)phenylalanyl-N-methylglycinate

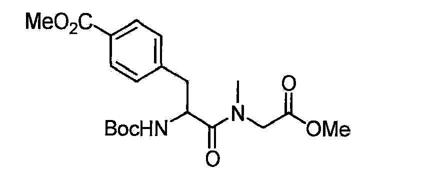

N-(tert-Butoxycarbonyl)-4-(methoxycarbonyl)phenylalanine (4.85g, 15mmol) and triethylamine (2.40ml, 17mmol) were dissolved in dimethylformamide (60ml), and the solution was stirred. Thereto were added methyl N-methylglycinate hydrochloride (2.37g, 17mmol), HOBt (2.60g, 17mmol), and WSC (3.84g, 20mmol), successively and the mixture was stirred overnight. After addition of 10% aqueous citric acid-ethyl acetate, the mixture was stirred and separated by a separating funnel.
The organic layer was washed with aqueous 10% citric acid, water, aqueous saturated sodium bicarbonate and saturated brine, and dried over sodium sulfate. The solvent was removed by distillation to give the subtitled compound 4.75g as white crystals. Yield 77%
¹H NMR (CDCl₃) δ 7.99-7.92 (2H, m), 7.33-7.21 (2H, m), 5.33 (1H x3/4, d, J = 8.6 Hz), 5.23 (1H x1/4, d, J = 8.6 Hz), 4.95-4.86 (1H x3/4, m), 4.72-4.63 (1H x1/4, m), 4.20 (1H x3/4, d, J = 17.2 Hz), 4.03 (1H x1/4, d, J = 17.2 Hz), 3.98-3.85 (1H, m), 3.90 (3H, s), 3.74 (3H x3/4, s), 3.72 (3H x1/4, s), 3.17-3.04 (1H, m), 3.03-2.93 (1H, m), 2.94 (3H x1/4, s), 2.91 (3H x3/4, s), 1.40 (9H x3/4, s), 1.37 (9H x1 /4, s).

### Step (iv)

### Methyl 4-[(4-methyl-3,6-dioxopiperazin-2-yl)methyl]benzoate

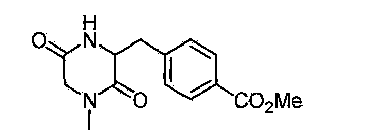

N-(tert-Butoxycarbonyl)-4-(methoxycarbonyl)phenylalanyl-N-methylglycine methyl ester (4.75g, 11.6mmol) was treated with trifluoroacetic acid (30ml) at room temperature for 1 hour, and after removal of the reaction solvent by distillation, the mixture was subjected to azeotropic distillation with chloroform three times. The residue was dissolved in methanol (120ml), thereto was added triethylamine (8.4ml, 60mmol) and the mixture was refluxed for 4 hours. After being cooled, the reaction solvent was removal by distillation, and the residue was diluted with ethyl acetate. The mixture was subjected to azeotropic distillation with aqueous 10% citric acid. The aqueous layer was salted out and extracted with ethyl acetate. The combined organic layer was dried over sodium sulfate, the solvent was removed by distillation and the residue was purified by silica gel column chromatography to give the subtitled compound 3.13g as white crystals. Yield 97%
¹H NMR (CDCl₃) δ 8.00 (2H, dd, J = 6.5, 1.6 Hz), 7.30-7.24 (2H, m), 6.69 (1H, s), 4.37-4.30 (1H, m), 3.92 (3H, s), 3.60 (1H, d, J = 17.6 Hz), 3.25 (1H, dd, J = 13.6, 5.8 Hz), 3.17 (1H, dd, J = 13.6, 4.1 Hz), 3.06 (1H, d, J = 17.6 Hz), 2.85 (3H, s).

### Step (v)

### tert-Butyl 2-[4-(hydroxymethyl)benzyl]-4-methylpiperazine-1-carboxylate

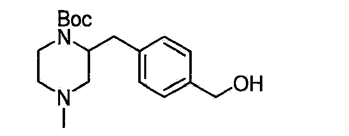

To a suspension of lithium aluminum hydride (2.15g, 56mmol) in tetrahydrofuran (84ml) was added dropwise under heating under stirring under an atmosphere of nitrogen a suspension of 4-[(4-methyl-3,6-dioxopiperazine-2-yl)methyl]benzoic acid methyl (3.13g, 11.3mmol) in tetrahydrofuran (92ml). After refluxing for 4 hours, the reaction mixture was ice cooled, quenched with aqueous sodium sulfate and made alkaline with aqueous 2N sodium hydroxide. After filtration, the filtrate was concentrated and thereto was added dioxane (100ml). Thereto was added under ice cooling di-tert-butyldicarbonate (3.49g, 16mmol) and the mixture was stirred at room temperature overnight. Thereto was added at room temperature additional di-tert-butyldicarbonate (3.49g, 16mmol) and the mixture was stirred for 2 hours. After addition of saturated brine, the mixture was extracted with ethyl acetate, the organic layer was washed with saturated brine, and dried over sodium sulfate. The solvent was removed by distillation and the residue was purified by silica gel column chromatography to give the subtitled compound 3.15g as a pale yellow oil.
Yield 87%
¹H NMR (CDCl₃) δ 7.32-7.20 (4H, m), 4.66 (2H, d, J = 5.9 Hz), 4.25-4.17 (1H, br), 3.97-3.89 (1H, br), 3.27-3.17 (1H, br), 3.13-3.03 (1H, br), 2.94-2.86 (1H, br), 2.85-2.75 (1H, br), 2.67-2.60 (1H, br), 2.27 (3H, brs), 2.04-1.94 (2H, br), 1.74 (1H, t, J = 5.9 Hz), 1.38 (9H, s).

### Step (vi)

### tert-Butyl 2-[4-(cyanomethyl)benzyl]-4-methylpiperazme- 1-carboxylate

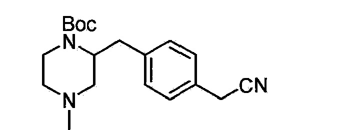

tert-Butyl 2-[4-(hydroxymethyl)benzyl]-4-methylpiperazine-1-carboxylate (1.28g, 4mmol), and triethylamine (0.56mmol, 4mmol) were dissolved in tetrahydrofuran (20ml) and thereto was added dropwise under ice cooling methanesulfonyl chloride (0.31ml, 4mmol), followed by stirring under ice cooling for 1 hour. After filtration by a filter (0.5µm), the filtrate was concentrated in vacuo and the residue was dissolved in dimethyl sulfoxide (10ml). The solution was added to a suspension of sodium cyanide (588mg, 12mmol) and sodium carbonate (848mg, 8mmol) in dimethyl sulfoxide, and the mixture was reacted at room temperature for 22 hours. Thereto was added water-ethyl acetate and the mixture was separated by a separating funnel. The organic layer was washed with water and saturated brine, and dried over sodium sulfate. After removal of the solvent by distillation, the residue was purified by silica gel column chromatography to give the subtitled compound 1.05g as an orange oil.
Yield 79%
¹H NMR (CDCl₃) δ 7.30-7.22 (4H, m), 4.27-4.17 (1H, br), 3.99-3.89 (1H, br), 3.70 (2H, s), 3.26-3.16 (1H, br), 3.11-3.01 (1H, br), 2.98-2.89 (1H, br), 2.85-2.75 (1H, br), 2.65-2.55 (1H, br), 2.27 (3H, brs), 2.05-1.97 (2H, br), 1.36 (9H, s).

### Step (vii)

### Methyl {4-[(4-methylpiperazin-2-yl)methyl]phenyl}acetate)

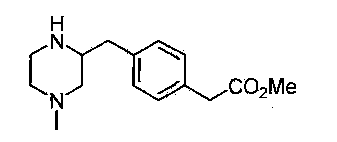

To methanol (6.08ml, 150mmol) was added dropwise under an atmosphere of nitrogen gas chloro(trimethyl)silane (9.5 ml, 75mmol) and the mixture was stirred at room temperature. Thereto was added tert-butyl 2-[4-(cyanomethyl)benzyl]-4-methylpiperazine-1-carboxylate (984mg, 2.99mmol) and the mixture was stirred at 50°C for 2 hours. After addition of water, the mixture was stirred for a while, made alkaline with aqueous saturated sodium bicarbonate and salted out with a small amount of sodium chloride. After extraction with chloroform, the organic layer was dried over sodium sulfate, and the solvent was removed by distillation to give the subtitled compound 536mg as an orange oil. Yield 68%
¹H NMR (CDCl₃) δ 7.21 (2H, d, J = 8.0 Hz), 7.16 (2H, d, J = 8.0 Hz), 3.69 (3H, s), 3.60 (2H, s), 3.02-2.90 (2H, m), 2.87-2.65 (4H, m), 2.62-2.54 (1H, m), 2.27 (3H, s), 2.10-1.87 (2H, m), 1.80 (1H, t, J = 10.4 Hz).

### Step (viii)

### [4-({1-[3-(6-Amino-2-butoxy-8-methoxy-9H-purin-9-yl)propyl]-4-methylpiperazin-2-yl}methyl)phenyl]acetic acid methyl ester

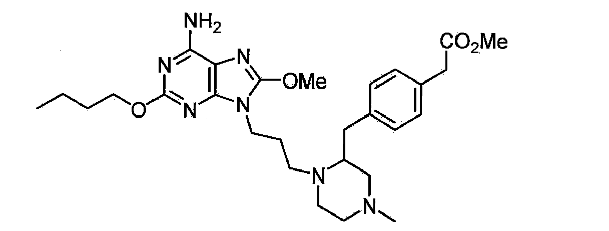

3-(6-Amino-2-butoxy-8-methoxy-9H-purin-9-yl)propyl methansulfonate (373mg, 1mmol), potassium iodide (166mg, 1mmol) potassium carbonate (207mg, 1.5mmol) and methyl {4-[(4-methylpiperazin-2-yl)methyl]phenyl}acetate (315 mg, 1.2mmol) were dissolved in dimethylformamide (4ml) and the suspension was stirred at 50°C for 1 hour, and at 70°C for 2 hours. After removal of the reaction solvent by distillation, to the residue was added water-ethyl acetate and the mixture was separated by a separating funnel. The organic layer was washed with saturated brine and sodium sulfate, the solvent was removed by distillation and the residue was purified by PTLC to give the subtitled compound 24mg as colorless oil. Yield 4%
¹H NMR (CDCl₃) δ 7.16 (2H, d, J = 8.0 Hz), 7.06 (2H, d, J = 8.0 Hz), 5.15 (2H, s), 4.29-4.21 (2H, m), 4.10 (3H, s), 4.03-3.93 (2H, m), 3.68 (3H, s), 3.58 (2H, s), 2.88-2.79 (3H, m), 2.73-2.21 (6H, m), 2.17 (3H, s), 2.09-1.78 (4H, m), 1.77-1.68 (2H, m), 1.54-1.42 (2H, m), 0.94 (3H, t, J = 7.3 Hz).

### Step (ix)

### [4-({1-[3-(6-Amino-2-butoxy-8-oxo-7,8-dihydro-9H-purin-9-yl)propyl]-4-methylpiperazin-2-yl}methyl)phenyl]acetic acid methyl ester

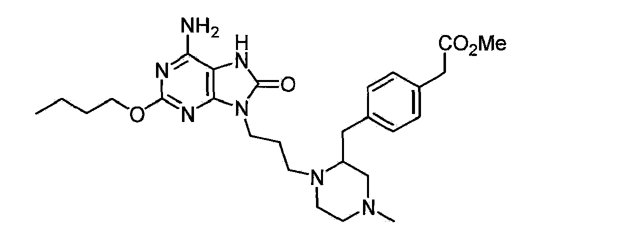

To methyl [4-({1-[3-(6-amino-2-butoxy-8-methoxy-9H-purin-9-yl)propyl]-4-methylpiperazin-2-yl}methyl)phenyl]acetate (24mg, 0.044mmol) were added 4N hydrochloric acid-dioxane (4ml) and 10% hydrochloric acid-methanol (1ml) and the mixture was stirred at room temperature for 5 hours. After concentrated, the residue was subjected to azeotropic distillation with methanol four times. The residue was neutralized with aqueous saturated sodium bicarbonate and resulting solid was filtered and dried to give the subtitled compound 18mg as a white solid. Yield 77%
¹H NMR (DMSO-d₆) δ 9.80 (1H, s), 7.13 (2H, d, J = 7.8 Hz), 7.03 (2H, d, J = 7.8 Hz), 6.31 (2H, s), 4.16-4.09 (2H, m), 3.78-3.69 (2H, m), 3.61 (3H, s), 3.60 (2H, s), 2.82-2.66 (3H, m), 2.62-2.40 (3H, m), 2.36-2.14 (3H, m), 2.09-1.92 (2H, m), 2.05 (3H, s), 1.92-1.74 (2H, m), 1.65-1.58 (2H, m), 1.41-1.31 (2H, m), 0.90 (3H, t, J = 7.3 Hz).

### Example 66

### [4-({3-[(6-Amino-2-butoxy-8-oxo-7,8-dihydro-9H-purin-9-yl)methyl]piperidin-1-yl}methyl)-3-(dimethylamino)phenyl]acetic acid methyl ester

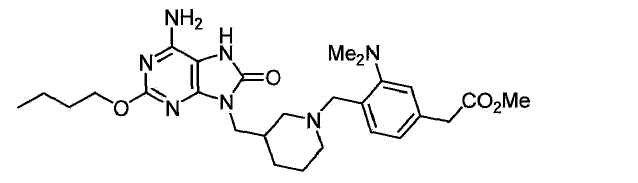

### Step (i)

### Methyl 4-({[tert-butyl(dimethyl)silyl]oxy}methyl)-3-(formylamino)benzoate

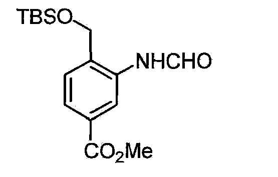

Methyl 3-amino-4-({[tert-butyl(dimethyl)silyl]oxy}methyl)benzoate (1.13g, 3.82mmol) was refluxed in an excess of ethyl formate until disappearance of the starting material. After removal of the reaction mixture, the residue was purified by silica gel column chromatography to give the subtitled compound 967 mg. Yield 78%
¹H NMR (CDCl₃) δ 8.95-8.62 (2H, m), 8.46 (S), 7.90 (s), 8.46-7.96 (1H), 7.31-7.19 (1H, m), 4.85-4.70 (2H, m), 3.99-3.84 (2H, m), 1.58 (2H, s), 0.90 (9H, s), 0.09 (6H, s).

### Step (ii)

### Methyl 4-({[tert-butyl(dimethyl)silyl]oxy}methyl)-3-[formyl(methyl)amino]benzoate

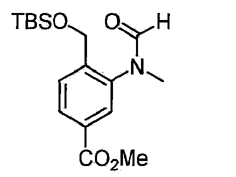

To methyl 4-({[tert-butyl(dimethyl)silyl]oxy}methyl)-3-(formylamino)benzoate (967mg, 2.99mmol) in dimethylformamide (10ml) was added sodium hydride (157mg, 3.6mmol) under ice cooling and the mixture was stirred for 1 hour. Thereto was added methyl iodide (37µl, 6mmol) and the mixture was reacted at room temperature for 6 hours. After addition of aqueous sodium bicarbonate, the mixture was extracted with ethyl acetate and the organic layer was washed with water and saturated brine. After removal of the solvent by distillation, the residue was purified by silica gel column chromatography to give the subtitled compound 885mg as a white solid. Yield 87%
¹H NMR (CDCl₃) δ 8.31-8.00 (2H, m), 7.81 (1H, s), 7.72-7.66 (1H, m), 4.67 (s), 4.63 (s), 4.67-4.63 (2H), 3.93 (s), 3.91 (s), 3.93-3.91 (3H), 3.32 (s), 3.22 (s), 3.32-3.22 (3H), 0.93 (9H, s), 0.11 (6H, s).

### Step (iii)

### [4-({3-[(6-Amino-2-butoxy-8-methoxy-9H-purin-9-yl)methyl]piperidin-1-yl}methyl)-3-(dimethylamino)phenyl]methanol

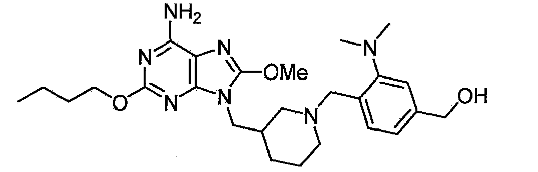

Methyl 4-({[tert-butyl(dimethyl)silyl]oxy}methyl)-3-[formyl(methyl)amino]benzoate (337mg, 1mmol) was dissolved in acetic acid (7.8ml)-water (4.2ml)-tetrahydrofuran (1.8 ml) and the solution was reacted overnight. After removal of the reaction solvent by distillation, the residue was subjected to azeotropic distillation with methanol three times, and dried in vacuo. The residue was dissolved in tetrahydrofuran (3ml) under an atmosphere of nitrogen and thereto was added triethylamine (0.093ml, 1.2mmol). Thereto was added dropwise under ice cooling methanesulfonyl chloride (0.21ml, 1.5mmol), and the mixture was reacted under ice cooling for 1 hour. The reaction was quenched with ice cooled aqueous 10% citric acid, and extracted with ethyl acetate. The organic layer was washed with ice cooled aqueous 10% citric acid, cold water, ice cooled aqueous saturated sodium bicarbonate and ice cooled saturated brine and dried over sodium sulfate. After removal of the solvent by distillation, the residue, namely methyl 3-[formyl(methyl)amino]-4-{[(methylsulfonyl)oxy]methyl}benzoate was dissolved in dimethylformamide (1ml), and the solution was added to a suspension of 2-butoxy-8-methoxy-9-(piperidin-3-ylmethyl)-9H-purin-6-amine (334mg, 1mmol), potassium carbonate (207mg, 1.5mmol) and dimethylformamide (2 ml). After reacting at room temperature for 6 hours, the reaction mixture was concentrated. Thereto was added water-ethyl acetate and the mixture was separated by a separating funnel. The organic layer was washed with water, aqueous saturated sodium bicarbonate and saturated brine and dried over sodium sulfate. After removal of the solvent by distillation, the residue was purified by silica gel column chromatography to give methyl 4-({3-[(6-amino-2-butoxy-8-methoxy-9H-purin-9-yl)methyl]piperidin-1-yl}methyl)-3-[formyl(methyl)amino]benzoate 445mg as a pale yellow oil.
Yield 80%

To a suspension of lithium aluminum hydride (45mg, 1.2mmol) in tetrahydrofuran (2ml) was added dropwise a suspension of methyl 4-({3-[(6-amino-2-butoxy-8-methoxy-9H-purin-9-yl)methyl]piperidin-1-yl}methyl)-3-[formyl(methyl)amino]benzoate (455mg, 0.84mmol) in tetrahydrofuran lml at room temperature under an atmosphere of nitrogen gas. After 2 hours, the reaction mixture was ice cooled, quenched with aqueous sodium sulfate and filtered. After the filtrate was extracted with ethyl acetate, the organic layer was washed with aqueous saturated sodium bicarbonate and saturated brine dried over sodium sulfate. After removal of the solvent by distillation, the residue was purified by silica gel column chromatography to give the subtitled compound 259mg as a white solid. Yield 62%
¹H NMR (CDCl₃) δ 7.55-7.35 (1H, br), 7.25-6.90 (2H, m), 5.33-5.18 (2H, m), 4.70-4.60 (2H, m), 4.35-4.21 (2H, m), 4.18-4.00 (3H, m), 3.88-3.73 (2H, m), 3.64-2.98 (2H, br), 2.96-2.62 (7H, m), 2.52-2.30 (1H, br), 2.29-1.99 (2H, br), 1.97-1.58 (7H, m), 1.55-1.45 (2H, m), 1.00-0.92 (3H, m).

### Step (iv)

### Methyl ([4-({3-[(6-amino-2-butoxy-8-oxo-7,8-dihydro-9H-purin-9-yl)methyl]piperidin-1-yl}methyl)-3-(dimethylamino)phenyl]acetate

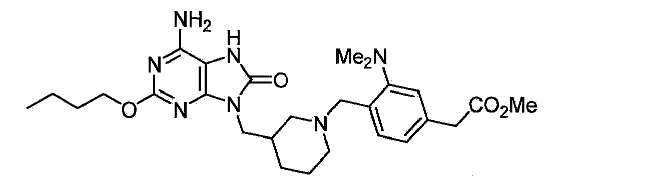

[4-({3-[(6-Amino-2-butoxy-8-methoxy-9H-purin-9-yl)methyl]piperidine-1-yl}methyl)-3-(dimethylamino)phenyl]methanol (259mg, 0.52mmol) was dissolved in tetrahydrofuran (4ml) under an atmosphere of nitrogen gas, and thereto was added triethylamine (0.13ml, 0.9mmol). Thereto was added dropwise methanesulfonyl chloride (0.062ml, 0.8mmol) under ice cooling, and the mixture was reacted under ice cooling for 1 hour. After filtration, the filtrate was concentrated and the residue, namely 4-({3-[(6-amino-2-butoxy-8-methoxy-9H-purin-9-yl)methyl]piperidin-1-yl}methyl)-3-(dimethylamino)benzyl methanesulfonate was dissolved in DMF (2ml). The solution was added to a suspension of sodium cyanide (34mg, 0.7mmol), and potassium carbonate (138mg, 1mmol) in DMF (2ml), and the mixture was reacted at room temperature overnight. The mixture was further stirred at 70°C for 4 hours. After being cooled, the mixture was concentrated, to the residue was added water-chloroform and separated by a separating funnel. The organic layer was washed with saturated brine, and dried over sodium sulfate. After removal of the solvent by distillation, the residue was purified by silica gel column chromatography [4-({3-[(6-amino-2-butoxy-8-oxo-7,8-dihydro-9H-purin-9-yl)methyl]piperidin-1-yl}methyl)-3-(dimethylamino)phenyl]acetonitrile as a pale brown solid. Under an atmosphere of nitrogen gas, to methanol (0.4ml, 10mmol) was added dropwise chloro(trimethyl)silane (0.63ml, 5mmol) and the mixture was stirred at room temperature. Thereto was added [4-({3-[(6-amino-2-butoxy-8-oxo-7,8-dihydro-9H-purin-9-yl)methyl]piperidin-1-yl}methyl)-3-(dimethylamino)phenyl]acetonitrile (64mg, 0.12mmol) and the mixture was stirred at 50°C for 2 hours. After addition of water the mixture was stirred for a while, made alkaline with aqueous saturated sodium bicarbonate and extracted with chloroform. The organic layer was dried over sodium sulfate and the solvent was removed by distillation to give the titled compound 21mg as a pale brown solid. Yield 30%
¹H NMR (DMSO-d₆) δ 9.83 (1H, s), 7.27 (1H, d, J = 7.6 Hz), 6.88 (1H, s), 6.83 (1H, d, J = 7.6 Hz), 6.36 (2H, s), 4.14 (2H, t, J = 6.6 Hz), 3.67-3.37 (9H, m), 2.73-2.42 (4H, m), 2.60 (6H, s), 2.18-1.78 (3H, m), 1.72-1.49 (3H, m), 1.46-1.20 (3H, m), 0.92 (3H, t, J = 7.3 Hz).

### Example 67

### 2-Butoxy-7,8-dihydro-9-[2-(1-[2-({3-[3-(methoxycarbonylmethyl)phenoxy]propyl}N-methylamino)ethyl]piperidin-2-yl)ethyl]-8-oxoadenine

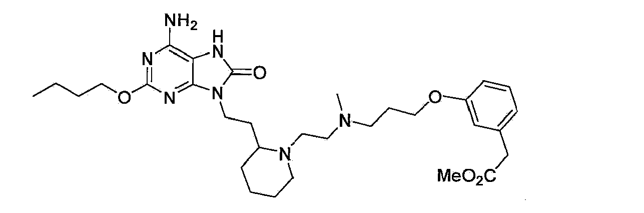

### Step (i)

### 2-({3-[3-(Methoxycarbonylmethyl)phenoxy]propyl}N-methylamino)ethanol

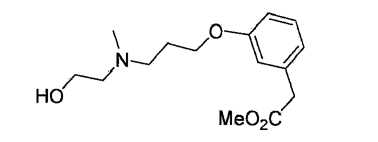

To 3-[3-(Methoxycarbonylmethyl)phenoxy]propyl bromide 287mg (1.0mmol) in DMF (5.0ml) were added diisopropylethylamine 387mg (3.0mmol) and N-methylethanolamine 75 mg (1.0mmol), and the mixture was stirred at room temperature for 19 hours. After the reaction was completed, the mixture was concentrated in vacuo and the residue was purified and isolated by column chromatography (Si0₂, CHCl₃→2%MeOH-CHCl₃→5%MeOH-CHCl₃→10%MeOH-CHCl₃) to give the subtitled compound 240mg as a colorless oil. Yield 85%
¹H NMR (CDCl₃) δ 7.22 (1H, t J= 7.82 Hz), 6.86 (1H, d J= 7.61 Hz), 6.78-6.82 (1H, m), 4.06 (2H, t J= 5.79 Hz), 3.89 (2H, t J= 4.91 Hz), 3.69 (3H, s), 3.60 (2H, s), 3.13 (2H, t J= 7.61 Hz), 3.05 (2H, t J= 5.09 Hz), 2.71 (3H, s), 2.20-2.27 (2H, m), R_{f} = 0.2 (10%MeOH-CHCl₃).

### Step (ii)

### N-{3-[3-(Methoxycarbonylmethyl)phenoxy]propyl}-N-methylamino)ethylchloride

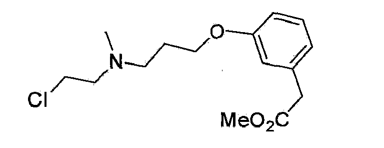

To the compound 140mg (0.50mmol) obtained in step (i) in CDCl₃ (10.0ml) was added thionyl chloride 213mg (1.8mmol) and the mixture was stirred at 60°C for 4 hours. After the reaction is completed, the mixture was concentrated in vacuo to give the subtitled compound 150mg as a yellow oil. Yield 100%
¹H NMR (CDCl₃) δ 7.26 (1H, t, J= 9.80 Hz), 6.88 (1H, d, J= 7.35 Hz), 6.77-6.80 (1H, m), 4.08 (4H, brs), 3.70 (3H, s), 3.60 (2H, s), 3.55-3.60 (1H, m), 3.37-3.43 (2H, m), 3.30-3.33 (1H, m), 2.93 (2H, t, J= 5.09 Hz), 2.40 (2H, bs), R_{f} = 0.8 (10%MeOH-CHCl₃).

### Step (iii)

### 2-Butoxy-7,8-dihydro-9-[2-(1-[2-({3-[3-(Methoxycarbonylmethyl)phenoxy]propyl}N-methylamino)ethyl]piperidin-2-yl)ethyl]-8-oxoadenine

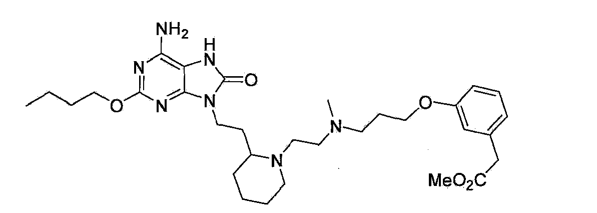

To the compound 150mg (0.5mmol) obtained in step (ii) in MeOH (2mL) were added NaHCO₃ (101mg, 3.0eq) and KI catalyst (10mg) and further added NaHCO₃ (101mg, 3.0eq) and KI catalyst (10mg), and further added 2-butoxy-7,8-dihydro-9-[2-piperidine-2-yl)ethyl]-8-oxoadenin 134mg (0.4mmol), and the mixture was stirred in a bath at 60°C for 8 hours.
When the ratio of decrease of the starting material and increase of the product was not almost changed by LC, the mixture was cooled to room temperature, and the reaction product was isolated by PTLC to give the titled compound 17mg as a white solid. Yield 7.1%
¹H NMR (δ, MeOH-d₃) 7.05 (1H, t), 6.64-6.69 (3H, m), 4.15 (2H, t), 3.85 (2H, t), 3.74 (2H, t), 3.55 (3H, s), 3.23 (2H, s), 2.60-2.75 (2H, m), 2.21-2.55 (7H, m), 2.12 (3H, s), 2.00-2.10 (1H, m), 1.75-1:79 (3H, m), 1.57-1.62 (4H, m), 1.17-1.50 (6H, m), 1.06 (1H, t), 0.85 (3H, t). R_{f}= 0.6 (10% methanol,
1%NH₃ aq.- chloroform, NH PTLC).

### Example 68

### 2-Butoxy-7,8-dihydro-9-{[3-{N-[2-(3-methoxycarbonylmethylphenyl-1-yl)ethyl])piperidin-4-yl)aminopropyl}-8-oxoadenine

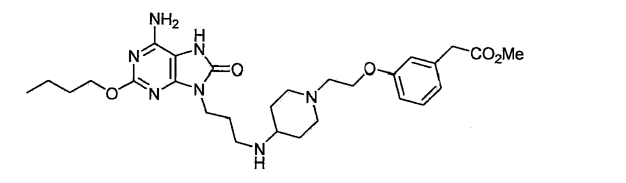

### Step (i)

### [3-(2-{4-[(3-Chrolopropyl)-(2-nitrobenzenesulfonyl)amino]piperidin-1-yl}-ethoxy)phenyl] acetic acid methyl ester

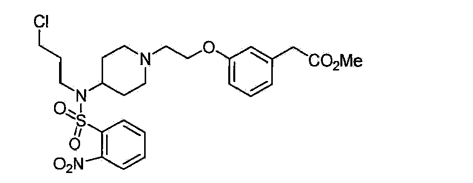

To 4-(tert-butyloxycarbonylamino)piperidine 0.82g (4.09mmol) in DMF (30ml) were added diisopropylethylamine 1.4ml (8.12mmol and [3-(2-bromoethoxy)phenyl]acetic acid methyl ester 1.12mg (4.10mmol) at room temperature, and the mixture was stirred for 30 hours. After addition of aqueous saturated sodium bicarbonate 30ml, the mixture was extracted with ethyl acetate (50ml x 3). The organic layer was dried over anhydrous magnesium sulfate, concentrated in vacuo and purified by silica gel column chromatography to give {2-[4-(tert-butyloxycarbonylamino)piperidin-1-yl]-ethoxy}phenyl)acetic acid methyl ester 1.12g as a colorless oil. Yield 70%

The obtained compound 1.06g (2.69mmol) was dissolved in trifluoroacetic acid 5ml at room temperature. The mixture was stirred for 1 hour and concentrated in vacuo to give {[2-(aminopiperidin-1-yl)-ethoxy]phenyl}acetic acid methyl ester 842mg as a colorless oily crude product. To the obtained crude product 842mg in dichloromethane (10ml) were added triethylamine 0.94ml (6.74mmol) and o-nitrobenzenesulfonyl chloride 713 mg (3.22mmol) at room temperature and the mixture was stirred for 1.5 hours. After addition of aqueous saturated sodium bicarbonate 30ml, the mixture was extracted with chloroform (50ml x 3). The organic layer was dried over anhydrous magnesium sulfate, concentrated in vacuo and the residue was purified by silica gel column chromatography to give [(2-{4-[2-(nitrobenzenesulfonyl)amino]piperidin-1-yl}-ethoxy)phenyl]acetic acid methyl ester 1.27g as a colorless oil. Yield 99%
To the obtained compound 800mg (1.68mmol) in DMF (10ml) was added 1-bromo-3-chloropropane 1.66ml (16.8mmol) and potassium carbonate 697mg (5.04mmol) at room temperature and the mixture was stirred for 15 hours. After addition of aqueous saturated sodium bicarbonate 30ml, the mixture was extracted with chloroform (50ml x 3). The organic layer was dried over anhydrous magnesium sulfate, dried over, concentrated in vacuo and purified by silica gel column chromatography to give the subtitled compound 914mg as colorless oil. Yield 99%
¹H NMR (DMSO-d₆) δ 8.12-8.03 (1H, m), 7.98-7.75 (5H, m), 7.24-7.16 (1H, m), 6.80-6.70 (3H, m), 4.02-3.94 (2H, m), 3.66-3.52 (5H, m), 3.58 (3H, s), 3.40-3.30 (2H, m), 2.98-2.86 (2H, m), 2.70-2.55 (2H, m), 2.10-1.85 (4H, m), 1.76-1.58 (2H, m), 1.50-1.43 (2H, m).

### Step (ii)

### 2-Butoxy-9-{[3-{N-[2-(3-methoxycarbonylmethylphenyl-1-yl)ethyl])piperidin-4-yl)aminopropyl}-8-methoxyadenine

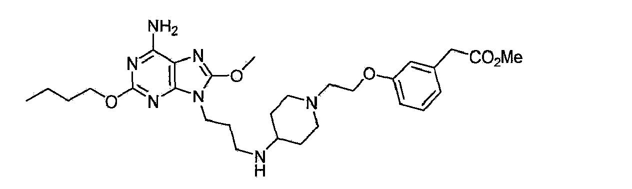

To 2-butoxy-8-methoxyadenine 724mg (2.06mmol) in DMF (15ml) were added the compound 914mg (1.64mmol) obtained step (i) and potassium carbonate 750mg (5.43mmol), and the mixture was stirred at 80°C for 15 hours. After removal of the solvent by distillation, thereto was added aqueous saturated sodium bicarbonate 50ml, and the mixture was extracted with chloroform (50ml x 3). The organic layer was dried over anhydrous magnesium sulfate, concentrated in vacuo and purified by silica gel column chromatography to give 2-butoxy-9-{[3-{N-[2-(3-methoxycarbonylmethylphenyl-1-yl)ethyl])piperidin-4-yl)aminopropyl-N-(2-nitrobenzenesulfonyl)}-8-methoxyadenine 795mg as a colorless amorphous.
Yield 51%

To the obtained compound 648mg (0.858mmol) in DMF (10ml) were added 2-mercaptoethanol 0.18ml (2.57mmol) and potassium carbonate 360mg (2.60mmol) and the mixture was stirred at room temperature for 21 hours. After removal of the solvent by distillation, thereto added aqueous saturated sodium bicarbonate 50ml and the mixture was extracted with chloroform (50ml x 3). The organic layer was dried over anhydrous magnesium sulfate, concentrated in vacuo and purified by silica gel column chromatography to give the subtitled compound 398mg as a colorless amorphous. Yield 81%
¹H NMR (DMSO-d₆) δ 7.23-7.19 (1H, m), 6.84-6.80 (3H, m), 6.77 (2H, brs), 4.17 (2H, t), 4.05 (3H, s), 4.02 (2H, t), 3.88 (2H, t), 3.64 (2H, s), 3.61 (3H, s), 2.83-2.80 (2H, m), 2.64 (2H, t), 2.45 (2H, t), 2.32-2.22 (1H, m), 2.04-1.97 (2H, m), 1.81-1.60 (6H, m), 1.45-1.35 (2H, m), 1.23-1.12 (2H, m), 0.92 (3H, t).

### Step (iii)

### 2-Butoxy-7, 8-dihydro-9-{[3-{N-[2-(3-methoxycarbonylmethylphenyl-1-yl)ethyl])piperidin-4-yl)aminopropyl}-8-oxoadenine

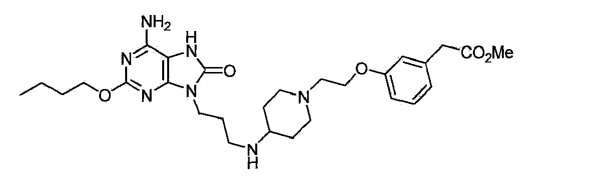

To the compound 155mg (0.272mmol) obtained in step (iii) in methanol (5ml) was added concentrated sulfuric acid (0.2ml) and the mixture was refluxed for 5 hours. After neutralized with aqueous saturated sodium bicarbonate, the resulting solid was filtered to give the titled compound 141mg as a white solid. Yield 93%
¹H NMR (DMSO-d₆) δ 9.92 (1H, brs), 7.23-7.19 (1H, m), 6.84-6.81 (3H, m), 6.44 (2H, brs), 4.16 (2H, t), 4.02 (2H, brt), 3.72 (2H, brt), 3.64 (2H, s), 3.61 (3H, s), 3.35 (2H, brs), 2.92-2.84 (2H, m), 2.65 (2H, t), 2.56 (1H, brs), 2.07-1.98 (2H, m), 1.82-1.72 (4H, m), 1.67-1.61 (2H, m), 1.44-1.34 (2H, m), 1.30-1.20 (2H, m), 0,91 (3H, t).

### Example 69

### 2-Butoxy-7, 8-dihydro-9-{3-(N-{N-[2-(3-methoxycarbonylmethylphenoxy)ethyl]piperidin-4-yl}-N-(2H-imidazol-4-ylmethyl)amino)propyl}-8-oxoadenine

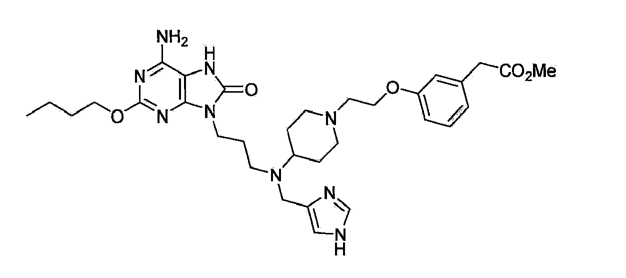

To the compound 256 mg (0.448mmol) obtained by example 68 step (ii) in NMP (5ml) were added 4-formylimidazole 131mg (1.37mmol), sodium triacetoxyborohydride 288mg (1.36mmol) at room temperature, and the mixture was stirred for 24 hours. Thereto added aqueous saturated sodium bicarbonate 50ml and the mixture was extracted with chloroform (60ml x 3). The organic layer was dried over anhydrous magnesium sulfate, concentrated in vacuo and purified by silica gel column chromatography to give the titled compound 163 mg as a colorless amorphous. Yield 56%

The obtained compound 158mg was reacted in the same manner as example 1 step (iii) to give the titled compound 122mg as a white solid.
Yield 79%
¹H NMR (DMSO-d₆) δ 9.85 (1H, brs), 7.52 (1H, brs), 7.23-7.19 (1H, m), 6.83-6.80 (4H, m), 6.42 (2H, brs), 4.14 (2H, t), 4.01 (2H, brt), 3.68-3.62 (2H, m), 3.64 (2H, s), 3.61 (3H, s), 3.56 (2H, brs), 2.98-2.90 (2H, m), 2.66-2.60 (2H, m), 1.97-1.88 (2H, m), 1.81-1.72 (2H, m), 1.67-1.58 (4H, m), 1.47-1.32 (4H, m), 0.90 (3H, t).

### Example 70

### {3-[2-(4-{[3-(6-Amino-2-butoxy-8-oxo-7,8-dihydro-purin-9-yl)propyl]isobutylamino}piperidin-1-yl)ethoxy]phenyl}acetic acid

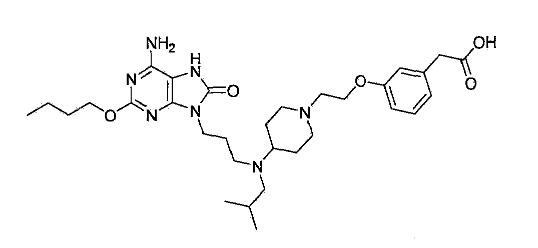

Using the compound 20mg (0.03mmol) obtained by example 63, in the same manner as example 35, there was obtained the titled compound 12mg as a white solid. Yield 62%
¹H NMR (DMSO-d₆) δ 12.16 (1H, brs), 7.12-7.08 (3H, m), 6.95 (1H, s), 6.74 (1H, d, J= 7.5 Hz), 6.68 (1H, d, J= 7.9 Hz), 4.15 (2H, t, J= 6.6 Hz), 3.98 (2H, t, J= 5.3 Hz), 3.66 (2H, t, J= 6.1 Hz), 3.23 (2H, s), 2.86 (2H, d, J= 10.7 Hz), 2.59 (2H, t, J= 5.3 Hz), 2.35 (2H, t, J= 7.0 Hz), 2.30-2.24 (1H, m), 2.11 (2H, d, J= 7.0 Hz), 1.92 (2H, t, J= 10.7 Hz), 1.78 (2H, brs), 1.67-1.55 (3H, m), 1.46-1.34 (4H, m), 1.29-1.16 (2H, m), 0.91 (3H, t, J= 7.4 Hz), 0.81 (6H, d, J= 6.5 Hz).

### Example 71

### [4-({1-[3-(6-Amino-2-butoxy-8-oxo-7,8-dihydro-purin-9-yl)propyl]piperidin-4-ylamino}methyl)phenyl]acetic acid dihydrochloride

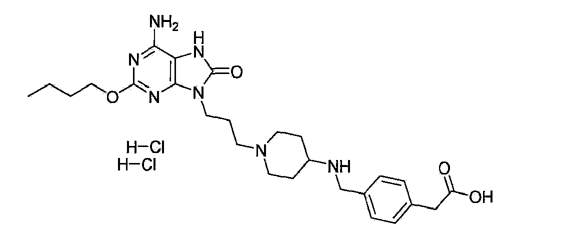

Using the compound 15mg (0.03mmol) obtained by example 64, in the same manner as example 35, there was obtained the titled compound 16mg as a white solid. Yield 97%
¹H NMR (DMSO-d₆) δ 10.66 (1H, s), 10.35 (1H, brs), 9.61 (2H, s), 7.53 (2H, d, J= 8.0 Hz), 7.32 (2H, d, J= 8.0Hz), 7.03 (1H, brs), 4.21 (2H, t, J= 6.6 Hz), 4.13 (2H, brs), 3.76 (2H, t, J= 6.1 Hz), 3.60 (2H, s), 3.56 (2H, d, J= 11.3 Hz), 3.22 (1H, brs), 3.03-2.93 (4H, m), 2.31 (2H, t, J= 13.0 Hz), 2.09-2.02 (4H, m), 1.68-1.63 (2H, m), 1.44-1.38 (2H, m), 0.93 (3H, t, J= 7.4 Hz).

### Example 72

### 2-Butoxy-7,8-dihydro-9-{[3-{N-[2-(3-hydroxycarbonylmethylphenoxy-1-ethyl])piperidin-4-yl)aminopropyl}-8-oxoadenine

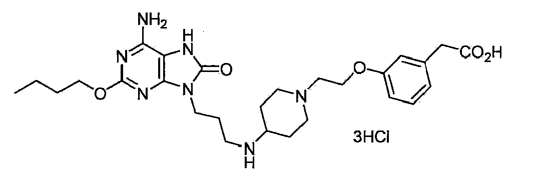

To the compound 42.6mg (0.0767mmol) obtained by example 68 was added concentrated hydrochloric acid (2ml) and the mixture was added for 3.5 hours. After diluted with 1,4-dioxane (2ml), the mixture was concentrated in vacuo to give the titled compound 72.3mg as a white solid.
Yield 100%
¹H NMR (DMSO-d₆) δ 9.92 (1H, m), 7.23-7.19 (1H, m), 6.84-6.81 (3H, m), 6.44 (2H, m), 4.16 (2H, m), 4.02 (2H, m), 3.72 (2H, m), 3.64 (2H, brs), 3.35 (2H, m), 2.92-2.84 (2H, m), 2.65 (2H, m), 2.56 (1H, m), 2.07-1.98 (2H, m), 1.82-1.72 (4H, m), 1.67-1.61 (2H, m), 1.44-1.34 (2H, m), 1.30-1.20 (2H, m), 0,87 (3H, t).

### Example 73

### 2-Butoxy-7, 8-dihydro-9-{3-(N-{N-[2-(3-carboxylmethylphenoxy)ethyl]piperidin-4-yl}-N-(2H-imidazol-4-ylmethyl)amino)propyl}-8-oxoadenine

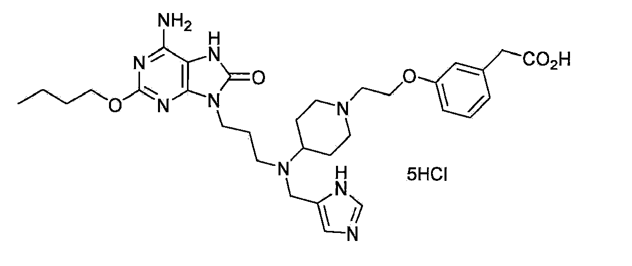

To the compound 34.3mg (0.0539mmol) obtained by example 69 was added concentrated hydrochloric acid (2ml) and the mixture was stirred for 5.5 hours. After diluted with 1,4-dioxane (2ml), the mixture was concentrated in vacuo to give the titled compound 58.9mg as a white solid. Yield 100%
¹H NMR (DMSO-d₆) δ 9.85 (1H, brs), 7.52 (1H, m), 7.28-7.19 (1H, m), 6.83-6.80 (4H, m), 6.42 (2H, m), 4.14 (2H, m), 4.01 (2H, m), 3.68-3.62 (2H, m), 3.64 (2H, m), 3.56 (2H, m), 2.98-2.90 (2H, m), 2.66-2.60 (2H, m), 1.97-1.88 (2H, m), 1.81-1.72 (2H, m), 1.67-1.58 (4H, m), 1.47-1.32 (4H, m), 0.87 (3H, t).

## Claims

1. An adenine compound represented by the following formula (1): 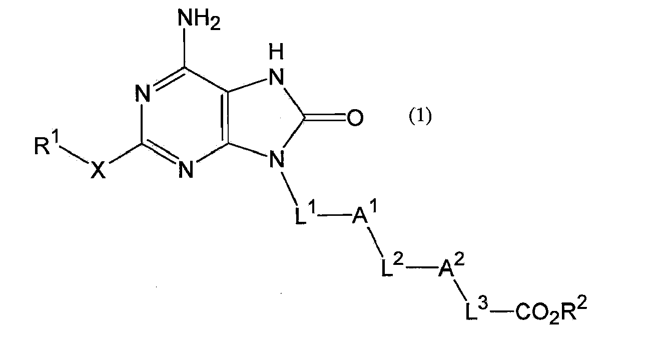 [wherein
R¹ is halogen atom, optionally substituted alkyl group, optionally substituted alkenyl group, optionally substituted alkynyl group, optionally substituted cycloalkyl group, optionally substituted aryl group or optionally substituted heteroaryl group;
R² is hydrogen atom, optionally substituted alkyl group, optionally substituted alkenyl group, optionally substituted alkynyl group or optionally substituted cycloalkyl group;
X is oxygen atom, sulfur atom, NR⁴ (wherein R⁴ is hydrogen atom or C₁₋₆ alkyl group), SO, SO₂ or a single bond, provided that X is a single bond when R¹ is halogen atom;
A¹ is optionally substituted saturated or unsaturated 4 to 8 membered heterocyclic group containing 1 to 2 hetero atoms selected from 1 to 2 nitrogen atoms, 0 to 1 oxygen atom, and 0 to 1 sulfur atom;
A² is optionally substituted 6 to 10 membered aromatic carbocyclic group or optionally substituted 5 to 10 membered aromatic heterocyclic group;
L³ is optionally substituted straight or branched alkylene or a single bond; and
L¹ and L² are independently, straight or branched alkylene or a single bond and any 1 to 3 methylene groups in said alkylene group may be replaced by oxygen atom, sulfur atom, NR⁵ (R⁵ is hydrogen atom, optionally substituted alkyl group, optionally substituted cycloalkyl group, optionally substituted aryl group or optionally substituted heteroaryl group), SO, SO₂, C=NR⁶ (wherein R⁶ is optionally substituted alkyl group, optionally substituted aryl group or optionally substituted heteroaryl group), or carbonyl group.]
or its pharmaceutically acceptable salt.

2. The adenine compound or its pharmaceutically acceptable salt according to claim 1, wherein
substituted alkyl group, substituted alkenyl group or substituted alkynyl group in R¹ and R², and substituted alkyl group in R⁵ and R⁶ are substituted by one or more substituents independently selected from the group consisting of groups (a) to (c) below;
(a) halogen atom, hydroxy group, carboxy group, mercapto group and C₁₋₆ haloalkoxy group;
(b) C₁₋₆ alkoxy group, C₂₋₆ alkylcarbonyl group, C₂₋₆ alkoxycarbonyl group, C₁₋₆ alkylsulfonyl group, C₁₋₆ alkylsulfinyl group, C₂₋₆ alkylcarbonyloxy group, and C₁₋₆ alkylthio group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
(c) optionally substituted amino group, optionally substituted carbamoyl group and optionally substituted sulfamoyl group (wherein the group of this group may be substituted by 1 or 2 substituents selected from the group consisting of groups (k), (l) and (m) below), optionally substituted 3 to 8 membered cycloalkyl group and optionally substituted 4 to 8 membered saturated heterocyclic group (wherein the group of this group may be substituted by one or more substituents selected from the group consisting of groups (d), (e) and (f) below), and optionally substituted 6 to 10 membered aryl group, optionally substituted 5 to 10 membered heteroaryl group, optionally substituted 6 to 10 membered aryloxy group and optionally substituted 5 to 10 membered heteroaryloxy group (wherein the group of this group may be substituted by one or more substituents selected from the group consisting of groups (g), (h) (i) and (j) below);
substituted cycloalkyl group in R¹, R² and R⁵ is substituted by one or more substituents independently selected from the group consisting of groups (d) to (f) below;
(d) halogen atom, hydroxy group, carboxy group, mercapto group, cyano group, nitro group, C₁₋₆ haloalkyl group and C₁₋₆ haloalkoxy group;
(e) C₁₋₆ alkyl group, C₁₋₆ alkoxy group, C₂₋₆ alkenyl group, C₂₋₆ alkynyl group, C₂₋₆ alkoxycarbonyl group, and C₁₋₆ alkylthio group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆alkyl groups, sulfamoyl group optionally substituted by the same or different and one or two C₁₋₆ alkyl groups, and C₁₋₆ alkylsulfonyl group);
(f) optionally substituted amino group, optionally substituted carbamoyl group and optionally substituted sulfamoyl group (wherein the group of this group may be substituted by one or two substituents selected groups (k), (l) and (m) below), optionally substituted 6 to 10 membered aryl group and optionally substituted 5 to 10 membered heteroaryl group (the group of this group may be substituted by one or more substituents selected from the group consisting of groups (g), (h), (i) and (j) below);
substituted aryl group and substituted heteroaryl group in R¹, R⁵ and R⁶ are substituted by one or more substituents independently selected from the group consisting of groups (g) to (j) below;
(g) halogen atom, hydroxy group, mercapto group, cyano group, nitro group, C₁₋₆ haloalkyl group, and C₁₋₆ haloalkoxy group;
(h) C₁₋₆ alkyl group, C₁₋₆ alkoxy group, C₂₋₆ alkenyl group, C₂₋₆ alkynyl group, and C₁₋₆ alkylthio group (wherein the group of this group may be substituted by one or more substituents independently selected from a group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, and C₁₋₆ alkylsulfonyl group);
(i) 3 to 8 membered cycloalkyl group and 4 to 8 membered saturated heterocyclic group (the group of this group may be substituted by one or more substituents independently selected from group consisting of halogen atom, hydroxy group, carboxy group, C₁₋₆ alkyl group and C₁₋₆ alkoxy group);
(j) optionally substituted amino group, optionally substituted carbamoyl group, and optionally substituted sulfamoyl group (the group of this group may be substituted by one or two substituents selected from group consisting of groups (k), (l) and (m) below);
the substituted amino group, substituted carbamoyl group and substituted sulfamoyl group mentioned above are substituted by one or two substituents independently selected from the group consisting of groups (k) to (m) below;
(k) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, and 3 to 8 membered cycloalkylsulfinyl group (wherein the group of this group may be substituted by one or more substituents independently selected from the group consisting of halogen atom, hydroxy group, carboxy group, C₁₋₆ alkoxy group, and C₂₋₆ alkoxycarbonyl group);
(l) 6 to 10 membered aryl group, 6 to 10 membered arylcarbonyl group, 6 to 10 membered aryloxycarbonyl group, 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, mercapto group, cyano group, nitro group, C₁₋₆ alkyl group, C₁₋₆ alkoxy group or C₁₋₆ alkylthio group);
(m) 4 to 7 membered saturated heterocyclic group containing 1 to 4 hetero atoms selected from 1 to 2 nitrogen atoms, 0 to 1 oxygen atom, and 0 to 1 sulfur atom which is formed by combining two substituents with the nitrogen atom (said saturated heterocyclic group may be substituted on any carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group); substituted 4 to 8 membered heterocyclic group in A¹ may be substituted by one or more substituents independently selected from a group consisting of halogen atom, hydroxy group, oxo group, C₁₋₆ alkyl group, C₁₋₆ alkoxy group, C₂₋₆ alkylcarbonyl group and C₂₋₆ alkoxycarbonyl group; substituted 6 to 10 membered aromatic carbocyclic group or substituted 5 to 10 membered aromatic heterocyclic group in A² may be substituted by one or more substituents independently selected from a group consisting of halogen atom, C₁₋₆ alkyl group, C₁₋₆ alkoxy group, C₁₋₆ haloalkyl group, C₁₋₆ haloalkoxy group, amino group optionally substituted by the same or different and one or two C₁₋₆ alkyl groups, 4 to 8 membered saturated heterocyclic group containing 1 to 2 hetero atoms selected from 1 to 2 nitrogen atoms, 0 to 1 oxygen atom, and 0 to 1 sulfur atom (said saturated heterocyclic group may be substituted by one or more substituents independently selected from a group consisting of halogen atom, hydroxy group, oxo group, C₁₋₆ alkyl group, C₁₋₆ alkoxy group, C₂₋₆ alkylcarbonyl group and C₂₋₆ alkoxycarbonyl group); and L³ is straight or branched C₁₋₆ alkylene, or a single bond.

3. The adenine compound or its pharmaceutically acceptable salt according to claim 1 or 2, wherein in the formula (1), A¹ is pyrrolidine, piperidine, azetidine, piperazine, morpholine, thiomorpholine, thiomorpholine-1-oxide, thiomorpholine-1,1-dioxide, 2,6-dimethylpiperidine, 3,5-dimethylpiperidine, 2,6-dimethylpiperazine, 2,6-dimethylmorpholine, 3,5-dimethylmorpholine, 2,6-dimethylthiomorpholine, or 3,5-dimethylthiomorpholine.

4. The adenine compound or its pharmaceutically acceptable salt according to any one of claims 1 to 3, wherein in the formula (1), A² is benzene, pyridine, furan, imidazole or thiophene.

5. The adenine compound or its pharmaceutically acceptable salt according to any one of claims 1 to 4, wherein in the formula (1), R² is C₁₋₄ alkyl group.

6. The adenine compound or its pharmaceutically acceptable salt according to claim 5, wherein in the formula (1), R² is methyl group.

7. The adenine compound or its pharmaceutically acceptable salt according to any one of claims 1 to 4, wherein in the formula (1), R² is C₂₋₈ alkyl group substituted by optionally substituted amino group.

8. The adenine compound or its pharmaceutically acceptable salt according to any one of claims 1 to 7, wherein in the formula (1), L¹ is the following formula:
(CH₂)ₙ-(Y⁴)ₘ-(CH₂)₁ₐ
[wherein, n and 1a are independently an integer of 0 to 5, m is 0 or 1, Y⁴ is oxygen atom or NR⁵ (wherein R⁵ is the same as defined in claim 1)],
L² is a single bond, oxygen atom, C₁₋₁₀ straight alkylene or the following formula:
(CH₂)ₐ-(Y¹)ₚ-(CH₂)_{q}-(Y²),-(CH₂)ₜ-(Y³)ᵤ
[wherein Y¹ is carbonyl group, Y² is NR⁵' (R⁵' is the same as the definition of R⁵), Y³ is oxygen atom, a, t and q are independently, an integer of 0 to 4, p, r and u are independently 0 or 1, provided that t is 2 or more when r and u are 1], and
L3 is a single bond or C₁₋₄ straight alkylene.

9. The adenine compound or its pharmaceutically acceptable salt according to claim 8, wherein R⁵ is hydrogen atom, C₁₋₆ alkyl group, C₁₋₆ alkylcarbonyl group or C₁₋₆ alkylsulfonyl group (these groups may be substituted by one or more substituents independently selected from a group consisting of halogen atom, hydroxy group, alkoxy group, 3 to 8 membered cycloalkyl group, 6 to 10 membered aryl group, 6 to 10 membered arylcarbonyl group and 5 to 10 membered heteroaryl group (this group may be substituted by one or more substituents independently selected from a group consisting of halogen atom, hydroxy group, nitro group, cyano group, C₁₋₆ alkyl group, C₁₋₆ alkoxy group, C₁₋₆ haloalkyl group and C₁₋₆ haloalkoxy group)).

10. The adenine compound or its pharmaceutically acceptable salt according to claim 1 selected from the group of the following compounds:
2-Butoxy-7,8-dihydro-9-{[2-(3-{N-[(3-methoxycarbonylmethylphenyl-1-yl)methyl]-N-methylamino}propyl)piperidin-4-yl]-methyl}-8-oxoadenine;
2-Butoxy-7,8-dihydro-9- {[2-(3-{N-[(3-methoxycarbonylmethylphenyl-1-yl)methyl]amino}propyl)piperidin-4-yl]methyl}-8-oxoadenine;
7, 8-Dihydro-9-(1-{2-[3-(methoxycarbonylmethyl)phenoxy]ethyl}piperidin-4-ylmethyl)-2-(2-methoxyehoxy)-8-oxoadenine;
7,8-Dihydro-9-{1-[3-(N-{2-[3-(methoxycarbonylmethyl)phenoxy]ethyl}-N-methylamino)propyl]piperidin-4-ylmethyl}-2-(2-methoxyehoxy)-8-oxoadenine;
7,8-Dihydro-9-[1-(3-{N-[3-(methoxycarbonylmethyl)benzyl]-N-methylamino}propyl)piperidin-4-ylmethyl]-8-oxoadenine;
2-Butoxy-7,8-dihydro-9-(1-{2-[3-(methoxycarbonylmethyl)phenoxy]ethyl}piperidin-4-ylmethyl)-8-oxoadenine;
2-Butoxy-7,8-dihydro-9-{1-[3-(N-{2-[3-(methoxycarbonylmethyl)phenoxy]ethyl}-N-methylamino)propyl]piperidin-4-ylmethyl}-8-oxoadenine;
7,8-Dihydro-9-(1-{2-[3-(methoxycarbonylmethyl)phenoxy]ethyl}piperidin-4-ylethyl)-2-(2-methoxyehoxy)-8-oxoadenine;
7, 8-Dihydro-9-[1-(3-[{N-methyl-N-[3-(methoxycarbonylmethyl)benzyl]}amino]propyl)piperidin-4-ylethyl]-2-(2-methoxyehoxy)-8-oxoadenine;
7,8-Dihydro-9-{1-[3-([N-methyl-N-{2-[3-(methoxycarbonylmethyl)phenoxy]ethyl}]amino)propyl]piperidin-4-ylethyl}-2-(2-methoxyehoxy)-8-oxoadenine;
2-Butoxy-7,8-dihydro-9-(1-{2-[3-(methoxycarbonylmethyl)phenoxy]ethyl}piperidin-4-ylethyl)-8-oxoadenine;
2-Butoxy-7,8-dihydro-9-{1-[8-([N-methyl-N-{2-[3-(methoxycarbonylmethyl) phenoxy] ethyl}] amino)propyl] piperidin-4-ylethyl}-8-oxoadenine;
7,8-Dihydro-9-(1-{[3-(methoxycarbonylmethyl)phenyl]aminocarbonylmethyl}piperidin-4-ylethyl)-2-(2-methoxyehoxy)-8-oxoadenine;
2-Butoxy-7,8-dihydro-9-(1-{[3-(methoxycarbonylmethyl)phenyl]aminocarbonylmethyl}piperidin-4-ylmethyl)-8-oxoadenine;
2-Butoxy-7,8-dihydro-9-{1-[(N-{2-[3-(methoxycarbonylmethyl)phenoxy]ethyl}-N-methyl)aminomethylcarbonyl]piperidin-4-ylmethyl}-8-oxoadenine;
7,8-Dihydro-2-(2-methoxyehoxy)-9-{1-[(N-{2-[3-(methoxycarbonylmethyl)phenoxy]ethyl}-N-methyl)aminomethylcarbonyl]piperidin-4-ylmethyl}-8-oxoadenine;
7,8-Dihydro-2-(2-methoxyehoxy)-9-[1-(N-{2-[3-(methoxycarbonylmethyl)phenoxy]ethyl}-N-methylaminomethylcarbonyl)piperidin-4-ylmethyl]-8-oxoadenine;
2-Butoxy-7,8-dihydro-9-(2-{1-[(N-{2-[3-(methoxycarbonylmethyl)phenoxy]ethyl}-N-methyl)aminocarbonylmethyl]piperidin-4-yl}ethyl)-8-oxoadenine;
2-Butoxy-7,8-dihydro-9-{1-[(N-{2-[2-methoxy-5-(methoxycarbonylmethyl)phenoxy]ethyl}-N-methyl)aminomethylcarbonyl]piperidin-4-ylmethyl}-8-oxoadenine;
2-Butoxy-7,8-dihydro-9-[5-(4-{2-[N-methyl-N-(3-methoxycarbonylmethyl)benzyl] aminoethyl}piperazin-1-yl)pentyl]-8-oxoadenine;
2-Butoxy-7,8-dihydro-9-[7-(4-{2-[N-methyl-N-(3-methoxycarbonylmethyl)benzyl]aminoethyl}piperazin-1-yl)heptyl]-8-oxoadenine;
2-Butoxy-7,8-dihydro-9-(5-{4-[2-(3-methoxycarbonylmethylphenyloxy)ethyl]piperazin-1-yl}pentyl)-8-oxoadenine;
2-Butoxy-7,8-dihydro-9-(7-{4-[2-(3-methoxycarbonylmethylphenyloxy)ethyl]piperazin-1-yl}heptyl)-8-oxoadenine;
2-Butoxy-7,8-dihydro-9-[2-{4-(3-methoxycarbonylmethylphenoxy)piperidin-1-yl}ethyl]-8-oxoadeni.ne;
2-Butoxy-7,8-dihydro-9-[2-{4-(3-methoxycarbonylmethylphenyl)pipendin-1-yl}ethyl]-8-oxoadenine;
2-Butoxy-7, 8-dihydro-9-[2-{4-(3-methoxycarbonylmethylbenzyl)piperidin-1-yl}ethyl]-8-oxoadenine;
2-Butoxy-7,8-dihydro-9-[2-{4-(4-methoxycarbonylmethylpyridin-2-yl)piperazin-1-yl}ethyl]-8-oxoadenine;
2-Butoxy-7,8-dihydro-9-(3-{4-[3-(2-methoxy-2-oxoethyl)phenoxy]piperidin-1-yl}propyl)-8-oxoadenine;
2-Butoxy-7, 8-dihydro-9-{4-[4-(3-methoxycarbonylmethyl)benzylpiperazin-1-yl]butyl}-8-oxoadenine;
2-Butoxy-7,8-dihydro-9-{4-[4-(3-methoxycarbonylmethylbenzyl)piperazin-1-yl]butyl}-8-oxoadenine;
2-Butoxy-7,8-dihydro-9-{4-[4-(4-methoxycarbonylbenzylcarbonylbenzyl)piperidin-1-yl]butyl}-8-oxoadenine;
2-Butoxy-7,8-dihydro-9-[1-(5-methoxycarbonylfuran-2-ylmethyl)piperidin-4-ylmethyl]-8-oxoadenine;
2-Butoxy-7,8-dihydro-9-[5-{4-(3-methoxycarbonylmethylphenyl)piperidin-1-yl}pentyl]-8-oxoadenine;
7,8-Dihydro-2-(2-methoxyehoxy)-9-[2-{4-(3-methoxycarbonylmethylbenzyl)piperidin-1-yl}ethyl]-8-oxoadenine;
2-Butoxy-7,8-dihydro-9-{[2-(3-{N-[(3-hydroxycarbonylmethylphenyl-1-yl)methyl]-N-methylamino}-propyl)-piperidin-4-yl]-methyl}-8-oxoadenine;
2-Butoxy-7,8-dihydro-9-(1-{2-[3-(hydroxycarbonylmethyl)phenoxy]ethyl}piperidin-4-ylmethyl)-8-oxoadenine;
2-Butoxy-7,8-dihydro-9-{1-[3-(N-{2-[3-(hydroxycarbonylmethyl)phenoxy]ethyl}-N-methylamino)propyl]piperidin-4-ylmethyl}-8-oxoadenine;
9-(1-{2-[3-(carboxymethyl)phenoxy]ethyl}piperidin-4-ylethyl)-7,8-dihydro-2-(2-methoxyehoxy)-8-oxoadenine;
2-Butoxy-7,8-dihydro-9-(1-{2-[3-(carboxymethyl)phenoxy]ethyl}piperidin-4-ylethyl)-8-oxoadenine;
2-Butoxy-7, 8-dihydro-9-{1-[3-([N-methyl-N-{2-[3-(carboxymethyl)phenoxy]ethyl}]amino)propyl]piperidin-4-ylethyl}-8-oxoadenine;
7,8-Dihydro-9-(1-{[3-(hydroxycarbonylmethyl)phenyl]aminocarbonylmethyl}piperidin-4-ylethyl)-2-(2-methoxyehoxy)-8-oxoadenine;
2-Butoxy-7,8-dihydro-9-(1-{[3-(hydroxycarbonylmethyl)phenyl]aminocarbonylmethyl}piperidin-4-ylmethyl)-8-oxoadenine;
2-Butoxy-7,8-dihydro-9-{1-[(N-{2-[3-(hydroxycarbonylmethyl)phenoxy]ethyl}-N-methyl)aminomethylcarbonyl]piperidin-4-ylmethyl}-8-oxoadenine;
7,8-Dihydro-9-{1-[(N-{2-[3-(hydroxycarbonylmethyl)phenoxy]ethyl}-N-methyl)aminomethylcarbonyl]piperidin-4-ylmethyl}-2-(2-methoxyehoxy)-8-oxoadenine;
7,8-Dihydro-9-[1-(N-{2-[3-(hydroxycarbonylmethyl)phenoxy]ethyl}-N-methylaminomethylcarbonyl)piperidin-4-ylmethyl]-2-(2-methoxyehoxy)-8-oxoadenine;
2-Butoxy-7,8-dihydro-9-(2-{1-[(N-{2-[3-(hydroxycarbonylmethyl)phenoxy]ethyl}-N-methyl)aminocarbonylmethyl]piperidin-4-yl}ethyl)-8-oxoadenine;
2-Butoxy-7,8-dihydro-9-[5-(4-{2-[N-methyl-N-(3-hydroxycarbonylmethyl) benzyl] aminoethyl}piperazin-1-yl)pentyl]-8-oxoadenine;
2-Butoxy-7,8-dihydro-9-[7-(4-{2-[N-methyl-N-(3-hydroxycarbonylmethyl)benzyl]aminoethyl}piperazin-1-yl)heptyl]-8-oxoadenine;
2-Butoxy-7,8-dihydro-9-(5-{4-[2-(3-hydroxycarbonylmethylphenyloxy)ethyl]piperazin-1-yl}pentyl)-8-oxoadenine;
2-Butoxy-7,8-dihydro-9-(7-{4-[2-(3-hydroxycarbonylmethylphenyloxy)ethyl]piperazin-1-yl}heptyl)-8-oxoadenine;
2-Butoxy-7,8-dihydro-9-[2-{4-(3-hydroxycarbonylmethylphenoxy)piperidin-1-yl}ethyl]-8-oxoaclenine;
2-Butoxy-7 ,8-dihydro-9-[2-{4-(3-hydroxycarbonyltnethylphenyl)piperidin-1-yl}ethyl] -8-oxoadenine;
2-Butoxy-7 ,8-dihydro-9-[2-{4-(3-hydroxycarbonylmethylbenzyl)piperidin-1-yl}ethyl]-8-oxoadenine;
2-Butoxy-7,8-dihydro-9-[5-{4-(3-hydroxycarbonylmethylphenyl)piperidin-1-yl}pentyl] -8-oxoadenine;
2-Butoxy-7, 8-dihydro-9-(3-{4-[3-(2-hydroxy-2-oxoethyl)phenoxy]piperidin-1-yl}propyl)-8-oxoadenine;
2-Butoxy-7,8-dihydro-9-{4-[(3-hydroxycarbonylmethyl)benzylpiperazin-1-yl]butyl}-8-oxoadenine;
2-Butoxy-7, 8-dihydro-9-{4-[4-(3-hydroxycarbonylmethylbenzyl)piperazin-1-yl]butyl}-8-oxoadenine;
2-Butoxy-7,8-dihydro-9-{4-[4-(4-hydroxycarbonylbenzylcarbonylbenzyl)piperidin-1-yl]butyl}-8-oxoadenine;
2-Butoxy-7,8-dihydro-9-[5-{4-(3-hydroxycarbonylmethylphenyl)piperidin-1-yl}pentyl]-8-oxoadenine;
Methyl {3-[({1-[2-(6-amino-2-butoxy-8-oxo-7,8-dihydro-9H-purin-9-yl)ethyl]piperazin-4-yl}amino)methyl]phenyl}acetate;
{3-[({1-[2-(6-amino-2-butoxy-8-oxo-7,8-dihydro-9H-purin-9-yl)ethyl]piperidin-4-yl}amino)methyl]phenyl}acetic acid;
Methyl {3-[2-(4-{[3-(6-amino-2-butoxy-8-oxo-7,8-dihydro-purin-9-yl) propyl] isobutylamino}piperidin-1-yl) ethoxy] phenyl}acetate;
Methyl [4-({1-[3-(6-amino-2-butoxy-8-oxo-7,8-dihydro-purin-9-yl)propyl]piperidin-4-ylamino}methyl)phenyl]acetate;
Methyl [4-({1-[3-(6-amino-2-butoxy-8-oxo-7,8-dihydro-9H-purin-9-yl)propyl]-4-methylpiperazin-2-yl}methyl)phenyl]acetate;
Methyl [4-({3-[(6-amino-2-butoxy-8-oxo-7,8-dihydro-9H-purin-9-yl)methyl]piperidin-1-yl}methyl)-3-(dimethylamino)phenyl]acetate;
2-Butoxy-7, 8-dihydro-9-[2-(1-[2-({3-[3-(methoxycarbonylmethyl)phenoxy]propyl}-N-methylamino)ethyl]piperidin-2-yl)ethyl] -8-oxoadenine;
2-Butoxy-7,8-dihydro-9-{[3-{N-[2-(3-methoxycarbonylmethylphenoxy-1-yl)ethyl])piperidin-4-yl]aminopropyl}-8-oxoadenine;
2-Butoxy-7,8-dihydro-9-{3-(N-{N-[2-(3-methoxycarbonylmethylphenoxy)ethyl]piperidin-4-yl}-N-(2H-imidazol-4-ylmethyl)amino)propyl}-8-oxoadenine;
{3-[2-(4-{[3-(6-Amino-2-butoxy-8-oxo-7,8-dihydropurin-9-yl)propyl]isobutylamino}pipehdin-1-yl)ethoxy]phenyl}acetic acid;
[4-({1-[3-(6-Amino-2-butoxy-8-oxo-7,8-dihydropurin-9-yl)propyl]piperidin-4-ylamino}methyl)phenyl]acetic acid 2 hydrochloride;
2-Butoxy-7,8-dihydro-9-{[3-{N-[2-(3-hydroxycarbonylmethylphenoxy-1-yl)ethyl])piperidin-4-yl]aminopropyl}-8-oxoadenine; and
2-Butoxy-7,8-dihydro-9-{3-(N-{N-[2-(3-methoxycarbonylmethylphenoxy)ethyl]piperidin-4-yl}-N-(2H-imidazol-4-ylmethyl)amino)propyl}-8-oxoadenine.

11. A pharmaceutical composition containing the adenine compound or a pharmaceutically acceptable salt thereof as described in any one of claims 1 to 10 as an active ingredient.

12. A TLR7 activator containing the adenine compound or a pharmaceutically acceptable salt thereof as described in any one of claims 1 to 10 as an active ingredient.

13. An immuno-modifier containing the adenine compound or a pharmaceutically acceptable salt thereof as described in in any one of claims 1 to 10 as an active ingredient.

14. A therapeutic or prophylactic agent for allergic diseases, viral diseases or cancers containing the adenine compound or a pharmaceutically acceptable salt thereof as described in in any one of claims 1 to 10 as an active ingredient.

15. A therapeutic or prophylactic agent for asthma, COPD, allergic rhinitis, allergic conjunctivitis, atopic dermatosis, cancer, hepatitis B, hepatitis C, HIV, HPV, a bacterial infectious disease, or dermatosis containing the adenine compound or a pharmaceutically acceptable salt thereof as described in in any one of claims 1 to 10 as an active ingredient.

16. A medicament for topical administration containing the adenine compound or a pharmaceutically acceptable salt thereof as described in in any one of claims 1 to 10 as an active ingredient.
